# EUROPEAN PATENT APPLICATION

(11) **EP 4 741 389 A1**
(43) Date of publication of application: **13.05.2026**
(21) Application number: 24835354.2
(22) Date of filing: 03.07.2024
(51) Int. Cl.: C07D 413/14, C07D 413/00, C07D 498/04, C07D 498/00, A61K 31/423, A61K 31/42, A61P 35/00

(54) **KAT6 INHIBITOR**

(30) Priority: 04.07.2023 CN 202310816102; 08.09.2023 CN 202311161448; 29.11.2023 CN 202311618729; 08.01.2024 CN 202410024692; 15.04.2024 CN 202410452483
(71) Applicant: Shanghai Qilu Pharmaceutical Research and Development Centre Ltd., Shanghai 201203 (CN)
(72) Inventor: YANG, Chundao, Shanghai 201203 (CN); ZHENG, Xuejian, Shanghai 201203 (CN); ZHANG, Fumao, Shanghai 201203 (CN); QIAN, Wenyuan, Shanghai 201203 (CN); SHI, Guqin, Shanghai 201203 (CN); ZHANG, Yuxing, Shanghai 201203 (CN); GUO, Huijie, Shanghai 201203 (CN); SUN, Weimei, Shanghai 201203 (CN); SUN, Daqing, Shanghai 201203 (CN); TAO, Weikang, Shanghai 201203 (CN)
(74) Representative: Buzzi, Notaro & Antonielli d'Oulx S.p.A.
(86) International application number: PCT/CN2024/103239
(87) International publication number: WO 2025/007873

(57) **Abstract**

Provided in the present application is a new compound having KAT6 imhibitory activity, a pharmaceutical composition containing the compound, a useful intermediate for preparimg the compound, and the use of the compound of the present application in the preparation of a drug for treating cancer.

## Description

The present application claims priorities to Chinese Patent Application No. CN202310816102X, filed with the China National Intellectual Property Administration on July 04, 2023, titled "KAT6 INHIBITOR"; Chinese Patent Application No. CN2023111614487, filed with the China National Intellectual Property Administration on September 08, 2023, titled "KAT6 INHIBITOR"; Chinese Patent Application No. CN2023116187290, filed with the China National Intellectual Property Administration on November 29, 2023, titled "KAT6 INHIBITOR"; Chinese Patent Application No. CN2024100246927, filed with the China National Intellectual Property Administration on January 08, 2024, titled "KAT6 INHIBITOR"; and Chinese Patent Application No. CN2024104524832, filed with the China National Intellectual Property Administration on April 15, 2024, titled "KAT6 INHIBITOR", which are incorporated herein by reference in their entirety.

### TECHNICAL FIELD

The present application belongs to the field of medicinal chemistry and relates to a KAT6 inhibitor, specifically to a novel compound with KAT6 inhibitory activity, a pharmaceutical composition containing the compound, a useful intermediate for preparing the compound, and a method of using the compound of the present application for treating a disease mediated by KAT6.

### BACKGROUND

KAT6A (Lysine Acetyltransferase 6A, also known as MOZ) and KAT6B (Lysine Acetyltransferase 6B, also known as MORF) are members of the MYST family of acetyltransferases. KAT6A is involved in chromosomal translocations in acute myeloid leukemia and exhibits amplification mutations in various cancers such as lung cancer, breast cancer, ovarian cancer, endometrial carcinoma, bladder cancer, and esophageal cancer. Similarly, KAT6B is also involved in chromosomal translocation mutations in various cancer types. MOZ- and MORF-linked fusion proteins identified in acute myeloid leukemia include MOZ-CBP, MOZ-p300, MOZ-TIF2, MOZ-NcoA3, MOZ-LEUTX, and MORF-CBP. Among these, MOZ-TIF2 exhibits transforming activity in cultured cells and can induce acute myeloid leukemia in mice. In tumor cells with KAT6A and KAT6B amplification, the expression of KAT6A and KAT6B is closely related to gene copy number, indicating selective pressure to maintain their activity during tumorigenesis. Furthermore, in cell proliferation assays, tumor cells with high expression of KAT6A and KAT6B are generally more dependent on the activity of KAT6A and KAT6B.

KAT6A and KAT6B typically widely acetylate histone H3 at lysine 23 (H3K23), while KAT6A can also specifically acetylate at H3K9 of its regulated genes. KAT6A interacts with transcription factors such as p53 and RUNX1, acetylates histones, and regulates the expression of downstream genes. KAT6A binds to the proximal promoter region of the Estrogen Receptor α (ERα) gene and activates ERα gene expression. In ER-positive breast cancer cells with KAT6A amplification mutations or high expression, inhibiting the acetyltransferase activity of KAT6A or knocking down KAT6A can significantly inhibit the proliferation of breast cancer cells. In addition, the acetyltransferase activity of KAT6A is crucial for promoting the expression of MEIS1 and HOXa9 genes, which are often overexpressed in some lymphoma and leukemia cells. In a MYC-induced lymphoma mouse model, deletion of one KAT6A allele significantly prolonged the median survival of the mice. In mice, mutation of the KAT6B allele leads to a significant reduction in the division and differentiation of cortical progenitor cells, severely affecting brain cortex development. KAT6B also plays an important role in maintaining the number of adult neural stem cells. KAT6B also has gene mutations in some rare types of leukemia.

Although numerous research efforts have been directed toward this mechanism of action, but no KAT6 inhibitor has been approved for clinical use to date. Consequently, there is an urgent need to develop effective KAT6 inhibitors for clinical application.

### SUMMARY

The present application provides a compound of formula (I), or an isomer, deuterated form, or pharmaceutically acceptable salt thereof, 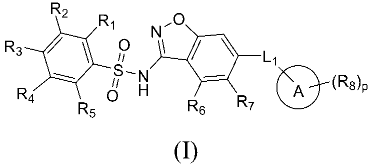 wherein,
R₁ and R₂ are each independently selected from the group consisting of hydrogen, halogen, C₁₋₄ alkyl, C₁₋₄ haloalkyl, C₁₋₄ alkoxy, substituted or unsubstituted C₃₋₆ cycloalkyl, -C₁₋₄ alkyl-O-C₁₋₄ alkyl, hydroxy, -C₁₋₄ alkylhydroxy, -C₁₋₄ alkyl-S-C₁₋₄ alkyl, amino, and C₁₋₄ alkylamino;
R₃ is selected from the group consisting of hydrogen, halogen, C₁₋₄ alkyl, C₁₋₄ haloalkyl, C₁₋₄ alkoxy, hydroxy, and amino; R₄ and R₅ together with carbon atoms to which they are bonded form a 7- or 8-membered heterocyclyl, the heterocyclyl is optionally substituted by m Rₐ, two Rₐ located on the same carbon atom can form a C₃₋₆ cycloalkyl or a 3- to 6-membered heterocyclyl, or two Rₐ located on adjacent carbon atoms can form a C₃₋₆ cycloalkyl or a 3- to 6-membered heterocyclyl;
or R₅ is selected from the group consisting of hydrogen, halogen, C₁₋₄ alkyl, C₁₋₄ haloalkyl, C₁₋₄ alkoxy, hydroxy, and amino; R₃ and R₄ together with carbon atoms to which they are bonded form a 7- or 8-membered heterocyclyl, the heterocyclyl is optionally substituted by m Rₐ, two Rₐ located on the same carbon atom can form a C₃₋₆ cycloalkyl or a 3- to 6-membered heterocyclyl, or two Rₐ located on adjacent carbon atoms can form a C₃₋₆ cycloalkyl or a 3- to 6-membered heterocyclyl;
Rₐ is selected from the group consisting of hydrogen, halogen, C₁₋₄ alkyl, C₁₋₄ haloalkyl, C₁₋₄ alkoxy, hydroxy, and amino;
m is 1, 2, 3, or 4;
R₆ and R₇ are each independently selected from the group consisting of hydrogen, halogen, C₁₋₄ alkyl, C₁₋₄ haloalkyl, C₁₋₄ alkoxy, C₁₋₄ haloalkoxy, C₃₋₆ cycloalkyl, C₃₋₆ cycloalkyloxy, and deuterated C₁₋₄ alkoxy; or R₆ and R₇ together with the carbon atoms to which they are bonded form a 5- to 8-membered heterocyclyl, the heterocyclyl is optionally substituted by n R_{b};
R_{b} is selected from the group consisting of hydrogen, halogen, C₁₋₄ alkyl, C₁₋₄ haloalkyl, C₁₋₄ alkoxy, hydroxy, and amino;
n is 1, 2, 3, or 4;
L₁ is selected from the group consisting of C₁₋₄ alkylene, -O-, and deuterated C₁₋₄ alkylene;
ring A is a 5- or 6-membered heteroaryl;
R₈ is selected from the group consisting of hydrogen, halogen, C₁₋₄ alkyl, C₁₋₄ haloalkyl, C₁₋₄ alkoxy, substituted or unsubstituted C₃₋₆ cycloalkyl, amino, -C₁₋₄ alkylamino, and hydroxy; and
p is 1, 2, 3, or 4.

The present application provides a compound of formula (I), or an isomer, deuterated form, or pharmaceutically acceptable salt thereof, 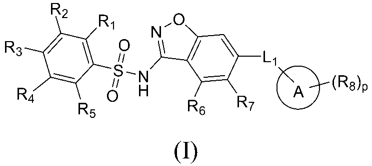 wherein,
R₁ and R₂ are each independently selected from the group consisting of hydrogen, halogen, C₁₋₄ alkyl, C₁₋₄ haloalkyl, C₁₋₄ alkoxy, C₃₋₆ cycloalkyl, -C₁₋₄ alkyl-O-C₁₋₄ alkyl, hydroxy, -C₁₋₄ alkylhydroxy, -C₁₋₄ alkyl-S-C₁₋₄ alkyl, amino, and C₁₋₄ alkylamino;
R₃ is selected from the group consisting of hydrogen, halogen, C₁₋₄ alkyl, C₁₋₄ haloalkyl, C₁₋₄ alkoxy, hydroxy, and amino; R₄ and R₅ together with carbon atoms to which they are bonded form a 7- or 8-membered heterocyclyl, the heterocyclyl is optionally substituted by m Rₐ, two Rₐ located on the same carbon atom can form a C₃₋₆ cycloalkyl or a 3- to 6-membered heterocyclyl, or two Rₐ located on adjacent carbon atoms can form a C₃₋₆ cycloalkyl or a 3- to 6-membered heterocyclyl;
or R₅ is selected from the group consisting of hydrogen, halogen, C₁₋₄ alkyl, C₁₋₄ haloalkyl, C₁₋₄ alkoxy, hydroxy, and amino; R₃ and R₄ together with carbon atoms to which they are bonded form a 7- or 8-membered heterocyclyl, the heterocyclyl is optionally substituted by m Rₐ, two Rₐ located on the same carbon atom can form a C₃₋₆ cycloalkyl or a 3- to 6-membered heterocyclyl, or two Rₐ located on adjacent carbon atoms can form a C₃₋₆ cycloalkyl or a 3- to 6-membered heterocyclyl;
Rₐ is selected from the group consisting of hydrogen, halogen, C₁₋₄ alkyl, C₁₋₄ haloalkyl, C₁₋₄ alkoxy, hydroxy, and amino;
m is 1, 2, 3, or 4;
R₆ and R₇ are each independently selected from the group consisting of hydrogen, halogen, C₁₋₄ alkyl, C₁₋₄ haloalkyl, C₁₋₄ alkoxy, C₁₋₄ haloalkoxy, C₃₋₆ cycloalkyl, C₃₋₆ cycloalkyloxy, and deuterated C₁₋₄ alkoxy; or R₆ and R₇ together with carbon atoms to which they are bonded form a 5- to 8-membered heterocyclyl, the heterocyclyl is optionally substituted by n R_{b};
R_{b} is selected from the group consisting of hydrogen, halogen, C₁₋₄ alkyl, C₁₋₄ haloalkyl, C₁₋₄ alkoxy, hydroxy, and amino;
n is 1, 2, 3, or 4;
L₁ is selected from the group consisting of C₁₋₄ alkylene, -O-, and deuterated C₁₋₄ alkylene;
ring A is a 5- or 6-membered heteroaryl; heteroatom(s) in the 5- or 6-membered heteroaryl is/are each independently selected from the group consisting of S and N;
R₈ is selected from the group consisting of hydrogen, halogen, C₁₋₄ alkyl, C₁₋₄ haloalkyl, C₁₋₄ alkoxy, C₃₋₆ cycloalkyl, amino, -C₁₋₄ alkylamino, and hydroxy;
p is 1, 2, 3, or 4; and
heteroatom(s) in the heterocyclyl is/are each independently selected from the group consisting of O, S and N.

The present application provides a compound of formula (I), or an isomer, deuterated form, or pharmaceutically acceptable salt thereof, 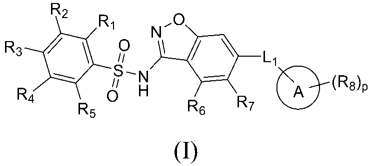 wherein,
R₁ and R₂ are each independently selected from the group consisting of hydrogen, halogen, C₁₋₄ alkyl, C₁₋₄ haloalkyl, C₁₋₄ alkoxy, substituted or unsubstituted C₃₋₆ cycloalkyl, -C₁₋₄ alkyl-O-C₁₋₄ alkyl, hydroxy, -C₁₋₄ alkylhydroxy, -C₁₋₄ alkyl-S-C₁₋₄ alkyl, amino, and C₁₋₄ alkylamino;
R₃ is selected from the group consisting of hydrogen, halogen, C₁₋₄ alkyl, C₁₋₄ haloalkyl, C₁₋₄ alkoxy, hydroxy, and amino; R₄ and R₅ together with carbon atoms to which they are bonded form a 7- or 8-membered heterocyclyl, the heterocyclyl is optionally substituted by m Rₐ, two Rₐ located on the same carbon atom can form a C₃₋₆ cycloalkyl or a 3- to 6-membered heterocyclyl, or two Rₐ located on adjacent carbon atoms can form a C₃₋₆ cycloalkyl or a 3- to 6-membered heterocyclyl;
or R₅ is selected from the group consisting of hydrogen, halogen, C₁₋₄ alkyl, C₁₋₄ haloalkyl, C₁₋₄ alkoxy, hydroxy, and amino; R₃ and R₄ together with carbon atoms to which they are bonded form a 7- or 8-membered heterocyclyl, the heterocyclyl is optionally substituted by m Rₐ, two Rₐ located on the same carbon atom can form a C₃₋₆ cycloalkyl or a 3- to 6-membered heterocyclyl, or two Rₐ located on adjacent carbon atoms can form a C₃₋₆ cycloalkyl or a 3- to 6-membered heterocyclyl;
Rₐ is selected from the group consisting of hydrogen, halogen, C₁₋₄ alkyl, C₁₋₄ haloalkyl, C₁₋₄ alkoxy, hydroxy, and amino;
m is 1, 2, 3, or 4;
R₆ and R₇ are each independently selected from the group consisting of hydrogen, halogen, C₁₋₄ alkyl, C₁₋₄ haloalkyl, C₁₋₄ alkoxy, C₁₋₄ haloalkoxy, C₃₋₆ cycloalkyl, and C₃₋₆ cycloalkyloxy; or R₆ and R₇ together with carbon atoms to which they are bonded form a 5- to 8-membered heterocyclyl, the heterocyclyl is optionally substituted by n R_{b};
R_{b} is selected from the group consisting of hydrogen, halogen, C₁₋₄ alkyl, C₁₋₄ haloalkyl, C₁₋₄ alkoxy, hydroxy, and amino;
n is 1, 2, 3, or 4;
L₁ is selected from the group consisting of C₁₋₄ alkylene and -O-;
ring A is a 5- or 6-membered heteroaryl;
R₈ is selected from the group consisting of hydrogen, halogen, C₁₋₄ alkyl, C₁₋₄ haloalkyl, C₁₋₄ alkoxy, substituted or unsubstituted C₃₋₆ cycloalkyl, amino, -C₁₋₄ alkylamino, and hydroxy; and
p is 1, 2, 3, or 4.

In some embodiments of the present application, in the aforementioned compound of formula (I), or an isomer, deuterated form, or pharmaceutically acceptable salt thereof, R₁ is selected from the group consisting of CH₃O-, and hydrogen.

In some embodiments of the present application, in the aforementioned compound of formula (I), or an isomer, deuterated form, or pharmaceutically acceptable salt thereof, R₁ is CH₃O-.

In some embodiments of the present application, in the aforementioned compound of formula (I), or an isomer, deuterated form, or pharmaceutically acceptable salt thereof, R₂ is H.

In some embodiments of the present application, in the aforementioned compound of formula (I), or an isomer, deuterated form, or pharmaceutically acceptable salt thereof, the 7- or 8-membered heterocyclyl formed by R₃ and R₄ together with the carbon atoms to which they are bonded is selected from the group consisting of

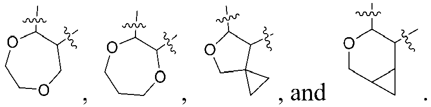

In some embodiments of the present application, in the aforementioned compound of formula (I), or an isomer, deuterated form, or pharmaceutically acceptable salt thereof, the 7- or 8-membered heterocyclyl formed by R₃ and R₄ together with the carbon atoms to which they are bonded is selected from the group consisting of

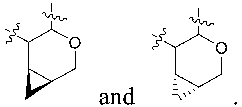

In some embodiments of the present application, in the aforementioned compound of formula (I), or an isomer, deuterated form, or pharmaceutically acceptable salt thereof, the 7- or 8-membered heterocyclyl formed by R₄ and R₅ together with the carbon atoms to which they are bonded is selected from the group consisting of

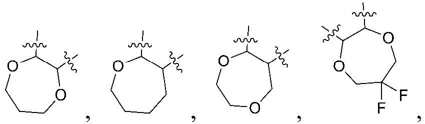

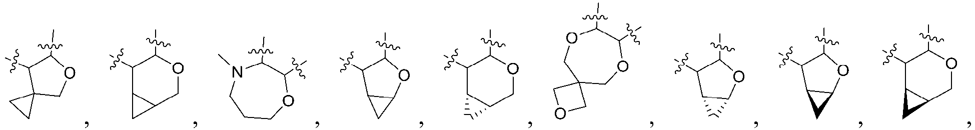

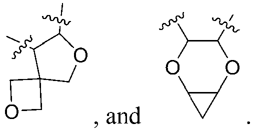

In some embodiments of the present application, in the aforementioned compound of formula (I), or an isomer, deuterated form, or pharmaceutically acceptable salt thereof, the 7- or 8-membered heterocyclyl formed by R₄ and R₅ together with the carbon atoms to which they are bonded is selected from the group consisting of

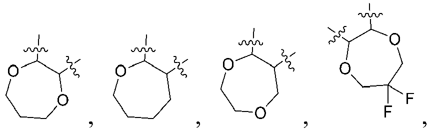

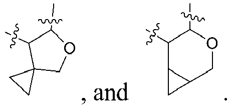

In some embodiments of the present application, in the aforementioned compound of formula (I), or an isomer, deuterated form, or pharmaceutically acceptable salt thereof, the 7- or 8-membered heterocyclyl formed by R₄ and R₅ together with the carbon atoms to which they are bonded is selected from the group consisting of

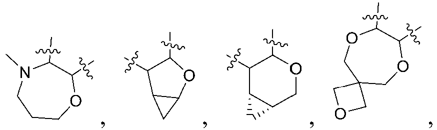

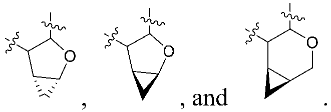

In some embodiments of the present application, in the aforementioned compound of formula (I), or an isomer, deuterated form, or pharmaceutically acceptable salt thereof, the 7- or 8-membered heterocyclyl formed by R₄ and R₅ together with the carbon atoms to which they are bonded is selected from the group consisting of

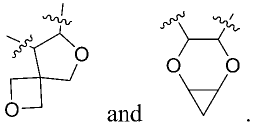

In some embodiments of the present application, in the aforementioned compound of formula (I), or an isomer, deuterated form, or pharmaceutically acceptable salt thereof, the 7- or 8-membered heterocyclyl formed by R₃ and R₄ together with the carbon atoms to which they are bonded is

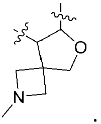

In some embodiments of the present application, in the aforementioned compound of formula (I), or an isomer, deuterated form, or pharmaceutically acceptable salt thereof, Rₐ is -F.

In some embodiments of the present application, in the aforementioned compound of formula (I), or an isomer, deuterated form, or pharmaceutically acceptable salt thereof, Rₐ is -CH₃.

In some embodiments of the present application, in the aforementioned compound of formula (I), or an isomer, deuterated form, or pharmaceutically acceptable salt thereof, 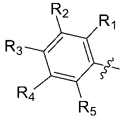 is selected from the group consisting of 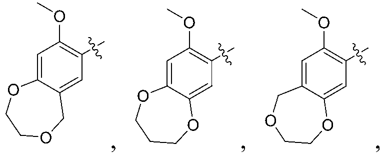 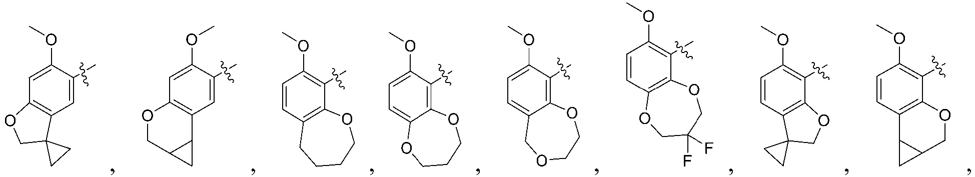 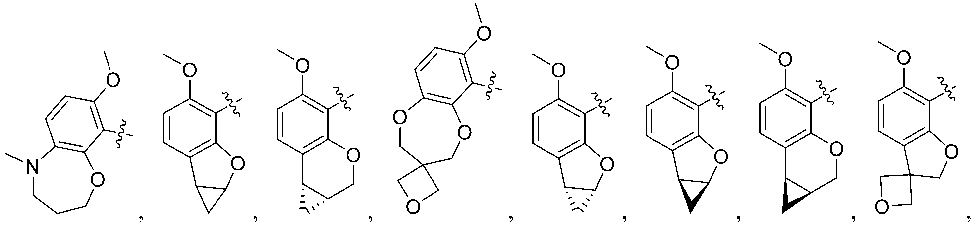 and

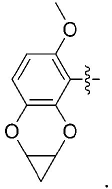

In some embodiments of the present application, in the aforementioned compound of formula (I), or an isomer, deuterated form, or pharmaceutically acceptable salt thereof, 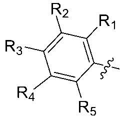 is selected from the group consisting of

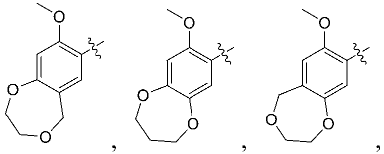

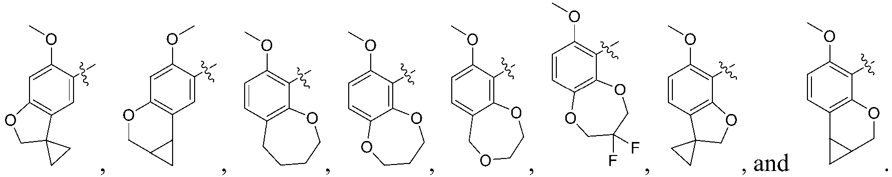

In some embodiments of the present application, in the aforementioned compound of formula (I), or an isomer, deuterated form, or pharmaceutically acceptable salt thereof, 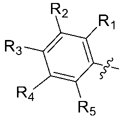 is selected from the group consisting of

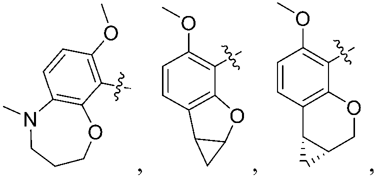

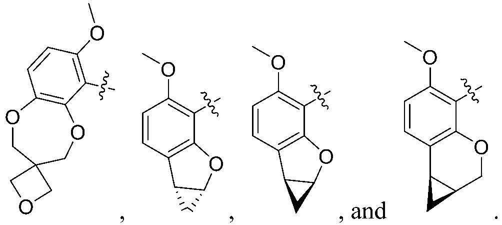

In some embodiments of the present application, in the aforementioned compound of formula (I), or an isomer, deuterated form, or pharmaceutically acceptable salt thereof, 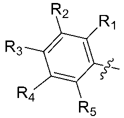 is selected from the group consisting of

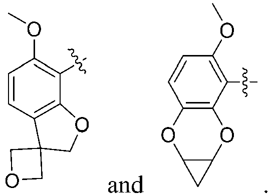

In some embodiments of the present application, in the aforementioned compound of formula (I), or an isomer, deuterated form, or pharmaceutically acceptable salt thereof, 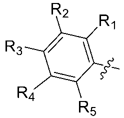 is selected from the group consisting of 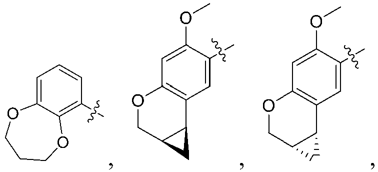 and

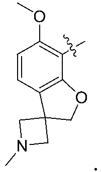

In some embodiments of the present application, in the aforementioned compound of formula (I), or an isomer, deuterated form, or pharmaceutically acceptable salt thereof, R₆ and R₇ are each independently selected from the group consisting of -F, -CH₃, CH₃O-, C₂H₅O-, CHF₂O, 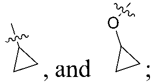 preferably, R₆ is CH₃O-.

In some embodiments of the present application, in the aforementioned compound of formula (I), or an isomer, deuterated form, or pharmaceutically acceptable salt thereof, R₆ and R₇ are each independently selected from the group consisting of -C₂H₅ and -OCD₃.

In some embodiments of the present application, in the aforementioned compound of formula (I), or an isomer, deuterated form, or pharmaceutically acceptable salt thereof, the 5- to 8-membered heterocyclyl formed by R₆ and R₇ together with the carbon atoms to which they are bonded is selected from the group consisting of

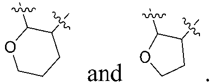

In some embodiments of the present application, in the aforementioned compound of formula (I), or an isomer, deuterated form, or pharmaceutically acceptable salt thereof, the 5- to 8-membered heterocyclyl formed by R₆ and R₇ together with the carbon atoms to which they are bonded is

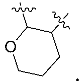

In some embodiments of the present application, in the aforementioned compound of formula (I), or an isomer, deuterated form, or pharmaceutically acceptable salt thereof, the 5- to 8-membered heterocyclyl formed by R₆ and R₇ together with the carbon atoms to which they are bonded is

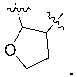

In some embodiments of the present application, in the aforementioned compound of formula (I), or an isomer, deuterated form, or pharmaceutically acceptable salt thereof, Li is -CH₂-.

In some embodiments of the present application, in the aforementioned compound of formula (I), or an isomer, deuterated form, or pharmaceutically acceptable salt thereof, Li is -CD₂-.

In some embodiments of the present application, in the aforementioned compound of formula (I), or an isomer, deuterated form, or pharmaceutically acceptable salt thereof, L₁ is -O-.

In some embodiments of the present application, in the aforementioned compound of formula (I), or an isomer, deuterated form, or pharmaceutically acceptable salt thereof, ring A is selected from the group consisting of

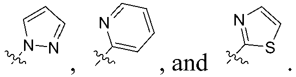

The present application provides a benzoisoxazole derivative, a pharmaceutically acceptable salt thereof, a pharmaceutical composition thereof, and use thereof in medicine.

The compound, or an isomer, deuterated form, or pharmaceutically acceptable salt thereof provided by the present application, wherein the compound is selected from the group consisting of the following compounds:

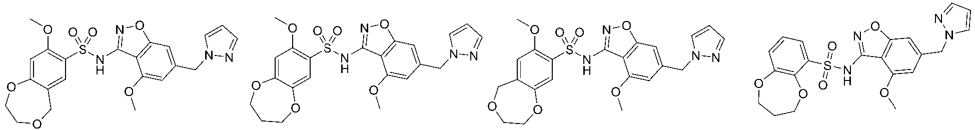

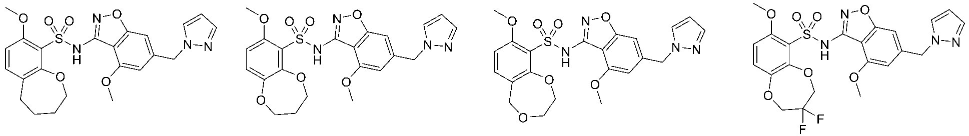

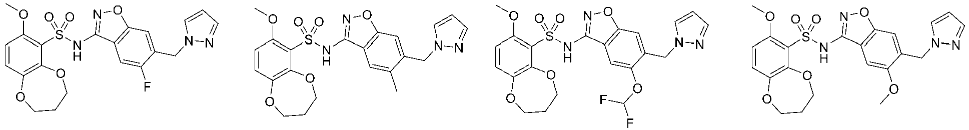

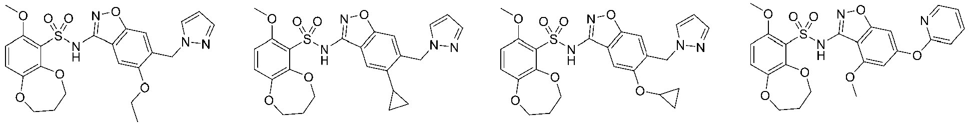

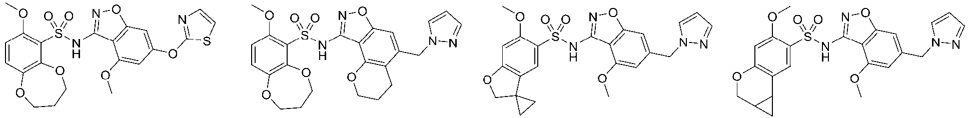

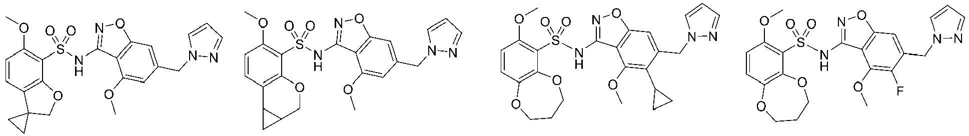

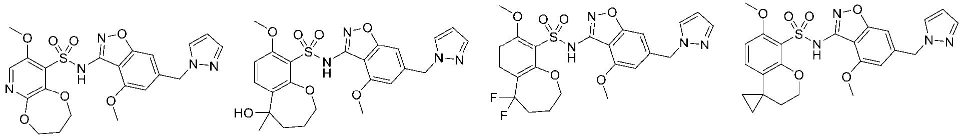

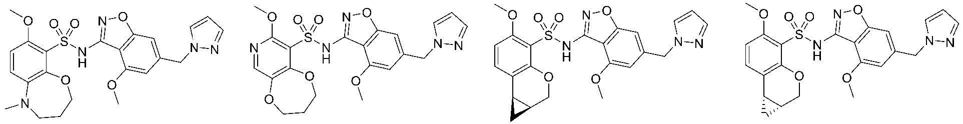

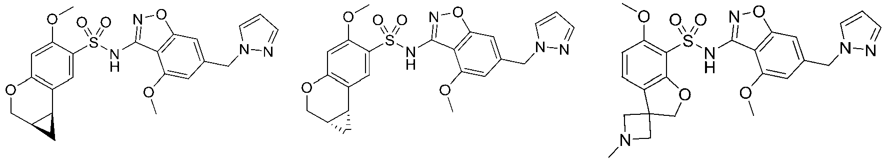

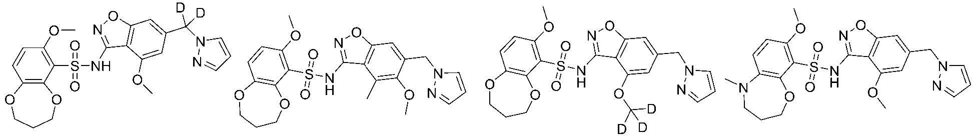

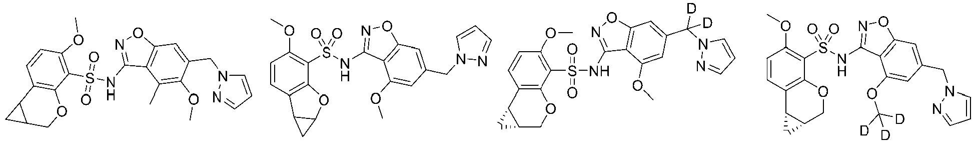

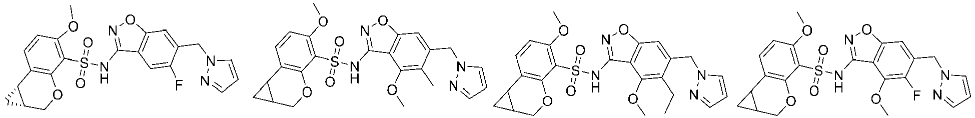

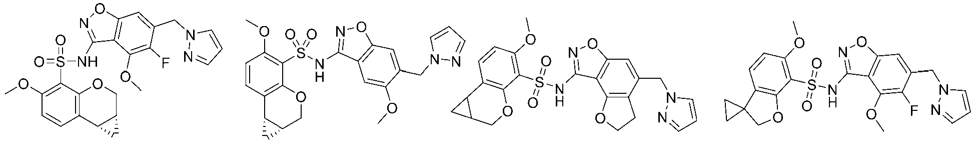

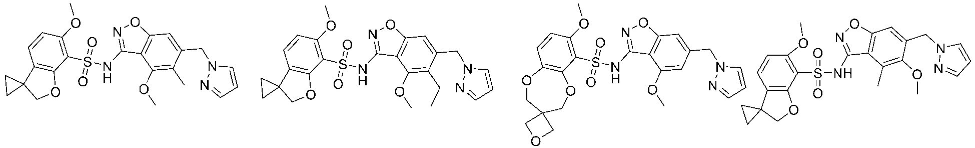

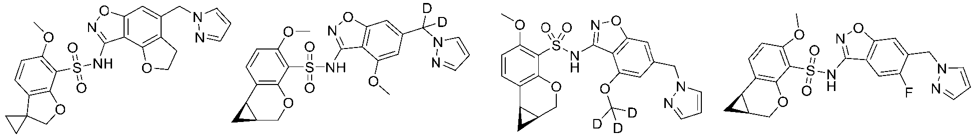

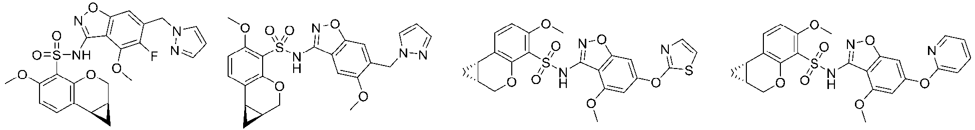

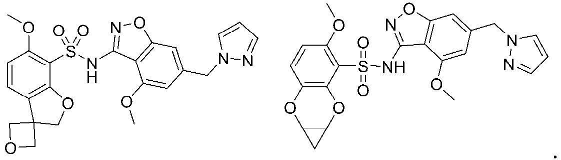

The present application also provides a pharmaceutical composition comprising a therapeutically effective amount of the aforementioned compound, or an isomer, deuterated form, or pharmaceutically acceptable salt thereof, and a pharmaceutically acceptable carrier.

In certain embodiments of the present application, in the pharmaceutical composition, the content of the compound, or an isomer, deuterated form, or pharmaceutically acceptable salt thereof ranges from 0.1 mg to 1000 mg.

In certain embodiments of the present application, in the pharmaceutical composition, the pharmaceutically acceptable carrier comprises one or more selected from the group consisting of a filler, a disintegrant, a binder, a glidant, and a lubricant.

The present application also provides the use of the aforementioned compound, or an isomer, deuterated form, or pharmaceutically acceptable salt thereof, or the aforementioned pharmaceutical composition, in the manufacture of a medicament for treating a KAT6-mediated disease, wherein the KAT6-mediated disease includes cancer.

The present application also provides a method for treating a KAT6-mediated disease, comprising administering a therapeutically effective amount of the aforementioned compound, or an isomer, deuterated form, or pharmaceutically acceptable salt thereof, or the aforementioned pharmaceutical composition, to a subject in need thereof; preferably, the KAT6-mediated disease includes cancer.

### TECHNICAL EFFECTS

The compound provided by the present application exhibits significant KAT6 enzymatic inhibitory activity, which can be used for the treatment of cancer.

### BRIF DESCRIPTION OF THE DRAWINGS

The drawings described herein are provided to further understand the present application, constitute a part of the present application, and the exemplary embodiments of the present application and their details are used to explain the present application and do not constitute an undue limitation of the present application.
FIG. 1 is a graph showing the body weight changes of mice in different groups in the in vivo pharmacodynamic study of the present application;
FIG. 2 is a graph showing the tumor growth curves of mice in different groups in the in vivo pharmacodynamic study of the present application.

### DETAILED DESCRIPTION

### Description and Definitions

Unless otherwise stated, the following terms and phrases used herein are intended to have the following meanings. A specific term or phrase without a specific definition should not be considered uncertain or unclear, but should be understood according to its ordinary meaning.

The compounds of the present application exist as isomers including geometric isomers and stereoisomers, such as cis-trans isomers, enantiomers, diastereomers, and racemic mixtures and other mixtures thereof, all of which fall within the scope of the present application.

The term "enantiomer" refers to stereoisomers that are mirror images of each other.

The term "diastereomer" refers to stereoisomers wherein the molecules have two or more chiral centers and the molecules are non-mirror images of each other.

The term "cis-trans isomer" refers to a configuration existing due to the inability of double bonds or single bonds of ring carbon atoms in a molecule to rotate freely.

Unless otherwise specified, a wedged solid bond 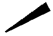 and a wedged hashed bond 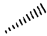 are used to indicate the absolute configuration of a stereocenter, while a straight solid bond 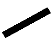 and a straight hashed bond 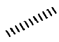 are used to indicate the relative configuration of a stereocenter. For example, 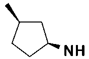 represents that the methyl group and the amino group are located on the same side of the cyclopentane ring. Stereoisomers of the compounds of the present application can be prepared by chiral synthesis, chiral reagents, or other conventional techniques. For instance, one enantiomer of a compound of the present application can be prepared using asymmetric catalytic techniques or chiral auxiliary derivatization techniques. Alternatively, a single stereoisomeric compound can be obtained from a mixture using chiral resolution techniques. Or, it can be prepared directly using chiral starting materials. The separation of optically pure compounds in the present application is typically accomplished using preparative chromatography with a chiral column to achieve the separation of chiral compounds.

The absolute stereoconfiguration of a compound can be confirmed by conventional technical means in the art. For example, single-crystal X-ray diffraction can be used, or the absolute configuration of the compound can be confirmed based on the chiral structure of the starting material and the reaction mechanism of asymmetric synthesis. Compounds labeled as "absolute configuration not determined" in this application are typically obtained by resolving a racemic compound into a single isomer using preparative SFC (Supercritical Fluid Chromatography) with chiral separation, followed by characterization and testing.

Unless otherwise specified, "substituted or unsubstituted" means it may be substituted or may not be substituted. Examples of the "substituted or unsubstituted C₃₋₆ cycloalkyl" include, but are not limited to

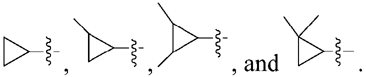

The term "halogen" denotes a fluorine, chlorine, bromine, or iodine atom.

The term "C₁₋₄ alkyl" is used to denote a C₁₋₄ straight or branched chain saturated hydrocarbon group. Examples of alkyl groups include, but are not limited to, methyl, ethyl, n-propyl, isopropyl, butyl, isobutyl, and the like.

The term "C₁₋₄ haloalkyl" refers to an alkyl group in which one or more hydrogen atoms are replaced by halogen atoms. Examples of C₁₋₄ haloalkyl include, but are not limited to fluoromethyl, difluoromethyl, trifluoromethyl, trichloromethyl, tribromomethyl, 2,2,2-trifluoroethyl, 2,2,2-trichloroethyl, and the like.

The term "C₁₋₄ alkylene" is used to denote a C₁₋₄ straight or branched chain saturated divalent hydrocarbon group. Examples of C₁₋₄ alkylene include, but are not limited to -CH₂-, -(CH₂)₂-, -(CH₂)₃-, -(CH₂)₄-, -CH(CH₃)-, and -C(CH₃)₂-.

The term "C₁₋₄ deuterated alkylene" is used to denote a C₁₋₄ straight or branched chain saturated divalent hydrocarbon group in which hydrogen atoms are substituted by D atoms. Examples of C₁₋₄ deuterated alkylene include, but are not limited to -CD₂- and -CH(CD₃)-.

The term "C₁₋₄ alkoxy" refers to a C₁₋₄ alkyl group connected via an oxygen bridge. Examples of the C₁₋₄ alkoxy include, but are not limited to methoxy, ethoxy, n-propoxy, isopropoxy, n-butoxy, sec-butoxy, and tert-butoxy.

The term "deuterated alkoxy" refers to a deuterated C₁₋₄ alkyl group connected via an oxygen bridge. Herein, the deuterated C₁₋₄ alkyl denotes a straight or branched chain hydrocarbon group in which hydrogen atoms are completely or partially replaced by D atoms. Examples of deuterated C₁₋₄ alkoxy include, but are not limited to -OCD₃, -OCD₂CD₃, -OCD₂(CD₃), and -OCH₂(CD₃).

The term "C₃₋₆ cycloalkyl" refers to a 3- to 6-membered monocyclic alkyl group. Examples of C₃₋₆ cycloalkyl include, but are not limited to cyclopropyl, cyclobutyl, cyclopentyl, cyclohexyl, cycloheptyl, and cyclooctyl.

The term "heterocyclyl" refers to a non-aromatic cyclic group containing at least one heteroatom as a ring atom, derived by removal of one hydrogen atom; it includes saturated or partially saturated monocyclic heterocyclyl and bicyclic heterocyclyl groups. The terms "5- to 8-membered heterocyclyl" and "7- or 8-membered heterocyclyl" refer to substituted or unsubstituted 5- to 8-membered saturated or unsaturated non-aromatic rings or substituted and unsubstituted 7- or 8-membered saturated or unsaturated non-aromatic rings, containing 1-3 heteroatoms selected from the group consisting of N, O, and S. The nitrogen atom(s) may optionally be quaternized, and the nitrogen and sulfur heteroatoms may optionally be oxidized (i.e., NO and S(O)ₚ, where p is 1 or 2). The heterocyclyl is not limited by the point of attachment (i.e., it can be bonded via a carbon atom or a heteroatom). Examples of "heterocyclyl" include, but are not limited to

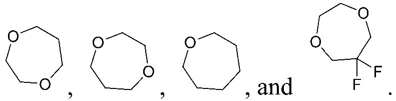

The term "spiroheterocyclyl" refers to a saturated or partially saturated cyclic structure formed by two or more ring structures sharing one common ring atom, containing at least one ring atom being a heteroatom. The heteroatoms are generally selected from the group consisting of CO, N, O, S, NO, SO, and S(O)₂. The "7- to 10-membered spiroheterocyclyl" described in the present application includes "7- to 10-membered saturated spiroheterocyclyl" and "7- to 10-membered partially saturated spiroheterocyclyl", preferably 7- to 10-membered oxygen-containing saturated spiroheterocyclyl. Specific examples include, but are not limited to

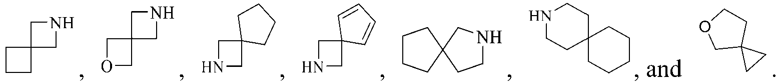

The term "fused heterocyclyl" refers to a saturated or partially saturated non-aromatic cyclic group formed by two or more ring structures sharing two adjacent atoms, containing at least one ring atom being a heteroatom; the heteroatoms are generally selected from the group consisting of N, O, and S; the ring carbon atoms and heteroatoms in the fused heterocycle may be further oxidized to form cyclic groups containing C(O), NO, SO, S(O)₂ groups, which are also included within the definition of the heterocyclyl described in the present application. The "non-aromatic" described in this definition means that the group does not possess aromaticity when it exists independently. The present application does not restrict that the group, when connected to other structures via intra-ring or extra-ring unsaturated bonds, or connected to other unsaturated structures via single bonds, or under specific conditions (such as in specific solvents), may exhibit aromaticity. The "7- to 10-membered fused heterocyclyl" described in the present application includes "7- to 10-membered saturated fused heterocyclyl" and "7- to 10-membered partially saturated fused heterocyclyl". The fusion mode can be a 5- or 6-membered heterocyclyl fused with a 3- to 4-membered heterocyclyl, a 5- or 6-membered heterocyclyl fused with a 3- to 4-membered cycloalkyl, a 5- or 6-membered heteroaryl fused with a 3- to 4-membered heterocyclyl, or a 5- or 6-membered heteroaryl fused with a 3- to 4-membered cycloalkyl. Specific examples of the fused heterocyclyl include, but are not limited to

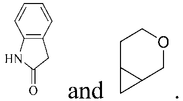

The term "heteroaryl" refers to a monocyclic group possessing aromaticity in which at least one ring atom is a heteroatom and/or heteroatomic group; the heteroatoms and/or heteroatomic groups are generally selected from the group consisting of N, O, S, P, NO, SO, S(O)₂, P(O), and NR, where R is H or any possible substituent group, wherein carbon atoms in the heterocycle may optionally be oxidized, i.e., forming -C(O); preferably, the heteroatoms are independently selected from the group consisting of 1-3 N atom(s) and/or O atom(s). The heteroaryl includes "5- or 6-membered heteroaryl"; specific examples include, but are not limited to, pyrrolyl, furanyl, thienyl, pyrazolyl, imidazolyl, pyrazinyl, pyridazinyl, triazinyl, oxazolyl, isoxazolyl, thiazolyl, isothiazolyl, pyridyl, pyrimidinyl.

The term "pharmaceutically acceptable" refers to those compounds, materials, compositions, and/or dosage forms that are, within the scope of sound medical judgment, suitable for contact with the tissues of humans and animals without excessive toxicity, irritation, allergic reactions, or other problems or complications, commensurate with a reasonable benefit/risk ratio.

The term "pharmaceutically acceptable salt" refers to a derivative of the compound of the present application prepared by reaction with a relatively non-toxic acid or base. These salts can be prepared during the synthesis, isolation, or purification of the compound, or separately by reacting the purified compound in its free form with a suitable acid or base. When the compound contains a relatively acidic functional group, a base addition salt can be obtained by reaction with an alkali metal, alkaline earth metal hydroxide, or organic amine, including cations based on alkali metals and alkaline earth metals, as well as non-toxic ammonium, quaternary ammonium, and amine cations, and also encompasses salts of amino acids, etc. When the compound contains a relatively basic functional group, an acid addition salt can be obtained by reaction with an organic or inorganic acid.

The term "pharmaceutically acceptable carrier" refers to a medium generally accepted in the art for delivering a biologically active agent to an animal, particularly a mammal, and includes, depending on the route of administration and the nature of the dosage form, for example, adjuvants, excipients, or vehicles, such as diluents, preservatives, fillers, flow regulators, disintegrants, wetting agents, emulsifiers, suspending agents, sweeteners, flavoring agents, perfuming agents, antibacterial agents, antifungal agents, lubricants, and dispersing agents. Pharmaceutically acceptable carriers are formulated within the purview of one of ordinary skill in the art considering a multitude of factors. These include, but are not limited to: the type and nature of the active agent being formulated; the subject to whom the composition containing the agent is to be administered; the intended route of administration of the composition; and the targeted therapeutic indication. Pharmaceutically acceptable carriers include both aqueous and non-aqueous media, as well as various solid and semi-solid dosage forms. Besides the active agent, such carriers include many different ingredients and additives; such additional ingredients included in the formulation for various reasons (e.g., stabilizing the active agent, binders, etc.) are well known to those of ordinary skill in the art.

The compounds of the present application can be prepared by various synthetic methods well-known to those skilled in the art, including the specific embodiments listed below, embodiments formed by their combination with other chemical synthesis methods, and equivalent substitution methods well-known to those skilled in the art. Preferred embodiments include, but are not limited to, the examples of the present application.

The solvents used in the present application are commercially available.

The structures of the compounds of the present application were determined by nuclear magnetic resonance (NMR) and/or liquid chromatography-mass spectrometry (LC-MS), or ultra-performance liquid chromatography-mass spectrometry (UPLC-MS). NMR chemical shifts (δ) are given in parts per million (ppm). NMR measurements were performed using a Bruker Neo 400M or Bruker Ascend 400 NMR instrument, with deuterated dimethyl sulfoxide (DMSO-*d*₆), deuterated methanol (CD₃OD), deuterated chloroform (CDCl₃), or heavy water (D₂O) as the solvent, and tetramethylsilane (TMS) as the internal standard.

Liquid chromatography-mass spectrometry (LC-MS) measurements were performed using an Agilent 1260-6125B single quadrupole mass spectrometer (ion source: electrospray ionization).

Ultra-performance liquid chromatography-mass spectrometry (UPLC-MS) measurements were performed using a Waters UPLC H-class SQD mass spectrometer (ion source: electrospray ionization).

HPLC measurements were performed using a Waters e2695-2998 or Waters ARC and Agilent 1260 or Agilent Poroshell HPH high-performance liquid chromatography system.

Preparative HPLC was performed using Waters 2555-2489 (10 µm, ODS 250 cm × 5 cm) or GILSON Trilution LC.

Chiral HPLC measurements were performed using a waters acquity UPC2 system; the column was Daicel chiral Celpak AD-H (5 µm, 4.6 × 250 mm).

Supercritical fluid chromatography (SFC) was performed using a waters SFC 80Q system.

The starting materials in the examples of the present application are known and commercially available, or can be synthesized using or according to methods known in the art.

Unless otherwise specified, all reactions in the present application were carried out under continuous magnetic stirring, under a dry nitrogen or argon atmosphere, using dry solvents, and reaction temperatures are in degrees Celsius.

The progress of reactions in the examples was monitored by conventional methods such as thin-layer chromatography (TLC), LC-MS, and the like. The eluent systems for column chromatography used in purification and the developing solvent systems for TLC consisted of one or more of the following solvents: dichloromethane, methanol, n-hexane, ethyl acetate, petroleum ether, ethyl acetate, acetone, chloroform, and the like. The volume ratios of the solvents were adjusted according to the polarity of the compounds, and small amounts of basic or acidic reagents such as triethylamine, acetic acid, trifluoroacetic acid, and the like, could also be added for adjustment.

### Intermediate INT-1: 6-((1H-Pyrazol-1-yl)methyl)-4-methoxybenzo[d]isoxazol-3-amine

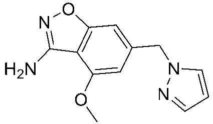

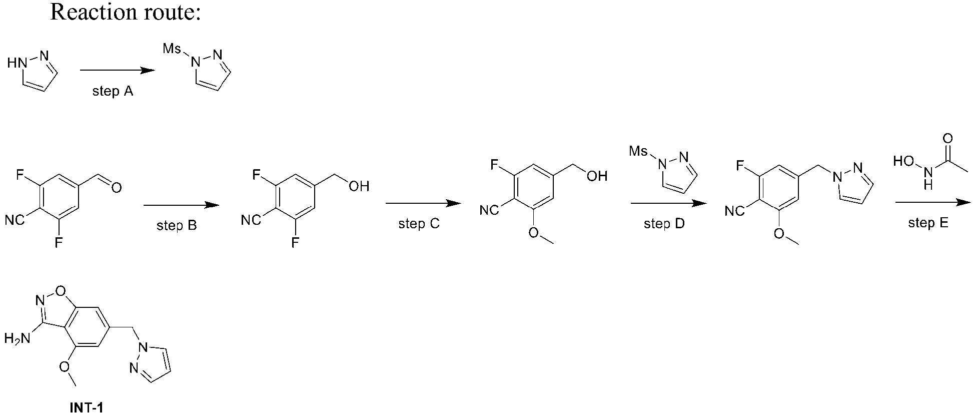

### Operating steps:

**Step A:** Under nitrogen protection, 1H-pyrazole (5 g, 73.5 mmol) and triethylamine (11.1 g, 110.3 mmol) were dissolved in dichloromethane (50 mL). After cooling to 0°C, methanesulfonyl chloride (10.9 g, 95.6 mmol) was added dropwise. The system was stirred at room temperature for 1 hour.

After the disappearance of the starting material as monitored by LCMS, the reaction mixture was quenched by adding saturated ammonium chloride solution. Dichloromethane (300 mL) was added, and the mixture was washed with water (100 mL) and saturated brine (100 mL × 3). The organic phase was dried over anhydrous magnesium sulfate, filtered, and the filtrate was concentrated under reduced pressure to afford 10.7 g of 1-(methylsulfonyl)-1H-pyrazole.

LCMS (ESI) m/z: 147.3 [M+H]⁺.

¹H NMR (400 MHz, DMSO-*d*₆) δ 8.28 (d, *J =* 2.6 Hz, 1H), 7.96 (d, *J =* 1.2 Hz, 1H), 6.61 (dd, *J =* 2.6, 1.6 Hz, 1H), 3.52 (s, 3H).

**Step B:** Under nitrogen protection, 2,6-difluoro-4-formylbenzonitrile (1 g, 6.0 mmol) was dissolved in ethanol (10 mL). After cooling to 0°C, sodium borohydride (226.5 mg, 6.0 mmol) was added dropwise. The system was stirred at 0°C for 2 hours.

After the disappearance of the starting material as monitored by LCMS, the reaction mixture was added to saturated citric acid solution. The ethanol was evaporated under reduced pressure, and the residue was extracted with dichloromethane (50 mL × 3). The combined organic phases were washed with saturated sodium bicarbonate (100 mL), water (100 mL), and saturated brine (100 mL). The organic phase was dried over anhydrous magnesium sulfate, filtered, and the filtrate was concentrated under reduced pressure. The resulting mixture was purified by silica gel column chromatography to afford 680 mg of 2,6-difluoro-4-(hydroxymethyl)benzonitrile.

LCMS (ESI) m/z: 170.1 [M+H]⁺.

**Step C:** Under nitrogen protection, 2,6-difluoro-4-(hydroxymethyl)benzonitrile (1 g, 5.9 mmol) was dissolved in methanol (10 mL). The solution was cooled to 0°C, and a solution of sodium methoxide (4.26 g, 23.67 mmol) was added dropwise. The temperature was heated to room temperature, and the mixture was stirred for 8 hours.

After the disappearance of the starting material as monitored by LCMS, the reaction mixture was poured into saturated aqueous citric acid solution (50 mL) to quench the reaction. Ethyl acetate (100 mL) was added for extraction. The organic phase was washed with saturated brine (100 mL × 3), dried over anhydrous sodium sulfate, filtered, and the filtrate was concentrated under reduced pressure to afford 990 mg of 2-fluoro-4-(hydroxymethyl)-6-methoxybenzonitrile.

LCMS (ESI) m/z: 182.1 [M+H]⁺.

¹H NMR (400 MHz, DMSO-*d*₆) δ 7.05 (s, 1H), 6.98 (d, *J =* 10.0 Hz, 1H), 5.68 (t, *J=* 5.8 Hz, 1H), 4.58 (d, *J =* 5.8 Hz, 2H), 3.94 (s, 3H).

**Step D:** Under nitrogen protection, 2-fluoro-4-(hydroxymethyl)-6-methoxybenzonitrile (6.33 g, 35 mmol), 1-(methylsulfonyl)-1H-pyrazole (6.13 g, 42 mmol), and cesium carbonate (13.7 g, 42 mmol) were dissolved in acetonitrile (80 mL). The system was stirred at 70°C for 1 hour.

After the disappearance of the starting material as monitored by LCMS, the reaction mixture was added to ethyl acetate (50 mL) and filtered. The filtrate was concentrated under reduced pressure. The resulting mixture was purified by silica gel column chromatography to afford 6.2 g of 4-((1H-pyrazol-1-yl)methyl)-2-fluoro-6-methoxybenzonitrile.

LCMS (ESI) m/z: 232.2 [M+H]⁺.

¹H NMR (400 MHz, DMSO-*d*₆) δ 7.90 (d, *J =* 2.2 Hz, 1H), 7.53 (d, *J =* 1.4 Hz, 1H), 6.99 (s, 1H), 6.69 (d, *J =* 9.6 Hz, 1H), 6.33 (t, *J =* 2.0 Hz, 1H), 5.44 (s, 2H), 3.91 (s, 3H).

Step E: Under nitrogen protection, N-hydroxyacetamide (6.8 g, 90.9 mmol) and potassium tert-butoxide (10.2 g, 90.9 mmol) were dissolved in N,N-dimethylformamide (70 mL). The system was stirred at room temperature for 1 hour. Then, 4-((1H-pyrazol-1-yl)methyl)-2-fluoro-6-methoxybenzonitrile (7 g, 30.3 mmol) was added, and the system was stirred at 60°C for 4 hours.

After the disappearance of the starting material as monitored by LCMS, the reaction mixture was quenched by adding water (100 mL). Dichloromethane (100 mL) was added for extraction. The organic phase was washed with saturated brine (100 mL × 3), dried over anhydrous magnesium sulfate, filtered, and the filtrate was concentrated under reduced pressure. The resulting mixture was purified by silica gel column chromatography to afford 4 g of 6-((1H-pyrazol-1-yl)methyl)-4-methoxybenzo[d]isoxazol-3-amine.

LCMS (ESI) m/z: 245.3 [M+H]⁺.

¹H NMR (400 MHz, DMSO-*d*₆) δ 7.88 (d, *J* = 2.0 Hz, 1H), 7.50 (d, *J =* 1.4 Hz, 1H), 6.69 (s, 1H), 6.63 (s, 1H), 6.30 (t, *J =* 2.0 Hz, 1H), 5.95 (s, 2H), 5.41 (s, 2H), 3.86 (s, 3H).

### Intermediate INT-2: 7-Methoxy-3,4-dihydro-2H-benzo[b][1,4]dioxepine-6-sulfonyl chloride

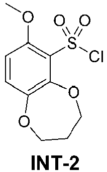

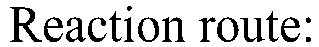

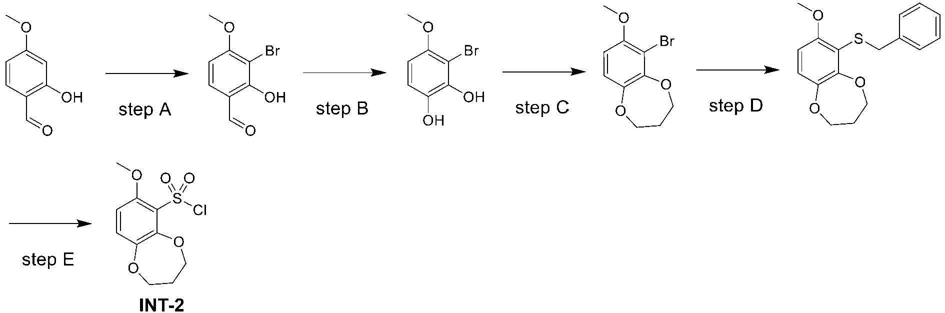

### Operating steps:

**Step A:** Under nitrogen protection, 2-hydroxy-4-methoxybenzaldehyde (15 g, 98.7 mmol) and aluminum trichloride (13.17 g, 98.7 mmol) were dissolved in dichloromethane (150 mL). After cooling to -20°C, bromine (15.8 g, 98.7 mmol) was added dropwise. The system was stirred at room temperature for 16 hours.

After the disappearance of the starting material as monitored by LCMS, the reaction mixture was quenched by adding 1M hydrochloric acid (200 mL). Dichloromethane (150 mL × 3) was added for extraction. The combined organic phases were washed with saturated sodium sulfite solution (100 mL), water (100 mL), and saturated brine (150 mL). The organic phase was dried over anhydrous magnesium sulfate, filtered, and the filtrate was concentrated under reduced pressure. The resulting mixture was purified by silica gel column chromatography to afford 9.5 g of 3-bromo-2-hydroxy-4-methoxybenzaldehyde.

MS (ESI) m/z: 231.0, 233.0 [M+H]⁺.

¹H NMR (400 MHz, DMSO-*d*₆): δ 11.69 (s, 1H), 9.88 (s, 1H), 7.82 (d, *J =* 8.8 Hz, 1H), 6.91 (d, *J =* 8.8 Hz, 1H), 3.97 (s, 3H).

**Step B:** Under nitrogen protection, 3-bromo-2-hydroxy-4-methoxybenzaldehyde (9.5 g, 41.09 mmol) was dissolved in tetrahydrofuran (100 mL). A sodium hydroxide solution (32 mL) and hydrogen peroxide (5.59 g, 49.18 mmol) were separately added to the reaction mixture at 0°C. The system was stirred at 0°C for 40 minutes.

After the disappearance of the starting material as monitored by LCMS, the reaction mixture was quenched by adding saturated sodium sulfite solution (200 mL). Dichloromethane (300 mL) was added for dilution. The mixture was washed with 1M hydrochloric acid solution (100 mL), water (100 mL), and saturated brine (100 mL × 3). The organic phase was dried over anhydrous magnesium sulfate, filtered, and the filtrate was concentrated under reduced pressure. The resulting mixture was purified by silica gel column chromatography to afford 9 g of 3-bromo-4-methoxybenzene-1,2-diol.

MS (ESI) m/z: 219.0, 220.9 [M+H]⁺.

¹H NMR (400 MHz, DMSO-*d*₆): δ 9.22 (s, 1H), 9.12 (s, 1H), 6.70 (d, *J =* 8.8 Hz, 1H), 6.37 (d, *J =* 8.8 Hz, 1H), 3.70 (s, 3H).

Step C: Under nitrogen protection, 3-bromo-4-methoxybenzene-1,2-diol (9 g, 41.1 mmol), 1,3-dibromopropane (12.45 g, 61.5 mmol), potassium carbonate (11.4 g, 82.2 mmol), and potassium fluoride (1.19 g, 20.4 mmol) were dissolved in N,N-dimethylformamide (90 mL). The system was stirred at 135°C for 2 hours.

After the disappearance of the starting material as monitored by LCMS, the reaction mixture was added to saturated ammonium chloride solution (500 mL). Ethyl acetate (300 mL) was added for dilution. The mixture was washed with water (100 mL) and saturated brine (100 mL). The organic phase was dried over anhydrous sodium sulfate, filtered, and the filtrate was concentrated under reduced pressure. The resulting mixture was purified by silica gel column chromatography to afford 6.7 g of 6-bromo-7-methoxy-3,4-dihydro-2H-benzo[b][1,4]dioxepine.

MS (ESI) m/z: 259.0, 261.0 [M+H]⁺.

¹H NMR (400 MHz, DMSO-d₆): δ 6.98 (d, *J =* 9.0 Hz, 1H), 6.70 (d, *J =* 9.0 Hz, 1H), 4.18 - 4.02 (m, 4H), 3.78 (s, 3H), 2.14 - 2.04 (m, 2H).

**Step D:** Under nitrogen protection, 6-bromo-7-methoxy-3,4-dihydro-2H-benzo[b][1,4] dioxepine (6.7 g, 25.8 mmol) was dissolved in tetrahydrofuran (50 mL). The solution was cooled to -78°C, and tert-butyllithium (17.74 mL, 28.38 mmol, 1.6M) was added dropwise. The mixture was stirred at this temperature for 30 minutes. A solution of 1,2-dibenzyldisulfane (7.64 g, 30.82 mmol) in tetrahydrofuran (15 mL) was added dropwise at -78°C. The mixture was stirred at the same temperature for 30 minutes and then warmed to room temperature and stirred for 1 hour.

After the disappearance of the starting material as monitored by LCMS, the reaction mixture was poured into a mixture of ethyl acetate (150 mL) and saturated ammonium chloride (150 mL). The mixture was extracted, and the organic phase was washed with saturated brine (100 mL), dried over anhydrous sodium sulfate, filtered, and the filtrate was concentrated under reduced pressure. The resulting mixture was purified by silica gel column chromatography to afford 5.5 g of 6-(benzylthio)-7-methoxy-3,4-dihydro-2H-benzo[b][1,4]dioxepine.

MS (ESI) m/z: 303.1 [M+H]⁺.

¹H NMR (400 MHz, DMSO-*d*₆): δ 7.25 - 7.11 (m, 5H), 6.90 (d, *J =* 8.8 Hz, 1H), 6.61 (d, *J* = 9.0 Hz, 1H), 3.99 (s, 2H), 3.96 - 3.79 (m, 4H), 3.75 (s, 3H), 2.03 - 1.92 (m, 2H).

**Step E:** Under nitrogen protection, 6-(benzylthio)-7-methoxy-3,4-dihydro-2H-benzo[b][1,4] dioxepine (1.7 g, 5.63 mmol) and acetic acid (2.7 g, 45.03 mmol) were dissolved in water (5.6 mL) and acetonitrile (61.9 mL). The solution was cooled to 0°C, and 1,3-dichloro-5,5-dimethylhydantoin (2.2 g, 11.26 mmol) was added. The system was stirred at 0°C for 1 hour.

After the disappearance of the starting material as monitored by LCMS, the reaction mixture was diluted with ethyl acetate (100 mL). The mixture was washed with water (100 mL × 3) and saturated brine (100 mL). The organic phase was dried over anhydrous magnesium sulfate, filtered, and the filtrate was concentrated under reduced pressure. The resulting mixture was purified by silica gel column chromatography to afford 1.49 g of 7-methoxy-3,4-dihydro-2H-benzo[b][1,4]dioxepine-6-sulfonyl chloride.

¹H NMR (400 MHz, DMSO-*d*₆): δ 6.93 (d, *J =* 8.8 Hz, 1H), 6.60 (d, *J =* 8.8 Hz, 1H), 4.00 - 3.86 (m, 4H), 3.66 (s, 3H), 2.05 - 1.94 (m, 2H).

### Intermediates INT-3 and INT-4:

### 6-((1H-Pyrazol-1-yl)methyl)-4-methoxy-5-methylbenzo[d]isoxazol-3-amine

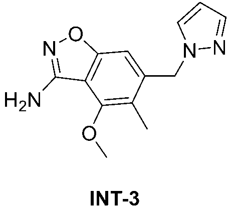

### 6-((1H-Pyrazol-1-yl)methyl)-5-ethyl-4-methoxybenzo [d] isoxazol-3-amine

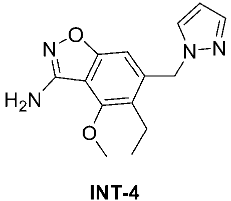

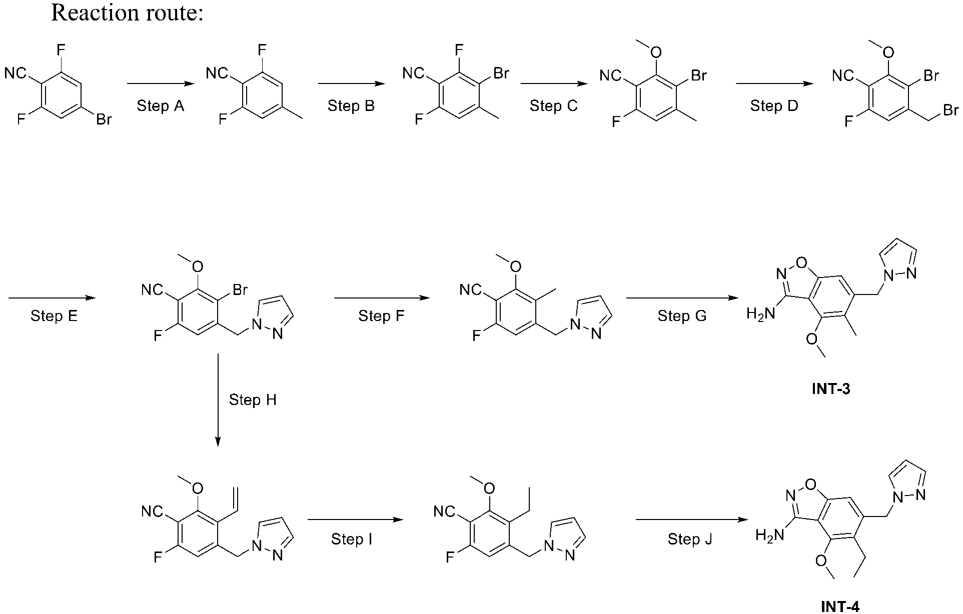

### Operating steps:

**Step A:** To a solution of 4-bromo-2,6-difluorobenzonitrile (20 g, 91.74 mmol) and methylboronic acid (16.47 g, 275.22 mmol) in dioxane (360 mL) and water (40 mL) were added tetrakis(triphenylphosphine)palladium (0.80 g, 0.69 mmol) and potassium carbonate (25.36 g, 183.48 mmol). The mixture was degassed with nitrogen three times and then stirred at 100°C for 12 hours. TLC showed the disappearance of the starting material and the formation of a new spot. The solvent was removed under reduced pressure, and the residue was purified by column chromatography to afford 8.5 g of 2,6-difluoro-4-methylbenzonitrile.

¹H NMR (400 MHz, Chloroform-d) δ 6.83 - 6.74 (m, 2H), 2.37 (s, 3H).

**Step B:** At 0°C, N-bromosuccinimide (8.31 g, 46.70 mmol) was added to a solution of 2,6-difluoro-4-methylbenzonitrile (6.5 g, 42.45 mmol) in sulfuric acid (32.5 mL). The mixture was then stirred at room temperature for 12 hours. TLC showed the disappearance of the starting material and the formation of a new spot. The reaction mixture was poured into 150 mL of ice water and extracted with ethyl acetate (200 mL × 2). The combined organic phases were washed with saturated brine, dried over sodium sulfate, filtered, concentrated, and purified by column chromatography to afford 8.4 g of 3-bromo-2,6-difluoro-4-methylbenzonitrile.

¹H NMR (400 MHz, Chloroform-d) δ 6.93 (dt, *J* = 9.0, 1.1 Hz, 1H), 2.45 (s, 3H).

**Step C:** At 0°C, sodium methoxide (651.91 mg, 12.07 mmol) was added to a solution of 3-bromo-2,6-difluoro-4-methylbenzonitrile (2 g, 8.62 mmol) in methanol (15 mL). The reaction was continued for 3 hours. TLC showed the disappearance of the starting material and the formation of a new spot. The solvent was removed under reduced pressure. 20 mL of water was added to the residue, and the mixture was extracted with 20 mL of ethyl acetate. The organic phase was washed with saturated brine, filtered, concentrated, and purified by column chromatography to afford 1.4 g of 3-bromo-6-fluoro-2-methoxy-4-methylbenzonitrile.

¹H NMR (400 MHz, Chloroform-d) δ 6.88 - 6.78 (m, 1H), 4.02 (s, 3H), 2.41 (s, 3H).

**Step D:** To a solution of 3-bromo-6-fluoro-2-methoxy-4-methylbenzonitrile (1.38 g, 5.65 mmol) and 2,2'-azobis(2-methylpropionitrile) (0.093 g, 0.57 mmol) in carbon tetrachloride (25 mL) was added N-bromosuccinimide (1.01 g, 5.65 mmol). The mixture was degassed with nitrogen three times and then stirred at 80°C for 3 hours. TLC showed the formation of a new spot. The solvent was removed under reduced pressure, and the residue was purified by column chromatography to afford 0.88 g of 3-bromo-4-(bromomethyl)-6-fluoro-2-methoxybenzonitrile.

¹H NMR (400 MHz, Chloroform-d) δ 7.06 (d, *J =* 8.7 Hz, 1H), 4.49 (s, 2H), 4.06 (s, 3H).

**Step E:** To a solution of 3-bromo-4-(bromomethyl)-6-fluoro-2-methoxybenzonitrile (610 mg, 1.89 mmol) and 1H-pyrazole (141.54 mg, 2.08 mmol) in acetonitrile (15 mL) was added cesium carbonate (677.38 mg, 2.08 mmol). The mixture was stirred at 80°C for 1.5 hours. After LCMS indicated completion of the reaction, 20 mL of water was added. The mixture was extracted with ethyl acetate (30 mL × 2). The combined organic phases were washed with saturated brine (30 mL × 2), dried over sodium sulfate, filtered, concentrated, and purified by column chromatography to afford 390 mg of 3-bromo-6-fluoro-2-methoxy-4-[(1H-pyrazol-1-yl) methyl]benzonitrile.

MS (ESI) m/z: 310.0 [M+H]⁺.

**Step F:** To a solution of 3-bromo-6-fluoro-2-methoxy-4-[(1H-pyrazol-1-yl)methyl] benzonitrile (130 mg, 0.42 mmol), methylboronic acid (50.28 mg, 0.84 mmol), and potassium carbonate (174.14 mg, 1.26 mmol) in dioxane (4 mL) and water (1 mL) was added [1,1'-bis(diphenylphosphino)ferrocene]dichloropalladium(II) (30.73 mg, 0.042 mmol). The mixture was degassed with nitrogen three times and stirred at 90°C for 3 hours. LCMS showed remaining starting material and the formation of the target peak. The solvent was removed under reduced pressure, and the residue was purified by column chromatography to afford 37 mg of 6-fluoro-2-methoxy-3-methyl-4-[(1H-pyrazol-1-yl)methyl]benzonitrile.

MS (ESI) m/z: 246.1 [M+H]⁺.

**Step G:** To a solution of 6-fluoro-2-methoxy-3-methyl-4-[(1H-pyrazol-1-yl)methyl] benzonitrile (37 mg, 0.15 mmol) and N-hydroxyacetamide (33.78 mg, 0.45 mmol) in N,N-dimethylformamide (1.5 mL) and water (0.22 mL) was added potassium carbonate (124.39 mg, 0.90 mmol). The mixture was stirred at 60°C for 12 hours. LCMS showed the disappearance of the starting material and the formation of the target peak. The mixture was concentrated under reduced pressure. The residue was diluted with water (10 mL) and extracted with ethyl acetate (10 mL × 2). The combined organic phases were washed with saturated brine (10 mL), dried over sodium sulfate, filtered, concentrated, and purified by preparative thin-layer chromatography to afford 30 mg of 6-((1H-pyrazol-1-yl)methyl)-4-methoxy-5-methylbenzo[d] isoxazol-3-amine.

MS (ESI) m/z: 259.1 [M+H]⁺.

**Step H:** To a solution of 3-bromo-6-fluoro-2-methoxy-4-[(1H-pyrazol-1-yl)methyl] benzonitrile (100 mg, 0.32 mmol), ethylboronic acid (4.73 mg, 0.064 mmol), and potassium carbonate (132.68 mg, 0.96 mmol) in dioxane (4 mL) and water (1 mL) was added [1,1'-bis(diphenylphosphino)ferrocene]dichloropalladium(II) (23.41 mg, 0.032 mmol). The mixture was degassed with nitrogen three times and stirred at 90°C for 3 hours. LCMS showed the disappearance of the starting material and the formation of the target peak. The solvent was removed under reduced pressure, and the residue was purified by column chromatography to afford 65 mg of 3-ethenyl-6-fluoro-2-methoxy-4-[(1H-pyrazol-1-yl)methyl]benzonitrile.

MS (ESI) m/z: 258.1 [M+H]⁺.

**Step I:** Under a nitrogen atmosphere, wet palladium on carbon (6.5 mg, 0.0061 mmol) was added to a solution of 3-ethenyl-6-fluoro-2-methoxy-4-[(1H-pyrazol-1-yl)methyl]benzonitrile (65 mg, 0.25 mmol) in methanol (5 mL). The atmosphere was replaced with hydrogen three times, and the mixture was stirred under a hydrogen atmosphere (15 psi) for 0.5 hours. LCMS showed the disappearance of the starting material and the formation of the target peak. The mixture was filtered, and the filtrate was concentrated to afford 70 mg of 3-ethyl-6-fluoro-2-methoxy-4-[(1H-pyrazol-l-yl)methyl]benzonitrile.

MS (ESI) m/z: 260.2 [M+H]⁺.

**Step J:** To a solution of 3-ethyl-6-fluoro-2-methoxy-4-[(1H-pyrazol-1-yl)methyl] benzonitrile (70 mg, 0.27 mmol) and N-hydroxyacetamide (60.81 mg, 0.81 mmol) in N,N-dimethylformamide (3 mL) and water (0.44 mL) was added potassium carbonate (186.58 mg, 1.35 mmol). The mixture was stirred at 60°C for 12 hours. LCMS showed the disappearance of the starting material and the formation of the target peak. The mixture was concentrated under reduced pressure. The residue was diluted with water (10 mL) and extracted with ethyl acetate (10 mL × 2). The combined organic phases were washed with saturated brine (10 mL), dried over sodium sulfate, filtered, concentrated, and purified by preparative thin-layer chromatography to afford 50 mg of 6-((1H-pyrazol-1-yl)methyl)-5-ethyl-4-methoxybenzo[d] isoxazol-3-amine.

MS (ESI) m/z: 273.1 [M+H]⁺.

### Intermediate INT-5: 6-((1H-Pyrazol-1-yl)methyl)-5-fluoro-4-methoxybenzo[d]isoxazol -3-amine

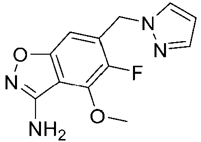

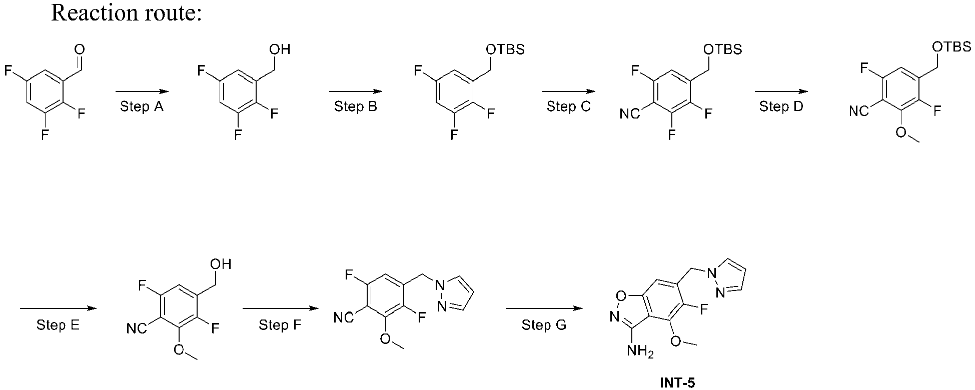

### Operating steps:

Step A: At 0°C, sodium borohydride (1.30 g, 34.35 mmol) was added to a solution of 2,3,5-trifluorobenzaldehyde (5 g, 31.23 mmol) in tetrahydrofuran (100 mL). The reaction was stirred at 0°C for 2 hours. TLC showed the disappearance of the starting material and the formation of a new spot. The reaction was quenched by slow addition of saturated ammonium chloride solution (5 mL). The mixture was filtered and concentrated to afford 5 g of (2,3,5-trifluorophenyl)methanol.

¹H NMR (400 MHz, Chloroform-d) δ 6.93 (ddt, *J =* 10.8, 5.1, 2.3 Hz, 1H), 6.83 - 6.74 (m, 1H), 4.81 - 4.62 (m, 2H).

Step B: To a solution of (2,3,5-trifluorophenyl)methanol (5 g, 30.84 mmol), triethylamine (4.68 g, 46.26 mmol), and 4-dimethylaminopyridine (0.38 g, 3.08 mmol) in dichloromethane (80 mL) was added tert-butyldimethylchlorosilane (6.04 g, 40.09 mmol). The mixture was stirred at room temperature for 2 hours. TLC showed the disappearance of the starting material and the formation of a new spot. Water (50 mL) was added to the reaction mixture, and the mixture was extracted with dichloromethane (50 mL). The combined organic phases were washed with saturated brine, dried over sodium sulfate, filtered, concentrated, and purified by column chromatography to afford 7.9 g of tert-butyldimethyl[(2,3,5-trifluorobenzyl)oxy]silane.

¹H NMR (400 MHz, Chloroform-d) δ 6.92 - 6.83 (m, 1H), 6.67 (dddd, *J =* 10.1, 8.1, 6.1, 3.2 Hz, 1H), 4.78 - 4.50 (m, 2H), 0.82 (s, 9H), -0.00 (s, 6H).

**Step C:** At -70°C, diisopropylamine (1.40 g, 13.03 mmol) was added dropwise to a solution of tert-butyldimethyl[(2,3,5-trifluorobenzyl)oxy]silane (3 g, 10.86 mmol) in tetrahydrofuran (30 mL). The mixture was stirred for 2 hours. Then, a solution of 4-methylbenzenesulfonyl cyanide (2164.73 mg, 11.95 mmol) in tetrahydrofuran (3 mL) was added dropwise, and the mixture was stirred for 1 hour. TLC showed the disappearance of the starting material and the formation of a new spot. Saturated ammonium chloride solution (5 mL) and water (20 mL) were added to the reaction mixture. The mixture was extracted with ethyl acetate (30 mL × 2). The combined organic phases were washed with saturated brine, dried over sodium sulfate, filtered, concentrated, and purified by column chromatography to afford 2 g of 4-{[(tert-butyldimethylsilyl)oxy]methyl}-2,3,6-trifluorobenzonitrile.

¹H NMR (400 MHz, Chloroform-d) δ 7.07 (dddd, *J =* 8.8, 4.7, 2.1, 1.0 Hz, 1H), 4.69 (q, *J* = 1.1 Hz, 2H), 0.81 (s, 9H), -0.00 (s, 6H).

**Step D:** At 0°C, sodium methoxide (286.85 mg, 5.31 mmol) was added to a solution of 4-{[(tert-butyldimethylsilyl)oxy]methyl}-2,3,6-trifluorobenzonitrile (2 g, 6.64 mmol) in tetrahydrofuran (20 mL). The mixture was stirred at 0°C for 1 hour. TLC showed the disappearance of the starting material and the formation of a new spot. At 0°C, dilute hydrochloric acid was added dropwise to adjust the pH to 6-7. Water (20 mL) was added, and the mixture was extracted with ethyl acetate (30 mL × 2). The combined organic phases were washed with saturated brine, dried over sodium sulfate, filtered, concentrated, and purified by column chromatography to afford 0.8 g of 4-{[(tert-butyldimethylsilyl)oxy]methyl}-3,6-difluoro-2-methoxybenzonitrile.

¹H NMR (400 MHz, Chloroform-d) δ 6.96 (dd, *J =* 8.9, 4.8 Hz, 1H), 4.71 (t, *J =* 1.2 Hz, 2H), 4.07 (d, *J =* 3.0 Hz, 3H), 0.88 (s, 9H), 0.06 (s, 6H).

**Step E:** Tetrabutylammonium fluoride (502.00 mg, 1.92 mmol) was added to a solution of 4-{[(tert-butyldimethylsilyl)oxy]methyl}-3,6-difluoro-2-methoxybenzonitrile (300 mg, 0.96 mmol) in tetrahydrofuran (10 mL). The mixture was stirred at room temperature for 1 hour. TLC showed the disappearance of the starting material and the formation of a new spot. 20 mL of water was added to the reaction mixture, and the mixture was extracted with ethyl acetate (30 mL × 2). The combined organic phases were washed with saturated brine, dried over sodium sulfate, filtered, and concentrated to afford 0.3 g of 3,6-difluoro-4-(hydroxymethyl)-2-methoxybenzonitrile.

¹H NMR (400 MHz, Chloroform-d) δ 7.02 - 6.95 (m, 1H), 4.75 (dd, *J =* 1.6, 0.8 Hz, 2H), 4.09 (d, *J =* 3.1 Hz, 3H).

**Step F:** To a solution of 3,6-difluoro-4-(hydroxymethyl)-2-methoxybenzonitrile (300 mg, 1.51 mmol) and 1-(methylsulfonyl)-1H-pyrazole (264.86 mg, 1.81 mmol) in acetonitrile (20 mL) was added cesium carbonate (590.39 mg, 1.81 mmol). The mixture was stirred at 70°C for 1 hour. TLC showed the disappearance of the starting material and the formation of a new spot. 20 mL of water was added to the reaction mixture, and the mixture was extracted with ethyl acetate (30 mL × 2). The combined organic phases were washed with saturated brine, dried over sodium sulfate, filtered, concentrated, and purified by column chromatography to afford 0.28 g of 3,6-difluoro-2-methoxy-4-[(1H-pyrazol-1-yl)methyl]benzonitrile.

MS (ESI) m/z: 250.1 [M+H]⁺.

**Step G:** To a solution of 3,6-difluoro-2-methoxy-4-[(1H-pyrazol-1-yl)methyl]benzonitrile (250 mg, 1.00 mmol) and N-hydroxyacetamide (225.21 mg, 3.00 mmol) in N,N-dimethylformamide (4 mL) and water (0.57 mL) was added potassium carbonate (691.05 mg, 5.00 mmol). The mixture was stirred at 60°C for 5 hours. The solvent was removed under reduced pressure. Water (20 mL) was added to the residue, and the mixture was extracted with ethyl acetate (30 mL × 2). The combined organic phases were washed with saturated brine, dried over sodium sulfate, filtered, and concentrated to afford 0.2 g of 6-((1H-pyrazol-1-yl)methyl)-5-fluoro-4-methoxybenzo[d]isoxazol-3-amine.

MS (ESI) m/z: 263.1 [M+H]⁺.

### Intermediate INT-6: 4-((1H-Pyrazol-1-yl)methyl)-2,3-dihydrobenzofuro[7,6-d]isoxazol -8-amine (see Patent WO2023016484)

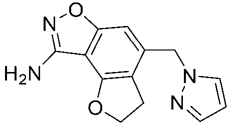

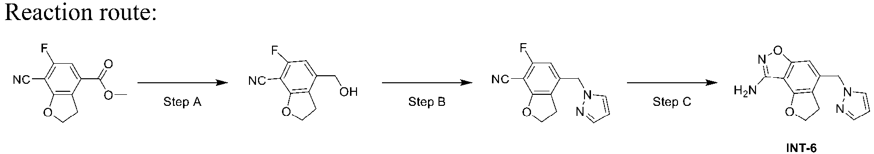

### Operating steps:

**Step A:** Under nitrogen protection, methyl 7-cyano-6-fluoro-2,3-dihydrobenzofuran-4-carboxylate (200 mg, 0.905 mmol) was dissolved in methanol (2 mL). Sodium borohydride (138 mg, 3.62 mmol) was added under ice-bath conditions. The temperature was raised to 50°C and the reaction was stirred for 18 hours. The reaction was monitored by LCMS. After completion, the reaction was quenched with ice water and extracted with ethyl acetate (10 mL) and water (10 mL). The organic phase was dried over anhydrous sodium sulfate and concentrated under reduced pressure. The residue was purified by column chromatography to afford 98 mg of the target product, 6-fluoro-4-(hydroxymethyl)-2,3-dihydrobenzofuran-7-carbonitrile.

MS (ESI) m/z: 194.2 [M+H]⁺.

**Step B:** 6-Fluoro-4-(hydroxymethyl)-2,3-dihydrobenzofuran-7-carbonitrile (95 mg, 0.492 mmol), 1-(methylsulfonyl)-1H-pyrazole (86 mg, 0.591 mmol), and cesium carbonate (240 mg, 0.738 mmol) were dissolved in acetonitrile (1 mL). The mixture was stirred at 80°C for 2 hours. The reaction was monitored by LCMS. After complete consumption of the starting material, the reaction mixture was extracted with ethyl acetate (10 mL) and water (10 mL). The organic phase was dried over anhydrous sodium sulfate and purified by column chromatography to afford 88 mg of 4-((1H-pyrazol-1-yl)methyl)-6-fluoro-2,3-dihydrobenzofuran-7-carbonitrile.

MS (ESI) m/z: 244.1 [M+H]⁺.

**Step C:** To a solution of 4-((1H-pyrazol-1-yl)methyl)-6-fluoro-2,3-dihydrobenzofuran-7-carbonitrile (88 mg, 0.362 mmol) in N,N-dimethylformamide (1 mL) were added acetohydroxamic acid (82 mg, 1.09 mmol) and potassium carbonate (250 mg, 1.81 mmol). The mixture was stirred at 60°C for 3 hours. TLC showed complete conversion of the starting material to the product. The reaction mixture was concentrated under reduced pressure. The residue was extracted with ethyl acetate (10 mL) and water (10 mL). The organic phase was washed with saturated brine, dried over anhydrous sodium sulfate, and concentrated under vacuum. The residue was purified by column chromatography to afford 44 mg of 4-((1H-pyrazol-1-yl)methyl)-2,3-dihydrobenzofuro[7,6-d]isoxazol-8-amine.

MS (ESI) m/z: 257.1 [M+H]⁺.

### Intermediate INT-7: 6-((1H-Pyrazol-1-yl)methyl)-5-methoxy-4-methylbenzo[d] isoxazol-3-amine

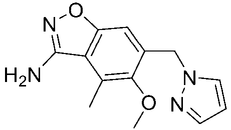

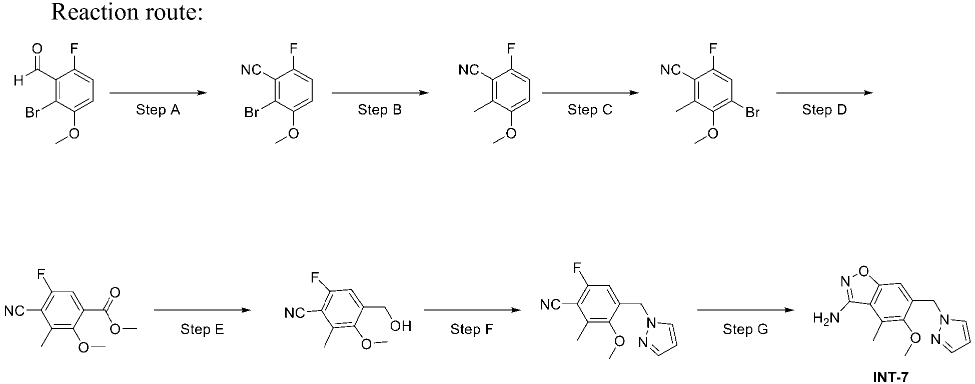

### Operating steps:

**Step A:** Under ice-bath conditions, sulfamic acid (3.40 g, 30.05 mmol) was added to a solution of 2-bromo-6-fluoro-3-methoxybenzaldehyde (4 g, 17.17 mmol) in water (20 mL). The reaction was stirred at 50°C for 12 hours. After LCMS indicated complete consumption of the starting material, the reaction mixture was filtered directly and washed with water to afford 3.8 g of 2-bromo-6-fluoro-3-methoxybenzonitrile.

¹H NMR (400 MHz, DMSO-*d*₆) δ 7.64 - 7.48 (m, 2H), 3.91 (s, 3H).

**Step B:** 2-Bromo-6-fluoro-3-methoxybenzonitrile (3.8 g, 16.59 mmol), methylboronic acid (3.13 g, 49.78 mmol), [1,1'-bis(diphenylphosphino)ferrocene]dichloropalladium (0.64 g, 0.87 mmol), and potassium carbonate (4.58 g, 33.2 mmol) were dissolved in 1,4-dioxane (50 mL) and water (5 mL). The mixture was refluxed at 110°C overnight. The reaction was monitored by TLC. After complete consumption of the starting material, the reaction mixture was extracted with ethyl acetate (150 mL) and water (150 mL). The combined organic phases were washed with saturated brine (50 mL × 3), dried over anhydrous sodium sulfate, filtered, concentrated under reduced pressure, and purified by column chromatography to afford 2.47 g of 6-fluoro-3-methoxy-2-methylbenzonitrile.

¹H NMR (400 MHz, DMSO-*d*₆) δ 7.39 - 7.29 (m, 2H), 3.84 (s, 3H), 2.33 (s, 3H).

**Step C:** N-Bromosuccinimide (388.00 mg, 2.18 mmol) was added to a solution of 6-fluoro-3-methoxy-2-methylbenzonitrile (330 mg, 2.00 mmol) in dichloromethane (5 mL). The reaction was stirred at room temperature. The reaction was monitored by TLC. After complete consumption of the starting material, the reaction mixture was poured into ice water to quench the reaction. The mixture was extracted with ethyl acetate (10 mL) and water (10 mL). The combined organic phases were washed with saturated brine (10 mL × 3), dried over anhydrous sodium sulfate, filtered, concentrated under reduced pressure, and purified by column chromatography to afford 426 mg of 4-bromo-6-fluoro-3-methoxy-2-methylbenzonitrile.

¹H NMR (400 MHz, DMSO-*d*₆) δ 7.84 (d, *J =* 8.6 Hz, 1H), 3.76 (s, 3H), 2.46 (s, 3H).

**Step D:** Under a carbon monoxide atmosphere at standard atmospheric pressure, tetrakis(triphenylphosphine)palladium (663 mg, 0.574 mmol) and triethylamine (2.3 g, 22.96 mmol) were added to a solution of 4-bromo-6-fluoro-3-methoxy-2-methylbenzonitrile (2.79 g, 11.48 mmol) in methanol (20 mL). The reaction was stirred at 65°C for 12 hours. The reaction was monitored by LCMS. After completion, the reaction mixture was extracted with ethyl acetate (60 mL) and water (60 mL). The organic phase was dried over anhydrous sodium sulfate, concentrated under reduced pressure, and purified by column chromatography (petroleum ether/ethyl acetate = 3/1) to afford 2.1 g of methyl 4-cyano-5-fluoro-2-methoxy-3-methylbenzoate.

¹H NMR (400 MHz, Chloroform-d) δ 7.43 (d, *J =* 8.6 Hz, 1H), 3.95 (s, 3H), 3.83 (s, 3H), 2.53 (s, 3H).

**Step E:** Sodium borohydride (407.05 mg, 10.76 mmol) was added under ice-bath conditions to a solution of methyl 4-cyano-5-fluoro-2-methoxy-3-methylbenzoate (600 mg, 2.69 mmol) in methanol (10 mL). The temperature was raised to 50°C and the reaction was stirred for 18 hours. The reaction was monitored by LCMS. After completion, the reaction was quenched with ice water and extracted with ethyl acetate (10 mL) and water (10 mL). The organic phase was dried over anhydrous sodium sulfate, concentrated under reduced pressure, and purified by column chromatography to afford 420 mg of 6-fluoro-4-(hydroxymethyl)-3-methoxy-2-methylbenzonitrile.

MS (ESI) m/z: 196.1 [M+H]⁺.

**Step F:** 6-Fluoro-4-(hydroxymethyl)-3-methoxy-2-methylbenzonitrile (420 mg, 2.15 mmol), 1-(methylsulfonyl)-1H-pyrazole (0.38 g, 2.58 mmol), and cesium carbonate (1050.77 mg, 3.22 mmol) were dissolved in acetonitrile (10 mL). The mixture was stirred at 80°C for 2 hours. The reaction was monitored by LCMS. After complete consumption of the starting material, the reaction mixture was extracted with ethyl acetate (30 mL) and water (30 mL). The organic phase was dried over anhydrous sodium sulfate and purified by column chromatography to afford 570 mg of 4-((1H-pyrazol-1-yl)methyl)-6-fluoro-3-methoxy-2-methylbenzonitrile.

MS (ESI) m/z: 246.1 [M+H]⁺.

**Step G:** Acetohydroxamic acid (0.70 g, 9.28 mmol) and potassium carbonate (1.92 g, 13.92 mmol) were added to a solution of 4-((1H-pyrazol-1-yl)methyl)-6-fluoro-3-methoxy-2-methylbenzonitrile (570 mg, 2.32 mmol) in N,N-dimethylformamide (10 mL). The mixture was stirred at 60°C for 3 hours. TLC showed complete conversion of the starting material to the product. The reaction mixture was concentrated under reduced pressure. The residue was extracted with ethyl acetate (20 mL) and water (20 mL). The organic phase was dried over anhydrous sodium sulfate, concentrated under vacuum, and purified by column chromatography to afford 310 mg of 6-((1H-pyrazol-1-yl)methyl)-5-methoxy-4-methylbenzo[d]isoxazol-3-amine.

MS (ESI) m/z: 259.1 [M+H]⁺.

### Intermediate INT-8: 6-((1H-Pyrazol-1-yl)methyl-d2)-4-methoxybenzo[d]isoxazol-3-amine

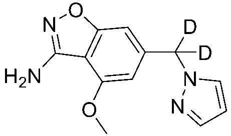

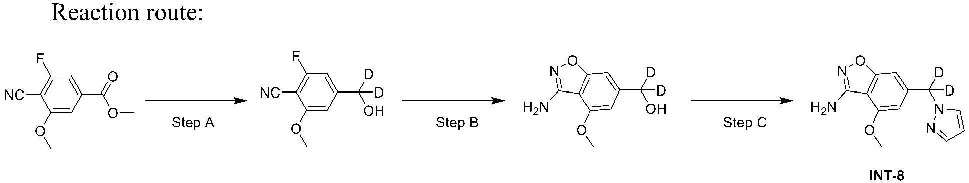

### Operating steps:

**Step A:** Under nitrogen protection, methyl 7-cyano-6-fluoro-2,3-dihydrobenzofuran-4-carboxylate (418 mg, 2 mmol) was dissolved in deuterated methanol (2 mL). Sodium borodeuteride (336 mg, 8 mmol) was added under ice-bath conditions. The temperature was raised to 50°C and the reaction was stirred for 18 hours. The reaction was monitored by LCMS. After completion, the reaction was quenched with deuterium oxide and extracted with ethyl acetate (10 mL) and deuterium oxide (10 mL). The organic phase was dried over anhydrous sodium sulfate and concentrated under reduced pressure. The residue was purified by column chromatography to afford 390 mg of 2-fluoro-4-(hydroxymethyl-d2)-6-methoxybenzonitrile.

MS (ESI) m/z: 184.1 [M+H]⁺.

**Step B:** Acetohydroxamic acid (480 mg, 6.32 mmol) and potassium carbonate (1.47 g, 10.6 mmol) were added to a solution of 2-fluoro-4-(hydroxymethyl-d2)-6-methoxybenzonitrile (390 mg, 2.12 mmol) in N,N-dimethylformamide (10 mL). The mixture was stirred at 60°C for 3 hours. TLC showed complete conversion of the starting material to the product. The reaction mixture was concentrated under reduced pressure. The residue was extracted with ethyl acetate (10 mL) and water (10 mL). The organic phase was washed with saturated brine, dried over anhydrous sodium sulfate, concentrated under vacuum, and purified by column chromatography to afford 198 mg of (3-amino-4-methoxybenzo[d]isoxazol-6-yl)methan-d2-ol.

MS (ESI) m/z: 197.1 [M+H]⁺.

**Step C:** (3-Amino-4-methoxybenzo[d]isoxazol-6-yl)methan-d2-ol (196 mg, 1 mmol), 1-(methylsulfonyl)-1H-pyrazole (175 mg, 1.2 mmol), and cesium carbonate (487 mg, 1.5 mmol) were dissolved in acetonitrile (1 mL). The mixture was stirred at 80°C for 2 hours. The reaction was monitored by LCMS. After complete consumption of the starting material, the reaction mixture was extracted with ethyl acetate (10 mL) and water (10 mL). The organic phase was dried over anhydrous sodium sulfate and purified by column chromatography to afford 200 mg of 6-((1H-pyrazol-1-yl)methyl-d2)-4-methoxybenzo[d]isoxazol-3-amine.

MS (ESI) m/z: 247.1 [M+H]⁺.

### Intermediate INT-9: 6-((Pyrazol-1-yl)methyl)-4-(trideuteromethoxy)-1,2-benzoxazol-3-amine

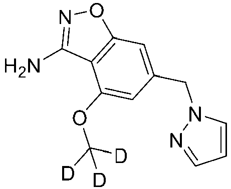

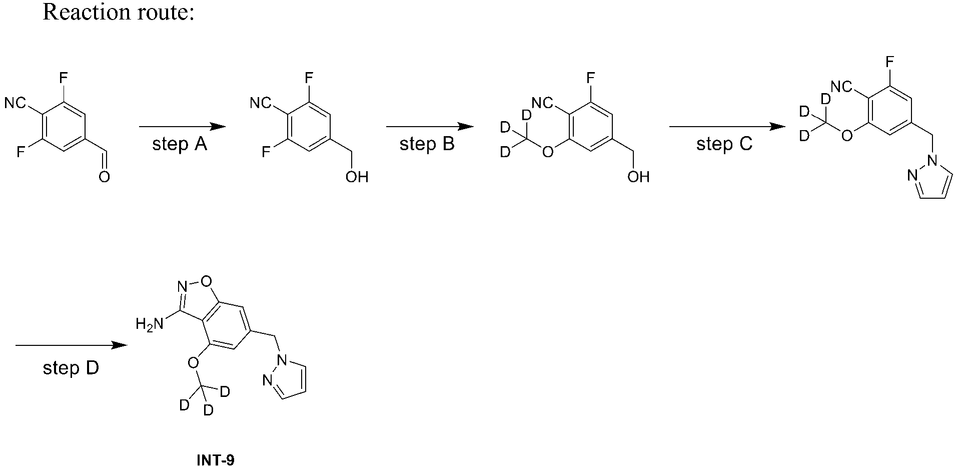

### Operating steps:

**Step A:** 2,6-Difluoro-4-formylbenzonitrile (5 g, 29.92 mmol) was dissolved in ethanol (50 mL) and cooled to 0°C. Sodium borohydride (2.3 g, 60.79 mmol) was added in portions slowly. The system was stirred at 0°C for 2 hours. After the disappearance of the starting material as monitored by LCMS, the reaction mixture was quenched with saturated ammonium chloride solution and extracted with ethyl acetate. The organic phase was washed with saturated brine, dried over anhydrous sodium sulfate, filtered, and concentrated under reduced pressure to afford 5.01 g of 2,6-difluoro-4-(hydroxymethyl)benzonitrile.

MS (ESI) m/z: 170.0 [M+H]⁺.

¹H NMR (400 MHz, DMSO-*d*₆) δ 4.60 (d, *J =* 5.8 Hz, 2H), 5.63 - 5.75 (m, 1H), 7.16 - 7.42 (m, 2H).

**Step B:** 2,6-Difluoro-4-(hydroxymethyl)benzonitrile (500 mg, 2.96 mmol) was dissolved in deuterated methanol (2.5 mL) and cooled to 0°C. Sodium hydride (177.36 mg, 4.43 mmol, 60% purity) was added in portions. The system was stirred at 20°C for 12 hours. After the disappearance of the starting material as monitored by LCMS, the reaction mixture was quenched with saturated ammonium chloride solution and extracted with ethyl acetate. The organic phase was washed with saturated brine, dried over anhydrous sodium sulfate, filtered, and concentrated under reduced pressure. The resulting mixture was purified by silica gel column chromatography to afford 303 mg of 2-fluoro-4-(hydroxymethyl)-6-(trideuteromethoxy)benzonitrile.

MS (ESI) m/z: 185.1 [M+H]⁺.

¹H NMR (400 MHz, DMSO-*d*₆) δ 4.51 - 4.60 (m, 2H), 5.55 - 5.61 (m, 1H), 6.94 - 7.01 (m, 1H), 7.04 (s, 1H).

**Step C:** 2-Fluoro-4-(hydroxymethyl)-6-(trideuteromethoxy)benzonitrile (300 mg, 1.60 mmol) was dissolved in acetonitrile (5 mL). Cesium carbonate (676.11 mg, 2.08 mmol) and 1-methylsulfonylpyrazole (256.65 mg, 1.76 mmol) were added. The system was stirred at 70°C for 1 hour. After the disappearance of the starting material as monitored by LCMS, the reaction mixture was diluted with water and extracted with ethyl acetate. The organic phase was dried over anhydrous sodium sulfate, filtered, and concentrated under reduced pressure. The resulting mixture was purified by silica gel column chromatography to afford 345 mg of 2-fluoro-4-(pyrazol-1-ylmethyl)-6-(trideuteromethoxy)benzonitrile.

MS (ESI) m/z: 235.1 [M+H]⁺.

¹H NMR (400 MHz, DMSO-*d*₆) δ 5.36 - 5.49 (m, 2H), 6.28 - 6.37 (m, 1H), 6.62 - 6.75 (m, 1H), 6.91 - 7.03 (m, 1H), 7.48 - 7.56 (m, 1H), 7.85 - 7.92 (m, 1H).

**Step D:** 2-Fluoro-4-(pyrazol-1-ylmethyl)-6-(trideuteromethoxy)benzonitrile (340 mg, 1.45 mmol), potassium carbonate (1.20 g, 8.71 mmol), and acetohydroxamic acid (326.87 mg, 4.35 mmol) were dissolved in N,N-dimethylformamide (6 mL) and water (0.9 mL). The mixture was stirred at 60°C for 12 hours. After the disappearance of the starting material as monitored by LCMS, the reaction mixture was concentrated under reduced pressure, extracted with ethyl acetate and water. The organic phase was washed with saturated brine, dried over anhydrous sodium sulfate, filtered, and concentrated under reduced pressure. The resulting mixture was purified by silica gel column chromatography to afford 256 mg of 6-((1H-pyrazol-1-yl)methyl)-4-(trideuteromethoxy)-1,2-benzoxazol-3-amine.

MS (ESI) m/z: 247.7 [M+H]⁺.

¹H NMR (400 MHz, DMSO-d₆) δ 5.35 - 5.45 (m, 2H), 5.86 - 5.98 (m, 2H), 6.30 (t, *J =* 1.9 Hz, 1H), 6.59 - 6.65 (m, 1H), 6.69 (s, 1H), 7.43 - 7.52 (m, 1H), 7.87 (d, *J= 2.0* Hz, 1H).

### Intermediate INT-10-P1: (1aR,7bS)-5-Methoxy-1,1a,2,7b-tetrahydrocyclopropa[c] chromene-4-sulfonyl chloride / (1aS,7bR)-5-Methoxy-1,1a,2,7b-tetrahydrocyclopropa[c] chromene-4-sulfonyl chloride

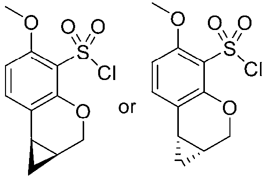

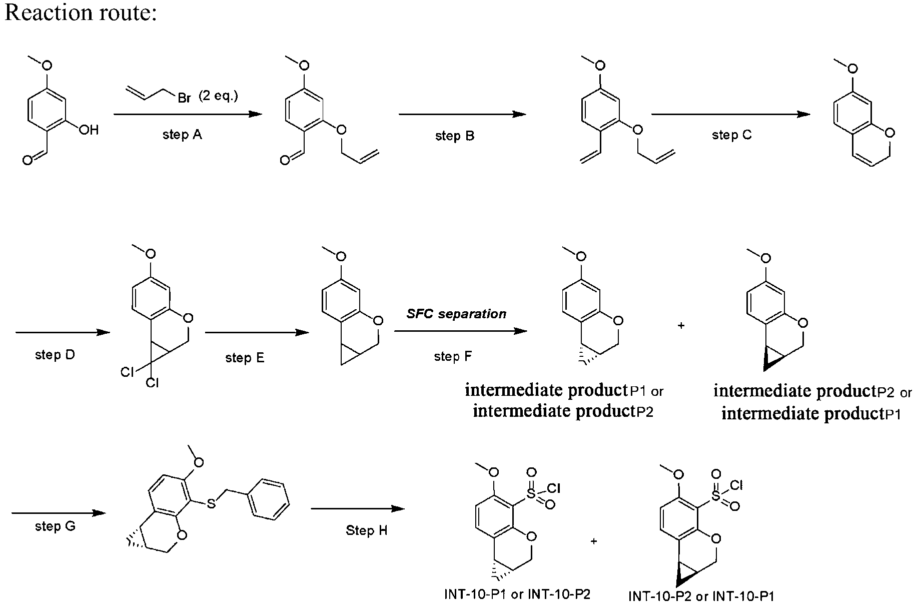

### Operating steps:

**Step A:** 2-Hydroxy-4-methoxybenzaldehyde (10.0 g, 66 mmol) and 3-bromoprop-1-ene (12.0 g, 99 mmol) were dissolved in acetone (100 mL). Anhydrous potassium carbonate (18.2 g, 132 mmol) was added. The reaction mixture was gradually heated to 70°C and stirred for 3 hours. After the disappearance of the starting material as monitored by LCMS, the mixture was cooled to room temperature, diluted with water (50 mL), and extracted with ethyl acetate (50 mL × 2). The combined organic phases were washed with water (20 mL) and saturated brine (20 mL), dried over anhydrous sodium sulfate, filtered, and concentrated under reduced pressure to afford the crude product, 2-(allyloxy)-4-methoxybenzaldehyde (13 g).

MS (ESI) m/z: 193.3 [M+H]⁺.

**Step B:** Under ice-bath conditions, sodium hydride (3.5 g, 88 mmol, 60% wt) was added to a solution of (bromomethyl)triphenylphosphonium bromide (31 g, 88 mmol) in anhydrous N,N-dimethylformamide (100 mL). The mixture was stirred for half an hour. A solution of 2-(allyloxy)-4-methoxybenzaldehyde (13 g, 67.7 mmol) in N,Ndimethylformamide (30 mL) was slowly added dropwise to the mixture. The reaction system was slowly warmed to room temperature and stirred for 2 hours. After the disappearance of the starting material as monitored by LCMS, the mixture was cooled to 0°C, and water was added dropwise to quench the reaction. The filtrate was extracted with ethyl acetate (50 mL × 3). The combined organic phases were washed with water (50 mL) and saturated brine (50 mL), dried over anhydrous sodium sulfate, filtered, and concentrated under reduced pressure. The crude product was purified by flash silica gel column chromatography to afford 2-(allyloxy)-4-methoxy-1-vinylbenzene (11 g).

MS (ESI) m/z: 191.2 [M+H]⁺.

**Step C:** 2-(Allyloxy)-4-methoxy-1-vinylbenzene (11 g, 57.9 mmol) was dissolved in dichloromethane (80 mL). (1,3-Bis(2,4,6-trimethylphenyl)-2-imidazolidinylidene)dichloro(o-isopropoxyphenylmethylene)ruthenium (1.8 g, 2.9 mmol) was added. The reaction system was stirred at room temperature for 1 hour. After the disappearance of the starting material as monitored by LCMS, the mixture was concentrated under reduced pressure. The crude product was purified by flash silica gel column chromatography to afford 7-methoxy-2H-chromene (8.2 g)^{.}

MS (ESI) m/z: 163.3 [M+H]⁺.

¹H NMR (500 MHz, CDCl₃): δ 6.87 (d, *J=* 8.0 Hz, 1H), 6.42 (dd, *J =* 8.0, 2.4 Hz, 1H), 6.40 - 6.35 (m, 2H), 5.65 - 5.60 (m, 1H), 4.79 (dd, *J =* 3.6, 2.0 Hz, 2H), 3.77 (s, 3H).

**Step D:** 7-Methoxy-2H-chromene (8.2 g, 50.6 mmol) and benzyltriethylammonium chloride (1.2 g, 5.1 mmol) were dissolved in chloroform (80 mL). A 33 wt% sodium hydroxide aqueous solution was slowly added dropwise at room temperature. The reaction mixture was stirred at room temperature for 2 hours. After the disappearance of the starting material as monitored by LCMS, the reaction mixture was poured into water (100 mL) and extracted with dichloromethane (50 mL × 3). The combined organic phases were washed with water (50 mL) and saturated brine (50 mL), dried over anhydrous sodium sulfate, filtered, and concentrated under reduced pressure. The crude product was purified by flash silica gel column chromatography to afford 1,1-dichloro-5-methoxy-1,1a,2,7b-tetrahydrocyclopropa[c]chromene (9.5 g).

MS (ESI) m/z: 245.1 [M+H]⁺.

**Step E:** At room temperature, metallic sodium (7.1 g, 310 mmol) was dissolved in anhydrous diethyl ether (70 mL). A solution of 1,1-dichloro-5-methoxy-1,1a,2,7b-tetrahydrocyclopropa[c]chromene (9.5 g, 38.8 mmol) in a mixture of anhydrous diethyl ether (30 mL) and anhydrous methanol (15 mL) was slowly added dropwise to the reaction mixture. Methanol (5 mL) was added every hour until the starting material disappeared. After the disappearance of the starting material as monitored by LCMS, anhydrous ethanol (20 mL) was added to quench the reaction. The mixture was diluted with water (50 mL) and extracted with ethyl acetate (50 mL × 3). The combined organic phases were washed with water (50 mL) and saturated brine (50 mL), dried over anhydrous sodium sulfate, filtered, and concentrated under reduced pressure. The crude product was purified by flash silica gel column chromatography to afford 5-methoxy-1,1a,2,7b-tetrahydrocyclopropa[c]chromene (5.5 g).

MS (ESI) m/z: 244.2 [M+H]⁺.

**Step F:** 5-Methoxy-1,1a,2,7b-tetrahydrocyclopropa[c]chromene (30 g, 170.25 mmol, 1 eq) was separated and purified by SFC to afford 12.4 g of intermediate product P1. (Retention time: 2.651 min, Chromatographic conditions: Column: DAICEL CHIRALCEL OJ (250 mm × 50 mm, 10 µm); Mobile phase: [CO₂-EtOH (0.1% NH₃·H₂O)]; B%: 20%, isocratic elution mode. Herein, [CO₂-EtOH (0.1% NH₃·H₂O)] means CO₂ is phase A, and EtOH containing 0.1 vol% NH₃·H₂O is phase B; B%: 20% means A% : B% = 80% : 20% (volume ratio)).

**Step G:** Under nitrogen protection, the intermediate product obtained from the separation above (3 g, 17.03 mmol) and N,N,N',N'-tetramethylethane-1,2-diamine (197.85 mg, 1.70 mmol) were dissolved in tetrahydrofuran (45 mL). The temperature was lowered to 0°C, and n-butyllithium (2.5 M, 10.22 mL) was added dropwise. The mixture was stirred at this temperature for 1 hour. A solution of 1,2-dibenzyldisulfane (6.29 g, 25.54 mmol) in tetrahydrofuran (45 mL) was added dropwise at 0°C. The temperature was raised to room temperature, and the mixture was stirred for 3 hours. After the disappearance of the starting material as monitored by LCMS, the reaction mixture was poured into a mixture of ethyl acetate and saturated ammonium chloride. The mixture was extracted, washed with saturated brine (100 mL), dried over anhydrous sodium sulfate, filtered, and concentrated under reduced pressure. The resulting mixture was purified by silica gel column chromatography to afford 3.3 g of the intermediate product.

MS (ESI) m/z: 299.3 [M+H]⁺.

¹H NMR (400 MHz, CHLOROFORM-d) δ 0.86 - 0.98 (m, 2H), 1.63 (br d, *J =* 6.1 Hz, 1H), 1.83 - 1.93 (m, 1H), 3.54 (d, *J =* 10.5 Hz, 1H), 3.80 (s, 3H), 3.86 - 3.93 (m, 1H), 3.96 - 4.03 (m, 1H), 4.32 (d, *J =* 10.5 Hz, 1H), 6.46 (d, *J =* 8.3 Hz, 1H), 7.06 - 7.24 (m, 6H).

**Step H:** The intermediate product from Step G (3.3 g, 11.06 mmol) was dissolved in acetic acid (50 mL) and water (5 mL). The temperature was lowered to 0°C, and N-chlorosuccinimide (4.43 g, 33.18 mmol) was added. The system was stirred at 0°C for 0.5 hours.

After the disappearance of the starting material as monitored by TLC, the reaction mixture was diluted with water (50 mL) and extracted with ethyl acetate (50 × 2 mL). The combined organic phases were washed with saturated brine (100 mL), dried over anhydrous sodium sulfate, filtered, and concentrated under reduced pressure. The resulting mixture was purified by silica gel column chromatography (eluent: petroleum ether/ethyl acetate = 10/1) to afford 2.44 g of INT-10-P1.

¹H NMR (400 MHz, CHLOROFORM-d) δ 1.04 - 1.13 (m, 2H), 1.75 - 1.84 (m, 1H), 1.94 - 2.03 (m, 1H), 3.94 (s, 3H), 4.00 (dd, *J=* 10.7, 1.9 Hz, 1H), 4.56 (d, *J =* 10.5 Hz, 1H), 6.60 (d, *J=* 8.6 Hz, 1H), 7.45 (d, *J =* 8.6 Hz, 1H).

### Example 1:

### N-(6-((1H-Pyrazol-1-yl)methyl)-4-methoxybenzo[d]isoxazol-3-yl)-3,4-dihydro-2H-benz o[b] [1,4]dioxepine-6-sulfonamide

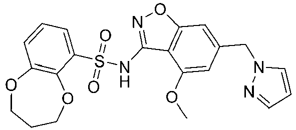

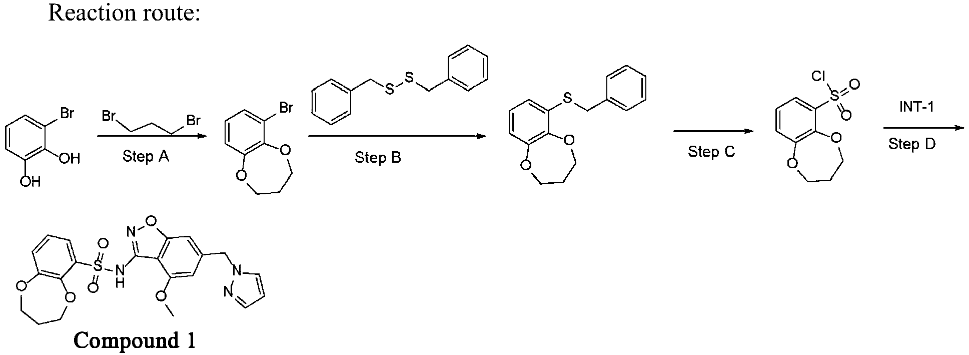

### Operating steps:

**Step A:** Under nitrogen protection, 3-bromobenzene-1,2-diol (1 g, 5.3 mmol), 1,3-dibromopropane (1.6 g, 8.0 mmol), potassium carbonate (1.46 g, 10.6 mmol), and potassium fluoride (153 mg, 2.6 mmol) were dissolved in N,N-dimethylformamide (10 mL). The system was stirred at 135°C for 2 hours.

After the disappearance of the starting material as monitored by LCMS, the reaction mixture was poured into saturated ammonium chloride solution (100 mL) and diluted with ethyl acetate (100 mL). The mixture was washed with water (100 mL) and saturated brine (100 mL), dried over anhydrous sodium sulfate, filtered, and concentrated under reduced pressure. The resulting mixture was purified by silica gel column chromatography to afford 750 mg of 6-bromo-3,4-dihydro-2H-benzo[b][1,4]dioxepine.

MS (ESI) m/z: 229.1, 231.0 [M+H]⁺.

¹H NMR (400 MHz, DMSO-*d*₆) δ 7.26 (dd, *J =* 7.8, 1.6 Hz, 1H), 6.99 (dd, *J =* 8.2, 1.6 Hz, 1H), 6.88 (t, *J =* 8.0 Hz, 1H), 4.23 - 4.10 (m, 4H), 2.19 - 2.06 (m, 2H).

**Step B:** Under nitrogen protection, 6-bromo-3,4-dihydro-2H-benzo[b][1,4]dioxepine (640 mg, 2.8 mmol) was dissolved in tetrahydrofuran (8 mL). The solution was cooled to -78°C, and tert-butyllithium (1.93 mL) was added dropwise. The mixture was stirred at this temperature for 30 minutes. A solution of 1,2-dibenzyldisulfane (825 mg, 3.4 mmol) in tetrahydrofuran (2 mL) was added dropwise at -78°C. The mixture was stirred at the same temperature for 30 minutes and then warmed to room temperature and stirred for 1 hour.

After the disappearance of the starting material as monitored by LCMS, the reaction mixture was poured into a mixture of ethyl acetate and saturated ammonium chloride. The mixture was extracted, washed with saturated brine (100 mL × 3), dried over anhydrous magnesium sulfate, filtered, and concentrated under reduced pressure. The resulting mixture was purified by silica gel column chromatography to afford 320 mg of 6-(benzylthio)-3,4-dihydro-2H-benzo[b][1,4]dioxepine.

MS (ESI) m/z: 273.1 [M+H]⁺.

¹H NMR (400 MHz, DMSO-*d*₆) δ 7.42 - 7.16 (m, 5H), 6.99 - 6.82 (m, 2H), 6.78 (dd, *J* = 7.8, 1.6 Hz, 1H), 4.14 (s, 2H), 4.08 (t, *J* = 5.2 Hz, 4H), 2.16 - 1.98 (m, 2H).

**Step C:** Under nitrogen protection, 6-(benzylthio)-3,4-dihydro-2H-benzo[b][1,4]dioxepine (320 mg, 1.2 mmol) and N-chlorosuccinimide (802.6 mg, 6.0 mmol) were dissolved in acetic acid (1.2 mL), water (1.2 mL), and acetonitrile (9 mL). The system was stirred at room temperature for 16 hours.

After the disappearance of the starting material as monitored by LCMS, the reaction mixture was diluted with ethyl acetate (100 mL). The mixture was washed with water (100 mL) and saturated brine (100 mL), dried over anhydrous magnesium sulfate, filtered, and concentrated under reduced pressure. The resulting mixture was purified by silica gel column chromatography to afford 200 mg of 3,4-dihydro-2H-benzo[b][1,4]dioxepine-6-sulfonyl chloride.

¹H NMR (400 MHz, DMSO-*d*₆) δ 7.36 (dd, *J =* 7.6, 1.8 Hz, 1H), 6.96 (dd, *J =* 7.8, 1.8 Hz, 1H), 6.86 (t, *J =* 7.8 Hz, 1H), 4.10 - 3.94 (m, 4H), 2.11 - 2.01 (m, 2H).

**Step D:** Under nitrogen protection, 3,4-dihydro-2H-benzo[b][1,4]dioxepine-6-sulfonyl chloride (200 mg, 0.81 mmol) and 6-((1H-pyrazol-1-yl)methyl)-4-methoxybenzo[d]isoxazol-3-amine (131 mg, 0.54 mmol) were dissolved in pyridine (5 mL). The system was stirred at 120°C for 2 hours.

After the disappearance of the starting material as monitored by LCMS, the reaction mixture was added to water (20 mL) and diluted with ethyl acetate (20 mL). The mixture was washed with saturated brine (50 mL), dried over anhydrous magnesium sulfate, filtered, and concentrated under reduced pressure. The resulting mixture was purified by silica gel column chromatography and preparative TLC to afford 5.5 mg of N-(6-((1H-pyrazol-1-yl)methyl)-4-methoxybenzo[d] isoxazol-3-yl)-3,4-dihydro-2H-benzo[b][1,4]dioxepine-6-sulfonamide.

MS (ESI) m/z: 457.1 [M+H]⁺.

¹H NMR (400 MHz, DMSO-*d*₆) δ 10.48 (s, 1H), 7.87 (d, *J =* 2.2 Hz, 1H), 7.48 (dd, *J =* 7.8, 1.6 Hz, 2H), 7.27 (s, 1H), 7.09 (s, 1H), 6.82 (s, 1H), 6.72 (s, 1H), 6.30 (t, *J =* 2.0 Hz, 1H), 5.43 (s, 2H), 4.21 - 4.05 (m, 4H), 3.80 (s, 3H), 2.15 - 2.02 (m, 2H).

### Example 2:

### N-(6-((1H-Pyrazol-1-yl)methyl)-4-methoxybenzo[d]isoxazol-3-yl)-7-methoxy-3,4-dihyd ro-2H-benzo[b][1,4]dioxepine-6-sulfonamide

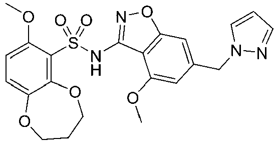

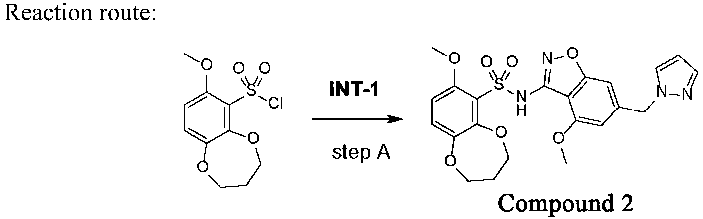

### Operating steps:

**Step A:** Under nitrogen protection, 7-methoxy-3,4-dihydro-2H-benzo[b][1,4]dioxepine-6-sulfonyl chloride (1.0 g, 3.60 mmol) and 6-((1H-pyrazol-1-yl)methyl)-4-methoxybenzo[d] isoxazol-3-amine (439 mg, 1.80 mmol) were dissolved in pyridine (8 mL). The system was stirred under microwave irradiation at 130°C for 12 hours.

After the disappearance of the starting material as monitored by LCMS, the reaction mixture was added to water (50 mL) and diluted with ethyl acetate (50 mL). The mixture was washed with saturated brine (30 mL), dried over anhydrous sodium sulfate, filtered, and concentrated under reduced pressure. The resulting mixture was purified by silica gel column chromatography and reverse-phase preparative separation to afford 90 mg of N-(6-((1H-pyrazol-1-yl)methyl)-4-methoxybenzo[d]isoxazol-3-yl)-7-methoxy-3,4-dihydro-2H-benzo[b][1,4]dioxepine-6-sulfonami de.

MS (ESI) m/z: 487.2 [M+H]⁺.

¹H NMR (400 MHz, DMSO-d₆): δ 9.95 (s, 1H), 7.88 (d, *J =* 2.0 Hz, 1H), 7.50 (d, *J =* 1.4 Hz, 1H), 7.23 (d, *J =* 9.2 Hz, 1H), 6.79 (dd, *J =* 21.6, 12.6 Hz, 3H), 6.30 (t, *J =* 2.0 Hz, 1H), 5.45 (s, 2H), 4.10 - 3.97 (m, 4H), 3.83 (s, 3H), 3.69 (s, 3H), 2.07 - 2.01 (m, 2H).

### Example 3 and Example 4:

### N-(6-((1H-Pyrazol-1-yl)methyl)-4-methoxybenzo[d]isoxazol-3-yl)-6-methoxy-2H-spiro[ benzofuran-3,1'-cyclopropane]-7-sulfonamide

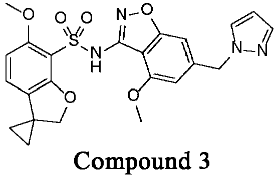

### N-(6-((1H-Pyrazol-1-yl)methyl)-4-methoxybenzo[d]isoxazol-3-yl)-6-methoxy-2H-spiro[ benzofuran-3,1'-cyclopropane]-5-sulfonamide

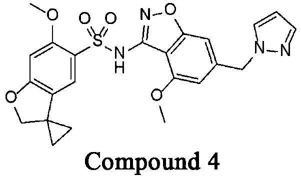

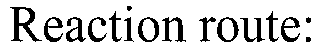

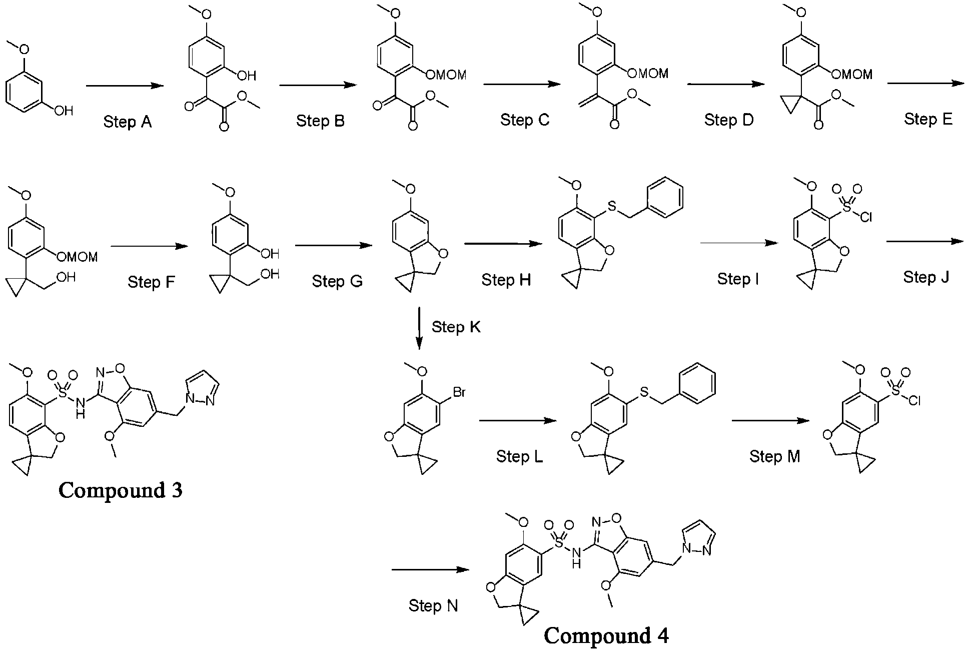

### Operating steps:

**Step A:** Under nitrogen protection, aluminum trichloride (11.82 g, 88.61 mmol) was suspended in dichloromethane (100 mL), and methyl oxalyl chloride (10.85 g, 88.61 mmol) was slowly added under ice-bath conditions. After stirring until the reaction mixture became clear, 3-methoxyphenol (5.0 g, 40.28 mmol) was slowly added dropwise to the reaction mixture. The system was slowly warmed to 25°C and stirred for 2 hours.

After the completion of the reaction as monitored by LCMS, ice cubes were slowly added to the reaction mixture under ice-bath conditions, followed by the addition of 2 M hydrochloric acid (50 mL). The mixture was extracted with dichloromethane (100 mL × 3). The combined organic phases were washed with 2 M sodium hydroxide solution (100 mL × 2) and saturated sodium chloride solution (100 mL), dried over anhydrous sodium sulfate, filtered, and concentrated under reduced pressure. The crude product was purified by silica gel column chromatography to afford 9.0 g of methyl 2-(2-hydroxy-4-methoxyphenyl)-2-oxoacetate.

MS (ESI) m/z: 211.1 [M+H]⁺.

¹H NMR (500 MHz, CDCl₃): δ 11.74 (s, 1H), 7.67 (d, *J =* 9.0 Hz, 1H), 6.51 - 6.45 (m, 2H), 4.12 (d, *J =* 7.2 Hz, 1H), 3.98 (s, 3H), 3.88 (s, 3H).

**Step B:** Under nitrogen protection, methyl 2-(2-hydroxy-4-methoxyphenyl)-2-oxoacetate (10.0 g, 47.58 mmol) and potassium carbonate (13.13 g, 95.15 mmol) were dissolved in N,N-dimethylformamide (80 mL). Bromomethyl methyl ether (5.02 mL, 61.85 mmol) was slowly added dropwise at 0°C. The reaction mixture was slowly warmed to 25°C and stirred for 1 hour.

After the completion of the reaction as monitored by LCMS, saturated sodium bicarbonate aqueous solution (100 mL) was slowly added dropwise at 0°C to quench the reaction. The mixture was extracted with ethyl acetate (120 mL × 3), washed with saturated sodium chloride solution (100 mL × 3), dried over anhydrous sodium sulfate, filtered, and concentrated under reduced pressure. The crude product was purified by silica gel column chromatography to afford 6.95 g of methyl 2-(4-methoxy-2-(methoxymethoxy)phenyl)-2-oxoacetate.

MS (ESI) m/z: 255.1 [M+H]⁺.

**Step C:** At 0°C, n-butyllithium (9.05 mL, 22.62 mmol, 2.5 M) was added to a solution of methyltriphenylphosphonium bromide (8431.82 mg, 23.60 mmol) in tetrahydrofuran (100 mL). The temperature was raised to room temperature and stirred for 1 hour, then cooled to -78°C. A solution of methyl 2-(4-methoxy-2-(methoxymethoxy)phenyl)-2-oxoacetate (5000 mg, 19.67 mmol) in tetrahydrofuran (20 mL) was added to the reaction mixture. The temperature was raised to room temperature and stirred for 12 hours.

After the completion of the reaction as monitored by LCMS, saturated ammonium chloride (5 mL) and water (100 mL) were added to the reaction mixture. The mixture was extracted with ethyl acetate (100 mL × 2). The combined organic phases were washed with saturated brine (100 mL × 2), dried over sodium sulfate, filtered, concentrated, and purified by column chromatography to afford 4.3 g of methyl 2-(4-methoxy-2-(methoxymethoxy)phenyl)acrylate.

MS (ESI) m/z: 253.1 [M+H]⁺.

**Step D:** Potassium tert-butoxide (3826.36 mg, 34.1 mmol) was added to a solution of trimethylsulfoxonium iodide (7504.39 mg, 34.1 mmol) in N,N-dimethylformamide (70 mL). The mixture was degassed with nitrogen three times and stirred at 20°C for 45 minutes. A solution of methyl 2-(4-methoxy-2-(methoxymethoxy)phenyl)acrylate (4300 mg, 17.05 mmol) in N,N-dimethylformamide (30 mL) was added to the reaction mixture. The temperature was raised to 45°C and the reaction was stirred for 1 hour.

After the completion of the reaction as monitored by LCMS, the reaction mixture was cooled to 0°C, and dilute hydrochloric acid was added dropwise to adjust the pH to 6-7. Water (200 mL) was added to the reaction mixture, and the mixture was extracted with ethyl acetate (100 mL × 2). The combined organic phases were washed with saturated brine (100 mL × 2), dried over sodium sulfate, filtered, concentrated, and purified by column chromatography to afford 3.1 g of methyl 1-(4-methoxy-2-(methoxymethoxy)phenyl)cyclopropane-1-carboxylate.

MS (ESI) m/z: 267.2 [M+H]⁺.

**Step E:** At 0°C, lithium aluminum hydride (12.8 mL, 12.80 mmol, 1 M) was added to a solution of methyl 1-(4-methoxy-2-(methoxymethoxy)phenyl)cyclopropane-1-carboxylate (3.1 g, 11.64 mmol) in tetrahydrofuran (10 mL). The reaction was stirred at 0°C for 1 hour.

After the completion of the reaction as monitored by LCMS, water (500 mL), 15 wt% sodium hydroxide aqueous solution (500 mL), and water (1500 mL) were sequentially added to the reaction mixture at 0°C. The mixture was filtered, and the filtrate was concentrated to afford 2.7 g of (1-(4-methoxy-2-(methoxymethoxy)phenyl)cyclopropyl)methanol.

MS (ESI) m/z: 251.2 [M+Na]⁺.

**Step F:** At 0°C, a solution of hydrochloric acid (1652.37 mg, 45.32 mmol) in dioxane was added to a solution of (1-(4-methoxy-2-(methoxymethoxy)phenyl)cyclopropyl)methanol (2.7 g, 11.33 mmol) in methanol (30 mL). The temperature was raised to 20°C and the reaction was stirred for 1 hour.

After the completion of the reaction as monitored by LCMS, the solvent was removed under reduced pressure. Water (20 mL) was added, and the mixture was extracted with ethyl acetate (30 mL × 2). The combined organic phases were washed with saturated brine (30 mL × 2), dried over sodium sulfate, filtered, concentrated, and purified by column chromatography to afford 1.9 g of 2-(1-(hydroxymethyl)cyclopropyl)-5-methoxyphenol.

MS (ESI) m/z: 195.2 [M+H]⁺.

**Step G:** Triphenylphosphine (3241.90 mg, 12.36 mmol) was added to a solution of 2-(1-(hydroxymethyl)cyclopropyl)-5-methoxyphenol (1600 mg, 8.24 mmol) in tetrahydrofuran (30 mL). The mixture was stirred for 10 minutes, then diisopropyl azodicarboxylate (2499.32 mg, 12.36 mmol) was added, and stirring was continued for 1 hour.

After the completion of the reaction as monitored by LCMS, petroleum ether (90 mL) was added to the reaction mixture, and the mixture was filtered through silica gel (50 g). The filtrate was concentrated and purified by column chromatography to afford 960 mg of 6-methoxy-2H-spiro[benzofuran-3,1'-cyclopropane].

MS (ESI) m/z: 177.1 [M+H]⁺.

**Step H:** At 0°C, n-butyllithium (0.75 mL, 1.87 mmol, 2.5 M) was added dropwise to a solution of 6-methoxy-2H-spiro[benzofuran-3,1'-cyclopropane] (300 mg, 1.70 mmol) and N,N,N',N'-tetramethylethylenediamine (0.22 g, 1.87 mmol) in tetrahydrofuran (8 mL). The mixture was maintained at this temperature for 0.5 hours, then cooled to -70°C. A solution of dibenzyldisulfane (460.75 mg, 1.87 mmol) in tetrahydrofuran (3 mL) was added dropwise to the reaction mixture. The reaction was stirred at -70°C for 0.5 hours, then warmed to room temperature and stirred for 1 hour.

After the completion of the reaction as monitored by LCMS, saturated ammonium chloride (5 mL) and water (10 mL) were added to the reaction mixture. The mixture was extracted with ethyl acetate (30 mL × 2). The combined organic phases were washed with saturated brine (30 mL × 2), dried over sodium sulfate, filtered, concentrated, and purified by column chromatography to afford 380 mg of 7-(benzylthio)-6-methoxy-2H-spiro[benzofuran-3,1'-cyclopropane].

MS (ESI) m/z: 299.2 [M+H]⁺.

**Step I:** At 0°C, N-chlorosuccinimide (136.20 mg, 1.02 mmol) was added to a solution of 7-(benzylthio)-6-methoxy-2H-spiro[benzofuran-3,1'-cyclopropane] (100 mg, 0.34 mmol) in acetic acid (3 mL) and water (0.3 mL). The reaction was stirred at 10°C for 1 hour.

TLC showed the disappearance of the starting material. Water (10 mL) was added to the reaction mixture, and the mixture was extracted with ethyl acetate (10 mL × 2). The combined organic phases were washed with saturated brine (10 mL × 2) and saturated sodium carbonate solution (10 mL × 2), dried over sodium sulfate, filtered, concentrated, and purified by column chromatography to afford 60 mg of 6-methoxy-2H-spiro[benzofuran-3,1'-cyclopropane]-7-sulfonyl chloride.

¹H NMR (400 MHz, Chloroform-d): δ 6.74 (d, *J =* 8.3 Hz, 1H), 6.38 (d, *J =* 8.3 Hz, 1H), 4.65 (s, 2H), 3.90 (s, 3H), 1.00 (dt, *J =* 5.7, 2.1 Hz, 4H).

Step **J:** 6-Methoxy-2H-spiro[benzofuran-3,1'-cyclopropane]-7-sulfonyl chloride (33.79 mg, 0.12 mmol) was added to a solution of 6-((1H-pyrazol-1-yl)methyl)-4-methoxybenzo[d]isoxazol-3-amine (20 mg, 0.082 mmol) in pyridine (1 mL). The reaction was carried out under microwave irradiation at 100°C for 3 hours.

LCMS showed the formation of the target peak. The solvent was removed under reduced pressure, and the residue was purified by preparative separation to afford 13 mg of N-(6-((1H-pyrazol-1-yl)methyl)-4-methoxybenzo[d]isoxazol-3-yl)-6-methoxy-2H-spiro[benzofu ran-3,1'-cyclopropane]-7-sulfonamide.

MS (ESI) m/z: 483.1 [M+H]⁺.

¹H NMR (400 MHz, DMSO-*d₆*): δ 7.86 (d, *J* = 2.3 Hz, 1H), 7.49 (d, *J* = 1.8 Hz, 1H), 6.84 (s, 1H), 6.76 (s, 1H), 6.68 (s, 1H), 6.45 (d, *J* = 8.2 Hz, 1H), 6.29 (t, *J* = 2.1 Hz, 1H), 5.42 (s, 2H), 4.47 (s, 2H), 3.84 (s, 3H), 3.65 (s, 3H), 1.08 - 0.91 (m, 4H).

**Step K:** N-Bromosuccinimide (332.82 mg, 1.87 mmol) was added to a solution of 6-methoxy-2H-spiro[benzofuran-3,1'-cyclopropane] (300 mg, 1.70 mmol) in acetonitrile (3 mL). The reaction was stirred at room temperature for 3 hours.

TLC showed complete consumption of the starting material. Water (10 mL) was added to the reaction mixture, and the mixture was extracted with ethyl acetate (10 mL × 2). The combined organic phases were washed with saturated brine (10 mL), dried over sodium sulfate, filtered, concentrated, and purified by column chromatography to afford 360 mg of 5-bromo-6-methoxy-2H-spiro[benzofuran-3,1'-cyclopropane].

¹H NMR (400 MHz, Chloroform-d): δ 6.71 (s, 1H), 6.38 (s, 1H), 4.43 (s, 2H), 3.78 (s, 3H), 0.96 - 0.93 (m, 2H), 0.91 (dd, *J* = 3.9, 1.4 Hz, 2H).

**Step L:** At 0°C, n-butyllithium (0.62 mL, 1.55 mmol, 2.5 M) was added dropwise to a solution of 5-bromo-6-methoxy-2H-spiro[benzofuran-3,1'-cyclopropane] (360 mg, 1.41 mmol) and N,N,N',N'-tetramethylethylenediamine (0.18 g, 1.55 mmol) in tetrahydrofuran (8 mL). The mixture was maintained at this temperature for 0.5 hours, then cooled to -70°C. A solution of dibenzyldisulfane (382.15 mg, 1.55 mmol) in tetrahydrofuran (3 mL) was added dropwise to the reaction mixture. The reaction was stirred at -70°C for 0.5 hours, then warmed to room temperature and stirred for 1 hour.

After the completion of the reaction as monitored by LCMS, saturated ammonium chloride (5 mL) and water (10 mL) were added to the reaction mixture. The mixture was extracted with ethyl acetate (30 mL × 2). The combined organic phases were washed with saturated brine (30 mL × 2), dried over sodium sulfate, filtered, concentrated, and purified by column chromatography to afford 200 mg of 5-(benzylthio)-6-methoxy-2H-spiro[benzofuran-3,1'-cyclopropane].

MS (ESI) m/z: 299.2 [M+H]⁺.

**Step M:** At 0°C, N-chlorosuccinimide (228.34 mg, 1.71 mmol) was added to a solution of 5-(benzylthio)-6-methoxy-2H-spiro[benzofuran-3,1'-cyclopropane] (170 mg, 0.57 mmol) in acetic acid (5 mL) and water (0.5 mL).

TLC showed the disappearance of the starting material. Water (10 mL) was added to the reaction mixture, and the mixture was extracted with ethyl acetate (10 mL × 2). The combined organic phases were washed with saturated brine (10 mL × 2) and saturated sodium carbonate solution (10 mL × 2), dried over sodium sulfate, filtered, concentrated, and purified by column chromatography to afford 60 mg of 6-methoxy-2H-spiro[benzofuran-3,1'-cyclopropane]-5-sulfonyl chloride.

¹H NMR (400 MHz, Chloroform-d) δ 7.14 (s, 1H), 6.44 (s, 1H), 4.56 (s, 2H), 3.94 (s, 3H), 1.09 - 0.96 (m, 4H).

**Step N:** 6-Methoxy-2H-spiro[benzofuran-3,1'-cyclopropane]-5-sulfonyl chloride (33.79 mg, 0.12 mmol) was added to a solution of 6-((1H-pyrazol-1-yl)methyl)-4-methoxybenzo[d]isoxazol-3-amine (20 mg, 0.082 mmol) in pyridine (1 mL). The reaction was carried out under microwave irradiation at 100°C for 3 hours.

LCMS showed the formation of the target peak. The solvent was removed under reduced pressure, and the residue was purified by preparative separation to afford 18 mg of N-(6-((1H-pyrazol-1-yl)methyl)-4-methoxybenzo[d]isoxazol-3-yl)-6-methoxy-2H-spiro[benzofu ran-3,1'-cyclopropane]-5-sulfonamide.

MS (ESI) m/z: 483.1 [M+H]⁺.

¹H NMR (400 MHz, DMSO-*d₆*) δ 9.65 (s, 1H), 7.86 (d, *J =* 2.3 Hz, 1H), 7.49 (d, *J* = 1.8 Hz, 1H), 7.18 (s, 1H), 6.80 (s, 1H), 6.71 (s, 1H), 6.64 (s, 1H), 6.30 (t, *J* = 2.1 Hz, 1H), 5.43 (s, 2H), 4.56 (s, 2H), 3.86 (s, 3H), 3.72 (s, 3H), 1.03 (d, *J* = 11.2 Hz, 4H).

### Example 5:

### N-(6-((1H-Pyrazol-1-yl)methyl)-4-methoxybenzo[d]isoxazol-3-yl)-5-methoxy-1,1a,2,7b-tetrahydrocyclopropa[c]chromene-4-sulfonamide

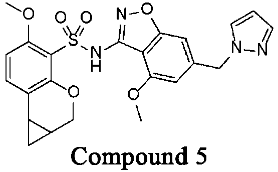

### N-(6-((1H-Pyrazol-1-yl)methyl)-4-methoxybenzo[d]isoxazol-3-yl)-5-methoxy-1,1a,2,7b-tetrahydrocyclopropa[c] chromene-6-sulfonamide

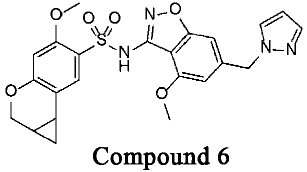

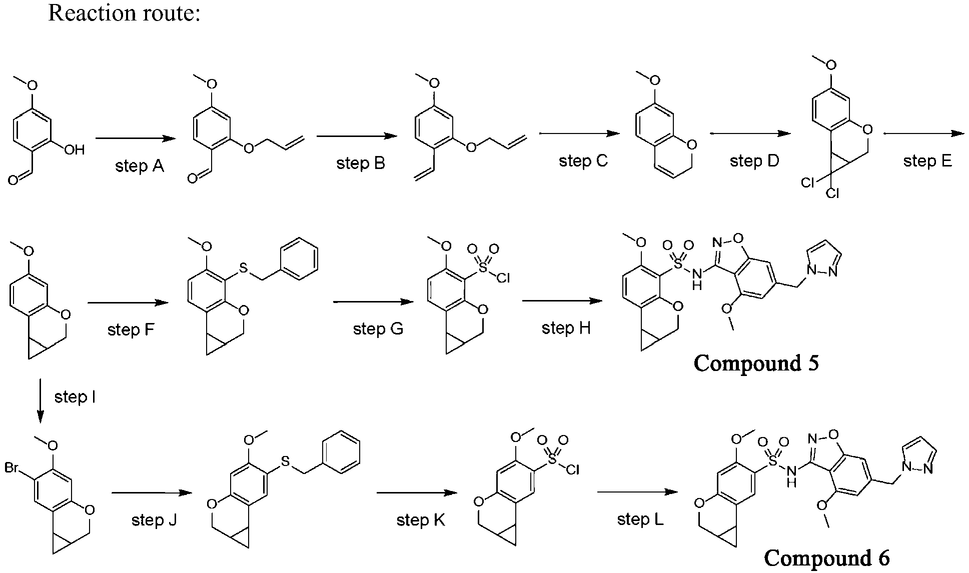

### Operating steps:

Step A: 2-Hydroxy-4-methoxybenzaldehyde (10.0 g, 66 mmol) and 3-bromoprop-1-ene (12.0 g, 99 mmol) were dissolved in acetone (100 mL). Anhydrous potassium carbonate (18.2 g, 132 mmol) was added. The reaction mixture was gradually heated to 70 °C and stirred for 3 hours.

After the disappearance of the starting material as monitored by LCMS, the mixture was cooled to room temperature, diluted with water (50 mL), and extracted with ethyl acetate (50 mL × 2). The combined organic phases were washed with water (20 mL) and saturated brine (20 mL), dried over anhydrous sodium sulfate, filtered, and concentrated under reduced pressure to afford 13 g of crude 2-(allyloxy)-4-methoxybenzaldehyde.

MS (ESI) m/z: 193.3 [M+H]⁺.

**Step B:** Under ice-bath conditions, sodium hydride (3.5 g, 88 mmol, 60 wt%) was added to a solution of (bromomethyl)triphenylphosphonium bromide (31 g, 71 mmol) in anhydrous N,N-dimethylformamide (100 mL). The mixture was stirred for half an hour. A solution of 2-(allyloxy)-4-methoxybenzaldehyde (13 g, 67.7 mmol) in N,N-dimethylformamide (30 mL) was slowly added dropwise to the mixture. The reaction system was slowly warmed to room temperature and stirred for 2 hours.

After the disappearance of the starting material as monitored by LCMS, the mixture was cooled to 0 °C, and water was added dropwise to quench the reaction. The filtrate was extracted with ethyl acetate (50 mL × 3). The combined organic phases were washed with water (50 mL) and saturated brine (50 mL), dried over anhydrous sodium sulfate, filtered, and concentrated under reduced pressure. The crude product was purified by flash silica gel column chromatography to afford 11 g of 2-(allyloxy)-4-methoxy-1-vinylbenzene.

MS (ESI) m/z: 191.2 [M+H]⁺.

**Step C:** 2-(Allyloxy)-4-methoxy-1-vinylbenzene (11 g, 57.9 mmol) was dissolved in dichloromethane (80 mL). (1,3-Bis(2,4,6-trimethylphenyl)-2-imidazolidinylidene)dichloro(o-isopropoxyphenylmethylene)ruthenium (1.8 g, 2.9 mmol) was added. The reaction system was stirred at room temperature for 1 hour.

After the disappearance of the starting material as monitored by LCMS, the mixture was concentrated under reduced pressure. The crude product was purified by flash silica gel column chromatography to afford 8.2 g of 7-methoxy-2H-chromene.

MS (ESI) m/z: 163.3 [M+H]⁺.

¹H NMR (500 MHz, CDCl₃): δ 6.87 (d, *J* = 8.0 Hz, 1H), 6.42 (dd, *J* = 8.0, 2.4 Hz, 1H), 6.40 - 6.35 (m, 2H), 5.65 - 5.60 (m, 1H), 4.79 (dd, *J =* 3.6, 2.0 Hz, 2H), 3.77 (s, 3H).

**Step D:** 7-Methoxy-2H-chromene (8.2 g, 50.6 mmol) and benzyltriethylammonium chloride (1.2 g, 5.1 mmol) were dissolved in chloroform (80 mL). A 33 wt% sodium hydroxide aqueous solution was slowly added dropwise at room temperature. The reaction mixture was stirred at room temperature for 2 hours.

After the disappearance of the starting material as monitored by LCMS, the reaction mixture was poured into water (100 mL) and extracted with dichloromethane (50 mL × 3). The combined organic phases were washed with water (50 mL) and saturated brine (50 mL), dried over anhydrous sodium sulfate, filtered, and concentrated under reduced pressure. The crude product was purified by flash silica gel column chromatography to afford 9.5 g of 1,1-dichloro-5-methoxy-1,1a,2,7b-tetrahydrocyclopropa[c]chromene.

MS (ESI) m/z: 245.1 [M+H]⁺.

**Step E:** At room temperature, metallic sodium (7.1 g, 310 mmol) was dissolved in anhydrous diethyl ether (70 mL). A solution of 1,1-dichloro-5-methoxy-1,1a,2,7b-tetrahydrocyclopropa[c]chromene (9.5 g, 38.8 mmol) in a mixture of anhydrous diethyl ether (30 mL) and anhydrous methanol (15 mL) was slowly added dropwise to the reaction mixture. Methanol (5 mL) was added every hour until the starting material disappeared.

After the disappearance of the starting material as monitored by LCMS, anhydrous ethanol (20 mL) was added to quench the reaction. The mixture was diluted with water (50 mL) and extracted with ethyl acetate (50 mL × 3). The combined organic phases were washed with water (50 mL) and saturated brine (50 mL), dried over anhydrous sodium sulfate, filtered, and concentrated under reduced pressure. The crude product was purified by flash silica gel column chromatography to afford 5.5 g of 5-methoxy-1,1a,2,7b-tetrahydrocyclopropalc]chromene.

MS (ESI) m/z: 244.2 [M+H]⁺.

**Step F:** The starting material 5-methoxy-1,1a,2,7b-tetrahydrocyclopropa[c]chromene (600 mg, 3.4 mmol) was dissolved in anhydrous tetrahydrofuran (10 mL). The atmosphere was replaced with nitrogen three times. n-Butyllithium (1.6 mL, 4.1 mmol) was added dropwise under ice-bath conditions, and the mixture was stirred for 1 hour. Then, a solution of 1,2-dibenzyldisulfane (1.0 g, 4.1 mmol) in anhydrous tetrahydrofuran (10 mL) was added dropwise to the reaction mixture. The temperature was gradually raised to room temperature and the reaction was stirred for 2 hours.

After the disappearance of the starting material as monitored by LCMS, water (30 mL) was added to the reaction system for dilution. The mixture was extracted with ethyl acetate (30 mL × 3), and the organic phases were combined. The combined organic phase was washed with saturated brine (30 mL × 3), dried over anhydrous sodium sulfate, filtered, and concentrated under reduced pressure. The crude product was purified by flash silica gel column chromatography to afford 250 mg of 4-(benzylthio)-5-methoxy-1,1a,2,7b-tetrahydrocyclopropa[c] chromene.

MS (ESI) m/z: 299.1 [M+H]⁺.

¹H NMR (500 MHz, DMSO-*d₆*): δ 7.24 - 7.10 (m, 6H), 6.54 (d, *J =* 8.0 Hz, 1H), 4.29 (d, *J* = 10.2 Hz, 1H), 3.96 (d, *J* = 12.4 Hz, 1H), 3.88 (d, *J* = 12.4 Hz, 1H), 3.72 (s, 3H), 3.51 (d, *J* = 10.0 Hz, 1H), 1.96 - 1.91 (m, 1H), 1.72 (d, *J =* 8.0 Hz, 1H), 0.97 - 0.91 (m, 1H), 0.72 - 0.69 (m, 1H).

**Step G:** 4-(Benzylthio)-5-methoxy-1,1a,2,7b-tetrahydrocyclopropa[c]chromene (250 mg, 0.84 mmol) was dissolved in acetonitrile (5 mL) and water (0.5 mL). Acetic acid (403 mg, 6.7 mmol) was added. After cooling under ice-bath conditions, 1,3-dichloro-5,5-dimethylhydantoin (331 mg, 1.7 mmol) was added to the reaction mixture. The reaction was stirred under the ice bath for half an hour.

After the disappearance of the starting material as monitored by TLC, water (30 mL) was added to the reaction system for dilution. The mixture was extracted with ethyl acetate (30 mL × 3), and the organic phases were combined. The combined organic phase was washed with saturated brine (30 mL × 3), dried over anhydrous sodium sulfate, filtered, and concentrated under reduced pressure. The crude product was purified by flash silica gel column chromatography to afford 100 mg of 5-methoxy-1,1a,2,7b-tetrahydrocyclopropa[c]chromene-4-sulfonyl chloride.

MS (ESI) m/z: 275.1 [M+H]⁺.

**Step H:** The starting material 5-methoxy-1,1a,2,7b-tetrahydrocyclopropa[c]chromene-4-sulfonyl chloride (100 mg, 0.36 mmol) and 6-((1H-pyrazol-1-yl)methyl)-4-methoxybenzo[d] isoxazol-3-amine (57 mg, 0.24 mmol) were dissolved in anhydrous pyridine (1.0 mL). The reaction was carried out under microwave irradiation at 100 °C for 3 hours.

After the disappearance of the starting material as monitored by LCMS, water (30 mL) was added to the reaction system for dilution. The mixture was extracted with ethyl acetate (30 mL × 3), and the organic phases were combined. The combined organic phase was washed with saturated brine (30 mL × 3), dried over anhydrous sodium sulfate, filtered, and concentrated under reduced pressure. The crude product was purified by preparative HPLC to afford 12.3 mg of N-(6-((1H-pyrazol-1-yl)methyl)-4-methoxybenzo[d]isoxazol-3-yl)-5-methoxy-1,1a,2,7b-tetrahydrocyclopropa[c]chromene-4-sulfonamide.

MS (ESI) m/z: 483.1 [M+H]⁺.

¹H NMR (400 MHz, DMSO-*d₆*): δ 9.69 (s, 1H), 7.88 (d, *J =* 2.0 Hz, 1H), 7.50 (d, *J =* 1.6 Hz, 1H), 7.44 (d, *J* = 8.4 Hz, 1H), 6.83 (s, 1H), 6.76 (s, 1H), 6.71 (d, *J* = 8.4 Hz, 1H), 6.31 (t, *J* = 2.0 Hz, 1H), 5.45 (s, 2H), 4.27 (d, *J =* 10.8 Hz, 1H), 3.87 (s, 3H), 3.72 (s, 3H), 3.59 (d, *J =* 10.4 Hz, 1H), 2.04 - 1.98 (m, 1H), 1.75 (d, *J* = 5.6 Hz, 1H), 0.98 - 0.91 (m, 1H), 0.67 - 0.65 (m, 1H).

**Step I:** The starting material 5-methoxy-1,1a,2,7b-tetrahydrocyclopropalc]chromene (600 mg, 3.4 mmol) was dissolved in anhydrous acetonitrile (10 mL). N-Bromosuccinimide (666 mg, 3.7 mmol) was added. The reaction was stirred at room temperature for 3 hours.

After the disappearance of the starting material as monitored by LCMS, water (30 mL) was added to the reaction system for dilution. The mixture was extracted with ethyl acetate (30 mL × 3), and the organic phases were combined. The combined organic phase was washed with saturated brine (30 mL × 3), dried over anhydrous sodium sulfate, filtered, and concentrated under reduced pressure. The crude product was purified by flash silica gel column chromatography to afford 700 mg of 6-bromo-5-methoxy-1,1a,2,7b-tetrahydrocyclopropalc] chromene.

MS (ESI) m/z: 255.1 [M+H]⁺.

**Step J:** 6-Bromo-5-methoxy-1,1a,2,7b-tetrahydrocyclopropa[c]chromene (700 mg, 2.8 mmol) was dissolved in anhydrous tetrahydrofuran (20 mL). The atmosphere was replaced with nitrogen three times. n-Butyllithium (1.2 mL, 3.0 mmol) was added dropwise under a dry ice-acetone bath, and the mixture was stirred for 1 hour. Then, a solution of 1,2-dibenzyldisulfane (738 mg, 3.0 mmol) in anhydrous tetrahydrofuran (10 mL) was added dropwise to the reaction mixture. The temperature was gradually raised to room temperature and the reaction was stirred for 2 hours.

After the disappearance of the starting material as monitored by LCMS, water (30 mL) was added to the reaction system for dilution. The mixture was extracted with ethyl acetate (30 mL × 3), and the organic phases were combined. The combined organic phase was washed with saturated brine (30 mL × 3), dried over anhydrous sodium sulfate, filtered, and concentrated under reduced pressure. The crude product was purified by flash silica gel column chromatography to afford 350 mg of 6-(benzylthio)-5-methoxy-1,1a,2,7b-tetrahydrocyclopropa[c] chromene.

MS (ESI) m/z: 299.1 [M+H]⁺.

¹H NMR (400 MHz, DMSO-*d₆*): δ 7.28 (d, *J* = 4.4 Hz, 4H), 7.25 - 7.19 (m, 1H), 7.15 (s, 1H), 6.44 (s, 1H), 4.25 (d, *J* = 10.4 Hz, 1H), 4.04 (s, 2H), 3.77 (d, *J* = 10.8 Hz, 1H), 3.73 (s, 3H), 1.94 - 1.87 (m, 1H), 1.74 (dd, *J* = 13.6, 8.4 Hz, 1H), 1.00 - 0.93 (m, 1H), 0.71 - 0.68 (m, 1H).

**Step K:** 6-(Benzylthio)-5-methoxy-1,1a,2,7b-tetrahydrocyclopropa[c]chromene (350 mg, 1.2 mmol) was dissolved in acetonitrile (5 mL) and water (0.5 mL). Acetic acid (564 mg, 9.4 mmol) was added. After cooling under ice-bath conditions, 1,3-dichloro-5,5-dimethylhydantoin (427 mg, 2.4 mmol) was added to the reaction mixture. The reaction was stirred under the ice bath for half an hour.

After the disappearance of the starting material as monitored by TLC, water (30 mL) was added to the reaction system for dilution. The mixture was extracted with ethyl acetate (20 mL × 3), and the organic phases were combined. The combined organic phase was washed with saturated brine (20 mL × 3), dried over anhydrous sodium sulfate, filtered, and concentrated under reduced pressure. The crude product was purified by flash silica gel column chromatography to afford 200 mg of 5-methoxy-1,1a,2,7b-tetrahydrocyclopropa[c]chromene-6-sulfonyl chloride.

MS (ESI) m/z: 297.1 [M+Na]⁺.

**Step L:** 5-Methoxy-1,1a,2,7b-tetrahydrocyclopropa[c]chromene-6-sulfonyl chloride (200 mg, 0.73 mmol) and 6-((1H-pyrazol-1-yl)methyl)-4-methoxybenzo[d]isoxazol-3-amine (119 mg, 0.49 mmol) were dissolved in anhydrous pyridine (1.0 mL). The reaction was carried out under microwave irradiation at 100 °C for 3 hours.

After the disappearance of the starting material as monitored by LCMS, water (30 mL) was added to the reaction system for dilution. The mixture was extracted with ethyl acetate (10 mL × 3), and the organic phases were combined. The combined organic phase was washed with saturated brine (10 mL × 3), dried over anhydrous sodium sulfate, filtered, and concentrated under reduced pressure. The crude product was purified by preparative HPLC to afford 14.8 mg of N-(6-((1H-pyrazol-1-yl)methyl)-4-methoxybenzo[d]isoxazol-3-yl)-5-methoxy-1,1a,2,7b-tetrahydrocyclopropa[c]chromene-6-sulfonamide.

MS (ESI) m/z: 483.1 [M+H]⁺.

¹H NMR (500 MHz, DMSO-*d₆*): δ 9.77 (s, 1H), 7.87 (s, 1H), 7.72 (s, 1H), 7.49 (s, 1H), 6.82 (s, 1H), 6.73 (s, 1H), 6.56 (s, 1H), 6.30 (t, *J* = 2.0 Hz, 1H), 5.43 (s, 2H), 4.33 (d, *J* = 11.0 Hz, 1H), 3.89 (d, *J* = 11.0 Hz, 1H), 3.86 (s, 3H), 3.69 (s, 3H), 2.10 (s, 1H), 1.85 - 1.76 (m, 1H), 1.08 - 0.99 (m, 1H), 0.73 - 0.71 (m, 1H).

### Example 6:

### (1aS,7bR)-N-(6-((1H-Pyrazol-1-yl)methyl)-4-methoxybenzo[d]isoxazol-3-yl)-5-methoxy -1,1a,2,7b-tetrahydrocyclopropa[c]chromene-4-sulfonamide ( Compound 5-P1 or Compound 5-P2)

### (1aR,7bS)-N-(6-((1H-Pyrazol-1-yl)methyl)-4-methoxybenzo[d]isoxazol-3-yl)-5-methoxy -1,1a,2,7b-tetrahydrocyclopropa[c]chromene-4-sulfonamide (Compound 5-P2 or Compound 5-P1)

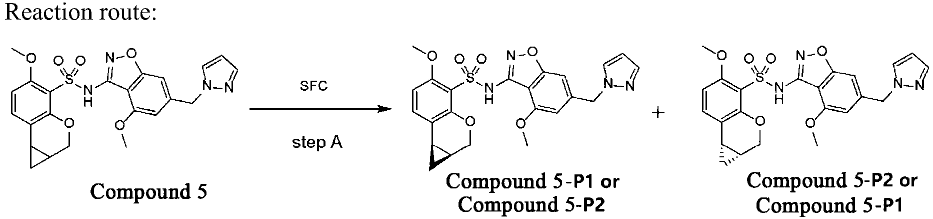

### Operating steps:

Step A: 498 mg of N-(6-((1H-Pyrazol-1-yl)methyl)-4-methoxybenzo[d]isoxazol-3-yl)-5-methoxy-1,1a,2,7b-tetrahydrocyclopropalc]chromene-4-sulfonamide was purified and separated by supercritical fluid chromatography (Column: REGIS (S,S) WHELK-O1 (250 mm × 25 mm, 10 µm); Mobile phase: [CO₂-EtOH (0.1% NH₃·H₂O)]; B%: 50%, isocratic elution mode) to afford 124.45 mg of 5-P1 (Retention time: 2.650 minutes) and 90.10 mg of 5-P2 (Retention time: 2.912 minutes).

### Compound 5-P1:

MS (ESI) m/z: 483.1 [M+H]⁺.

¹H NMR (400 MHz, METHANOL-d₄) δ 7.77 (d, *J* = 2.3 Hz, 1H), 7.56 (d, *J* = 1.6 Hz, 1H), 7.36 (d, *J* = 8.6 Hz, 1H), 6.76 (s, 1H), 6.62 - 6.70 (m, 2H), 6.37 (t, *J* = 2.1 Hz, 1H), 5.46 (s, 2H), 4.32 (d, *J* = 10.8 Hz, 1H), 3.99 (s, 3H), 3.79 (s, 3H), 3.72 (d, *J =* 10.5 Hz, 1H), 1.96 (td, *J =* 8.5, 4.4 Hz, 1H), 1.72 (br d, *J* = 5.5 Hz, 1H), 0.95 (td, *J =* 8.2, 4.7 Hz, 1H), 0.73 (q, *J* = 4.8 Hz, 1H).

### Compound 5-P2:

MS (ESI) m/z: 483.1 [M+H]⁺.

¹H NMR (400 MHz, METHANOL-d₄) δ 7.77 (d, *J* = 2.3 Hz, 1H), 7.56 (d, *J* = 1.6 Hz, 1H), 7.37 (d, *J* = 8.6 Hz, 1H), 6.77 (s, 1H), 6.63 - 6.70 (m, 2H), 6.37 (t, *J* = 2.1 Hz, 1H), 5.47 (s, 2H), 4.33 (d, *J* = 10.4 Hz, 1H), 3.99 (s, 3H), 3.79 (s, 3H), 3.73 (d, *J =* 10.1 Hz, 1H), 1.96 (td, *J =* 8.5, 4.3 Hz, 1H), 1.73 (br d, *J* = 5.4 Hz, 1H), 0.95 (td, *J* = 8.2, 4.7 Hz, 1H), 0.73 (q, *J =* 4.8 Hz, 1H).

### Example 7:

### N-(6-((1H-Pyrazol-1-yl)methyl)-4-methoxybenzo[d]isoxazol-3-yl)-8-methoxy-3,4-dihyd ro-2H-benzo[b][1,4]dioxepine-7-sulfonamide

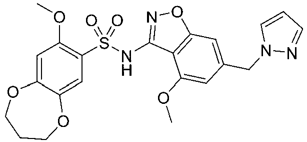

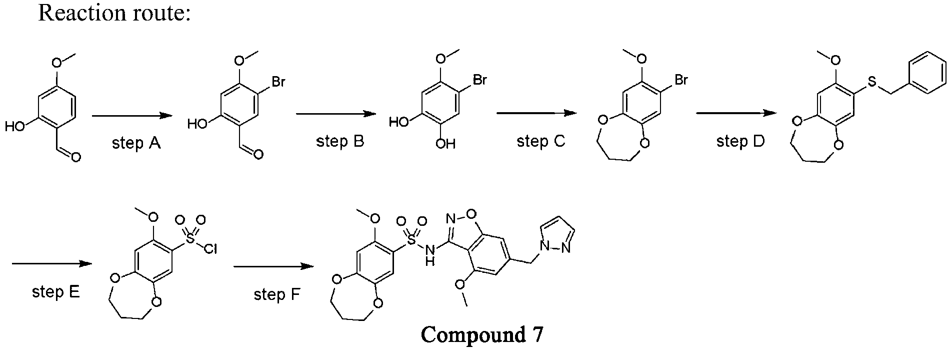

### Operating steps:

Step A: Under nitrogen protection, 2-hydroxy-4-methoxybenzaldehyde (5 g, 32.9 mmol) was dissolved in dichloromethane (50 mL). After cooling to 0°C, bromine (5.26 g, 32.9 mmol) was added dropwise. The system was stirred at room temperature for 16 hours.

After the disappearance of the starting material as monitored by LCMS, the reaction mixture was quenched with 1M hydrochloric acid (100 mL) and extracted with dichloromethane (100 mL). The organic phase was washed with saturated sodium sulfite solution (100 mL), water (100 mL), and saturated brine (100 mL × 3), dried over anhydrous magnesium sulfate, filtered, and concentrated under reduced pressure. The resulting mixture was purified by silica gel column chromatography to afford 3.0 g of 5-bromo-2-hydroxy-4-methoxybenzaldehyde.

MS (ESI) m/z: 231.1 [M+H]⁺.

**Step B:** Under nitrogen protection, 5-bromo-2-hydroxy-4-methoxybenzaldehyde (5.4 g, 23.5 mmol) was dissolved in tetrahydrofuran (50 mL). A sodium hydroxide solution (18.8 mL) and hydrogen peroxide (3.2 g, 28.2 mmol) were separately added to the reaction mixture at 0°C. The system was stirred at 0°C for 40 minutes.

After the disappearance of the starting material as monitored by LCMS, the reaction mixture was quenched with saturated sodium sulfite solution (100 mL) and diluted with dichloromethane (100 mL). The mixture was washed with 1M hydrochloric acid solution (100 mL), water (100 mL × 3), and saturated brine (100 mL × 3), dried over anhydrous magnesium sulfate, filtered, and concentrated under reduced pressure. The resulting mixture was purified by silica gel column chromatography to afford 5 g of 4-bromo-5-methoxybenzene-1,2-diol.

MS (ESI) m/z: 219.0 [M+H]⁺.

**Step C:** Under nitrogen protection, 4-bromo-5-methoxybenzene-1,2-diol (5 g, 22.9 mmol), 1,3-dibromopropane (6.9 g, 34.4 mmol), potassium carbonate (6.3 g, 45.9 mmol), and potassium fluoride (664.1 mg, 11.5 mmol) were dissolved in N,N-dimethylformamide (50 mL). The system was stirred at 135°C for 2 hours.

After the disappearance of the starting material as monitored by LCMS, the reaction mixture was poured into saturated ammonium chloride solution (100 mL) and diluted with ethyl acetate (100 mL). The mixture was washed with water (100 mL) and saturated brine (100 mL), dried over anhydrous magnesium sulfate, filtered, and concentrated under reduced pressure. The resulting mixture was purified by silica gel column chromatography to afford 380 mg of 7-bromo-8-methoxy-3,4-dihydro-2H-benzo[b][1,4]dioxepine.

MS (ESI) m/z: 259.0 [M+H]⁺.

**Step D:** Under nitrogen protection, 7-bromo-8-methoxy-3,4-dihydro-2H-benzo[b][1,4] dioxepine (380 mg, 2.1 mmol) was dissolved in tetrahydrofuran (5 mL). The solution was cooled to -78°C, and tert-butyllithium (1.4 mL, 2.3 mmol) was added dropwise. The mixture was stirred at this temperature for 30 minutes. A mixture of 1,2-dibenzyldisulfane (618 mg, 2.5 mmol) and tetrahydrofuran (2 mL) was added dropwise at -78°C. The mixture was stirred at the same temperature for 30 minutes and then warmed to room temperature and stirred for 1 hour.

After the disappearance of the starting material as monitored by LCMS, the reaction mixture was poured into a mixture of ethyl acetate and saturated ammonium chloride. The mixture was extracted, washed with saturated brine (100 mL), dried over anhydrous magnesium sulfate, filtered, and concentrated under reduced pressure. The resulting mixture was purified by silica gel column chromatography to afford 220 mg of 7-(benzylthio)-8-methoxy-3,4-dihydro-2H-benzo[b][1,4]dioxepine.

MS (ESI) m/z: 303.2 [M+H]⁺.

**Step E:** Under nitrogen protection, 7-(benzylthio)-8-methoxy-3,4-dihydro-2H-benzo[b][1,4] dioxepine (220 mg, 0.73 mmol) and acetic acid (350 mg, 5.8 mmol) were dissolved in water (0.7 mL) and acetonitrile (8 mL). The solution was cooled to 0°C, and 1,3-dichloro-5,5-dimethylhydantoin (287 mg, 1.46 mmol) was added. The system was stirred at 0°C for 1 hour.

After the disappearance of the starting material as monitored by LCMS, the reaction mixture was diluted with ethyl acetate (100 mL). The mixture was washed with water (100 mL × 3) and saturated brine (100 mL), dried over anhydrous magnesium sulfate, filtered, and concentrated under reduced pressure. The resulting mixture was purified by silica gel column chromatography to afford 170 mg of 8-methoxy-3,4-dihydro-2H-benzo[b][1,4]dioxepine-7-sulfonyl chloride.

¹H NMR (500 MHz, DMSO-*d₆*): δ 7.26 (s, 1H), 6.55 (s, 1H), 4.12 - 4.08 (m, 2H), 4.04 - 4.00 (m, 2H), 3.67 (s, 3H), 2.10 - 2.03 (m, 2H).

**Step F:** Under nitrogen protection, 8-methoxy-3,4-dihydro-2H-benzo[b][1,4]dioxepine-7-sulfonyl chloride (170 mg, 0.61 mmol) and 6-((1H-pyrazol-1-yl)methyl)-4-methoxybenzo[d] isoxazol-3-amine (74.6 mg, 0.31 mmol) were dissolved in pyridine (2 mL). The system was stirred under microwave irradiation at 100°C for 3 hours.

After the disappearance of the starting material as monitored by LCMS, the reaction mixture was added to water (50 mL) and diluted with ethyl acetate (50 mL). The mixture was washed with saturated brine (30 mL), dried over anhydrous sodium sulfate, filtered, and concentrated under reduced pressure. The resulting mixture was purified by reverse-phase preparative chromatography to afford 47 mg of N-(6-((1H-pyrazol-1-yl)methyl)-4-methoxybenzo[d]isoxazol-3-yl)-8-methoxy-3,4-dihydro-2H-benzo[b][1,4]dioxepine-7-sulfonami de.

MS (ESI) m/z: 487.2 [M+H]⁺.

¹H NMR (400 MHz, DMSO-*d₆*): δ 10.10 (s, 1H), 7.88 (d, *J =* 2.2 Hz, 1H), 7.50 (d, *J =* 1.6 Hz, 1H), 7.34 (s, 1H), 6.84 (s, 1H), 6.75 (d, *J =* 4.0 Hz, 2H), 6.30 (t, *J* = 2.0 Hz, 1H), 5.44 (s, 2H), 4.23 (t, *J* = 5.2 Hz, 2H), 4.10 (t, *J* = 5.4 Hz, 2H), 3.84 (s, 3H), 3.69 (s, 3H), 2.16 - 2.07 (m, 2H).

### Example 8:

### N-(6-((1H-Pyrazol-1-yl)methyl)-4-methoxybenzo|d]isoxazol-3-yl)-7-methoxy-2,3-dihyd ro-5H-benzo[e][1,4]dioxepine-8-sulfonamide

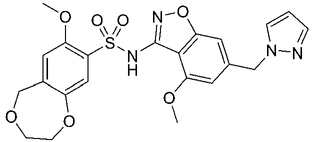

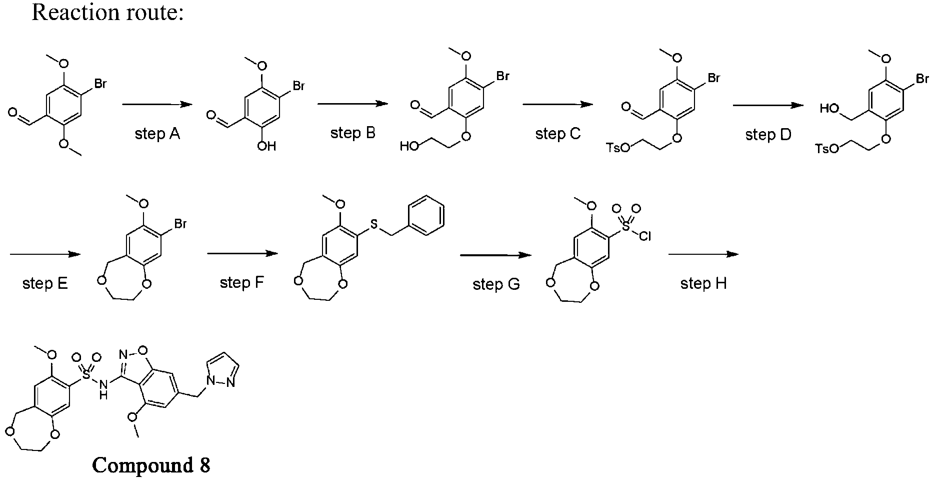

### Operating steps:

**Step A:** Under nitrogen protection, 4-bromo-2,5-dimethoxybenzaldehyde (10.0 g, 40.80 mmol) and anhydrous zinc chloride (33.36 g, 244.83 mmol) were dissolved in nitromethane (100 mL). Boron trichloride (244.83 mL, 244.83 mmol) was slowly added dropwise at 0°C. The reaction mixture was slowly warmed to 25°C and stirred for 8 hours.

After LCMS indicated completion of the reaction, methanol (200 mL) and water (150 mL) were slowly added dropwise at 0°C. After removal of most of the solvent, the residue was extracted with dichloromethane (100 mL × 3). The combined organic phases were washed with saturated sodium bicarbonate solution (100 mL) and saturated brine (100 mL), dried over anhydrous sodium sulfate, filtered, and concentrated. The resulting residue was purified by column chromatography to afford 3.1 g of the target product, 4-bromo-2-hydroxy-5-methoxybenzaldehyde.

MS (ESI) m/z: 231.0, 233.0 [M+H]⁺.

¹H NMR (500 MHz, CDCl₃): δ 10.73 (s, 1H), 9.85 (s, 1H), 7.28 (s, 1H), 6.99 (s, 1H), 3.91 (s, 3H).

**Step B:** Under nitrogen protection, potassium carbonate (3.70 g, 26.84 mmol) was added to a solution of 4-bromo-2-hydroxy-5-methoxybenzaldehyde (3.1 g, 13.42 mmol) and 2-bromoethan-1-ol (2.01 g, 16.10 mmol) in acetonitrile (25 mL). The reaction system was stirred at 80°C for 16 hours.

After the disappearance of most of the starting materials as monitored by LCMS, the reaction mixture was added to ethyl acetate (30 mL) and filtered. The filter cake was washed with ethyl acetate (50 mL), and the filtrate was concentrated under reduced pressure. The resulting residue was purified by silica gel column chromatography to afford 2.09 g of the target product, 4-bromo-2-(2-hydroxyethoxy)-5-methoxybenzaldehyde.

MS (ESI) m/z: 275.0, 277.0 [M+H]⁺.

**Step C:** Under nitrogen protection, triethylamine (3.2 mL, 22.79 mmol) was slowly added dropwise to a solution of 4-bromo-2-(2-hydroxyethoxy)-5-methoxybenzaldehyde (2.09 g, 7.60 mmol) in dichloromethane (20 mL) at 0°C. After stirring at 0°C for 15 minutes, p-toluenesulfonyl chloride (2.17 g, 11.40 mmol) was added. The reaction system was slowly warmed to 25°C and stirred for 16 hours.

After LCMS indicated completion of the reaction, the reaction mixture was quenched with saturated sodium bicarbonate solution and extracted with dichloromethane (50 mL × 3). The combined organic phases were washed with saturated brine (100 mL), dried over anhydrous sodium sulfate, filtered, and concentrated. The resulting residue was purified by column chromatography to afford 2.2 g of the target product, 2-(5-bromo-2-formyl-4-methoxyphenoxy) ethyl 4-methylbenzenesulfonate.

MS (ESI) m/z: 429.0, 431.0 [M+H]⁺.

**Step D:** Under nitrogen protection, lithium aluminum hydride (7.7 mL, 7.69 mmol) was slowly added dropwise to a solution of 2-(5-bromo-2-formyl-4-methoxyphenoxy)ethyl 4-methylbenzenesulfonate (2.2 g, 5.12 mmol) in anhydrous tetrahydrofuran (20 mL) under ice-bath conditions. The reaction mixture was stirred at 0°C for 0.5 hours.

After LCMS indicated completion of the reaction, water (8 mL), 10 wt% NaOH aqueous solution (8 mL), and water (24 mL) were slowly added dropwise to the reaction mixture. The mixture was extracted with ethyl acetate (50 mL × 3). The combined organic phases were washed with saturated sodium chloride solution (50 mL), dried over anhydrous sodium sulfate, filtered, and concentrated. The resulting residue was purified by column chromatography to afford 1.2 g of the target product, 2-(5-bromo-2-(hydroxymethyl)-4-methoxyphenoxy)ethyl 4-methylbenzenesulfonate.

MS (ESI) m/z: 413.0, 415.0 [M+H-18]⁺.

**Step E:** Under nitrogen protection, a solution of 2-(5-bromo-2-(hydroxymethyl)-4-methoxyphenoxy)ethyl 4-methylbenzenesulfonate (1.8 g, 4.17 mmol) in anhydrous N,N-dimethylformamide (5 mL) was slowly added dropwise to a suspension of sodium hydride (200 mg, 5.01 mmol, 60 wt% in mineral oil) and potassium iodide (208 mg, 1.25 mmol) in anhydrous N,N-dimethylformamide (10 mL). The reaction system was stirred at 80°C for 1 hour.

After the completion of the reaction as monitored by LCMS, the reaction mixture was cooled to room temperature and water (30 mL) was slowly added. The mixture was extracted with ethyl acetate (50 mL × 3). The combined organic phases were washed with saturated sodium chloride solution (100 mL × 3), dried over anhydrous sodium sulfate, filtered, and concentrated under reduced pressure. The resulting residue was purified by column chromatography to afford 520 mg of the target product, 8-bromo-7-methoxy-2,3-dihydro-5H-benzo[e][1,4]dioxepine.

MS (ESI) m/z: 259.0, 261.0 [M+H]⁺.

¹H NMR (400 MHz, CDCl₃): δ 7.28 (s, 1H), 6.71 (s, 1H), 4.63 (s, 2H), 4.06 - 4.02 (m, 2H), 3.99 - 3.98 (m, 2H), 3.86 (s, 3H).

**Step F:** 8-Bromo-7-methoxy-2,3-dihydro-5H-benzo[e][1,4]dioxepine (350 mg, 1.36 mmol) was dissolved in tetrahydrofuran (4 mL). The system was cooled to -78°C, and n-butyllithium (0.6 mL, 1.49 mmol, 2.5 M) was added dropwise at this temperature. After the addition was complete, the mixture was stirred at this temperature for 40 minutes. Then, a solution of dibenzyldisulfane (401 mg, 1.63 mmol) in tetrahydrofuran (1 mL) was added dropwise to the above reaction mixture. The resulting mixture was slowly warmed to -30°C under argon protection. The entire process took 1.5 hours.

After the disappearance of the starting material as monitored by LCMS, the reaction mixture was poured into ice-cold ammonium chloride solution (20 mL) and extracted with ethyl acetate (20 mL × 3). The organic phase was washed with brine (20 mL × 3) and dried over anhydrous sodium sulfate. After filtration and concentration, the crude product was purified by normal-phase chromatography to afford 110 mg of 8-(benzylthio)-7-methoxy-2,3-dihydro-5H-benzo[e][1,4]dioxepine.

MS (ESI) m/z: 303.1 [M+H]⁺.

¹H NMR (400 MHz, DMSO-*d₆*): δ 7.33 - 7.21 (m, 5H), 6.95 (s, 1H), 6.64 (s, 1H), 4.62 (s, 2H), 4.11 (s, 2H), 4.08 - 3.98 (m, 4H), 3.79 (s, 3H).

**Step G:** 8-(Benzylthio)-7-methoxy-2,3-dihydro-5H-benzo[e][1,4]dioxepine (110 mg, 0.36 mmol) was dissolved in acetonitrile/water (2.2 mL / 0.2 mL). The system was cooled to 0°C, and acetic acid (160 mg) was added to the above reaction mixture. Finally, 1,3-dichloro-5,5-dimethylhydantoin (142 mg, 0.72 mmol) was added to the reaction mixture. The resulting mixture was stirred at 0°C for 1 hour.

After the disappearance of the starting material as monitored by LCMS, water (10 mL) was added. The aqueous layer was washed with ethyl acetate (10 mL × 2), then washed with brine (20 mL × 2), dried over anhydrous sodium sulfate, filtered, and concentrated to afford the crude product. The crude product was purified by normal-phase chromatography to afford 60 mg of 7-methoxy-2,3-dihydro-5H-benzo[e][1,4]dioxepine-8-sulfonyl chloride.

MS (ESI) m/z: 301.0 [M+Na]⁺.

**Step H:** 7-Methoxy-2,3-dihydro-5H-benzo[e][1,4]dioxepine-8-sulfonyl chloride (60 mg, 0.22 mmol) and 6-((1H-pyrazol-1-yl)methyl)-4-methoxybenzo[d]isoxazol-3-amine (27 mg, 0.11 mmol) were dissolved in pyridine (1 mL). After nitrogen displacement, the mixture was heated to 100°C under microwave irradiation for 4 hours.

After the disappearance of the starting material as monitored by LCMS, the reaction mixture was cooled to room temperature and diluted with ethyl acetate (10 mL) and water (10 mL). The organic phase was washed with brine (20 mL), dried over sodium sulfate, filtered, and concentrated. The crude product was purified by reverse-phase preparative chromatography to afford 10 mg of N-(6-((1H-pyrazol-1-yl)methyl)-4-methoxybenzo[d]isoxazol-3-yl)-7-methoxy-2,3-dihydro-5H-benzo[e][1,4]dioxepine-8-sulfonamide.

MS (ESI) m/z: 487.2 [M+H]⁺.

¹H NMR (400 MHz, DMSO-*d₆*): δ 10.04 (s, 1H), 7.88 (d, *J* = 2.2 Hz, 1H), 7.71 (s, 1H), 7.49 (s, 1H), 6.83 (d, *J* = 4.6 Hz, 2H), 6.74 (s, 1H), 6.30 (t, *J* = 2.0 Hz, 1H), 5.44 (s, 2H), 4.63 (s, 2H), 4.14 (d, *J =* 4.8 Hz, 2H), 3.91 (d, *J* = 4.8 Hz, 2H), 3.84 (s, 3H), 3.75 (s, 3H).

### Example 9:

### N-(6-((1H-Pyrazol-1-yl)methyl)-4-methoxybenzo[d]isoxazol-3-yl)-8-methoxy-2,3-dihyd ro-5H-benzo[e][1,4]dioxepine-7-sulfonamide

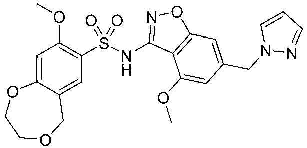

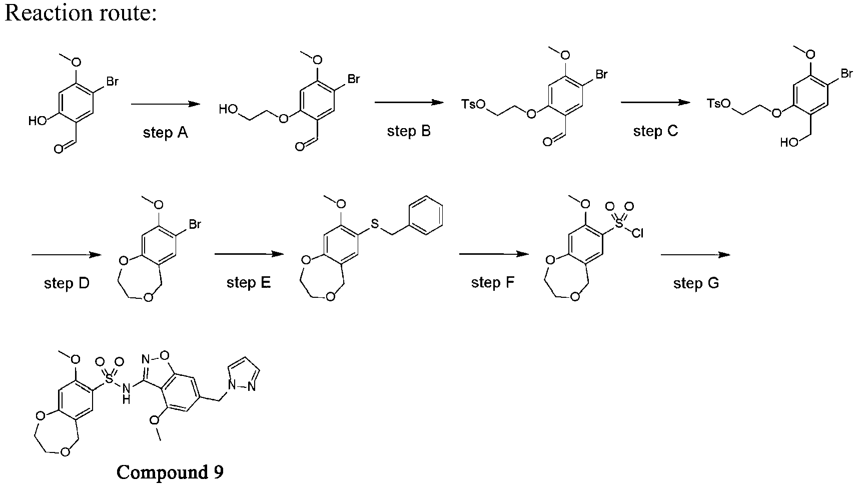

### Operating steps:

**Step A:** 5-Bromo-2-hydroxy-4-methoxybenzaldehyde (6 g, 25.97 mmol) was dissolved in N,N-dimethylformamide (50 mL). 2-Bromoethan-1-ol (4.22 g, 33.76 mmol) and potassium carbonate (10.75 g, 77.92 mmol) were added to the reaction mixture, followed by potassium iodide (0.2 g). The resulting mixture was heated to 80 °C and stirred for 48 hours.

After the disappearance of the starting material as monitored by LCMS, the reaction mixture was cooled to 0 °C, quenched with ammonium chloride solution (250 mL), and extracted with ethyl acetate (50 mL × 3). The organic phase was washed with water (50 mL) and brine (50 mL), dried over anhydrous sodium sulfate, filtered, and concentrated. The crude product was purified by normal-phase chromatography to afford 4.5 g of 5-bromo-2-(2-hydroxyethoxy)-4-methoxybenzaldehyde.

MS (ESI) m/z: 275.0 [M+H]⁺.

**Step B:** 5-Bromo-2-(2-hydroxyethoxy)-4-methoxybenzaldehyde (4.5 g, 16.3 mmol) was dissolved in dichloromethane (50 mL). Triethylamine (11.31 mL, 81.52 mmol) and p-toluenesulfonyl chloride (3.73 g, 19.56 mmol) were added to the above reaction mixture. The resulting mixture was stirred at room temperature for 16 hours.

After the disappearance of the starting material as monitored by LCMS, the reaction was quenched with ammonium chloride solution (250 mL) and extracted with ethyl acetate (50 mL × 3). The organic phase was washed with water (50 mL) and brine (50 mL), dried over anhydrous sodium sulfate, filtered, and concentrated. The crude product was purified by normal-phase chromatography to afford 4.8 g of 2-(4-bromo-2-formyl-5-methoxyphenoxy)ethyl 4-methylbenzenesulfonate.

MS (ESI) m/z: 429.0 [M+H]⁺.

¹H NMR (400 MHz, DMSO-*d₆*): δ 9.81 (s, 1H), 7.78 (d, *J* = 6.4 Hz, 2H), 7.74 (s, 1H), 7.42 (d, *J* = 6.8 Hz, 2H), 6.81 (s, 1H), 4.46 - 4.43 (m, 4H), 3.96 (s, 3H), 2.40 (s, 3H).

**Step C:** 2-(4-Bromo-2-formyl-5-methoxyphenoxy)ethyl 4-methylbenzenesulfonate (4.8 g, 11.21 mmol) was dissolved in tetrahydrofuran (50 mL). Lithium aluminum hydride (16.82 mL, 16.82 mmol) was added dropwise to the above reaction mixture at 0 °C. The resulting mixture was stirred at 0 °C for 1 hour.

After the disappearance of the starting material as monitored by LCMS, the mixture was diluted with tetrahydrofuran (50 mL) and quenched with sodium sulfate decahydrate (approximately 30 g). The mixture was stirred at room temperature for 30 minutes, filtered, and the filtrate was concentrated to afford the crude product. The crude product was purified by normal-phase column chromatography to afford 2.7 g of 2-(4-bromo-2-(hydroxymethyl)-5-methoxyphenoxy)ethyl 4-methylbenzenesulfonate.

MS (ESI) m/z: 453.1 [M+Na]⁺.

¹H NMR (400 MHz, DMSO-*d₆*): δ 7.79 (d, *J* = 8.2 Hz, 2H), 7.46 (d, *J* = 8.2 Hz, 2H), 7.43 (s, 1H), 6.65 (s, 1H), 4.36 - 4.31 (m, 2H), 4.29 (s, 2H), 4.26 - 4.22 (m, 2H), 3.81 (s, 3H), 2.41 (s, 3H).

**Step D:** Sodium hydride (0.31 g, 7.53 mmol, 60 wt%) was dissolved in N,N-dimethylformamide (20 mL). A solution of 2-(4-bromo-2-(hydroxymethyl)-5-methoxy phenoxy)ethyl 4-methylbenzenesulfonate (2.7 g, 6.27 mmol) in N,N- dimethylformamide (10 mL) was added dropwise to the above solution, followed by the addition of potassium iodide (0.1 g). The resulting mixture was heated to 80 °C and stirred for 2 hours.

After the completion of the reaction as monitored by LCMS, the reaction mixture was cooled to room temperature, diluted with water (150 mL), and extracted with ethyl acetate (100 mL × 3). The organic phase was washed with brine (100 mL × 3) and dried over anhydrous sodium sulfate. After filtration and concentration, the crude product was purified by normal-phase column chromatography to afford 1.3 g of 7-bromo-8-methoxy-2,3-dihydro-5H-benzo[e][1,4]dioxepine.

MS (ESI) m/z: 259.0 [M+H]⁺.

¹H NMR (400 MHz, DMSO-*d₆*): δ 7.49 (s, 1H), 6.80 (s, 1H), 4.55 (s, 2H), 4.05 - 4.04 (m, 2H), 3.90 - 3.88 (m, 2H), 3.81 (s, 3H).

**Step E:** 7-Bromo-8-methoxy-2,3-dihydro-5H-benzo[e][1,4]dioxepine (1.3 g, 5.04 mmol) was dissolved in tetrahydrofuran (20 mL). The system was cooled to -78 °C, and n-butyllithium (2.22 mL, 5.54 mmol, 2.5 M) was added dropwise at this temperature. After the addition was complete, the mixture was stirred at this temperature for 40 minutes. Then, a solution of dibenzyldisulfane (1.49 g, 6.05 mmol) in tetrahydrofuran (5 mL) was added dropwise to the above reaction mixture. The resulting mixture was slowly warmed to -30 °C under argon protection. The entire process took 1.5 hours.

After the disappearance of the starting material as monitored by LCMS, the reaction mixture was poured into ice-cold ammonium chloride solution (50 mL) and extracted with ethyl acetate (100 mL × 3). The organic phase was washed with brine (100 mL × 3) and dried over anhydrous sodium sulfate. After filtration and concentration, the crude product was purified by normal-phase chromatography to afford 1.0 g of 7-(benzylthio)-8-methoxy-2,3-dihydro-5H-benzo[e][1,4]dioxepine.

MS (ESI) m/z: 303.0 [M+H]⁺.

¹H NMR (400 MHz, DMSO-*d₆*): δ 7.29 - 7.21 (m, 5H), 7.16 (s, 1H), 6.69 (s, 1H), 4.51 (s, 2H), 4.08 (s, 2H), 4.02 - 4.00 (m, 2H), 3.88 - 3.78 (m, 2H), 3.79 (s, 3H).

**Step F:** 7-(Benzylthio)-8-methoxy-2,3-dihydro-5H-benzo[e][1,4]dioxepine (400 mg, 1.32 mmol) was dissolved in acetonitrile/water (8.8 mL / 0.8 mL). The system was cooled to 0 °C, and acetic acid (640 mg) was added to the above reaction mixture. Finally, 1,3-dichloro-5,5-dimethylhydantoin (520 mg, 2.64 mmol) was added to the reaction mixture. The resulting mixture was stirred at 0 °C for 1 hour.

After the disappearance of the starting material as monitored by LCMS, water (50 mL) was added. The aqueous layer was washed with ethyl acetate (100 mL × 2), then washed with brine (100 mL), dried over anhydrous sodium sulfate, filtered, and concentrated to afford the crude product. The crude product was purified twice by normal-phase chromatography to afford 160 mg of 8-methoxy-2,3-dihydro-5H-benzo[e][1,4]dioxepine-7-sulfonyl chloride.

MS (ESI) m/z: 301.1 [M+Na]⁺.

¹H NMR (400 MHz, DMSO-*d₆*): δ 7.78 (s, 1H), 6.78 (s, 1H), 4.66 (s, 2H), 4.23 - 4.21 (m, 2H), 4.15 - 4.02 (m, 5H), 3.79 (s, 3H).

**Step G:** 8-Methoxy-2,3-dihydro-5H-benzo[e][1,4]dioxepine-7-sulfonyl chloride (160 mg, 0.58 mmol) and 6-((1H-pyrazol-1-yl)methyl)-4-methoxybenzo[d]isoxazol-3-amine (70 mg, 0.29 mmol) were dissolved in pyridine (2 mL). After nitrogen displacement, the mixture was heated to 100 °C under microwave irradiation for 4 hours.

After the disappearance of the starting material as monitored by LCMS, the reaction mixture was cooled to room temperature and diluted with ethyl acetate (10 mL) and water (10 mL). The organic phase was washed with brine (50 mL), dried over sodium sulfate, filtered, and concentrated. The crude product was purified by reverse-phase preparative chromatography to afford 30 mg of N-(6-((1H-pyrazol-1-yl)methyl)-4-methoxybenzo[d]isoxazol-3-yl)-8-methoxy-2,3-dihydro-5H-benzo[e][1,4]dioxepine-7-sulfonamide.

MS (ESI) m/z: 487.2 [M+H]⁺.

¹H NMR (400 MHz, DMSO-*d₆*): δ 10.04 (s, 1H), 7.88 (d, *J =* 2.2 Hz, 1H), 7.71 (s, 1H), 7.49 (s, 1H), 6.83 (d, *J* = 4.6 Hz, 2H), 6.74 (s, 1H), 6.30 (t, *J* = 2.0 Hz, 1H), 5.44 (s, 2H), 4.63 (s, 2H), 4.14 (d, *J* = 4.8 Hz, 2H), 3.91 (d, *J* = 4.8 Hz, 2H), 3.84 (s, 3H), 3.75 (s, 3H).

### Example 10:

### N-(6-((1H-Pyrazol-1-yl)methyl)-4-methoxybenzo[d]isoxazol-3-yl)-8-methoxy-2,3-dihyd ro-5H-benzo [e] [1,4] dioxepine-9-sulfonamide

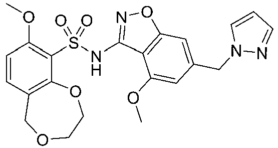

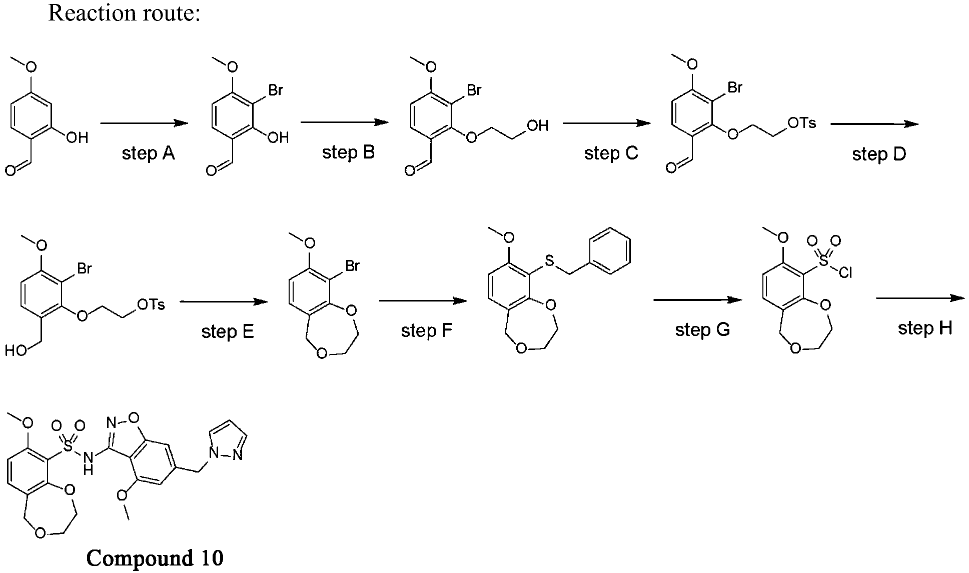

### Operating steps:

**Step A:** Under nitrogen protection, 2-hydroxy-4-methoxybenzaldehyde (5 g, 32.9 mmol) and aluminum trichloride (4.39 g, 32.9 mmol) were dissolved in dichloromethane (50 mL). After cooling to -20°C, bromine (5.26 g, 32.9 mmol) was added dropwise. The system was stirred at room temperature for 16 hours.

After the complete reaction of the starting material as monitored by LCMS, the reaction mixture was quenched by adding 1M hydrochloric acid (100 mL). Dichloromethane (100 mL) was added for extraction. The organic phase was washed with saturated sodium sulfite (100 mL), water (100 mL), and saturated brine (100 mL × 3), dried over anhydrous magnesium sulfate, filtered, and the filtrate was concentrated under reduced pressure. The resulting mixture was purified by silica gel column chromatography to afford 3.26 g of 3-bromo-2-hydroxy-4-methoxybenzaldehyde.

MS (ESI) M/Z: 231.0, 233.0 [M+H]⁺.

¹H NMR (400 MHz, DMSO-*d₆*): δ 11.69 (s, 1H), 9.88 (s, 1H), 7.82 (d, *J =* 8.8 Hz, 1H), 6.91 (d, *J =* 8.8 Hz, 1H), 3.97 (s, 3H).

**Step B:** Under nitrogen protection, potassium carbonate (12.6 g, 91.3 mmol) and potassium iodide (0.5 g, 3.0 mmol) were added to a solution of 3-bromo-2-hydroxy-4-methoxybenzaldehyde (7 g, 30.4 mmol) and 2-bromoethan-1-ol (5.7 g, 45.6 mmol) in acetonitrile (70 mL). The reaction system was stirred at 80°C for 16 hours.

After the disappearance of most of the starting materials as monitored by LCMS, the reaction mixture was added to ethyl acetate (100 mL) and filtered. The filter cake was washed with ethyl acetate (200 mL), and the filtrate was concentrated under reduced pressure. The resulting residue was purified by silica gel column chromatography to afford 6 g of the target product, 3-bromo-2-(2-hydroxyethoxy)-4-methoxybenzaldehyde.

MS (ESI) M/Z: 275.0, 277.0 [M+H]⁺.

**Step** C: Under nitrogen protection, 3-bromo-2-(2-hydroxyethoxy)-4-methoxybenzaldehyde (6 g, 21.9 mmol) and triethylamine (6.6 g, 65.7 mmol) were dissolved in dichloromethane (60 mL). After cooling to 0°C, p-toluenesulfonyl chloride (6.2 g, 32.8 mmol) was added dropwise slowly. The system was stirred at room temperature for 16 hours.

After the complete reaction of the starting material as monitored by LCMS, the reaction mixture was quenched by adding water (200 mL). Dichloromethane (200 mL) was added for extraction. The organic phase was washed with saturated brine (200 mL), dried over anhydrous magnesium sulfate, filtered, and the filtrate was concentrated under reduced pressure. The resulting mixture was purified by silica gel column chromatography to afford 5 g of 2-(2-bromo-6-formyl-3-methoxyphenoxy)ethyl 4-methylbenzenesulfonate.

MS (ESI) M/Z: 427.0, 429.0 [M+H]⁺.

**Step D:** Under nitrogen protection, a solution of 2-(2-bromo-6-formyl-3-methoxyphenoxy) ethyl 4-methylbenzenesulfonate (5 g, 11.7 mmol) in anhydrous tetrahydrofuran (50 mL) was cooled in an ice bath. Lithium aluminum hydride (14 mL, 14 mmol) was added dropwise slowly. The reaction mixture was stirred at 0°C for half an hour.

After LCMS showed the reaction was complete, sodium thiosulfate decahydrate (50 g) was added and stirred for 30 minutes until clear. The mixture was filtered, and the filter cake was washed with ethyl acetate (100 mL × 3). The filtrate was concentrated, and the resulting residue was purified by column chromatography to afford 3 g of the target product, 2-(2-bromo-6-(hydroxymethyl)-3-methoxyphenoxy)ethyl 4-methylbenzenesulfonate.

MS (ESI) M/Z: 413.0, 415.0 [M-18+H]⁺.

**Step E:** Under nitrogen protection, a solution of 2-(2-bromo-6-(hydroxymethyl)-3-methoxyphenoxy)ethyl 4-methylbenzenesulfonate (3 g, 7.0 mmol) in anhydrous N,N-dimethylformamide (5 mL) was added dropwise slowly to a suspension of sodium hydride (335 mg, 8.4 mmol, 60wt%) and potassium iodide (116 mg, 0.7 mmol) in anhydrous N,N-dimethylformamide (20 mL). The reaction system was stirred at 70°C for 1 hour.

After the completion of the reaction as monitored by LCMS, the reaction mixture was cooled to room temperature and water (100 mL) was added slowly. The mixture was extracted with ethyl acetate (100 mL × 3). The combined organic phases were washed with saturated sodium chloride solution (100 mL × 3), dried over anhydrous sodium sulfate, filtered, and the filtrate was concentrated under reduced pressure. The resulting residue was purified by column chromatography to afford 1 g of the target product, 9-bromo-8-methoxy-2,3-dihydro-5H-benzo[e] [1,4]dioxepine.

MS (ESI) M/Z: 259.0, 261.0 [M+H]⁺.

**Step F:** Under nitrogen protection, 9-bromo-8-methoxy-2,3-dihydro-5H-benzo[e][1,4] dioxepine (630 mg, 2.4 mmol) was dissolved in tetrahydrofuran (5 mL) and cooled to -78°C. tert-Butyllithium (2.1 mL, 2.68 mmol) was added dropwise, and the mixture was stirred at this temperature for 30 minutes. A mixture of 1,2-dibenzyldisulfane (720 mg, 2.9 mmol) and tetrahydrofuran (5 mL) was added dropwise at -78°C, stirred at the same temperature for 30 minutes, and then warmed to room temperature and stirred for 1 hour.

After the complete reaction of the starting material as monitored by LCMS, the reaction mixture was poured into a mixture of ethyl acetate and saturated ammonium chloride solution, extracted, washed with saturated brine (100 mL), dried over anhydrous magnesium sulfate, filtered, and the filtrate was concentrated under reduced pressure. The resulting mixture was purified by silica gel column chromatography to afford 300 mg of 9-(benzylthio)-8-methoxy-2,3-dihydro-5H-benzo[e][1,4]dioxepine.

MS (ESI) M/Z: 303.1 [M+H]⁺.

¹H NMR (400 MHz, DMSO-*d₆*): δ 7.26 - 7.08 (m, 6H), 6.69 (d, *J =* 8.4 Hz, 1H), 4.49 (s, 2H), 4.01 (s, 2H), 3.81 (s, 5H), 3.74 - 3.64 (m, 2H).

**Step G:** Under nitrogen protection, 9-(benzylthio)-8-methoxy-2,3-dihydro-5H-benzo[e][1,4] dioxepine (150 mg, 0.44 mmol) and acetic acid (213 mg, 3.55 mmol) were dissolved in water (0.44 mL) and acetonitrile (4.84 mL). The mixture was cooled to 0°C, and 1,3-dichloro-5,5-dimethylhydantoin (174.8 mg, 0.89 mmol) was added. The system was stirred at 0°C for 1 hour.

After the complete reaction of the starting material as monitored by LCMS, the reaction mixture was diluted with ethyl acetate (100 mL), washed with water (100 mL × 3) and saturated brine (100 mL), dried over anhydrous magnesium sulfate, filtered, and the filtrate was concentrated under reduced pressure. The resulting mixture was purified by silica gel column chromatography to afford 110 mg of 8-methoxy-2,3-dihydro-5H-benzo[e][1,4]dioxepine-9-sulfonyl chloride.

MS (ESI) M/Z: 301.0 [M+Na]⁺.

**Step H:** Under nitrogen protection, 8-methoxy-2,3-dihydro-5H-benzo[e][1,4]dioxepine-9-sulfonyl chloride (110 mg, 0.39 mmol) and 6-((1H-pyrazol-1-yl)methyl)-4-methoxybenzo[d] isoxazol-3-amine (48.3 mg, 0.20 mmol) were dissolved in pyridine (2 mL). The system was stirred under microwave conditions at 100 °C for 3 hours.

After the complete reaction of the starting material as monitored by LCMS, the reaction mixture was added to water (50 mL), diluted with ethyl acetate (50 mL), washed with saturated brine (30 mL), dried over anhydrous sodium sulfate, filtered, and the filtrate was concentrated under reduced pressure. The resulting mixture was purified by reverse-phase preparative chromatography to afford 13.6 mg of N-(6-((1H-pyrazol-1-yl)methyl)-4-methoxybenzo[d] isoxazol-3-yl)-8-methoxy-2,3-dihydro-5H-benzo[e][1,4]dioxepine-9-sulfonamide.

MS (ESI) M/Z: 487.2 [M+H]⁺.

¹H NMR (400 MHz, DMSO-*d₆*): δ 10.01 (s, 1H), 7.88 (d, *J* = 2.2 Hz, 1H), 7.49 (t, *J* = 5.6 Hz, 2H), 6.85 (d, *J =* 8.8 Hz, 2H), 6.75 (s, 1H), 6.30 (t, *J* = 2.0 Hz, 1H), 5.44 (s, 2H), 4.57 (s, 2H), 4.06 - 3.99 (m, 2H), 3.93 - 3.87 (m, 2H), 3.82 (s, 3H), 3.74 (s, 3H).

### Example 11:

### N-(6-((1H-Pyrazol-1-yl)methyl)-4-methoxybenzo[d]isoxazol-3-yl)-3,3-difluoro-7-metho xy-3,4-dihydro-2H-benzo[b] [1,4]dioxepine-6-sulfonamide

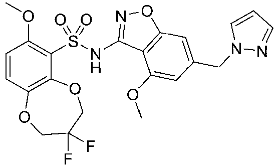

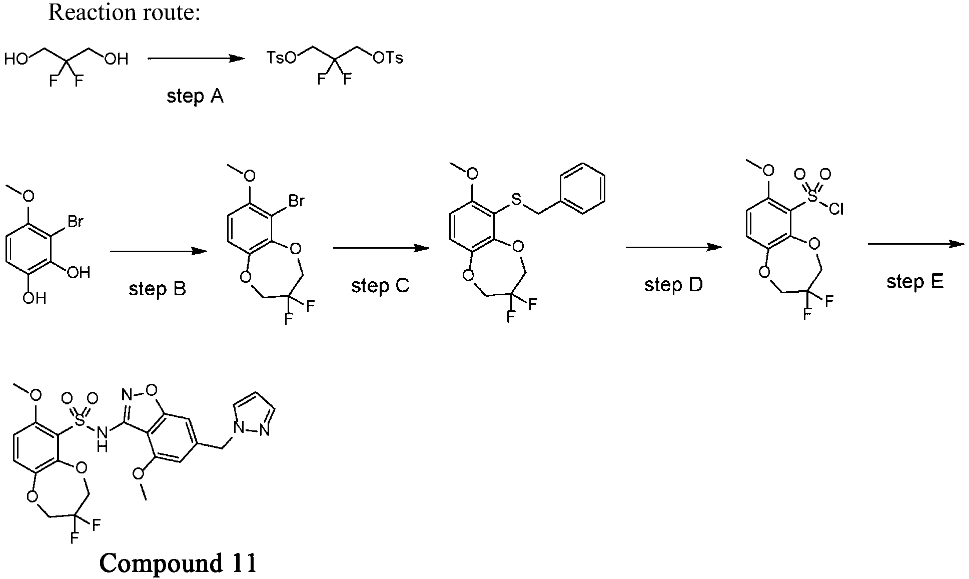

### Operating steps:

**Step** A: Under nitrogen protection, 2,2-difluoropropane-1,3-diol (1 g, 8.9 mmol) and triethylamine (6.2 mL, 44.6 mmol) were dissolved in dichloromethane (10 mL). After cooling to 0°C, p-toluenesulfonyl chloride (5.1 g, 26.8 mmol) was added dropwise slowly. The system was stirred at room temperature for 16 hours.

After the complete reaction of the starting material as monitored by LCMS, the reaction mixture was quenched by adding water (100 mL). Dichloromethane (100 mL) was added for extraction. The organic phase was washed with saturated brine (100 mL), dried over anhydrous magnesium sulfate, filtered, and the filtrate was concentrated under reduced pressure. The resulting mixture was purified by silica gel column chromatography to afford 4.39 g of 2,2-difluoropropane-1,3-diyl bis(4-methylbenzenesulfonate).

MS (ESI) M/Z: 421.0 [M+H]⁺.

**Step B:** Under nitrogen protection, 3-bromo-4-methoxybenzene-1,2-diol (7 g, 32.1 mmol), 2,2-difluoropropane-1,3-diyl bis(4-methylbenzenesulfonate) (20.3 g, 48.2 mmol), and potassium carbonate (6.7 g, 48.2 mmol) were dissolved in N,N-dimethylformamide (70 mL). The system was stirred at 120°C for 1 hour.

After the complete reaction of the starting material as monitored by LCMS, the reaction mixture was added to saturated ammonium chloride solution (200 mL), diluted with ethyl acetate (200 mL), washed with water (200 mL) and saturated brine (200 mL), dried over anhydrous magnesium sulfate, filtered, and the filtrate was concentrated under reduced pressure. The resulting mixture was purified by silica gel column chromatography to afford 2.12 g of 6-bromo-3,3-difluoro-7-methoxy-3,4-dihydro-2H-benzo[b][1,4]dioxepine.

MS (ESI) M/Z: 295.0, 297.0 [M+H]⁺.

**Step** C: Under nitrogen protection, 6-bromo-3,3-difluoro-7-methoxy-3,4-dihydro-2H-benzo [b][1,4]dioxepine (1.95 g, 6.6 mmol) was dissolved in tetrahydrofuran (20 mL) and cooled to -78°C. tert-Butyllithium (5.6 mL) was added dropwise, and the mixture was stirred at this temperature for 30 minutes. A mixture of 1,2-dibenzyldisulfane (1.96 g, 8.0 mmol) and tetrahydrofuran (5 mL) was added dropwise at -78°C, stirred at the same temperature for 30 minutes, and then warmed to room temperature and stirred for 1 hour.

After the complete reaction of the starting material as monitored by LCMS, the reaction mixture was poured into a mixture of ethyl acetate and saturated ammonium chloride solution, extracted, washed with saturated brine (100 mL), dried over anhydrous magnesium sulfate, filtered, and the filtrate was concentrated under reduced pressure. The resulting mixture was purified by silica gel column chromatography to afford 650 mg of 6-(benzylthio)-3,3-difluoro-7-methoxy-3,4-dihydro-2H-benzo[b][1,4]dioxepine.

MS (ESI) M/Z: 339.1 [M+H]⁺.

**Step D:** Under nitrogen protection, 6-(benzylthio)-3,3-difluoro-7-methoxy-3,4-dihydro-2H-benzo[b][1,4]dioxepine (150 mg, 0.44 mmol) and acetic acid (213 mg, 3.55 mmol) were dissolved in water (0.44 mL) and acetonitrile (4.84 mL). The mixture was cooled to 0°C, and 1,3-dichloro-5,5-dimethylhydantoin (174.8 mg, 0.89 mmol) was added. The system was stirred at 0°C for 1 hour.

After the complete reaction of the starting material as monitored by LCMS, the reaction mixture was diluted with ethyl acetate (100 mL), washed with water (100 mL × 3) and saturated brine (100 mL), dried over anhydrous magnesium sulfate, filtered, and the filtrate was concentrated under reduced pressure. The resulting mixture was purified by silica gel column chromatography to afford 120 mg of 3,3-difluoro-7-methoxy-3,4-dihydro-2H-benzo[b][1,4] dioxepine-6-sulfonyl chloride.

MS (ESI) M/Z: 337.0 [M+Na]⁺.

**Step E:** Under nitrogen protection, 3,3-difluoro-7-methoxy-3,4-dihydro-2H-benzo[b][1,4] dioxepine-6-sulfonyl chloride (120 mg, 0.38 mmol) and 6-((1H-pyrazol-1-yl)methyl)-4-methoxybenzo[d]isoxazol-3-amine (46.6 mg, 0.19 mmol) were dissolved in pyridine (2 mL). The system was stirred under microwave conditions at 100 °C for 3 hours.

After the complete reaction of the starting material as monitored by LCMS, the reaction mixture was added to water (50 mL), diluted with ethyl acetate (50 mL), washed with saturated brine (30 mL), dried over anhydrous sodium sulfate, filtered, and the filtrate was concentrated under reduced pressure. The resulting mixture was purified by reverse-phase preparative chromatography to afford 15.7 mg of N-(6-((1H-pyrazol-1-yl)methyl)-4-methoxybenzo[d] isoxazol-3-yl)-3,3-difluoro-7-methoxy-3,4-dihydro-2H-benzo[b][1,4]dioxepine-6-sulfonamide.

MS (ESI) M/Z: 523.2 [M+H]⁺.

¹H NMR (400 MHz, DMSO-*d₆*): δ 10.32 (s, 1H), 7.88 (d, *J =* 2.2 Hz, 1H), 7.50 (s, 1H), 7.36 (d, *J* = 9.2 Hz, 1H), 6.92 (d, *J =* 9.2 Hz, 1H), 6.84 (s, 1H), 6.75 (s, 1H), 6.30 (t, *J =* 1.8 Hz, 1H), 5.45 (s, 2H), 4.36 - 4.30 (m, 4H), 3.82 (s, 3H), 3.72 (s, 3H).

### Example 12:

### N-(6-((1H-Pyrazol-1-yl)methyl)-5-fluorobenzo[d]isoxazol-3-yl)-7-methoxy-3,4-dihydro-2H-benzo [b] [1,4] dioxepine-6-sulfonamide

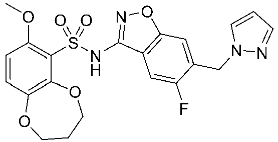

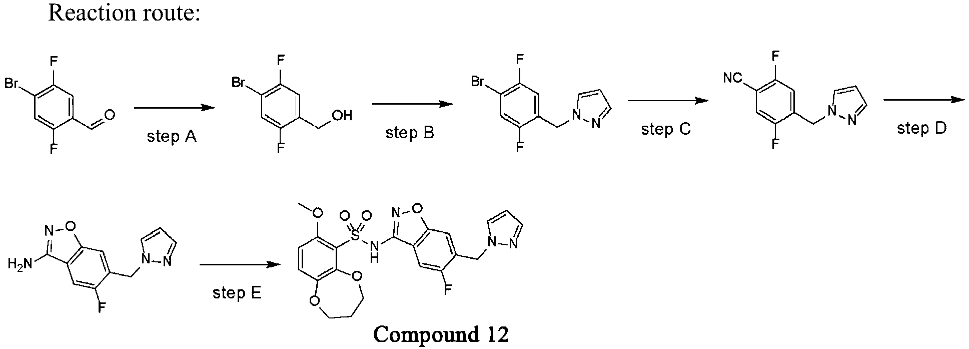

### Operating steps:

**Step A:** 4-Bromo-2,5-difluorobenzaldehyde (5 g, 22.62 mmol) was dissolved in tetrahydrofuran (50 mL). The system was cooled to 0°C, and then lithium aluminum hydride (24.88 mL, 24.88 mmol) was added dropwise to the above solution. The resulting mixture was stirred at the same temperature for 30 minutes.

After LCMS showed the reaction was complete, sodium sulfate decahydrate (20 g) was added and stirred for 30 minutes. The mixture was filtered, and the filter cake was washed twice with tetrahydrofuran (50 mL × 2). The organic phase was dried over anhydrous sodium sulfate, filtered, and concentrated to afford 4.3 g of crude 4-bromo-2,5-difluorobenzyl alcohol.

MS (ESI) M/Z: 205.0 [M-18+H]⁺.

**Step B:** 4-Bromo-2,5-difluorobenzyl alcohol (3.8 g, 17.04 mmol) was dissolved in acetonitrile (50 mL). Then cesium carbonate (8.33 g, 25.56 mmol) and N-methanesulfonylpyrazole (2.74 g, 18.74 mmol) were added to the reaction solution. The resulting mixture was heated to 70°C and stirred for an additional 2 hours.

After the disappearance of the starting material as monitored by LCMS, the reaction mixture was cooled to room temperature and filtered. The filtrate was concentrated to give a crude product, which was purified by normal-phase column chromatography to afford 3.5 g of 1-(4-bromo-2,5-difluorobenzyl)-1H-pyrazole.

MS (ESI) M/Z: 273.0 [M+H]⁺.

¹H NMR (400 MHz, DMSO-*d₆*): δ 7.85 (d, *J* = 2.2 Hz, 1H), 7.77 (dd, *J* = 9.0, 5.8 Hz, 1H), 7.49 (d, *J* = 1.6 Hz, 1H), 7.16 (dd, *J* = 8.8, 6.4 Hz, 1H), 6.29 (t, *J* = 2.0 Hz, 1H), 5.37 (s, 2H).

**Step C:** 1-(4-bromo-2,5-difluorobenzyl)-1H-pyrazole (3.5 g, 12.86 mmol) was dissolved in N,N-dimethylformamide (20 mL). Then copper(I) cyanide (2.3 g, 25.72 mmol) was added to the above reaction solution. The resulting mixture was heated to 150°C and stirred for 7 hours.

After the disappearance of the starting material as monitored by LCMS, the reaction mixture was cooled to room temperature. The mixture was filtered, and the filtrate was extracted with ethyl acetate (50 mL × 3). The combined organic phases were washed with water (30 mL × 2) and brine (50 mL × 2), dried over anhydrous sodium sulfate, filtered, and concentrated to give a crude product. The crude product was purified by normal-phase column chromatography to afford 1.05 g of 4-((1H-pyrazol-1-yl)methyl)-2,5-difluorobenzonitrile.

MS (ESI) M/Z: 220.0 [M+H]⁺.

¹H NMR (400 MHz, CDCl₃): δ 7.61 (d, *J* = 1.6 Hz, 1H), 7.50 (d, *J* = 2.2 Hz, 1H), 7.35 (dd, *J* = 8.4, 5.0 Hz, 1H), 6.80 (dd, *J* = 8.4, 5.6 Hz, 1H), 6.36 (t, *J* = 2.2 Hz, 1H), 5.41 (s, 2H).

**Step D:** 4-((1H-pyrazol-1-yl)methyl)-2,5-difluorobenzonitrile (150 mg, 0.68 mmol) was dissolved in a mixture of N,N-dimethylformamide and water (2.1 mL / 0.3 mL). N-Hydroxyacetamide (154 mg, 2.05 mmol) and potassium carbonate (283 mg, 2.05 mmol) were added to the above reaction solution. The resulting mixture was stirred at 60°C for 2 hours.

After the disappearance of the starting material as monitored by LCMS, the reaction mixture was cooled to room temperature, diluted with ethyl acetate (50 mL), and washed with water (30 mL × 3). The organic phase was then washed with brine (50 mL), dried over sodium sulfate, filtered, and concentrated. The crude product was purified by normal-phase column chromatography to afford 80 mg of 6-((1H-pyrazol-1-yl)methyl)-5-fluorobenzo[d]isoxazol-3-amine.

MS (ESI) M/Z: 233.2 [M+H]⁺.

¹H NMR (400 MHz, DMSO-*d₆*): δ 7.85 (d, *J* = 2.2 Hz, 1H), 7.65 (d, *J* = 9.2 Hz, 1H), 7.49 (d, *J* = 1.6 Hz, 1H), 7.13 (d, *J* = 5.6 Hz, 1H), 6.45 (s, 2H), 6.30 (t, *J =* 2.0 Hz, 1H), 5.48 (s, 2H).

**Step E:** 7-Methoxy-3,4-dihydro-2H-benzo[b][1,4]dioxepine-6-sulfonyl chloride (120 mg, 0.42 mmol) and 6-((1H-pyrazol-1-yl)methyl)-5-fluorobenzo[d]isoxazol-3-amine (50 mg, 0.21 mmol) were dissolved in pyridine (2 mL). After nitrogen displacement, the mixture was heated to 100°C under microwave irradiation and reacted for 4 hours.

After the disappearance of the starting material as monitored by LCMS, the reaction mixture was cooled to room temperature and diluted with ethyl acetate (10 mL) and water (10 mL). The organic phase was washed with brine (50 mL), dried over sodium sulfate, filtered, and concentrated. The crude product was purified by reverse-phase preparative column chromatography to afford 50 mg of N-(6-((1H-pyrazol-1-yl)methyl)-5-fluorobenzo[d]isoxazol-3-yl)-7-methoxy-3,4-dihydro-2H-benzo[b][1,4]dioxepine-6-sulfonamide.

MS (ESI) M/Z: 475.2 [M+H]⁺.

¹H NMR (400 MHz, DMSO-*d₆*): δ 11.66 (s, 1H), 7.88 - 7.84 (m, 2H), 7.49 (d, *J* = 1.6 Hz, 1H), 7.27 (d, *J* = 5.4 Hz, 1H), 7.21 (d, *J* = 9.2 Hz, 1H), 6.76 (d, *J* = 9.2 Hz, 1H), 6.30 (t, *J* = 2.2 Hz, 1H), 5.51 (s, 2H), 4.10 - 4.03 (m, 2H), 4.01 - 3.94 (m, 2H), 3.66 (s, 3H), 2.09 - 1.98 (m, 2H).

**Example 13:**

### N-(6-((1H-pyrazol-1-yl)methyl)-5-(difluoromethoxy)benzo[d]isoxazol-3-yl)-7-methoxy-3,4-dihydro-2H-benzo [b] [1,4] dioxepine-6-sulfonamide

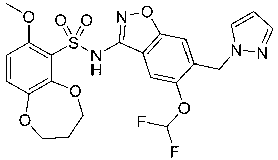

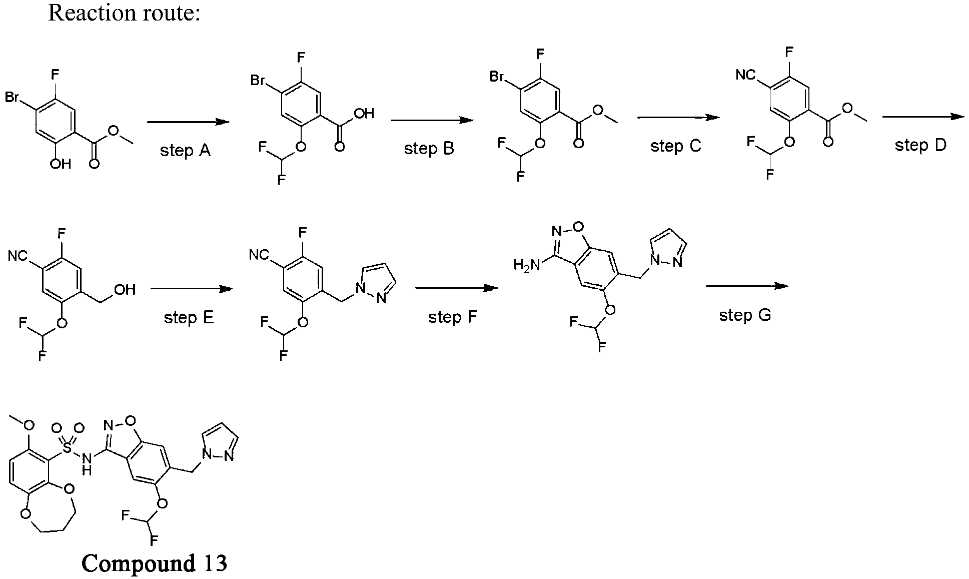

### Operating steps:

**Step A:** At room temperature, methyl 4-bromo-5-fluoro-2-hydroxybenzoate (4 g, 16.1 mmol) was dissolved in N,N-dimethylformamide (40 mL). Potassium hydroxide solution (10 mL, 2M) was added, followed by addition of (bromodifluoromethyl)phosphonic acid diethyl ester (6.4 g, 24.1 mmol) under ice-water bath conditions. The mixture was stirred under the ice-water bath conditions for 2 hours.

After the complete reaction of the starting material as monitored by LCMS, the reaction mixture was added to ammonium chloride solution (100 mL) and extracted with ethyl acetate (100 mL × 2). The combined organic phases were washed with water (100 mL) and saturated brine (100 mL), dried over anhydrous sodium sulfate, filtered, and concentrated under reduced pressure to afford 4 g of 4-bromo-2-(difluoromethoxy)-5-fluorobenzoic acid.

MS (ESI) M/Z: 285.0 [M+H]⁺.

**Step B:** 4-Bromo-2-(difluoromethoxy)-5-fluorobenzoic acid (4 g, 14.1 mmol) was dissolved in N,N-dimethylformamide (30 mL). Potassium carbonate (3.9 g, 28.2 mmol) was added, followed by dropwise addition of iodomethane (3.0 g, 21.1 mmol). The mixture was stirred at room temperature for 2 hours.

After the complete reaction of the starting material as monitored by LCMS, the reaction mixture was added to water (150 mL) and extracted with ethyl acetate (100 mL × 2). The combined organic phases were washed with water (100 mL) and saturated brine (100 mL), dried over anhydrous sodium sulfate, filtered, and concentrated under reduced pressure. The resulting crude product was purified by silica gel column chromatography to afford 3.2 g of methyl 4-bromo-2-(difluoromethoxy)-5-fluorobenzoate.

MS (ESI) M/Z: 299.1 [M+H]⁺.

¹H NMR (400 MHz, CDCl₃) δ 7.67 (d, *J* = 8.0 Hz, 1H), 7.52 (d, *J* = 5.6 Hz, 1H), 6.54 (t, *J* = 74 Hz, 1H), 3.93 (s, 3H).

**Step C:** Methyl 4-bromo-2-(difluoromethoxy)-5-fluorobenzoate (3.2 g, 10.7 mmol) was dissolved in N,N-dimethylformamide (15 mL). Copper(I) cyanide (1.93 g, 21.4 mmol) was added. The mixture was heated to 150 °C under microwave irradiation and stirred for 5 hours. After the disappearance of the starting material as monitored by LCMS, the reaction mixture was added to water (100 mL) and extracted with ethyl acetate (150 mL). The organic phase was washed with water (100 mL) and brine (100 mL), dried, and concentrated. The resulting crude product was purified by silica gel column chromatography to afford 2.1 g of methyl 4-cyano-2-(difluoromethoxy)-5-fluorobenzoate.

MS (ESI) M/Z: 246.1 [M+H]⁺.

**Step D:** At room temperature, methyl 4-cyano-2-(difluoromethoxy)-5-fluorobenzoate (500 mg, 2.04 mmol) was dissolved in methanol (10 mL). Sodium borohydride (155 mg, 4.08 mmol) was added, and the mixture was stirred at room temperature for 2 hours.

After the complete reaction of the starting material as monitored by LCMS, the reaction mixture was added to water (50 mL) and stirred for 10 minutes. It was then extracted with dichloromethane (50 mL × 2). The combined organic phases were washed with water (50 mL) and saturated brine (50 mL), dried over anhydrous sodium sulfate, filtered, and concentrated under reduced pressure to afford 420 mg of crude 5-(difluoromethoxy)-2-fluoro-4-(hydroxymethyl)benzonitrile.

MS (ESI) M/Z: 218.3 [M+H]⁺.

**Step E:** At room temperature, 5-(difluoromethoxy)-2-fluoro-4-(hydroxymethyl)benzonitrile (420 mg, 1.94 mmol) was dissolved in acetonitrile (10 mL). Cesium carbonate (1.26 g, 3.88 mmol) and 1-(methylsulfonyl)-1H-pyrazole (340 mg, 2.33 mmol) were added. The mixture was heated to 70 °C and stirred for 2 hours.

After the disappearance of the starting material as monitored by LCMS, the reaction mixture was added to water (50 mL) and extracted with ethyl acetate (100 mL). The organic phase was washed with water (50 mL) and saturated brine (50 mL), dried over anhydrous sodium sulfate, filtered, and the filtrate was dried and concentrated. The resulting crude product was purified by silica gel column chromatography to afford 380 mg of 4-((1H-pyrazol-1-yl)methyl)-5-(difluoromethoxy)-2-fluorobenzonitrile.

MS (ESI) M/Z: 268.1 [M+H]⁺.

¹H NMR (400 MHz, CDCl₃): δ 7.61 (d, *J* = 1.6 Hz, 1H), 7.49 (d, *J* = 2.4 Hz, 1H), 7.44 (d, *J* = 5.6 Hz, 1H), 6.72 (d, *J =* 8.8 Hz, 1H), 6.57 (t, *J =* 72 Hz, 1H), 6.37 (t, *J =* 2.0 Hz, 1H), 5.41 (s, 2H).

**Step F:** At room temperature, 4-((1H-pyrazol-1-yl)methyl)-5-(difluoromethoxy)-2-fluorobenzonitrile (380 mg, 1.42 mmol) was dissolved in a mixture of N,N-dimethylformamide (7 mL) and water (1 mL). N-Hydroxyacetamide (427 mg, 5.68 mmol) and potassium carbonate (783 mg, 5.68 mmol) were added. The mixture was heated to 60 °C and stirred for 3 hours.

After the disappearance of the starting material as monitored by LCMS, the reaction mixture was added to water (50 mL) and extracted with ethyl acetate (100 mL). The organic phase was washed with water (50 mL) and saturated brine (50 mL), dried over anhydrous sodium sulfate, filtered, the filtrate was dried and concentrated. The crude product was purified by silica gel column chromatography to afford 280 mg of 6-((1H-pyrazol-1-yl)methyl)-5-(difluoromethoxy)benzo[d]isoxazol-3-amine.

MS (ESI) M/Z: 281.3 [M+H]⁺.

¹H NMR (400 MHz, DMSO-*d₆*): δ 7.84 (d, *J* = 2.0 Hz, 1H), 7.74 (s, 1H), 7.51 (d, *J* = 1.6 Hz, 1H), 7.15 (t, *J* = 74 Hz, 1H), 6.95 (s, 1H), 6.50 (s, 2H), 6.32 (t, *J* = 2.0 Hz, 1H), 5.46 (s, 2H).

**Step G:** 6-((1H-pyrazol-1-yl)methyl)-5-(difluoromethoxy)benzo[d]isoxazol-3-amine (50 mg, 0.179 mmol) and 7-methoxy-3,4-dihydro-2H-benzo[b][1,4]dioxepine-6-sulfonyl chloride (99 mg, 0.358 mmol) were dissolved in anhydrous pyridine (1.5 mL). The reaction was carried out at 100 °C under microwave conditions for 3 hours.

After the disappearance of the starting material as monitored by LCMS, ammonium chloride solution (30 mL) was added to the reaction system, and extracted with ethyl acetate (30 mL × 2). The combined organic phases were first washed with water (30 mL), then washed with saturated brine (30 mL), dried over anhydrous sodium sulfate, filtered, and concentrated under reduced pressure. The crude product was purified by reverse-phase preparative chromatography to afford 55 mg of N-(6-((1H-pyrazol-1-yl)methyl)-5-(difluoromethoxy)benzo[d]isoxazol-3-yl)-7-methoxy-3,4-dihydro-2H-benzo[b][1,4]dioxepine-6-sulfonamide.

MS (ESI) M/Z: 523.2 [M+H]⁺.

¹H NMR (500 MHz, DMSO-*d₆*): δ 11.69 (s, 1H), 7.98 (s, 1H), 7.84 (d, *J =* 2.0 Hz, 1H), 7.51 (d, *J* = 1.2 Hz, 1H), 7.21 (d, *J* = 9.2 Hz, 1H), 7.18 (t, *J =* 73.2 Hz, 1H), 7.07 (s, 1H), 6.75 (d, *J* = 9.2 Hz, 1H), 6.32 (t, *J* = 2.0 Hz, 1H), 5.48 (s, 2H), 4.06 (t, *J* = 5.2 Hz, 2H), 3.98 (t, *J* = 5.6 Hz, 2H), 3.64 (s, 3H), 2.06 - 2.02 (m, 2H).

### Example 14:

### N-(6-((1H-Pyrazol-1-yl)methyl)-5-cyclopropylbenzo[d]isoxazol-3-yl)-7-methoxy-3,4-di hydro-2H-benzo[b][1,4]dioxepine-6-sulfonamide

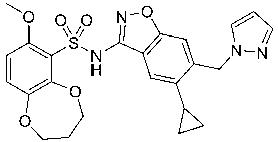

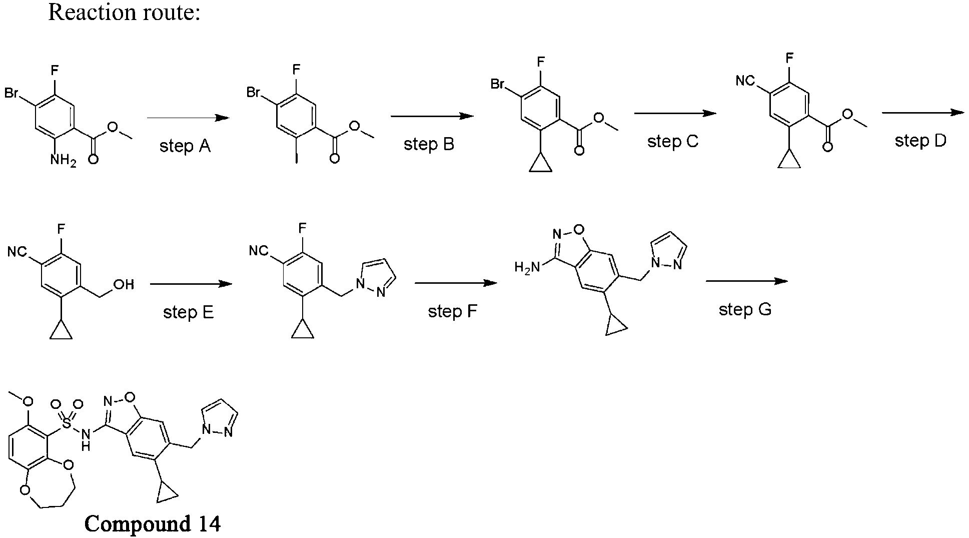

### Operating steps:

**Step A:** Under nitrogen protection, methyl 2-amino-4-bromo-5-fluorobenzoate (5.0 g, 20.2 mmol) was added to a mixture of 6M HCl aqueous solution (100 mL) and acetonitrile (50 mL). The mixture was cooled to 0°C, and a solution of sodium nitrite (1.47 g, 21.2 mmol) in water (10 mL) was added dropwise. The mixture was stirred at the same temperature for 1 hour. Then, a solution of potassium iodide (6.7 g, 40.5 mmol) in water (10 mL) was slowly added dropwise. The reaction mixture was slowly warmed to 25°C and stirred for an additional 16 hours.

After the complete reaction of the starting material as monitored by LCMS, the reaction mixture was added to ethyl acetate (100 mL) and filtered. The filter cake was washed with ethyl acetate (100 mL), and the filtrate was concentrated under reduced pressure. The resulting residue was purified by silica gel column chromatography to afford 6.4 g of methyl 4-bromo-5-fluoro-2-iodobenzoate.

MS (ESI) M/Z: 358.9, 360.9 [M+H]⁺.

**Step B:** Under nitrogen protection, methyl 4-bromo-5-fluoro-2-iodobenzoate (6.4 g, 17.9 mmol), cyclopropylboronic acid (1.84 g, 21.5 mmol), cesium carbonate (11.6 g, 42.4 mmol), and [1,1'-bis(diphenylphosphino)ferrocene]dichloropalladium (1.3 g, 1.8 mmol) were dissolved in 1,4-dioxane (100 mL). The reaction mixture was stirred at 100°C for 3 hours.

After the complete reaction of the starting material as monitored by LCMS, the reaction mixture was added to ethyl acetate (100 mL) and filtered. The filter cake was washed with ethyl acetate (100 mL), and the filtrate was concentrated under reduced pressure. The resulting residue was purified by silica gel column chromatography to afford 3.5 g of methyl 4-bromo-2-cyclopropyl-5-fluorobenzoate.

MS (ESI) M/Z: 273.0, 275.0 [M+H]⁺.

**Step C:** Under nitrogen protection, copper(I) cyanide (2.3 g, 25.7 mmol) was added to a solution of methyl 4-bromo-2-cyclopropyl-5-fluorobenzoate (3.5 g, 12.9 mmol) in anhydrous N,N-dimethylformamide (30 mL). The reaction mixture was stirred at 130°C for 16 hours.

After LCMS showed the reaction was complete, the reaction mixture was quenched by adding saturated sodium bicarbonate aqueous solution (50 mL) and filtered. The filtrate was extracted with ethyl acetate (100 mL × 3). The combined organic phases were washed with saturated sodium chloride solution (100 mL), dried over anhydrous sodium sulfate, filtered, and the solvent was removed under reduced pressure. The residue was purified by column chromatography to afford 1.3 g of methyl 4-cyano-2-cyclopropyl-5-fluorobenzoate.

MS (ESI) M/Z: 220.1 [M+H]⁺.

**Step D:** Under nitrogen protection, methyl 4-cyano-2-cyclopropyl-5-fluorobenzoate (1.3 g, 5.9 mmol) was dissolved in anhydrous tetrahydrofuran (15 mL). Lithium borohydride (5.9 mL, 11.9 mmol, 2M in tetrahydrofuran) was added dropwise slowly at 0°C. Then the reaction mixture was slowly warmed to 25°C and stirred for 2 hours.

After the completion of the reaction as monitored by LCMS, water (30 mL) was slowly added dropwise at 0°C. The mixture was extracted with ethyl acetate (50 mL × 3). The combined organic phases were washed with saturated sodium chloride aqueous solution (100 mL), dried over anhydrous sodium sulfate, filtered, and the filtrate was concentrated under reduced pressure to afford 1.1 g of 5-cyclopropyl-2-fluoro-4-(hydroxymethyl)benzonitrile.

MS (ESI) M/Z: 192.1 [M+H]⁺.

¹H NMR (400 MHz, DMSO-*d₆*): δ 7.46 (dd, *J* = 19.6, 8.6 Hz, 2H), 5.60 (t, *J* = 5.4 Hz, 1H), 4.75 (d, *J* = 5.4 Hz, 2H), 1.89 - 1.78 (m, 1H), 0.93 - 0.86 (m, 2H), 0.72 - 0.64 (m, 2H).

**Step E:** Under nitrogen protection, 5-cyclopropyl-2-fluoro-4-(hydroxymethyl)benzonitrile (1.1 g, 5.7 mmol), 1-(methylsulfonyl)-1H-pyrazole (1.0 g, 6.9 mmol), and cesium carbonate (3.75 g, 11.5 mmol) were dissolved in acetonitrile (10 mL). The system was stirred at 70°C for 2 hours.

After the complete reaction of the starting material as monitored by LCMS, the reaction mixture was added to ethyl acetate (50 mL) and filtered. The filter cake was washed with ethyl acetate (100 mL), and the filtrate was concentrated under reduced pressure. The resulting residue was purified by silica gel column chromatography to afford 1.0 g of 4-((1H-pyrazol-1-yl)methyl)-5-cyclopropyl-2-fluorobenzonitrile.

MS (ESI) M/Z: 242.2 [M+H]⁺.

**Step F:** Under nitrogen protection, N-hydroxyacetamide (186.7 mg, 2.5 mmol) and potassium carbonate (342.7 mg, 2.5 mmol) were dissolved in a mixture of N,N-dimethylformamide (7 mL) and water (1 mL). Then, 4-((1H-pyrazol-1-yl)methyl)-5-cyclopropyl-2-fluorobenzonitrile (200 mg, 0.83 mmol) was added. The system was stirred at 60°C for 3 hours.

After the complete reaction of the starting material as monitored by LCMS, the reaction mixture was quenched by adding water (20 mL) and extracted with ethyl acetate (50 mL × 3). The combined organic phases were washed with saturated sodium chloride aqueous solution (50 mL × 3), dried over anhydrous sodium sulfate, filtered, and the filtrate was concentrated under reduced pressure. The resulting residue was purified by silica gel column chromatography to afford 60 mg of 6-((1H-pyrazol-1-yl)methyl)-5-cyclopropylbenzo[d]isoxazol-3-amine.

MS (ESI) M/Z: 255.2 [M+H]⁺.

**Step G:** Under nitrogen protection, 6-((1H-pyrazol-1-yl)methyl)-5-cyclopropylbenzo[d] isoxazol-3-amine (50 mg, 0.20 mmol) and 7-methoxy-3,4-dihydro-2H-benzo[b][1,4]dioxepine-6-sulfonyl chloride (85 mg, 0.31 mmol) were dissolved in anhydrous pyridine (1 mL). The reaction system was stirred at 100°C under microwave conditions for 4 hours.

After the completion of the reaction as monitored by LCMS, the reaction was quenched by adding water (5 mL). The mixture was extracted with ethyl acetate (30 mL × 3). The combined organic phases were washed with saturated ammonium chloride solution (50 mL × 3) and saturated sodium chloride solution (50 mL × 3), dried over anhydrous sodium sulfate, filtered, and the filtrate was concentrated under reduced pressure. The resulting residue was purified by reverse-phase preparative chromatography (formic acid system) to afford 58.5 mg of the target product, N-(6-((1H-pyrazol-1-yl)methyl)-5-cyclopropylbenzo[d]isoxazol-3-yl)-7-methoxy-3,4-dihydro-2H-benzo[b][1,4]dioxepine-6-sulfonamide.

MS (ESI) M/Z: 497.3 [M+H]⁺.

¹H NMR (400 MHz, DMSO-*d₆*): δ 11.50 (s, 1H), 7.86 (d, *J* = 2.0 Hz, 1H), 7.79 (s, 1H), 7.54 (s, 1H), 7.21 (d, *J* = 9.2 Hz, 1H), 6.82 - 6.71 (m, 2H), 6.34 (t, *J* = 2.0 Hz, 1H), 5.66 (s, 2H), 4.10 - 3.93 (m, 4H), 3.64 (s, 3H), 2.08 - 1.98 (m, 3H), 1.02 - 0.95 (m, 2H), 0.65 - 0.54 (m, 2H).

### Example 15:

### N-(6-((1H-Pyrazol-1-yl)methyl)-5-methoxybenzo[d]isoxazol-3-yl)-7-methoxy-3,4-dihyd ro-2H-benzo[b][1,4] dioxepine-6-sulfonamide

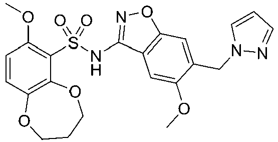

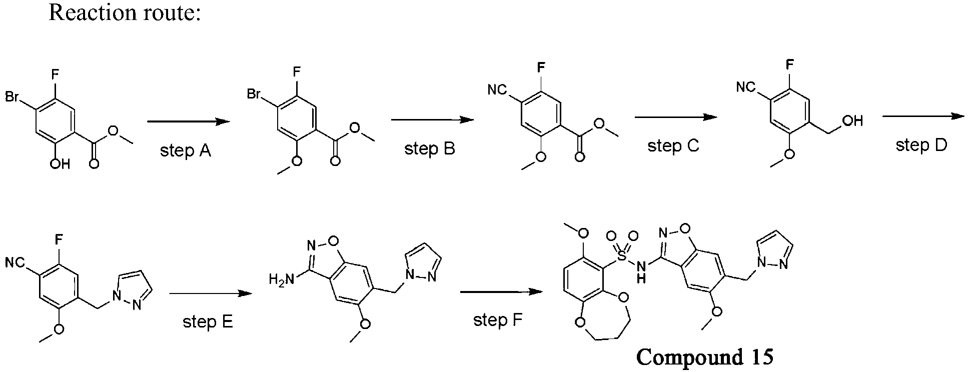

### Operating steps:

**Step A:** Under nitrogen protection and ice-bath conditions, iodomethane (1.20 mL, 19.27 mmol) was added dropwise slowly to a solution of methyl 4-bromo-5-fluoro-2-hydroxybenzoate (4.0 g, 16.06 mmol) and potassium carbonate (4.43 g, 32.12 mmol) in acetone (35 mL). The reaction mixture was slowly warmed to 25°C and stirred for an additional 16 hours.

After the complete reaction of the starting material as monitored by LCMS, the reaction mixture was added to ethyl acetate (30 mL) and filtered. The filter cake was washed with ethyl acetate (50 mL), and the filtrate was concentrated under reduced pressure. Purification by silica gel column chromatography afforded 3.9 g of methyl 4-bromo-5-fluoro-2-methoxybenzoate.

MS (ESI) M/Z: 263.0, 265.0 [M+H]⁺.

**Step B:** Under nitrogen protection, copper(I) cyanide (2.66 g, 29.66 mmol) was added slowly to a solution of methyl 4-bromo-5-fluoro-2-methoxybenzoate (3.9 g, 14.83 mmol) in anhydrous N,N-dimethylformamide (30 mL). The reaction mixture was stirred at 130°C for 16 hours.

After LCMS showed the reaction was complete, the reaction mixture was quenched by adding saturated sodium bicarbonate aqueous solution (50 mL) and extracted with ethyl acetate (100 mL × 3). The combined organic phases were washed with saturated sodium chloride solution (100 mL), dried over anhydrous sodium sulfate, filtered, and the solvent was removed under reduced pressure. Purification by column chromatography afforded 1.6 g of methyl 4-cyano-5-fluoro-2-methoxybenzoate.

MS (ESI) M/Z: 210 [M+H]⁺.

¹H NMR (400 MHz, CDCl₃) δ 7.61 (d, *J* = 8.6 Hz, 1H), 7.15 (d, *J* = 4.8 Hz, 1H), 3.92 (d, *J* = 2.4 Hz, 6H).

**Step C:** Under nitrogen protection, methyl 4-cyano-5-fluoro-2-methoxybenzoate (1.65 g, 7.89 mmol) was dissolved in anhydrous tetrahydrofuran (15 mL). Lithium borohydride (7.89 mL, 7.89 mmol, 2M) was added dropwise slowly at 0°C. After the addition was complete, the reaction mixture was slowly warmed to 25°C and stirred for 6 hours.

After the completion of the reaction as monitored by LCMS, water (30 mL) was slowly added dropwise at 0°C. The mixture was extracted with ethyl acetate (50 mL × 3). The combined organic phases were washed with saturated sodium chloride aqueous solution (100 mL), dried over anhydrous sodium sulfate, filtered, and the filtrate was concentrated under reduced pressure to afford 1.05 g of the target product, 2-fluoro-4-(hydroxymethyl)-5-methoxybenzonitrile.

MS (ESI) M/Z: 182.1 [M+H]⁺.

**Step D:** Under nitrogen protection, 2-fluoro-4-(hydroxymethyl)-5-methoxybenzonitrile (300 mg, 1.66 mmol), 1-(methylsulfonyl)-1H-pyrazole (291 mg, 1.99 mmol), and cesium carbonate (812 mg, 2.49 mmol) were dissolved in acetonitrile (10 mL). The system was stirred at 70°C for 3 hours.

After the complete reaction of the starting material as monitored by LCMS, the reaction mixture was added to ethyl acetate (30 mL) and filtered. The filter cake was washed with ethyl acetate (50 mL), and the filtrate was concentrated under reduced pressure. Purification by silica gel column chromatography afforded 200 mg of 4-((1H-pyrazol-1-yl)methyl)-2-fluoro-5-methoxybenzonitrile.

MS (ESI) M/Z: 232.2 [M+H]⁺.

**Step E:** Under nitrogen protection, N-hydroxyacetamide (259 mg, 3.46 mmol) and potassium carbonate (477 mg, 3.46 mmol) were dissolved in a mixture of N,N-dimethylformamide (7 mL) and water (1 mL). Then, 4-((1H-pyrazol-1-yl)methyl)-2-fluoro-5-methoxybenzonitrile (200 mg, 0.86 mmol) was added. The system was stirred at 60°C for 16 hours.

After the complete reaction of the starting material as monitored by LCMS, the reaction mixture was quenched by adding water (20 mL) and extracted with ethyl acetate (50 mL × 3). The combined organic phases were washed with saturated sodium chloride aqueous solution (50 mL × 3), dried over anhydrous sodium sulfate, filtered, and the filtrate was concentrated under reduced pressure. Purification by silica gel column chromatography afforded 50 mg of 6-((1H-pyrazol-1-yl)methyl)-5-methoxybenzo[d]isoxazol-3-amine.

MS (ESI) M/Z: 245.1 [M+H]⁺.

¹H NMR (400 MHz, DMSO-*d₆*): δ 7.80 (d, *J* = 2.2 Hz, 1H), 7.50 (d, *J* = 1.6 Hz, 1H), 7.42 (s, 1H), 6.72 (s, 1H), 6.30 (s, 3H), 5.38 (s, 2H), 3.84 (d, *J* = 12.6 Hz, 3H).

**Step F:** Under nitrogen protection, 6-((1H-pyrazol-1-yl)methyl)-5-methoxybenzo[d] isoxazol-3-amine (50 mg, 0.20 mmol) and 7-methoxy-3,4-dihydro-2H-benzo[b][1,4]dioxepine-6-sulfonyl chloride (85 mg, 0.31 mmol) were dissolved in anhydrous pyridine (1 mL). The reaction system was stirred at 100°C under microwave conditions for 4 hours.

After the completion of the reaction as monitored by LCMS, the reaction was quenched by adding water (5 mL). The mixture was extracted with ethyl acetate (10 mL × 3). The combined organic phases were washed with saturated ammonium chloride solution (10 mL × 3) and saturated sodium chloride solution (50 mL), dried over anhydrous sodium sulfate, filtered, and the filtrate was concentrated under reduced pressure. The crude product was purified by reverse-phase preparative chromatography to afford 49.2 mg of N-(6-((1H-pyrazol-1-yl)methyl)-5-methoxybenzo[d]isoxazol-3-yl)-7-methoxy-3,4-dihydro-2H-benzo[b] [1,4]dioxepine-6-sulfona mide.

MS (ESI) M/Z: 487.2 [M+H]⁺.

¹H NMR (400 MHz, DMSO-*d₆*): δ 11.44 (s, 1H), 7.82 (d, *J* = 1.6 Hz, 1H), 7.62 (s, 1H), 7.50 (d, *J* = 1.6 Hz, 1H), 7.21 (d, *J* = 9.2 Hz, 1H), 6.82 (s, 1H), 6.75 (d, *J* = 9.2 Hz, 1H), 6.30 (t, *J* = 2.0 Hz, 1H), 5.40 (s, 2H), 4.09 - 4.05 (m, 2H), 4.00 - 3.97 (m, 2H), 3.86 (s, 3H), 3.66 (s, 3H), 2.07 - 2.02 (m, 2H).

### Example 16:

### N-(6-((1H-Pyrazol-1-yl)methyl)-4-methoxybenzo[d]isoxazol-3-yl)-8-methoxy-2,3,4,5-tet rahydrobenzo[b]oxepine-9-sulfonamide

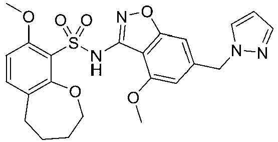

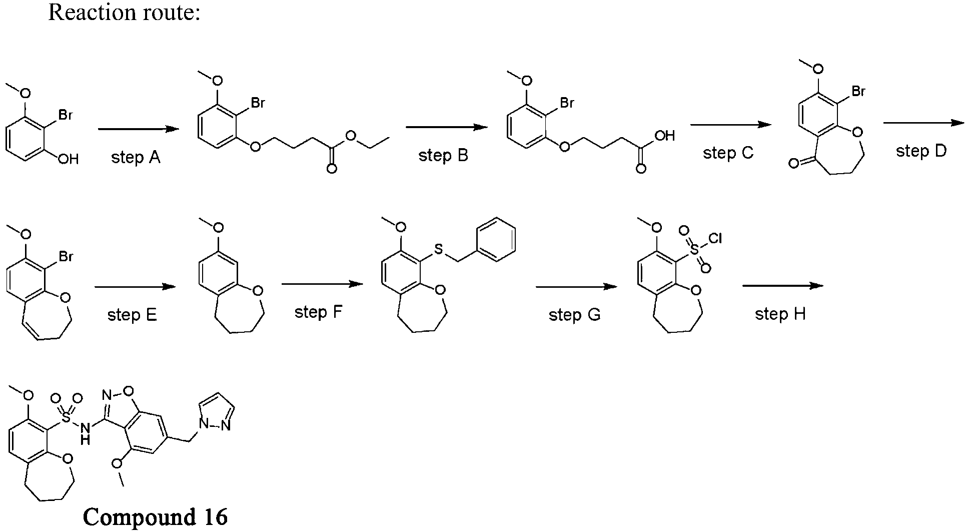

### Operating steps:

**Step A:** 2-Bromo-3-methoxyphenol (2 g, 9.9 mmol) was dissolved in N,N-dimethylformamide (50 mL). Potassium carbonate (2.8 g, 20 mmol) and ethyl 4-bromobutyrate (1.7 mL, 12 mmol) were added. The reaction system was stirred at room temperature overnight.

After the disappearance of the starting material as monitored by LCMS, the reaction mixture was quenched by adding water (50 mL). The mixture was extracted with ethyl acetate (70 mL × 2). The combined organic phases were washed with saturated brine (50 mL × 2), dried over anhydrous sodium sulfate, filtered, and concentrated under reduced pressure. The resulting residue was purified by silica gel column chromatography to afford 3 g of ethyl 4-(2-bromo-3-methoxyphenoxy)butanoate.

MS (ESI) M/Z: 317.1 [M+H]⁺.

**Step B:** Ethyl 4-(2-bromo-3-methoxyphenoxy)butanoate (3 g, 9.49 mmol) was dissolved in ethanol (20 mL). Sodium hydroxide (1.14 g, 28.475 mmol) was added. The reaction proceeded at room temperature overnight.

After the disappearance of the starting material as monitored by LCMS, dilute hydrochloric acid was added to the reaction system to adjust the pH to acidic. The mixture was extracted with ethyl acetate (70 mL × 2). The combined organic phases were washed with saturated brine (50 mL × 3), dried over anhydrous sodium sulfate, filtered, and concentrated under reduced pressure. The resulting residue was triturated with petroleum ether and dichloromethane to afford 2.9 g of 4-(2-bromo-3-methoxyphenoxy)butanoic acid.

MS (ESI) M/Z: 289.1 [M+H]⁺.

**Step C:** 4-(2-bromo-3-methoxyphenoxy)butanoic acid (2.9 g, 10.09 mmol) was dissolved in TFA (trifluoroacetic acid, 50 mL). TFAA (trifluoroacetic anhydride, 4.2 mL, 30 mmol) was added. The reaction system was stirred at room temperature overnight.

After the disappearance of the starting material as monitored by LCMS, the reaction mixture was quenched by adding water (50 mL). The mixture was extracted with ethyl acetate (70 mL × 2). The combined organic phases were washed with saturated brine (50 mL × 2), dried over anhydrous sodium sulfate, filtered, and concentrated under reduced pressure. The resulting residue was purified by silica gel column chromatography to afford 2.0 g of 9-bromo-8-methoxy-3,4-dihydrobenzo[b]oxepin-5(2H)-one.

¹H NMR (400 MHz, Chloroform-d): δ 7.78 (d, *J =* 8.8 Hz, 1H), 6.71 (d, *J =* 8.8 Hz, 1H), 4.33 (t, *J* = 6.7 Hz, 2H), 3.96 (s, 3H), 2.89 (dd, *J =* 7.4, 6.2 Hz, 2H), 2.30 - 2.10 (m, 2H).

**Step D:** 9-Bromo-8-methoxy-3,4-dihydrobenzo[b]oxepin-5(2H)-one (1.0 g, 7.4 mmol) was dissolved in methanol (30 mL). Sodium borohydride (0.14 g, 7.4 mmol) was added. The reaction system was stirred at room temperature for 3 hours.

After the disappearance of the starting material as monitored by LCMS, dilute HCl (5 mL) was added to the reaction mixture. The mixture was extracted with ethyl acetate (30 mL × 2). The combined organic phases were washed with saturated brine (30 mL × 2), dried over anhydrous sodium sulfate, filtered, and concentrated under reduced pressure. The resulting residue was purified by silica gel column chromatography to afford 0.53 g of 9-bromo-8-methoxy-2,3-dihydrobenzo[b]oxepine.

MS (ESI) M/Z: 255.1 [M+H]⁺.

**Step E:** 9-Bromo-8-methoxy-2,3-dihydrobenzo[b]oxepine (0.53 g, 2.1 mol) was dissolved in methanol (10 mL). Palladium on carbon (60 mg) was added. The air was evacuated and replaced with hydrogen 3-4 times. The reaction system was stirred at room temperature for 3 hours.

After the disappearance of the starting material as monitored by LCMS, the reaction mixture was filtered through Celite. The resulting residue was purified by silica gel column chromatography to afford 0.44 g of 8-methoxy-2,3,4,5-tetrahydrobenzo[b]oxepine.

¹H NMR (400 MHz, DMSO-*d₆*): δ 6.57 - 6.48 (m, 1H), 3.97 - 3.88 (m, 1H), 3.69 (s, 1H), 2.76 - 2.63 (m, 1H), 1.95 - 1.81 (m, 1H), 1.66 - 1.55 (m, 1H).

**Step F:** Under nitrogen protection, 8-methoxy-2,3,4,5-tetrahydrobenzo[b]oxepine (0.44 g, 2.44 mmol) and N,N,N',N'-tetramethylethylenediamine (0.42 mL, 2.68 mol) were dissolved in tetrahydrofuran (10 mL). The mixture was cooled to 0 °C, and n-butyllithium (1.68 mL, 2.68 mmol, 1.6M) was added dropwise. The mixture was stirred at this temperature for 20 minutes. A solution of 1,2-dibenzyldisulfane (0.66 g, 2.69 mmol) in tetrahydrofuran (5 mL) was added dropwise at -78 °C, stirred at the same temperature for 30 minutes, and then warmed to room temperature and stirred for 1 hour.

After the complete reaction of the starting material as monitored by LCMS, the reaction mixture was poured into a mixture of ethyl acetate (50 mL) and saturated ammonium chloride (50 mL), extracted, washed with saturated brine (50 mL), dried over anhydrous sodium sulfate, filtered, and the filtrate was concentrated under reduced pressure. The resulting mixture was purified by silica gel column chromatography (eluent: petroleum ether/ethyl acetate = 1/10) to afford 0.42 g of 9-(benzylthio)-8-methoxy-2,3,4,5-tetrahydrobenzo[b]oxepine.

MS (ESI) M/Z: 301.1 [M+H]⁺.

**Step G:** Under nitrogen protection, 9-(benzylthio)-8-methoxy-2,3,4,5-tetrahydrobenzo[b] oxepine (0.10 g, 0.33 mmol) was dissolved in acetic acid (3 mL). The mixture was cooled to 0°C, and 1,3-dichloro-5,5-dimethylhydantoin (0.13 g, 11.26 mmol) was added. The system was stirred at 0°C for 1 hour.

After the complete reaction of the starting material as monitored by LCMS, the reaction mixture was diluted with ethyl acetate (10 mL), washed with water (10 mL × 3) and saturated brine (10 mL), dried over anhydrous sodium sulfate, filtered, and the filtrate was concentrated under reduced pressure. The resulting mixture was purified by silica gel column chromatography (eluent: petroleum ether/ethyl acetate = 3/1) to afford 0.043 g of 8-methoxy-2,3,4,5-tetrahydrobenzo[b]oxepine-9-sulfonyl chloride.

¹H NMR (400 MHz, DMSO-*d₆*): δ 7.10 (d, *J* = 8.4 Hz, 1H), 6.62 (d, *J =* 8.4 Hz, 1H), 3.81 (t, *J* = 5.1 Hz, 1H), 2.69 - 2.61 (m, 1H), 1.87 - 1.78 (m, 1H), 1.60 - 1.54 (m, 1H).

**Step H:** Under nitrogen protection, 8-methoxy-2,3,4,5-tetrahydrobenzo[b]oxepine-9-sulfonyl chloride (0.035 g, 0.127 mmol) and 6-((1H-pyrazol-1-yl)methyl)-4-methoxybenzo[d] isoxazol-3-amine (0.015 g, 0.063 mmol) were dissolved in pyridine (0.5 mL). The system was stirred at 100 °C under microwave conditions for 3 hours.

After the complete reaction of the starting material as monitored by LCMS, the reaction mixture was added to water (5 mL), diluted with ethyl acetate (5 mL), washed with saturated brine (3 mL), dried over anhydrous sodium sulfate, filtered, and the filtrate was concentrated under reduced pressure. The resulting mixture was purified by silica gel column chromatography (eluent: petroleum ether/ethyl acetate = 4/5) and reverse-phase preparative chromatography (eluent: 0.01vol% formic acid in water / acetonitrile = 1/1) to afford 16 mg of N-(6-((1H-pyrazol-1-yl)methyl)-4-methoxybenzo[d]isoxazol-3-yl)-8-methoxy-2,3,4,5-tetrahydro benzo[b]oxepine-9-sulfonamide.

MS (ESI) M/Z: 485.2 [M+H]⁺.

¹H NMR (400 MHz, DMSO-*d₆*): δ 7.87 (d, *J* = 2.3 Hz, 1H), 7.49 (d, *J* = 1.8 Hz, 1H), 7.33 (d, *J* = 8.5 Hz, 1H), 6.76 (dd, *J* = 19.2, 10.7 Hz, 3H), 6.30 (t, *J* = 2.1 Hz, 1H), 5.43 (s, 2H), 3.93 (s, 2H), 3.84 (s, 3H), 3.68 (s, 3H), 2.69 (d, *J =* 9.9 Hz, 2H), 1.85 (s, 2H), 1.58 (s, 2H).

### Example 17:

### N-(6-((1H-Pyrazol-1-yl)methyl)-5-methylbenzo[d]isoxazol-3-yl)-7-methoxy-3,4-dihydro -2H-benzo [b] [1,4] dioxepine-6-sulfonamide

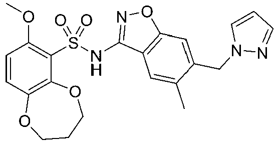

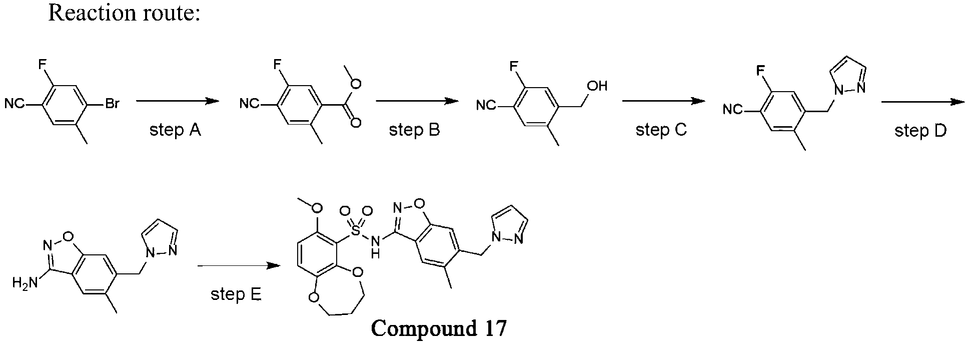

### Operating steps:

**Step A:** 4-Bromo-2-fluoro-5-methylbenzonitrile (500 mg, 2.34 mmol) was dissolved in methanol (15 mL). [1,1'-Bis(diphenylphosphino)ferrocene]dichloropalladium dichloromethane complex (382 mg, 0.47 mmol) and triethylamine (0.98 mL, 7.02 mmol) were added. After evacuating and refilling with carbon monoxide three times, the reaction mixture was stirred at 65°C under a carbon monoxide atmosphere for 16 hours.

After the completion of the reaction as monitored by LCMS, methanol was removed by concentration under reduced pressure. Ethyl acetate (30 mL) and water (50 mL) were added. The mixture was extracted with ethyl acetate (50 mL × 3). The combined organic phases were washed with saturated sodium chloride solution (100 mL), dried over anhydrous sodium sulfate, filtered, and the filtrate was concentrated under reduced pressure. The crude product was purified by silica gel column chromatography to afford 330 mg of methyl 4-cyano-5-fluoro-2-methylbenzoate.

MS (ESI) M/Z: 194.1 [M+H]⁺.

¹H NMR (400 MHz, DMSO-*d₆*): δ 7.98 (d, *J =* 6.4 Hz, 1H), 7.83 (d, *J =* 9.8 Hz, 1H), 3.87 (s, 3H), 2.48 (s, 3H).

**Step B:** Under nitrogen protection, methyl 4-cyano-5-fluoro-2-methylbenzoate (330 mg, 1.71 mmol) was dissolved in anhydrous tetrahydrofuran (15 mL). Lithium borohydride (1.71 mL, 3.42 mmol) was added dropwise slowly at 0°C. Then the reaction mixture was slowly warmed to 25°C and stirred for 2 hours.

After the completion of the reaction as monitored by LCMS, water (15 mL) was slowly added dropwise at 0°C. The mixture was extracted with ethyl acetate (30 mL × 3). The combined organic phases were washed with saturated sodium chloride aqueous solution (50 mL), dried over anhydrous sodium sulfate, filtered, and the filtrate was concentrated under reduced pressure to afford 190 mg of 2-fluoro-4-(hydroxymethyl)-5-methylbenzonitrile.

MS (ESI) M/Z: 166.1 [M+H]⁺.

**Step C:** Under nitrogen protection, 2-fluoro-4-(hydroxymethyl)-5-methylbenzonitrile (190 mg, 1.15 mmol), 1-(methylsulfonyl)-1H-pyrazole (402 mg, 1.38 mmol), and cesium carbonate (562 mg, 1.73 mmol) were dissolved in acetonitrile (10 mL). The system was stirred at 70°C for 3 hours.

After the complete reaction of the starting material as monitored by LCMS, the reaction mixture was added to ethyl acetate (30 mL) and filtered. The filter cake was washed with ethyl acetate (50 mL), and the filtrate was concentrated under reduced pressure. The crude product was purified by silica gel column chromatography to afford 220 mg of 4-((1H-pyrazol-1-yl)methyl)-2-fluoro-5-methylbenzonitrile.

MS (ESI) M/Z: 216.2 [M+H]⁺.

¹H NMR (400 MHz, DMSO-*d₆*): δ 7.85 (d, *J =* 1.8 Hz, 1H), 7.78 (d, *J =* 6.6 Hz, 1H), 7.54 (d, *J* = 1.4 Hz, 1H), 6.70 (d, *J* = 10.2 Hz, 1H), 6.34 (t, *J* = 2.2 Hz, 1H), 5.46 (s, 2H), 2.31 (s, 3H).

**Step D:** Under nitrogen protection, N-hydroxyacetamide (307 mg, 4.09 mmol) and potassium tert-butoxide (459 mg, 4.09 mmol) were dissolved in N,N-dimethylformamide (10 mL). The system was stirred at room temperature for 1 hour. Then, 4-((1H-pyrazol-1-yl)methyl)-2-fluoro-5-methylbenzonitrile (220 mg, 1.02 mmol) was added. The system was stirred at 60°C for 2 hours.

After the complete reaction of the starting material as monitored by LCMS, the reaction mixture was quenched by adding water (20 mL) and extracted with ethyl acetate (50 mL × 3). The combined organic phases were washed with saturated sodium chloride aqueous solution (50 mL × 3), dried over anhydrous sodium sulfate, filtered, and the filtrate was concentrated under reduced pressure. The crude product was purified by silica gel column chromatography to afford 110 mg of 6-((1H-pyrazol-1-yl)methyl)-5-methylbenzo[d]isoxazol-3-amine.

MS (ESI) M/Z: 229.2 [M+H]⁺.

**Step E:** Under nitrogen protection, 6-((1H-pyrazol-1-yl)methyl)-5-methylbenzo[d] isoxazol-3-amine (50 mg, 0.22 mmol) and 7-methoxy-3,4-dihydro-2H-benzo[b][1,4] dioxepine-6-sulfonyl chloride (91 mg, 0.33 mmol) were dissolved in anhydrous pyridine (1 mL). The reaction system was stirred at 100°C under microwave conditions for 3 hours.

After the completion of the reaction as monitored by LCMS, the reaction was quenched by adding water (5 mL). The mixture was extracted with ethyl acetate (30 mL × 3). The combined organic phases were washed with saturated ammonium chloride solution (50 mL × 3) and saturated sodium chloride solution (50 mL × 3), dried over anhydrous sodium sulfate, filtered, and the filtrate was concentrated under reduced pressure. The crude product was purified by reverse-phase preparative chromatography to afford 14.8 mg of N-(6-((1H-pyrazol-1-yl)methyl)-5-methylbenzo[d]isoxazol-3-yl)-7-methoxy-3,4-dihydro-2H-ben zo[b][1,4]dioxepine-6-sulfonamide.

MS (ESI) M/Z: 471.2 [M+H]⁺.

¹H NMR (400 MHz, DMSO-*d₆*): δ 11.52 (s, 1H), 7.83 (s, 1H), 7.81 (d, *J* = 1.8 Hz, 1H), 7.52 (d, *J* = 1.4 Hz, 1H), 7.20 (d, *J* = 9.2 Hz, 1H), 6.86 (s, 1H), 6.75 (d, *J* = 9.2 Hz, 1H), 6.32 (t, *J =* 2.0 Hz, 1H), 5.48 (s, 2H), 4.07 - 4.03 (m, 2H), 4.00 - 3.96 (m, 2H), 3.66 (s, 3H), 2.36 (s, 3H), 2.07 - 2.01 (m, 2H).

### Example 18:

### N-(6-((1H-Pyrazol-1-yl)methyl)-5-ethoxybenzo[d]isoxazol-3-yl)-7-methoxy-3,4-dihydro -2H-benzo [b] [1,4] dioxepine-6-sulfonamide

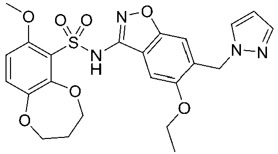

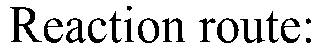

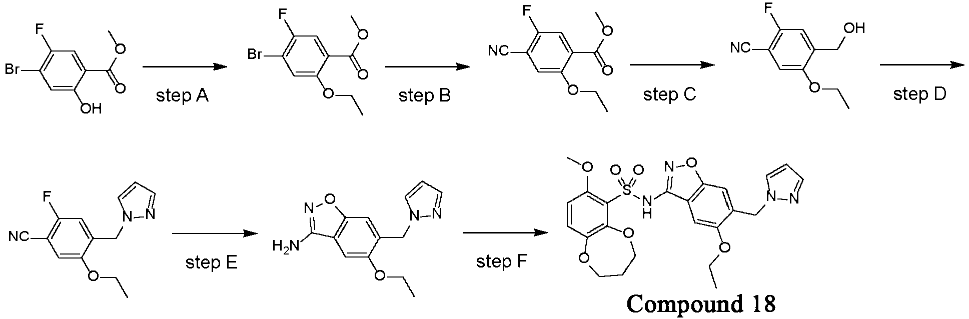

### Operating steps:

**Step A:** Methyl 4-bromo-5-fluoro-2-hydroxybenzoate (2.0 g, 8.0 mmol) and bromoethane (1.1 g, 10.4 mmol) were dissolved in acetonitrile (30 mL). Potassium carbonate (2.2 g, 16.0 mmol) was then added. The reaction mixture was gradually heated to 70 °C and reacted for 3 hours.

After the disappearance of the starting material as monitored by LCMS, the mixture was cooled to room temperature, diluted with water (50 mL), and extracted with ethyl acetate (50 mL × 2). The organic phase was washed with water (20 mL) and saturated brine (20 mL), dried over anhydrous sodium sulfate, filtered, and concentrated under reduced pressure to afford 2.0 g of crude methyl 4-bromo-2-ethoxy-5-fluorobenzoate.

MS (ESI) M/Z: 279.0 [M+H]⁺.

**Step B:** At room temperature, methyl 4-bromo-2-ethoxy-5-fluorobenzoate (2.0 g, 7.2 mmol) was dissolved in anhydrous N,N-dimethylformamide (20 mL). Copper(I) cyanide (1.7 g, 14.4 mmol) was added to the mixture. The reaction system was slowly heated to 150 °C and stirred for 5 hours.

After the disappearance of the starting material as monitored by LCMS, the mixture was cooled to room temperature, and water was added dropwise to quench the reaction. The mixture was filtered through Celite. The filtrate was extracted with ethyl acetate (30 mL × 3). The organic phase was washed with water (50 mL) and saturated brine (50 mL), dried over anhydrous sodium sulfate, filtered, and concentrated under reduced pressure. The resulting crude product was purified by flash silica gel column chromatography to afford 850 mg of methyl 4-cyano-2-ethoxy-5-fluorobenzoate.

MS (ESI) M/Z: 224.3 [M+H]⁺.

**Step C:** Methyl 4-cyano-2-ethoxy-5-fluorobenzoate (350 mg, 1.6 mmol) was dissolved in anhydrous tetrahydrofuran (10 mL). Under ice-bath conditions, lithium borohydride (2.0 mL, 4 mmol) was added dropwise. The reaction system was warmed to room temperature and stirred for 4 hours.

After the disappearance of the starting material as monitored by LCMS, the mixture was cooled to 0°C, and water was added dropwise to quench the reaction. The mixture was filtered through Celite. The filtrate was extracted with ethyl acetate (30 mL × 3). The organic phase was washed with water (50 mL) and saturated brine (50 mL), dried over anhydrous sodium sulfate, filtered, and concentrated under reduced pressure. The resulting crude product was purified by flash silica gel column chromatography to afford 250 mg of 5-ethoxy-2-fluoro-4-(hydroxymethyl)benzonitrile.

MS (ESI) M/Z: 196.1 [M+H]⁺.

**Step D:** 5-Ethoxy-2-fluoro-4-(hydroxymethyl)benzonitrile (250 mg, 1.3 mmol) and 1-(methylsulfonyl)-1H-pyrazole (209 mg, 1.4 mmol) were dissolved in N,N-dimethylformamide (8 mL). Cesium carbonate (848 mg, 2.6 mmol) was then added. The reaction mixture was gradually heated to 70 °C and reacted for 3 hours.

After the disappearance of the starting material as monitored by LCMS, the mixture was cooled to room temperature, diluted with water (50 mL), and extracted with ethyl acetate (50 mL × 2). The organic phase was washed with water (20 mL) and saturated brine (20 mL), dried over anhydrous sodium sulfate, filtered, and concentrated under reduced pressure to afford a crude product. The resulting crude product was purified by flash silica gel column chromatography to afford 350 mg of 4-((1H-pyrazol-1-yl)methyl)-5-ethoxy-2-fluorobenzonitrile.

MS (ESI) M/Z: 246.2 [M+H]⁺.

¹H NMR (400 MHz, DMSO-*d₆*): δ 7.83 (d, *J* = 2.0 Hz, 1H), 7.56 (d, *J* = 5.2 Hz, 1H), 7.51 (d, *J* = 1.2 Hz, 1H), 6.75 (d, *J =* 9.6 Hz, 1H), 6.31 (t, *J =* 2.0 Hz, 1H), 5.36 (s, 2H), 4.12 (q, *J* = 7.2 Hz, 2H), 1.34 (t, *J* = 7.2 Hz, 3H).

**Step E:** 4-((1H-Pyrazol-1-yl)methyl)-5-ethoxy-2-fluorobenzonitrile (200 mg, 0.82 mmol) and N-hydroxyacetamide (184 mg, 2.4 mmol) were dissolved in N,N-dimethylformamide (8 mL). Anhydrous potassium carbonate (338 mg, 2.4 mmol) was then added. The reaction mixture was gradually heated to 70 °C and reacted overnight.

After the completion of reaction as monitored by LCMS, the mixture was cooled to room temperature, diluted with water (50 mL), and extracted with ethyl acetate (50 mL × 2). The organic phase was washed with water (20 mL) and saturated brine (20 mL), dried over anhydrous sodium sulfate, filtered, and concentrated under reduced pressure to afford a crude product. The resulting crude product was purified by flash silica gel column chromatography to afford 350 mg of 6-((1H-pyrazol-1-yl)methyl)-5-ethoxybenzo[d]isoxazol-3-amine.

MS (ESI) M/Z: 259.2 [M+H]⁺.

**Step F:** The starting material 6-((1H-pyrazol-1-yl)methyl)-5-ethoxybenzo[d]isoxazol-3-amine (40 mg, 0.16 mmol) and 7-methoxy-3,4-dihydro-2H-benzo[b][1,4]dioxepine-6-sulfonyl chloride (86 mg, 0.32 mmol) were dissolved in anhydrous pyridine (1.0 mL). The reaction was carried out at 100 °C under microwave conditions for 3 hours.

After the disappearance of the starting material as monitored by LCMS, the reaction system was diluted with water (30 mL). The mixture was extracted with ethyl acetate (30 mL × 3), and the organic phases were combined. The combined organic phase was washed with saturated brine (30 mL × 3), dried over anhydrous sodium sulfate, filtered, and finally concentrated under reduced pressure. The crude product was purified by preparative liquid chromatography to afford 41.9 mg of N-(6-((1H-pyrazol-1-yl)methyl)-5-ethoxybenzo[d]isoxazol-3-yl)-7-methoxy-3,4-dihydro-2H-benzo[b][1,4]dioxepine-6-sulfonamide.

MS (ESI) M/Z: 501.2 [M+H]⁺.

¹H NMR (400 MHz, DMSO-*d₆*): δ 7.77 (d, *J* = 2.0 Hz, 1H), 7.47 (d, *J =* 1.6 Hz, 1H), 7.11 (s, 1H), 6.92 (d, *J* = 8.8 Hz, 1H), 6.62 - 6.55 (m, 2H), 6.27 (t, *J* = 2.0 Hz, 1H), 5.34 (s, 2H), 4.04 (q, *J* = 6.8 Hz, 2H), 3.92 - 3.85 (m, 2H), 3.84 - 3.77 (m, 2H), 1.92 (s, 2H), 1.36 (t, *J =* 6.8 Hz, 3H).

### Example 19:

### N-(6-((1H-Pyrazol-1-yl)methyl)-5-cyclopropoxybenzo[d]isoxazol-3-yl)-7-methoxy-3,4-d ihydro-2H-benzo [b] [1,4] dioxepine-6-sulfonamide

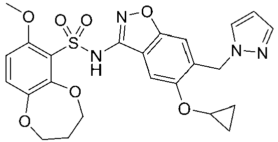

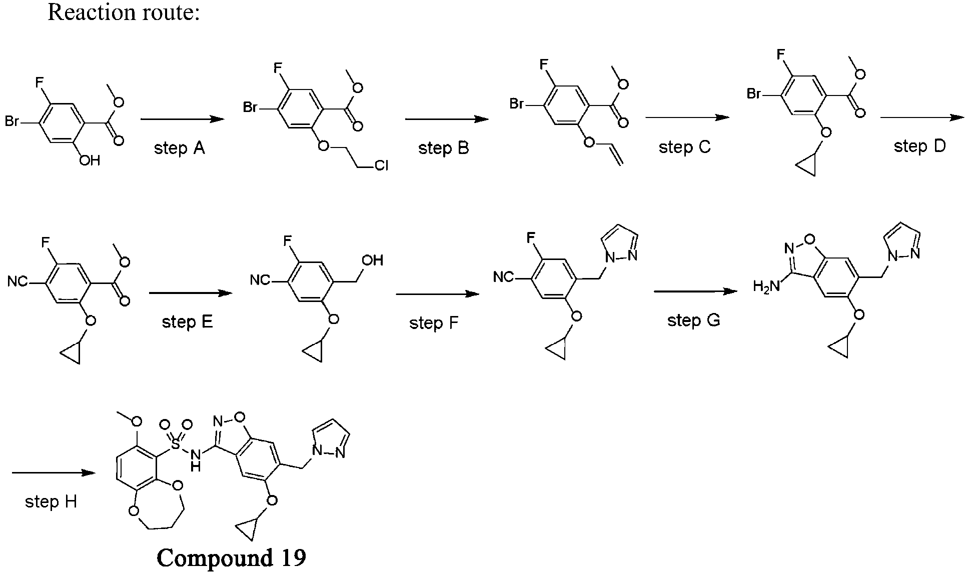

### Operating steps:

**Step A:** Methyl 4-bromo-5-fluoro-2-hydroxybenzoate (3.0 g, 12.0 mmol) and 2-chloroethyl 4-methylbenzenesulfonate (3.4 g, 14.5 mmol) were dissolved in N,N-dimethylformamide (30 mL). Anhydrous potassium carbonate (3.3 g, 24.0 mmol) was then added. The reaction mixture was gradually heated to 70 °C and reacted for 3 hours.

After the disappearance of the starting material as monitored by LCMS, the mixture was cooled to room temperature, diluted with water (50 mL), and extracted with ethyl acetate (50 mL × 2). The organic phase was washed with water (20 mL) and saturated brine (20 mL), dried over anhydrous sodium sulfate, filtered, and concentrated under reduced pressure to afford 3.2 g of crude methyl 4-bromo-2-(2-chloroethoxy)-5-fluorobenzoate.

MS (ESI) M/Z: 311.0 [M+H]⁺.

**Step B:** At room temperature, methyl 4-cyano-2-ethoxy-5-fluorobenzoate (3.2 g, 10.3 mmol) was dissolved in anhydrous tetrahydrofuran (30 mL). Under ice-bath conditions, potassium tert-butoxide (13.4 mL, 13.4 mmol) was slowly added dropwise to the mixture. The reaction system was slowly warmed to room temperature and stirred for 3 hours.

After the disappearance of the starting material as monitored by LCMS, water was added dropwise under ice-bath conditions to quench the reaction. The mixture was extracted with ethyl acetate (30 mL × 3). The organic phase was washed with water (50 mL) and saturated brine (50 mL), dried over anhydrous sodium sulfate, filtered, and concentrated under reduced pressure. The resulting crude product was purified by flash silica gel column chromatography to afford 1.75 g of methyl 4-bromo-5-fluoro-2-(vinyloxy)benzoate.

MS (ESI) M/Z: 277.0 [M+H]⁺.

**Step C:** At room temperature, methyl 4-bromo-5-fluoro-2-(vinyloxy)benzoate (1.75 g, 6.4 mmol) and chloroiodomethane (3.4 g, 19.1 mmol) were dissolved in anhydrous 1,2-dichloroethane (30 mL). Under ice-bath conditions, diethylzinc (1.3 g, 10.2 mmol) was added dropwise to the mixture. The reaction system was slowly warmed to room temperature and stirred for 2 hours.

After the disappearance of the starting material as monitored by LCMS, the mixture was cooled to 0 °C, and saturated ammonium chloride aqueous solution was added dropwise to quench the reaction. The mixture was extracted with ethyl acetate (30 mL × 3). The organic phase was washed with water (50 mL) and saturated brine (50 mL), dried over anhydrous sodium sulfate, filtered, and concentrated under reduced pressure. The resulting crude product was purified by flash silica gel column chromatography to afford 860 mg of methyl 4-bromo-2-cyclopropoxy-5-fluorobenzoate.

MS (ESI) M/Z: 289.1 [M+H]⁺.

¹H NMR (400 MHz, DMSO-*d₆*): δ 7.74 (d, *J* = 5.8 Hz, 1H), 7.63 (d, *J* = 8.8 Hz, 1H), 4.04 - 3.99 (m, 1H), 3.77 (s, 3H), 0.88 - 0.78 (m, 2H), 0.71 - 0.64 (m, 2H).

**Step D:** At room temperature, methyl 4-bromo-2-cyclopropoxy-5-fluorobenzoate (860 mg, 3.0 mmol) was dissolved in anhydrous N,N-dimethylformamide (10 mL). Copper(I) cyanide (696 mg, 6.0 mmol) was added to the mixture. The reaction system was slowly heated to 150 °C and stirred for 5 hours.

After the disappearance of the starting material as monitored by LCMS, the mixture was cooled to room temperature, and water was added dropwise to quench the reaction. The mixture was filtered through Celite. The filtrate was extracted with ethyl acetate (30 mL × 3). The organic phase was washed with water (50 mL) and saturated brine (50 mL), dried over anhydrous sodium sulfate, filtered, and concentrated under reduced pressure. The resulting crude product was purified by flash silica gel column chromatography to afford 360 mg of methyl 4-cyano-2-cyclopropoxy-5-fluorobenzoate.

MS (ESI) M/Z: 236.3 [M+H]⁺.

**Step E:** Methyl 4-cyano-2-cyclopropoxy-5-fluorobenzoate (360 mg, 1.5 mmol) was dissolved in anhydrous tetrahydrofuran (10 mL). Under ice-bath conditions, lithium borohydride (1.5 mL, 3.0 mmol) was added dropwise. The reaction system was warmed to room temperature and stirred for 4 hours.

After the disappearance of the starting material as monitored by LCMS, the mixture was cooled to 0 °C, and water was added dropwise to quench the reaction. The mixture was filtered through Celite. The filtrate was extracted with ethyl acetate (30 mL × 3). The organic phase was washed with water (50 mL) and saturated brine (50 mL), dried over anhydrous sodium sulfate, filtered, and concentrated under reduced pressure. The resulting crude product was purified by flash silica gel column chromatography to afford 230 mg of 5-cyclopropoxy-2-fluoro-4-(hydroxymethyl)benzonitrile.

MS (ESI) M/Z: 208.3 [M+H]⁺.

**Step F:** 5-Cyclopropoxy-2-fluoro-4-(hydroxymethyl)benzonitrile (230 mg, 1.1 mmol) and 1-(methylsulfonyl)-1H-pyrazole (178 mg, 1.2 mmol) were dissolved in N,N-dimethylformamide (8 mL). Anhydrous cesium carbonate (717 mg, 2.2 mmol) was then added. The reaction mixture was gradually heated to 70 °C and reacted for 3 hours.

After the disappearance of the starting material as monitored by LCMS, the mixture was cooled to room temperature, diluted with water (50 mL), and extracted with ethyl acetate (50 mL × 2). The organic phase was washed with water (20 mL) and saturated brine (20 mL), dried over anhydrous sodium sulfate, filtered, and concentrated under reduced pressure to afford a crude product. The resulting crude product was purified by flash silica gel column chromatography to afford 300 mg of 4-((1H-pyrazol-1-yl)methyl)-5-cyclopropoxy-2-fluorobenzonitrile.

MS (ESI) M/Z: 258.2 [M+H]⁺.

¹H NMR (400 MHz, DMSO-*d₆*): δ 7.78 (t, *J* = 4.0 Hz, 2H), 7.53 - 7.46 (m, 1H), 6.79 (d, *J* = 9.6 Hz, 1H), 6.30 (t, *J =* 2.0 Hz, 1H), 5.30 (s, 2H), 4.06 - 3.99 (m, 1H), 0.86 - 0.79 (m, 2H), 0.68 - 0.61 (m, 2H).

**Step G:** N-Hydroxyacetamide (88 mg, 1.2 mmol) was dissolved in N,N-dimethylformamide (5 mL). Potassium tert-butoxide (1.2 mL, 1.2 mmol) was then added, and the mixture was stirred at room temperature for 1 hour. Then, 4-((1H-pyrazol-1-yl)methyl)-5-cyclopropoxy-2-fluorobenzonitrile (100 mg, 0.39 mmol) was added to the mixture. The reaction mixture was heated to 60 °C and reacted for 3 hours.

After the completion of reaction as monitored by LCMS, the mixture was cooled to room temperature, diluted with water (50 mL), and extracted with ethyl acetate (50 mL × 2). The organic phase was washed with water (20 mL) and saturated brine (20 mL), dried over anhydrous sodium sulfate, filtered, and concentrated under reduced pressure to afford a crude product. The resulting crude product was purified by flash silica gel column chromatography to afford 40 mg of 6-((1H-pyrazol-1-yl)methyl)-5-cyclopropoxybenzo[d]isoxazol-3-amine.

MS (ESI) M/Z: 271.2 [M+H]⁺.

**Step H:** The starting material 6-((1H-pyrazol-1-yl)methyl)-5-cyclopropoxybenzo[d] isoxazol-3-amine (40 mg, 0.15 mmol) and 7-methoxy-3,4-dihydro-2H-benzo[b][1,4]dioxepine-6-sulfonyl chloride (83 mg, 0.3 mmol) were dissolved in anhydrous pyridine (1.0 mL). The reaction was carried out at 100 °C under microwave conditions for 3 hours.

After the disappearance of the starting material as monitored by LCMS, the reaction system was diluted with water (30 mL). The mixture was extracted with ethyl acetate (30 mL × 3), and the organic phases were combined. The combined organic phase was washed with saturated brine (30 mL × 3), dried over anhydrous sodium sulfate, filtered, and finally concentrated under reduced pressure. The crude product was purified by preparative liquid chromatography to afford 12.4 mg of N-(6-((1H-pyrazol-1-yl)methyl)-5-cyclopropoxybenzo[d]isoxazol-3-yl)-7-methoxy-3,4-dihydro-2H-benzo[b][1,4]dioxepine-6-sulfonamide.

MS (ESI) M/Z: 513.2 [M+H]⁺.

¹H NMR (400 MHz, DMSO-*d₆*): δ 11.53 (s, 1H), 7.93 (s, 1H), 7.77 (d, *J* = 2.0 Hz, 1H), 7.48 (d, *J =* 1.2 Hz, 1H), 7.21 (d, *J =* 9.2 Hz, 1H), 6.87 (s, 1H), 6.75 (d, *J =* 9.2 Hz, 1H), 6.29 (t, *J* = 2.0 Hz, 1H), 5.33 (s, 2H), 4.10 - 4.04 (m, 2H), 4.01 - 3.95 (m, 2H), 3.90 - 3.84 (m, 1H), 3.66 (s, 3H), 2.05 (dd, *J* = 10.4, 5.2 Hz, 2H), 0.85 (dd, *J* = 9.6, 3.6 Hz, 2H), 0.71 - 0.65 (m, 2H).

### Example 20:

### 7-Methoxy-N-(4-methoxy-6-(pyridin-2-yloxy)benzo[d]isoxazol-3-yl)-3,4-dihydro-2H-be nzo[b] [1,4]dioxepine-6-sulfonamide

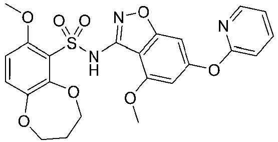

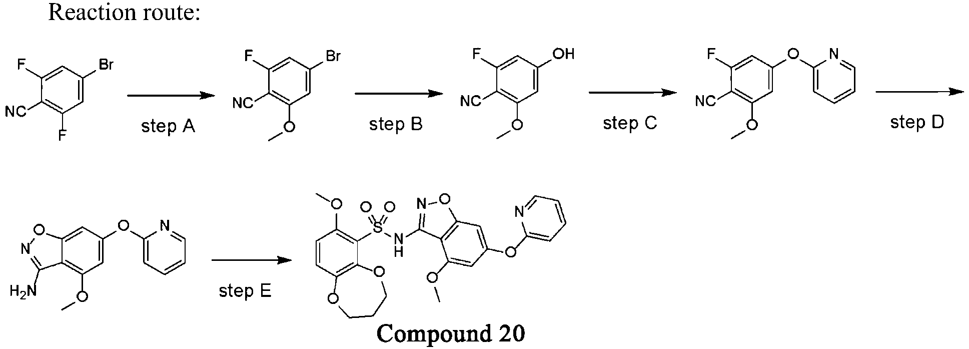

### Operating steps:

**Step A:** 4-Bromo-2,6-difluorobenzonitrile (4.34 g, 20 mmol) was dissolved in tetrahydrofuran (100 mL). Sodium methoxide (3.7 mL, 20 mmol) was added at 0 °C, and the reaction was stirred at this temperature for 1.5 hours.

After the disappearance of the starting material as monitored by LCMS, the mixture was extracted with ethyl acetate (100 mL × 2). The combined organic phases were washed with saturated brine (100 mL × 3), dried over anhydrous sodium sulfate, filtered, and concentrated under reduced pressure. The resulting residue was used directly in the next step without further purification.

MS (ESI) M/Z: 230.1 [M+H]⁺.

**Step B:** Under nitrogen protection, 4-bromo-2-fluoro-6-methoxybenzonitrile (4.5 g, 19.65 mmol), 2-dicyclohexylphosphino-2',4',6'-triisopropylbiphenyl (2.5 g, 5.89 mmol), tris(dibenzylideneacetone)dipalladium (5.4 g, 5.89 mmol), and potassium hydroxide (3.3 g, 58.95 mmol) were dissolved in 1,4-dioxane (40 mL) and water (40 mL). The reaction system was stirred at 100 °C overnight.

After the disappearance of the starting material as monitored by LCMS, the reaction mixture was adjusted to acidic pH with hydrochloric acid. The mixture was extracted with ethyl acetate (100 mL × 2). The combined organic phases were washed with saturated brine (150 mL × 2), dried over anhydrous sodium sulfate, filtered, and concentrated under reduced pressure. The resulting residue was purified by silica gel column chromatography to afford 2 g of 2-fluoro-4-hydroxy-6-methoxybenzonitrile.

MS (ESI) M/Z: 168.1 [M+H]⁺.

**Step C:** 2-Fluoro-4-hydroxy-6-methoxybenzonitrile (0.8 g, 4.79 mmol), methanesulfonic acid {[(R)-(-)-1-[(S)-2-(dicyclohexylphosphino)ferrocenyl]ethyldi-tert-butylphosphine}(2'-amino-1,1'-biphenyl-2-yl)palladium(II) (0.44 g, 0.479 mmol), cesium carbonate (1.56 g, 4.79 mmol), and 2-bromopyridine (1.37 mL, 14.37 mmol) were dissolved in toluene (20 mL). The reaction was carried out at 100 °C for 24 hours.

After the disappearance of the starting material as monitored by LCMS, the reaction mixture was quenched with water (10 mL) and extracted with ethyl acetate (20 mL × 2). The combined organic phases were washed with saturated brine (20 mL × 3), dried over anhydrous sodium sulfate, filtered, and concentrated under reduced pressure. The resulting residue was purified by silica gel column chromatography to afford 1.16 g of 2-fluoro-6-methoxy-4-(pyridin-2-yloxy)benzonitrile.

MS (ESI) M/Z: 245.1 [M+H]⁺.

**Step D:** 2-Fluoro-6-methoxy-4-(pyridin-2-yloxy)benzonitrile (1.16 g, 4.75 mmol), potassium carbonate (3.28 g, 23.75 mmol), and acetohydroxamic acid (1.07 g, 14.26 mmol) were dissolved in N,N-dimethylformamide (21 mL) and water (3 mL). The reaction system was stirred at 60 °C overnight.

After the disappearance of the starting material as monitored by LCMS, the reaction mixture was quenched by adding water (20 mL). The mixture was extracted with ethyl acetate (30 mL × 2). The combined organic phases were washed with saturated brine (30 mL × 2), dried over anhydrous sodium sulfate, filtered, and concentrated under reduced pressure. The resulting residue was used directly without further purification to afford 0.66 g of 4-methoxy-6-(pyridin-2-yloxy)benzo[d]isoxazol-3-amine.

MS (ESI) M/Z: 258.1 [M+H]⁺.

**Step E:** Under nitrogen protection, 7-methoxy-3,4-dihydro-2H-benzo[b][1,4]dioxepine-6-sulfonyl chloride (0.02 g, 0.072 mmol) and 4-methoxy-6-(pyridin-2-yloxy)benzo[d]isoxazol-3-amine (0.016 g, 0.06 mmol) were dissolved in pyridine (0.5 mL). The system was stirred at 100 °C under microwave conditions for 3 hours.

After the complete reaction of the starting material as monitored by LCMS, the reaction mixture was added to water (5 mL), diluted with ethyl acetate (5 mL), washed with saturated brine (3 mL), dried over anhydrous sodium sulfate, filtered, and the filtrate was concentrated under reduced pressure. The resulting mixture was purified by silica gel column chromatography (eluent: petroleum ether/ethyl acetate = 4/5) and reverse-phase preparative chromatography (eluent: 0.01vol% formic acid in water / acetonitrile = 1/1) to afford 2 mg of 7-methoxy-N-(4-methoxy-6-(pyridin-2-yloxy)benzo[d]isoxazol-3-yl)-3,4-dihydro-2H-benzo[b][ 1,4]dioxepine-6-sulfonamide.

MS (ESI) M/Z: 500.1 [M+H]⁺.

¹H NMR (400 MHz, DMSO-*d₆*): δ 8.20 (dd, *J =* 5.0, 1.9 Hz, 1H), 7.90 (td, *J=* 7.7, 2.0 Hz, 1H), 7.27 - 7.15 (m, 2H), 7.10 (d, *J* = 8.2 Hz, 1H), 6.89 (s, 1H), 6.78 (d, *J=* 9.1 Hz, 1H), 6.63 (s, 1H), 4.04 (dt, *J =* 28.4, 5.2 Hz, 4H), 3.83 (s, 3H), 3.71 (s, 3H).

### Example 21:

### 7-Methoxy-N-(4-methoxy-6-(thiazol-2-yloxy)benzo[d]isoxazol-3-yl)-3,4-dihydro-2H-be nzo[b] [1,4]dioxepine-6-sulfonamide

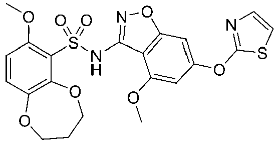

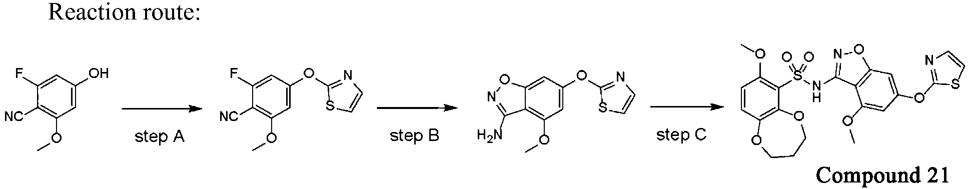

### Operating steps:

**Step A:** 2-Fluoro-4-hydroxy-6-methoxybenzonitrile (1.0 g, 5.99 mmol), methanesulfonic acid {[(R)-(-)-1-[(S)-2-(dicyclohexylphosphino)ferrocenyl]ethyldi-tert-butylphosphine}(2'-amino-1,1'-biphenyl-2-yl)palladium(II) (1.66 g, 1.80 mmol), cesium carbonate (5.8 g, 17.96 mmol), and 2-bromothiazole (1.64 mL, 17.96 mmol) were dissolved in toluene (20 mL). The reaction was carried out at 149 °C for 24 hours.

After the disappearance of the starting material as monitored by LCMS, the reaction mixture was quenched with water (10 mL) and extracted with ethyl acetate (20 mL × 2). The combined organic phases were washed with saturated brine (20 mL × 3), dried over anhydrous sodium sulfate, filtered, and concentrated under reduced pressure. The resulting residue was purified by silica gel column chromatography to afford 0.694 g of 2-fluoro-6-methoxy-4-(thiazol-2-yloxy)benzonitrile.

MS (ESI) M/Z: 251.1 [M+H]⁺.

**Step B:** 2-Fluoro-6-methoxy-4-(thiazol-2-yloxy)benzonitrile (0.694 g, 2.78 mmol), potassium carbonate (1.92 g, 13.88 mmol), and acetohydroxamic acid (0.625 g, 8.33 mmol) were dissolved in N,N-dimethylformamide (21 mL) and water (3 mL). The reaction system was stirred at 60 °C overnight.

After the disappearance of the starting material as monitored by LCMS, the reaction mixture was quenched by adding water (20 mL). The mixture was extracted with ethyl acetate (30 mL × 2). The combined organic phases were washed with saturated brine (30 mL × 2), dried over anhydrous sodium sulfate, filtered, and concentrated under reduced pressure. The resulting residue was used directly without further purification to afford 0.31 g of 4-methoxy-6-(thiazol-2-yloxy)benzo[d]isoxazol-3-amine.

MS (ESI) M/Z: 264.1 [M+H]⁺.

**Step C:** Under nitrogen protection, 7-methoxy-3,4-dihydro-2H-benzo[b][1,4]dioxepine-6-sulfonyl chloride (0.06 g, 0.228 mmol) and 4-methoxy-6-(thiazol-2-yloxy)benzo[d]isoxazol-3-amine (0.05 g, 0.19 mmol) were dissolved in pyridine (0.5 mL). The system was stirred at 100 °C under microwave conditions for 3 hours.

After the complete reaction of the starting material as monitored by LCMS, the reaction mixture was added to water (5 mL), diluted with ethyl acetate (5 mL), washed with saturated brine (3 mL), dried over anhydrous sodium sulfate, filtered, and the filtrate was concentrated under reduced pressure. The resulting mixture was purified by silica gel column chromatography (eluent: petroleum ether/ethyl acetate = 4/5) and reverse-phase preparative chromatography (eluent: 0.01vol% formic acid in water / acetonitrile = 1/1) to afford 2 mg of 7-methoxy-N-(4-methoxy-6-(thiazol-2-yloxy)benzo[d]isoxazol-3-yl)-3,4-dihydro-2H-benzo[b][1, 4]dioxepine-6-sulfonamide.

MS (ESI) M/Z: 506.0 [M+H]⁺.

¹H NMR (400 MHz, DMSO-*d₆*): δ 7.36 - 7.28 (m, 2H), 7.22 - 7.12 (m, 2H), 6.87 - 6.73 (m, 2H), 4.05 - 3.96 (m, 3H), 3.87 (s, 3H), 3.70 (s, 3H), 2.12 - 1.96 (m, 2H).

### Example 22:

### N-(5-((1H-Pyrazol-1-yl)methyl)-3,4-dihydro-2H-chromeno[8,7-d]isoxazol-9-yl)-7-meth oxy-3,4-dihydro-2H-benzo[b][1,4]dioxepine-6-sulfonamide

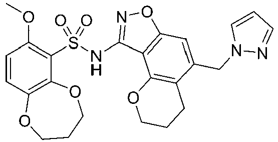

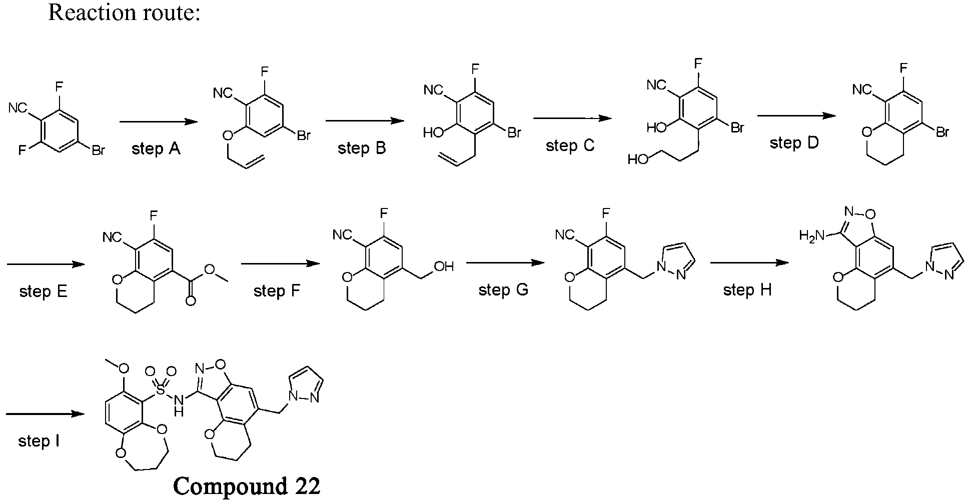

### Operating steps:

**Step A:** Under nitrogen protection, 4-bromo-2,6-difluorobenzonitrile (5.0 g, 23.04 mmol) and allyl alcohol (1.36 g, 23.54 mmol) were dissolved in a mixture of tetrahydrofuran (25 mL) and dioxane (25 mL). Sodium hydride (942 mg, 23.54 mmol, 60 wt%) was slowly added at 0°C. The reaction mixture was slowly warmed to 25°C and stirred for an additional 3 hours.

After LCMS showed the reaction was complete, the pH was adjusted to 3 with 1 M hydrochloric acid solution. The mixture was extracted with ethyl acetate (100 mL × 3). The combined organic phases were washed with saturated brine (100 mL) and dried over anhydrous sodium sulfate. After filtration and solvent removal under reduced pressure, the residue was purified by column chromatography to afford 6.3 g of 2-(allyloxy)-4-bromo-6-fluorobenzonitrile.

MS (ESI) M/Z: 256.0, 258.0 [M+H]⁺.

**Step B:** Under nitrogen protection, a solution of 2-(allyloxy)-4-bromo-6-fluorobenzonitrile (6.3 g, 24.61 mmol) in 1,2-dichlorobenzene (50 mL) was stirred at 175°C for 8 hours.

After the disappearance of most of the starting materials as monitored by LCMS, the reaction mixture was cooled to room temperature. The solvent was removed under reduced pressure and the residue was purified by column chromatography to afford 4.2 g of 3-allyl-4-bromo-6-fluoro-2-hydroxybenzonitrile.

MS (ESI) M/Z: 256.0, 258.0 [M+H]⁺.

¹H NMR (400 MHz, DMSO-*d₆*): δ 11.49 (s, 1H), 7.38 (d, *J* = 8.9 Hz, 1H), 5.87 - 5.77 (m, 1H), 5.04 - 5.01 (m, 1H), 4.92 - 4.87 (m, 1H), 3.52 (d, *J* = 5.7 Hz, 2H).

**Step C:** Under nitrogen protection, borane-tetrahydrofuran complex (18.1 mL, 18.07 mmol, 1M in tetrahydrofuran) was slowly added dropwise to a solution of 3-allyl-4-bromo-6-fluoro-2-hydroxybenzonitrile (4.2 g, 16.41 mmol) in tetrahydrofuran at 0°C. After stirring at 0°C for 1 hour, 30wt% hydrogen peroxide solution (50 mL) and saturated sodium bicarbonate aqueous solution (50 mL) were added and the mixture was stirred at 0°C for 1 hour.

After LCMS showed the reaction was complete, the mixture was quenched at 0°C with saturated sodium thiosulfate aqueous solution and acidified to pH=2 with concentrated hydrochloric acid. The mixture was extracted with ethyl acetate (100 mL × 3). The combined organic phases were washed with saturated brine (100 mL) and dried over anhydrous sodium sulfate. After filtration and solvent removal, the residue was purified by column chromatography to afford 2.7 g of 4-bromo-6-fluoro-2-hydroxy-3-(3-hydroxypropyl)benzonitrile.

MS (ESI) M/Z: 274.0 [M+H]⁺.

**Step D:** Under nitrogen protection, diethyl azodicarboxylate (1.43 mL, 9.85 mmol) was slowly added dropwise to a solution of 4-bromo-6-fluoro-2-hydroxy-3-(3-hydroxypropyl) benzonitrile (2.7 g, 9.85 mmol) and triphenylphosphine (2.58 g, 9.85 mmol) in anhydrous tetrahydrofuran (100 mL) under ice-bath conditions. The reaction mixture was stirred at 0°C for 0.5 hours.

After LCMS showed the reaction was complete, saturated ammonium chloride aqueous solution (50 mL) was added to quench the reaction. The mixture was extracted with ethyl acetate (100 mL × 3). The combined organic phases were washed with saturated sodium chloride solution (100 mL), dried over anhydrous sodium sulfate, filtered, and concentrated under reduced pressure. The residue was purified by column chromatography to afford 1.5 g of 5-bromo-7-fluorochroman-8-carbonitrile.

MS (ESI) M/Z: 256.0, 258.0 [M+H]⁺.

**Step E:** 5-Bromo-7-fluorochroman-8-carbonitrile (1.5 g, 5.86 mmol) was dissolved in methanol (30 mL). Palladium acetate (263 mg, 1.17 mmol) and triethylamine (2.44 mL, 17.58 mmol) were added. After evacuating and refilling with carbon monoxide three times, the reaction mixture was stirred at 65°C under a carbon monoxide atmosphere for 16 hours.

After the completion of the reaction as monitored by LCMS, methanol was removed by concentration under reduced pressure. Ethyl acetate (30 mL) and water (50 mL) were added. The mixture was extracted with ethyl acetate (50 mL × 3). The combined organic phases were washed with saturated sodium chloride solution (100 mL), dried over anhydrous sodium sulfate, filtered, and the filtrate was concentrated under reduced pressure. The resulting residue was purified by silica gel column chromatography to afford 1.0 g of the target product, methyl 8-cyano-7-fluorochroman-5-carboxylate.

MS (ESI) M/Z: 236.1 [M+H]⁺.

¹H NMR (400 MHz, DMSO-*d₆*): δ 7.32 (d, *J* = 9.6 Hz, 1H), 4.45 - 4.31 (m, 2H), 3.86 (s, 3H), 2.92 (t, *J =* 6.0 Hz, 2H), 1.99 - 1.90 (m, 2H).

**Step F:** Under nitrogen protection, methyl 8-cyano-7-fluorochroman-5-carboxylate (800 mg, 3.40 mmol) was dissolved in anhydrous tetrahydrofuran (15 mL). Lithium borohydride (3.40 mL, 6.80 mmol, 2M in tetrahydrofuran) was added dropwise slowly at 0°C. Then the reaction mixture was stirred at 0°C for 1 hour.

After the completion of the reaction as monitored by LCMS, water (20 mL) was slowly added dropwise at 0°C. The mixture was extracted with ethyl acetate (50 mL × 3). The combined organic phases were washed with saturated sodium chloride solution (100 mL), dried over anhydrous sodium sulfate, filtered, and the filtrate was concentrated under reduced pressure to afford 700 mg of the target product, 7-fluoro-5-(hydroxymethyl)chroman-8-carbonitrile.

MS (ESI) M/Z: 208.1 [M+H]⁺.

**Step G:** Under nitrogen protection, 7-fluoro-5-(hydroxymethyl)chroman-8-carbonitrile (700 mg, 3.38 mmol), 1-(methylsulfonyl)-1H-pyrazole (593 mg, 4.06 mmol), and cesium carbonate (1.65 g, 5.07 mmol) were dissolved in acetonitrile (10 mL). The system was stirred at 70°C for 2 hours.

After the complete reaction of the starting material as monitored by LCMS, the reaction mixture was added to ethyl acetate (30 mL) and filtered. The filter cake was washed with ethyl acetate (50 mL), and the filtrate was concentrated under reduced pressure. The resulting residue was purified by silica gel column chromatography to afford 546 mg of the target product, 5-((1H-pyrazol-1-yl)methyl)-7-fluorochroman-8-carbonitrile.

MS (ESI) M/Z: 258.1 [M+H]⁺.

**Step H:** Under nitrogen protection, N-hydroxyacetamide (478 mg, 6.37 mmol) and potassium tert-butoxide (714 mg, 6.37 mmol) were dissolved in N,N-dimethylformamide (10 mL). The system was stirred at room temperature for 1 hour. Then, 5-((1H-pyrazol-1-yl)methyl)-7-fluorochroman-8-carbonitrile (546 mg, 2.12 mmol) was added. The system was stirred at 60°C for 6 hours.

After the complete reaction of the starting material as monitored by LCMS, the reaction mixture was quenched by adding water (30 mL) and extracted with ethyl acetate (50 mL × 3). The combined organic phases were washed with saturated brine (50 mL × 3), dried over anhydrous sodium sulfate, filtered, and the filtrate was concentrated under reduced pressure. The resulting residue was purified by silica gel column chromatography to afford 50 mg of the target product, 5-((1H-pyrazol-1-yl)methyl)-3,4-dihydro-2H-chromeno[8,7-d]isoxazol-9-amine.

MS (ESI) M/Z: 271.2 [M+H]⁺.

¹H NMR (400 MHz, DMSO-*d₆*): δ 7.78 (d, *J =* 2.2 Hz, 1H), 7.51 (d, *J=* 1.6 Hz, 1H), 6.33 - 6.31 (m, 2H), 5.86 (s, 2H), 5.38 (s, 2H), 4.31 - 4.17 (m, 2H), 2.67 (t, *J =* 6.4 Hz, 2H), 2.06 - 1.92 (m, 2H).

**Step I:** Under nitrogen protection, 5-((1H-pyrazol-1-yl)methyl)-3,4-dihydro-2H-chromeno[8,7-d]isoxazol-9-amine (50 mg, 0.18 mmol) and 7-methoxy-3,4-dihydro-2H-benzo[b][1,4]dioxepine-6-sulfonyl chloride (78 mg, 0.28 mmol) were dissolved in anhydrous pyridine (1 mL). The reaction system was stirred at 100°C for 16 hours.

After the completion of the reaction as monitored by LCMS, the reaction was quenched by adding water (5 mL). The mixture was extracted with ethyl acetate (30 mL × 3). The combined organic phases were washed with saturated ammonium chloride solution (50 mL × 3) and saturated sodium chloride solution (50 mL × 3), dried over anhydrous sodium sulfate, filtered, and the filtrate was concentrated under reduced pressure. The resulting residue was purified by reverse-phase preparative chromatography to afford 32.4 mg of the target product, N-(5-((1H-pyrazol-1-yl)methyl)-3,4-dihydro-2H-chromeno[8,7-d]isoxazol-9-yl)-7-methoxy-3,4-dihydro-2H-benzo[b][1,4]dioxepine-6-sulfonamide.

MS (ESI) M/Z: 513.2 [M+H]⁺.

¹H NMR (400 MHz, DMSO-*d₆*): δ 9.74 (s, 1H), 7.79 (d, *J=* 2.2 Hz, 1H), 7.51 (d, *J =* 1.6 Hz, 1H), 7.23 (d, *J =* 9.2 Hz, 1H), 6.79 (d, *J =* 9.2 Hz, 1H), 6.44 (s, 1H), 6.32 (t, *J =* 2.0 Hz, 1H), 5.42 (s, 2H), 4.21 - 4.15 (m, 2H), 4.12 - 4.06 (m, 2H), 4.02 - 3.97 (m, 2H), 3.71 (s, 3H), 2.69 (t, *J=* 6.4 Hz, 2H), 2.05 (d, *J=* 5.0 Hz, 2H), 2.02 - 1.96 (m, 2H).

### Example 23:

### N-(6-((1H-Pyrazol-1-yl)methyl)-4-methoxy-5-methylbenzo[d]isoxazol-3-yl)-7-methoxy-3,4-dihydro-2H-benzo[b][1,4]dioxepine-6-sulfonamide

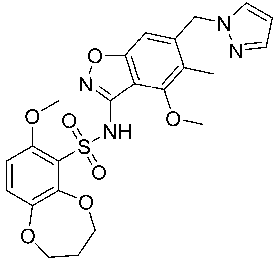

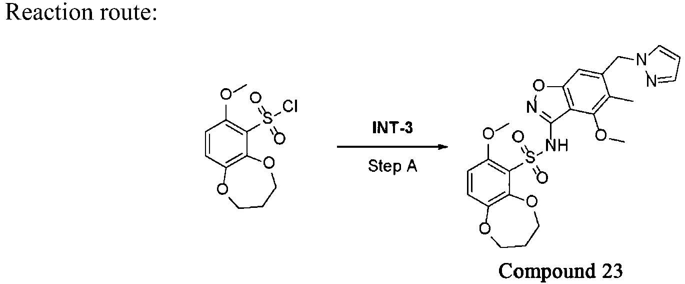

### Operating steps:

**Step A:** To a solution of 4-methoxy-5-methyl-6-((1H-pyrazol-1-yl)methyl)-1,2-benzoxazol-3-amine (30 mg, 0.12 mmol) in pyridine (1 mL) was added 7-methoxy-3,4-dihydro-2H-1,5-benzodioxepine-6-sulfonyl chloride (66.89 mg, 0.24 mmol). The reaction was carried out at 100 °C under microwave conditions for 3 hours. LCMS showed remaining starting material and formation of the target peak. The solvent was removed by concentration under reduced pressure, and purification by preparative chromatography afforded 3 mg of N-(6-((1H-pyrazol-1-yl)methyl)-4-methoxy-5-methylbenzo[d]isoxazol-3-yl)-7-methoxy-3,4-dihydro-2H-benzo[b][1,4]dioxepine-6-sulfonamide.

MS (ESI) M/Z: 501.1 [M+H]⁺.

¹H NMR (400 MHz, DMSO-*d₆*) δ 8.17 (s, 1H), 7.75 (d, *J* = 2.3 Hz, 1H), 7.50 (d, *J* = 1.8 Hz, 1H), 6.95 (s, 1H), 6.59 (d, *J =* 9.2 Hz, 1H), 6.37 (s, 1H), 6.30 (t, *J* = 2.1 Hz, 1H), 5.39 (s, 2H), 4.03 (s, 3H), 3.89 (d, *J =* 16.7 Hz, 4H), 3.56 (s, 3H), 2.17 (s, 3H), 1.94 (s, 2H).

### Example 24:

### N-(6-((1H-Pyrazol-1-yl)methyl)-4-methoxy-5-methylbenzo[d]isoxazol-3-yl)-5-methoxy-1,1a,2,7b-tetrahydrocyclopropa[c]chromene-4-sulfonamide

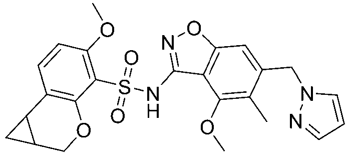

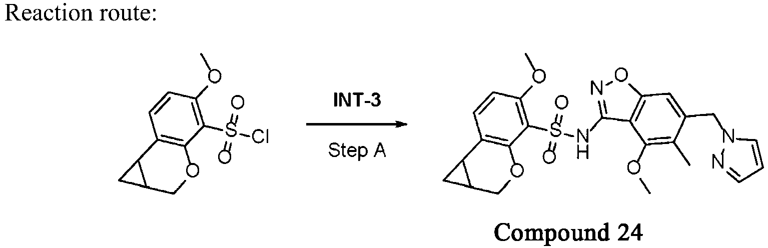

### Operating steps:

**Step A:** To a solution of 4-methoxy-5-methyl-6-((1H-pyrazol-1-yl)methyl)-1,2-benzoxazol-3-amine (20 mg, 0.077 mmol) in pyridine (0.3 mL) was added 9-methoxy-6-oxatricyclo[5.4.0.0^{2,4}]undeca-1(7),8,10-triene-8-sulfonyl chloride (31.73 mg, 0.12 mmol). The reaction was carried out at 100 °C under microwave conditions for 6 hours. LCMS showed consumption of the starting material and formation of the target peak. The solvent was removed by concentration under reduced pressure, and purification by preparative chromatography afforded 13 mg of N-(6-((1H-pyrazol-1-yl)methyl)-4-methoxy-5-methylbenzo[d] isoxazol-3-yl)-5-methoxy-1,1a,2,7b-tetrahydrocyclopropa[c]chromene-4-sulfonamide.

MS (ESI) M/Z: 497.1 [M+H]⁺.

¹H NMR (400 MHz, DMSO-*d₆*) δ 10.23 (s, 1H), 7.80 (d, *J =* 2.3 Hz, 1H), 7.53 (d, *J =* 1.8 Hz, 1H), 7.43 (s, 1H), 6.69 (s, 2H), 6.32 (t, *J =* 2.1 Hz, 1H), 5.48 (s, 2H), 4.23 (s, 1H), 3.76 (d, *J* = 34.1 Hz, 6H), 2.23 (s, 3H), 2.02 (s, 1H), 1.75 (s, 1H), 0.96 (s, 1H), 0.73 (q, *J =* 4.6 Hz, 1H).

### Example 25:

### N-(6-((1H-Pyrazol-1-yl)methyl)-4-methoxy-5-methylbenzo[d]isoxazol-3-yl)-6-methoxy-2H-spiro[benzofuran-3,1'-cyclopropane]-7-sulfonamide

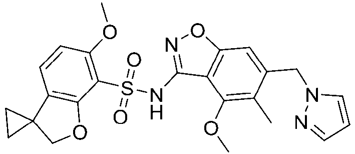

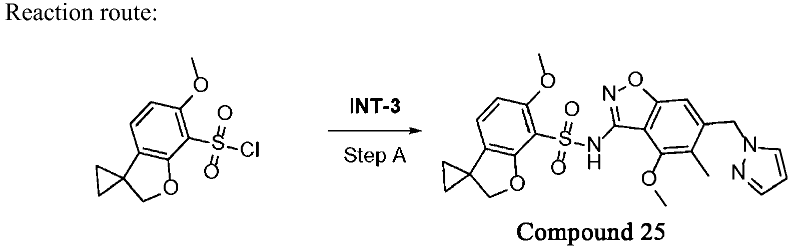

### Operating steps:

**Step A:** To a solution of 4-methoxy-5-methyl-6-((1H-pyrazol-1-yl)methyl)-1,2-benzoxazol-3-amine (20 mg, 0.077 mmol) in pyridine (0.3 mL) was added 6-methoxy-2H-spiro[benzofuran-3,1'-cyclopropane]-7-sulfonyl chloride (25.38 mg, 0.092 mmol). The reaction was carried out at 100 °C under microwave conditions for 6 hours. LCMS showed remaining starting material and formation of the target molecular peak. The solvent was removed by concentration under reduced pressure, and purification by preparative chromatography afforded 8 mg of N-(6-((1H-pyrazol-1-yl)methyl)-4-methoxy-5-methylbenzo[d]isoxazol-3-yl)-6-methoxy-2H-spiro[benzofuran-3,1'-cyclopropane]-7-sulfonamide.

MS (ESI) M/Z: 497.1 [M+H]⁺.

¹H NMR (400 MHz, DMSO-*d₆*) δ 10.28 (s, 1H), 7.79 (s, 1H), 7.52 (d, *J =* 1.8 Hz, 1H), 6.68 (s, 2H), 6.52 (s, 1H), 6.32 (t, *J* = 2.1 Hz, 1H), 5.48 (s, 2H), 4.48 (s, 2H), 3.87 (s, 3H), 3.71 (s, 3H), 2.23 (s, 3H), 1.01 (d, *J =* 12.2 Hz, 4H).

### Example 26:

### N-(6-((1H-Pyrazol-1-yl)methyl)-5-ethyl-4-methoxybenzo[d]isoxazol-3-yl)-7-methoxy-3, 4-dihydro-2H-benzo[b][1,4]dioxepine-6-sulfonamide

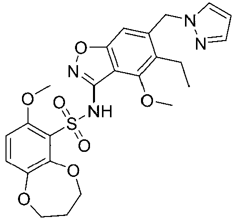

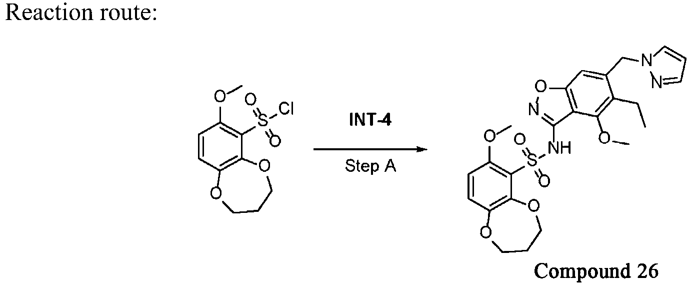

### Operating steps:

**Step A:** To a solution of 5-ethyl-4-methoxy-6-((1H-pyrazol-1-yl)methyl)-1,2-benzoxazol-3-amine (20 mg, 0.073 mmol) in pyridine (1 mL) was added 7-methoxy-3,4-dihydro-2H-1,5-benzodioxepine-6-sulfonyl chloride (61.04 mg, 0.22 mmol). The reaction was carried out at 100 °C under microwave conditions for 3 hours. LCMS showed formation of the target peak. The solvent was removed by concentration under reduced pressure, and purification by preparative chromatography afforded 13 mg of N-(6-((1H-pyrazol-1-yl)methyl)-5-ethyl-4-methoxybenzo[d]isoxazol-3-yl)-7-methoxy-3,4-dihyd ro-2H-benzo[b][1,4]dioxepine-6-sulfonamide.

MS (ESI) M/Z: 515.2 [M+H]⁺.

¹H NMR (400 MHz, DMSO-*d₆*) δ 10.50 (s, 1H), 7.84 (s, 1H), 7.51 (s, 1H), 7.24 (s, 1H), 6.79 (s, 2H), 6.32 (s, 1H), 5.52 (s, 2H), 4.03 (s, 5H), 3.87 (s, 3H), 3.72 (s, 2H), 2.75 (s, 2H), 2.06 (d, *J* = 10.8 Hz, 2H), 1.02 (s, 3H).

### Example 27:

### N-(6-((1H-Pyrazol-1-yl)methyl)-5-ethyl-4-methoxybenzo[d]isoxazol-3-yl)-5-methoxy-1, 1a,2,7b-tetrahydrocyclopropa[c]chromene-4-sulfonamide

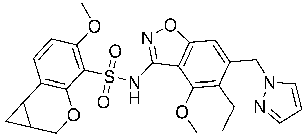

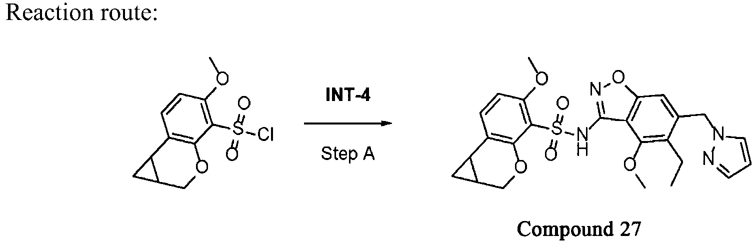

### Operating steps:

**Step A:** To a solution of 5-ethyl-4-methoxy-6-((1H-pyrazol-1-yl)methyl)-1,2-benzoxazol-3-amine (20 mg, 0.073 mmol) in pyridine (0.3 mL) was added 5-methoxy-1,1a,2,7b-tetrahydrocyclopropa[c]chromene-4-sulfonyl chloride (30.08 mg, 0.11 mmol). The reaction was carried out at 100 °C under microwave conditions for 6 hours. LCMS showed remaining starting material and formation of the target peak. The solvent was removed by concentration under reduced pressure, and the residue was purified by preparative chromatography to afford 12 mg of N-(6-((1H-pyrazol-1-yl)methyl)-5-ethyl-4-methoxybenzo[d] isoxazol-3-yl)-5-methoxy-1,1a,2,7b-tetrahydrocyclopropa[c]chromene-4-sulfonamide.

MS (ESI) M/Z: 511.2 [M+H]⁺.

¹H NMR (400 MHz, DMSO-*d₆*) δ 10.35 (s, 1H), 7.83 (s, 1H), 7.51 (d, *J =* 1.8 Hz, 2H), 6.71 (s, 2H), 6.31 (t, *J =* 2.1 Hz, 1H), 5.50 (s, 2H), 4.24 (s, 1H), 3.88 (s, 3H), 3.70 (s, 4H), 2.74 (d, *J=* 7.7 Hz, 2H), 2.01 (s, 1H), 1.74 (s, 1H), 1.01 (t, *J =* 7.3 Hz, 4H), 0.73 (q, *J =* 4.6 Hz, 1H).

### Example 28:

### N-(6-((1H-Pyrazol-1-yl)methyl)-5-ethyl-4-methoxybenzo[d]isoxazol-3-yl)-6-methoxy-2 H-spiro[benzofuran-3,1'-cyclopropane]-7-sulfonamide

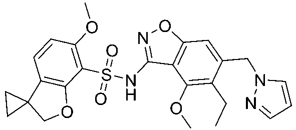

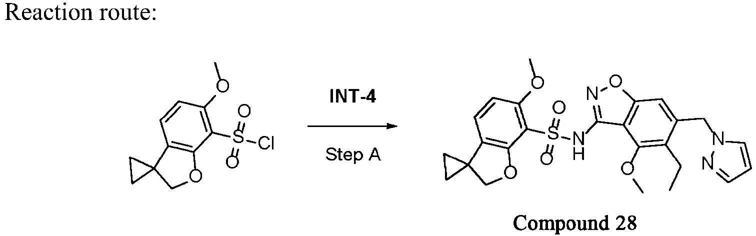

### Operating steps:

**Step A:** To a solution of 5-ethyl-4-methoxy-6-((1H-pyrazol-1-yl)methyl)-1,2-benzoxazol-3-amine (20 mg, 0.073 mmol) in pyridine (0.3 mL) was added 6-methoxy-2H-spiro[benzofuran-3,1'-cyclopropane]-7-sulfonyl chloride (24.07 mg, 0.088 mmol). The reaction was carried out at 100 °C under microwave conditions for 6 hours. LCMS showed remaining starting material and formation of the target peak. The solvent was removed by concentration under reduced pressure, and purification by preparative chromatography afforded 8 mg of N-(6-((1H-pyrazol-1-yl)methyl)-5-ethyl-4-methoxybenzo[d]isoxazol-3-yl)-6-methoxy-2H-spiro[benzofuran-3,1'-cyclopropane]-7-sulfonamide.

MS (ESI) M/Z: 511.2 [M+H]⁺.

¹H NMR (400 MHz, DMSO-*d₆*) δ 7.78 (s, 1H), 7.49 (d, *J* = 1.8 Hz, 1H), 6.54 (d, *J* = 125.0 Hz, 3H), 6.30 (d, *J* = 2.1 Hz, 1H), 5.44 (s, 2H), 4.39 (s, 2H), 4.07 (s, 3H), 3.59 (s, 3H), 2.75 - 2.67 (m, 2H), 0.97 (d, *J=* 11.3 Hz, 7H).

### Example 29:

### N-(6-((1H-Pyrazol-1-yl)methyl)-5-fluoro-4-methoxybenzo[d]isoxazol-3-yl)-7-methoxy-3,4-dihydro-2H-benzo[b][1,4]dioxepine-6-sulfonamide

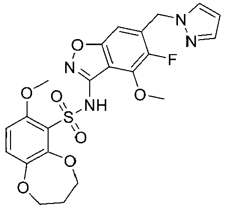

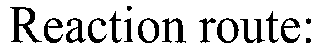

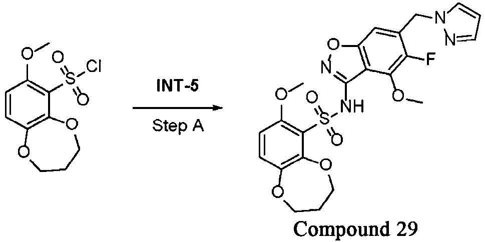

### Operating steps:

**Step A:** To a solution of 5-fluoro-4-methoxy-6-((1H-pyrazol-1-yl)methyl)-1,2-benzoxazol-3-amine (60 mg, 0.23 mmol) in pyridine (1 mL) was added 7-methoxy-3,4-dihydro-2H-1,5-benzodioxepine-6-sulfonyl chloride (76.92 mg, 0.28 mmol). The reaction was carried out at 100 °C under microwave conditions for 3 hours. LCMS showed consumption of the starting material and formation of the product peak. The solvent was removed by concentration under reduced pressure, and purification by preparative chromatography afforded 35 mg of N-(6-((1H-pyrazol-1-yl)methyl)-5-fluoro-4-methoxybenzo[d]isoxazol-3-yl)-7-methoxy-3,4-dihydro-2H-benzo[b][1,4]dioxepine-6-sulfonami de.

MS (ESI) M/Z: 505.1 [M+H]⁺.

¹H NMR (400 MHz, DMSO-*d₆*) δ 10.36 (s, 1H), 7.86 (d, *J* = 2.2 Hz, 1H), 7.50 (d, *J =* 1.8 Hz, 1H), 7.24 (d, *J =* 9.1 Hz, 1H), 6.92 (s, 1H), 6.80 (d, *J =* 9.1 Hz, 1H), 6.30 (d, *J =* 2.1 Hz, 1H), 5.50 (s, 2H), 4.04 (d, *J =* 19.0 Hz, 7H), 3.71 (s, 3H), 2.06 (d, *J =* 10.2 Hz, 2H).

### Example 30:

### N-(6-((1H-Pyrazol-1-yl)methyl)-5-fluoro-4-methoxybenzo[d]isoxazol-3-yl)-5-methoxy-1,1a,2,7b-tetrahydrocyclopropa[c]chromene-4-sulfonamide

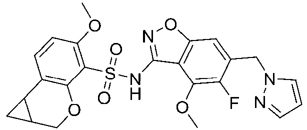

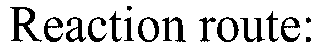

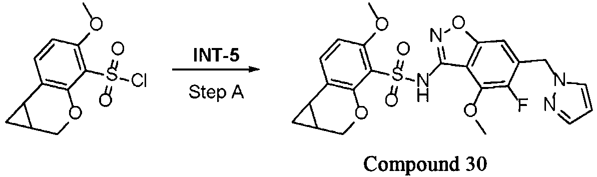

### Operating steps:

**Step A:** To a solution of 5-fluoro-4-methoxy-6-((1H-pyrazol-1-yl)methyl)-1,2-benzoxazol-3-amine (20 mg, 0.076 mmol) in pyridine (0.3 mL) was added 5-methoxy-1,1a,2,7b-tetrahydrocyclopropa[c]chromene- 4-sulfonyl chloride (31.32 mg, 0.11 mmol). The reaction was carried out at 100 °C under microwave conditions for 6 hours. LCMS showed consumption of the starting material and formation of the target peak. The solvent was removed by concentration under reduced pressure, and the residue was purified by preparative chromatography to afford 8 mg of N-(6-((1H-pyrazol-1-yl)methyl)-5-fluoro-4-methoxybenzo[d]isoxazol-3-yl)-5-methoxy-1,1a,2,7b-tetrahydrocyclopropa[c]chromene-4-sulfon amide.

MS (ESI) M/Z: 501.1 [M+H]⁺.

¹H NMR (400 MHz, DMSO-*d₆*) δ 7.85 (d, *J =* 2.3 Hz, 1H), 7.50 (d, *J=* 1.8 Hz, 1H), 7.35 (s, 1H), 6.73 (d, *J* = 55.8 Hz, 2H), 6.30 (t, *J* = 2.1 Hz, 1H), 5.48 (s, 2H), 4.07 (s, 4H), 3.68 (s, 3H), 3.52 (s, 1H), 1.98 (s, 1H), 1.71 (s, 1H), 0.93 (s, 1H), 0.71 (q, *J =* 4.6 Hz, 1H).

### (1aR,7bS)-N-(6-((1H-Pyrazol-1-yl)methyl)-5-fluoro-4-methoxybenzo[d]isoxazol-3-yl)-5 -methoxy-1,1a,2,7b-tetrahydrocyclopropa[c]chromene-4-sulfonamide / (1aS,7bR)-N-(6-((1H-pyrazol-1-yl)methyl)-5-fluoro-4-methoxybenzo[d]isoxazol-3-yl)-5-methoxy-1,1a,2,7b-t etrahydrocyclopropa[c]chromene-4-sulfonamide

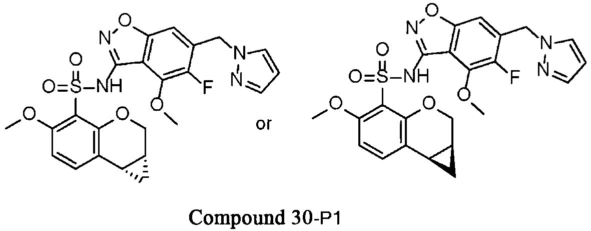

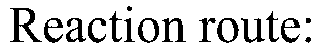

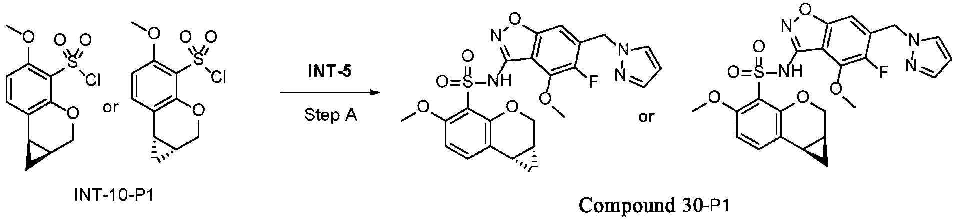

### Operating steps:

**Step A:** To a solution of 5-fluoro-4-methoxy-6-((1H-pyrazol-1-yl)methyl)-1,2-benzoxazol-3-amine (20 mg, 0.076 mmol) in pyridine (0.5 mL) was added INT-10-P1 (31.32 mg, 0.11 mmol). The reaction was carried out at 100 °C under microwave conditions for 6 hours. LCMS showed remaining starting material and formation of the target peak. The solvent was removed by concentration under reduced pressure, and purification by preparative chromatography afforded 9 mg of Compound 30-P1.

MS (ESI) M/Z: 501.1 [M+H]⁺.

¹H NMR (400 MHz, DMSO-*d₆*) δ 10.21 (s, 1H), 7.85 (d, *J =* 2.3 Hz, 1H), 7.55 - 7.35 (m, 2H), 6.79 (d, *J* = 73.5 Hz, 2H), 6.30 (t, *J* = 2.1 Hz, 1H), 5.49 (s, 2H), 4.24 (s, 1H), 4.05 (s, 3H), 3.70 (s, 4H), 2.01 (s, 1H), 1.74 (s, 1H), 0.95 (s, 1H), 0.71 (q, *J =* 4.6 Hz, 1H).

### Example 31:

### N-(6-((1H-Pyrazol-1-yl)methyl)-5-fluoro-4-methoxybenzo[d]isoxazol-3-yl)-6-methoxy-2H-spiro[benzofuran-3,1'-cyclopropane]-7-sulfonamide

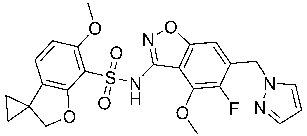

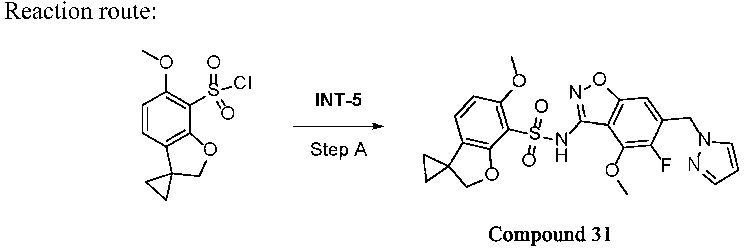

### Operating steps:

**Step A:** To a solution of 5-fluoro-4-methoxy-6-((1H-pyrazol-1-yl)methyl)-1,2-benzoxazol-3-amine (20 mg, 0.076 mmol) in pyridine (0.3 mL) was added 6-methoxy-2H-spiro[benzofuran-3,1'-cyclopropane]-7-sulfonyl chloride (31.32 mg, 0.11 mmol). The reaction was carried out at 100 °C under microwave conditions for 6 hours. LCMS showed consumption of the starting material and formation of the target peak. The solvent was removed by concentration under reduced pressure, and purification by preparative chromatography afforded 6 mg of N-(6-((1H-pyrazol-1-yl)methyl)-5-fluoro-4-methoxybenzo[d]isoxazol-3-yl)-6-methoxy-2H-spiro[benzofuran-3,1'-cyclopropane]-7-sulfonamide.

MS (ESI) M/Z: 501.1 [M+H]⁺.

¹H NMR (400 MHz, DMSO-*d₆*) δ 7.85 (d, *J* = 2.3 Hz, 1H), 7.49 (d, *J =* 1.8 Hz, 1H), 6.81 (s, 2H), 6.46 (s, 1H), 6.30 (t, *J =* 2.1 Hz, 1H), 5.47 (s, 2H), 4.45 (s, 2H), 4.09 (s, 3H), 3.65 (s, 3H), 1.10 - 0.90 (m, 4H).

### Example 32:

### N-(6-((1H-Pyrazol-1-yl)methyl)-4-methoxybenzo[d]isoxazol-3-yl)-7-methoxy-2H,4H-sp iro[benzo[b][1,4]dioxepine-3,3'-oxetane]-6-sulfonamide

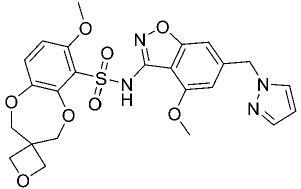

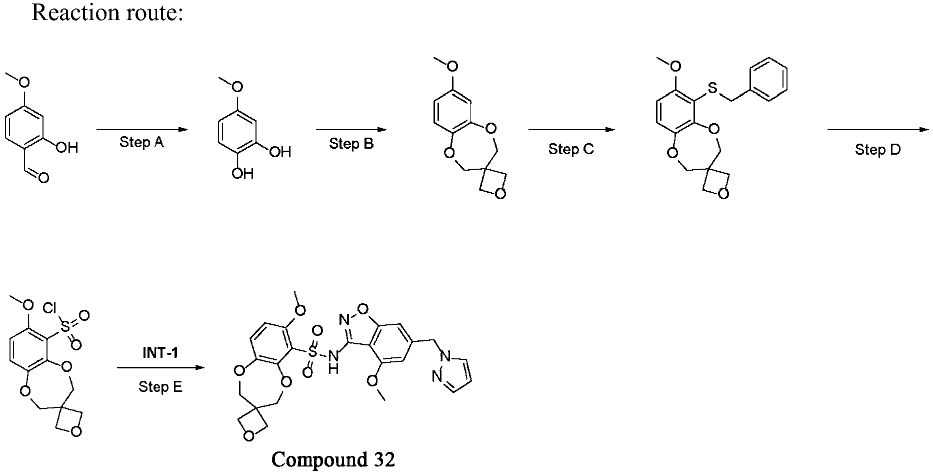

### Operating steps:

**Step A:** At 0 °C, sodium percarbonate (2:3) (5.16 g, 16.43 mmol) was added to a solution of 2-hydroxy-4-methoxybenzaldehyde (5 g, 32.86 mmol) in tetrahydrofuran (125 mL) and water (50 mL). The reaction was then stirred at 25 °C for 1 hour. LCMS showed the starting material had disappeared and the target peak had formed. The pH was adjusted to 2-3 with 6M hydrochloric acid, and the mixture was extracted with ethyl acetate (150 mL × 2). The combined organic phases were washed with saturated brine, dried over sodium sulfate, filtered, concentrated, and purified by column chromatography to afford 3.8 g of 4-methoxybenzene-1,2-diol.

¹H NMR (400 MHz, DMSO-*d₆*) δ 8.85 (s, 1H), 8.34 (s, 1H), 6.61 (d, *J =* 8.6 Hz, 1H), 6.34 (d, *J =* 2.9 Hz, 1H), 6.18 (dd, *J* = 8.6, 3.0 Hz, 1H), 3.61 (s, 3H).

**Step B:** Potassium carbonate (5916.77 mg, 42.81 mmol) and potassium iodide (236.88 mg, 1.43 mmol) were added to a solution of 4-methoxybenzene-1,2-diol (2 g, 14.27 mmol) and 3,3-bis(bromomethyl)oxetane (5221.11 mg, 21.41 mmol) in N,N-dimethylformamide (40 mL). The mixture was stirred at 100 °C for 5 hours. LCMS showed that the main peak was the target peak. Water (80 mL) was added to the reaction mixture, which was then extracted with ethyl acetate (40 mL × 2). The combined organic phases were washed with saturated brine (40 mL), dried over sodium sulfate, filtered, concentrated, and purified by column chromatography to afford 2.1 g of 7-methoxy-2,4-dihydrospiro[1,5-benzodioxepine-3,3'-oxetane].

MS (ESI) M/Z: 223.1 [M+H]⁺.

**Step C:** At 0 °C, n-butyllithium (319.98 mg, 5.00 mmol) was added dropwise to a solution of 7-methoxy-2,4-dihydrospiro[1,5-benzodioxepine-3,3'-oxetane] (1000 mg, 4.50 mmol) and N,N,N',N'-tetramethylethylenediamine (575.19 mg, 4.95 mmol) in tetrahydrofuran (15 mL). The reaction was stirred at 0 °C for 1 hour. The temperature was lowered to -70 °C, and a solution of [(benzyldisulfanyl)methyl]benzene (1219.63 mg, 4.95 mmol) in tetrahydrofuran (5 mL) was added to the reaction mixture. The reaction was stirred at -70 °C for 0.5 hours, warmed to room temperature, and then stirred for 1 hour. LCMS showed that the main peak was the target molecular peak. At 0 °C, saturated ammonium chloride (5 mL) and water (20 mL) were added to the reaction mixture, which was then extracted with ethyl acetate (20 mL × 2). The combined organic phases were washed with saturated brine, dried over sodium sulfate, filtered, concentrated, and purified by column chromatography to afford 1.4 g of the product, 6-(benzylthio)-7-methoxy-2,4-dihydrospiro[1,5-benzodioxepine-3,3'-oxetane].

MS (ESI) M/Z: 345.1 [M+H]⁺.

**Step D:** At 0 °C, N-chlorosuccinimide (176.26 mg, 1.32 mmol) was added to a solution of 6-(benzylthio)-7-methoxy-2,4-dihydrospiro[1,5-benzodioxepine-3,3'-oxetane] (150 mg, 0.44 mmol) in acetic acid (5 mL) and water (0.5 mL). The mixture was stirred at 0-10 °C for 1 hour. TLC (petroleum ether:ethyl acetate = 2:1) showed the starting material had disappeared and a new spot had formed, consistent with the pilot product's polarity. Water (10 mL) was added to the reaction mixture, which was then extracted with ethyl acetate (5 mL × 2). The combined organic phases were washed with saturated brine, dried over sodium sulfate, filtered, concentrated, and purified by column chromatography to afford 75 mg of 7-methoxy-2,4-dihydrospiro[1,5-benzodioxepine-3,3'-oxetane]-6-sulfonyl chloride.

¹H NMR (400 MHz, Chloroform-d) δ 7.23 (d, *J* = 9.2 Hz, 1H), 6.62 (d, *J* = 9.2 Hz, 1H), 4.55 (d, *J =* 6.7 Hz, 2H), 4.48 (d, *J* = 6.8 Hz, 2H), 4.43 (s, 2H), 4.29 (s, 2H), 3.87 (s, 3H).

**Step E:** 7-Methoxy-2,4-dihydrospiro[1,5-benzodioxepine-3,3'-oxetane]-6-sulfonyl chloride (39.45 mg, 0.12 mmol) was added to a solution of 4-methoxy-6-((1H-pyrazol-1-yl)methyl)-1,2-benzoxazol-3-amine (20 mg, 0.082 mmol) in pyridine (0.5 mL). The mixture was stirred at 100 °C for 12 hours. LCMS showed formation of the target peak. The mixture was concentrated under reduced pressure and purified by preparative chromatography to afford 3.3 mg of the product, N-(6-((1H-pyrazol-1-yl)methyl)-4-methoxybenzo[d]isoxazol-3-yl)-7-methoxy-2H,4H-spiro[benzo[b][1,4]dioxepine-3,3'-oxetane]-6-sulfonamide.

MS (ESI) M/Z: 529.2 [M+H]⁺.

¹H NMR (400 MHz, DMSO-*d₆*) δ 7.85 (d, *J =* 2.3 Hz, 1H), 7.48 (d, *J =* 1.9 Hz, 1H), 7.05 (s, 1H), 6.67 - 6.55 (m, 3H), 6.28 (t, *J =* 2.1 Hz, 1H), 5.39 (s, 2H), 4.32 (s, 4H), 4.13 (d, *J=* 7.8 Hz, 4H), 3.83 (s, 3H), 3.59 (s, 3H).

### Example 33:

### (1aR,7bS)-N-(6-((1H-Pyrazol-1-yl)methyl)-5-methoxybenzo[d]isoxazol-3-yl)-5-methoxy -1,1a,2,7b-tetrahydrocyclopropa[c]chromene-4-sulfonamide / (1aS,7bR)-N-(6-((1H-pyrazol-1-yl)methyl)-5-methoxybenzo[d]isoxazol-3-yl)-5-methoxy-1,1a,2,7b-tetrahydrocyclopropa[c ]chromene-4-sulfonamide

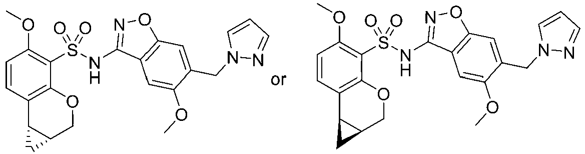

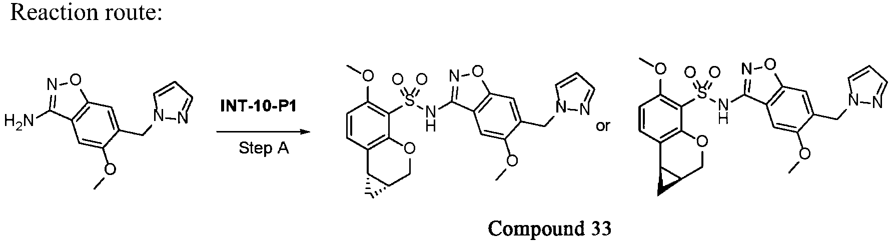

### Operating steps:

**Step A:** To a solution of 5-methoxy-6-((1H-pyrazol-1-yl)methyl)-1,2-benzoxazol-3-amine (15 mg, 0.061 mmol) in pyridine (0.5 mL) was added (1aR,7bS)-5-methoxy-1,1a,2,7b-tetrahydrocyclopropa[c]chromene- 4-sulfonyl chloride (25.14 mg, 0.091 mmol). The reaction was carried out at 100 °C under microwave conditions for 6 hours. TLC showed the starting material had disappeared and a new spot had formed. The solvent was removed by concentration under reduced pressure, and purification by preparative chromatography afforded 21 mg of (1aR,7bS)-N-(6-((1H-pyrazol-1-yl)methyl)-5-methoxybenzo[d]isoxazol-3-yl)-5-methoxy-1,1a,2,7b-tetrahydrocyclopropa[c]chromene-4-sulfon amide.

MS (ESI) M/Z: 483.1 [M+H]⁺.

¹H NMR (400 MHz, DMSO-*d₆*) δ 11.35 (s, 1H), 7.81 (d, *J =* 2.3 Hz, 1H), 7.59 (s, 1H), 7.50 (d, *J =* 1.8 Hz, 1H), 7.40 (d, *J =* 8.5 Hz, 1H), 6.79 (s, 1H), 6.67 (d, *J =* 8.6 Hz, 1H), 6.30 (t, *J =* 2.1 Hz, 1H), 5.39 (s, 2H), 4.22 (s, 1H), 3.85 (s, 3H), 3.69 (s, 3H), 3.47 (d, *J=* 10.9 Hz, 1H), 1.98 (td, *J =* 8.4, 4.3 Hz, 1H), 1.72 (d, *J =* 6.7 Hz, 1H), 0.93 (q, *J =* 3.8 Hz, 1H), 0.64 (q, *J =* 4.7 Hz, 1H).

### Example 34:

### (1aR,7bS)-N-(6-((1H-pyrazol-1-yl)methyl)-5-fluorobenzo[d]isoxazol-3-yl)-5-methoxy-1, 1a,2,7b-tetrahydrocyclopropa[c]chromene-4-sulfonamide / (laS,7bR)-N-(6-((1H-pyrazol-1-yl)methyl)-5-fluorobenzo[d]isoxazol-3-yl)-5-methoxy-1,1a,2,7b-tetrahydrocyclopropa[c]c hromene-4-sulfonamide

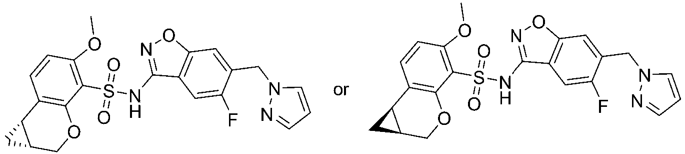

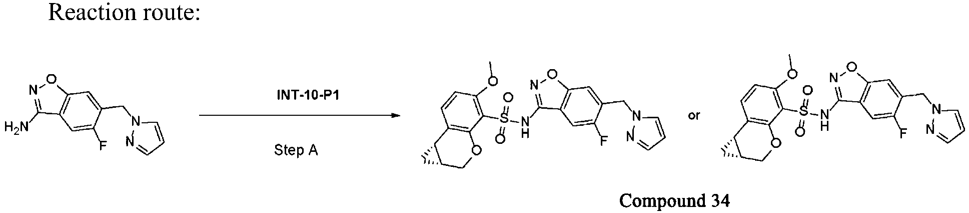

### Operating steps:

**Step A:** Under nitrogen protection, 6-((1H-pyrazol-1-yl)methyl)-5-fluorobenzo[d]isoxazol-3-amine (9 mg, 0.039 mmol) and (1aR,7bS)-5-methoxy-1,1a,2,7b-tetrahydrocyclopropa[c] chromene-4-sulfonyl chloride (16 mg, 0.058 mmol) were dissolved in pyridine (0.4 mL). The system was stirred at 100 °C under microwave conditions for 6 hours. After the complete reaction of the starting material as monitored by LCMS, the reaction mixture was added to water (5 mL), diluted with ethyl acetate (5 mL), washed with saturated brine (3 mL), dried over anhydrous sodium sulfate, filtered, and the filtrate was concentrated under reduced pressure. The resulting mixture was purified by silica gel column chromatography and reverse-phase preparative chromatography to afford 18 mg of (1aR,7bS)-N-(6-((1H-pyrazol-1-yl)methyl)-5-fluorobenzo[d]isoxazol-3-yl)-5-methoxy-1,1a,2,7b-tetrahydrocyclopropa[c]chromene-4-sulfona mide.

MS (ESI) M/Z: 471.1 [M+H]⁺.

¹H NMR (400 MHz, DMSO-*d₆*) δ 11.56 (s, 1H), 7.85 (d, *J =* 2.3 Hz, 1H), 7.77 (s, 1H), 7.49 (d, *J =* 1.8 Hz, 1H), 7.36 (s, 1H), 7.19 (s, 1H), 6.65 (s, 1H), 6.30 (t, *J=* 2.1 Hz, 1H), 5.49 (s, 2H), 4.16 (s, 1H), 3.67 (s, 3H), 2.00 (dd, *J =* 15.6, 8.2 Hz, 2H), 1.73 (d, *J =* 18.0 Hz, 1H), 0.98 - 0.88 (m, 1H), 0.66 (d, *J* = 4.9 Hz, 1H).

### Example 35:

### N-(4-((1H-Pyrazol-1-yl)methyl)-2,3-dihydrobenzofuro[7,6-d]isoxazol-8-yl)-7-methoxy-3,4-dihydro-2H-benzo[b][1,4]dioxepine-6-sulfonamide

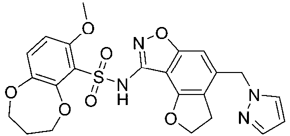

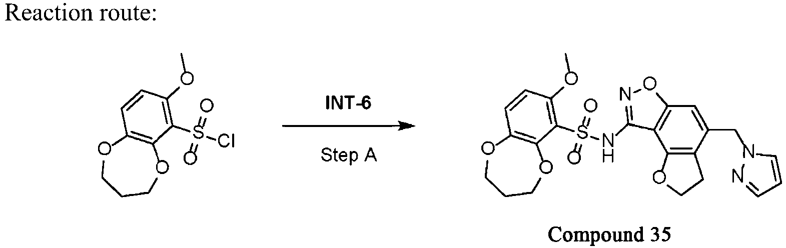

### Operating steps:

**Step A:** Under nitrogen protection, 4-((1H-pyrazol-1-yl)methyl)-2,3-dihydrobenzofuro [7,6-d]isoxazol-8-amine (8 mg, 0.031 mmol) and 7-methoxy-3,4-dihydro-2H-benzo[b][1,4]dioxepine-6-sulfonyl chloride (20 mg, 0.072 mmol) were dissolved in pyridine (0.4 mL). The system was stirred at 100 °C under microwave conditions for 6 hours. After the complete reaction of the starting material as monitored by LCMS, the reaction mixture was added to water (5 mL), diluted with ethyl acetate (5 mL), washed with saturated brine (3 mL), dried over anhydrous sodium sulfate, filtered, and the filtrate was concentrated under reduced pressure. The resulting mixture was purified by silica gel column chromatography and reverse-phase preparative chromatography to afford 6 mg of N-(4-((1H-pyrazol-1-yl)methyl)-2,3-dihydrobenzofuro[7,6-d]isoxazol-8-yl)-7-methoxy-3,4-dihydro-2H-benzo[b][1,4]dioxepine-6 -sulfonamide.

### MS (ESI) M/Z: 499.1 [M+H]⁺.

¹H NMR (400 MHz, DMSO-*d₆*) δ 10.68 (s, 1H), 7.82 (d, *J =* 2.3 Hz, 1H), 7.49 (d, *J* = 1.8 Hz, 1H), 7.20 (s, 1H), 6.71 (d, *J* = 33.6 Hz, 2H), 6.35 - 6.23 (m, 1H), 5.39 (s, 2H), 4.68 (d, *J* = 10.7 Hz, 2H), 3.98 (s, 4H), 3.67 (s, 3H), 3.14 - 3.01 (m, 2H), 2.00 (dd, *J* = 13.8, 6.8 Hz, 2H).

### Example 36:

### N-(4-((1H-Pyrazol-1-yl)methyl)-2,3-dihydrobenzofuro[7,6-d]isoxazol-8-yl)-6-methoxy-2H-spiro[benzofuran-3,1'-cyclopropane]-7-sulfonamide

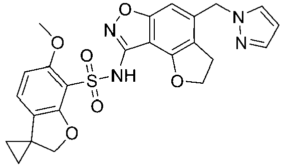

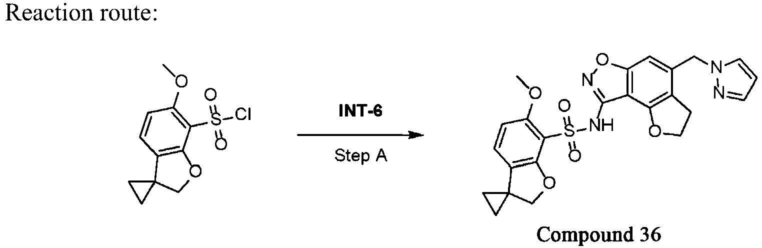

### Operating steps:

**Step A:** Under nitrogen protection, 4-((1H-pyrazol-1-yl)methyl)-2,3-dihydrobenzofuro[7,6-d]isoxazol-8-amine (12 mg, 0.047 mmol) and 6-methoxy-2H-spiro[benzofuran-3,1'-cyclopropane]-7-sulfonyl chloride (26 mg, 0.095 mmol) were dissolved in pyridine (0.4 mL). The system was stirred at 100 °C under microwave conditions for 6 hours. After the complete reaction of the starting material as monitored by LCMS, the reaction mixture was added to water (5 mL), diluted with ethyl acetate (5 mL), washed with saturated brine (3 mL), dried over anhydrous sodium sulfate, filtered, and the filtrate was concentrated under reduced pressure. The resulting mixture was purified by silica gel column chromatography and reverse-phase preparative chromatography to afford 8 mg of N-(4-((1H-pyrazol-1-yl)methyl)-2,3-dihydrobenzofuro[7,6-d]isoxazol-8-yl)-6-methoxy-2H-spiro [benzofuran-3,1'-cyclopropane]-7-sulfonamide.

MS (ESI) M/Z: 495.1 [M+H]⁺.

¹H NMR (400 MHz, DMSO-*d₆*) δ 10.59 (s, 1H), 7.82 (d, *J =* 2.2 Hz, 1H), 7.49 (s, 1H), 6.88 (s, 1H), 6.66 (s, 1H), 6.49 (d, *J =* 8.3 Hz, 1H), 6.30 (t, *J =* 2.2 Hz, 1H), 5.40 (s, 2H), 4.70 (t, *J* = 8.9 Hz, 2H), 4.47 (s, 2H), 3.68 (s, 3H), 3.09 (t, *J* = 8.9 Hz, 2H), 1.00 (d, *J =* 12.4 Hz, 4H).

### Example 37:

### N-(4-((1H-Pyrazol-1-yl)methyl)-2,3-dihydrobenzofuro[7,6-d]isoxazol-8-yl)-5-methoxy-1,1a,2,7b-tetrahydrocyclopropa[c]chromene-4-sulfonamide

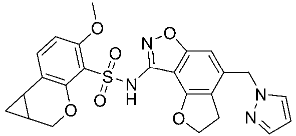

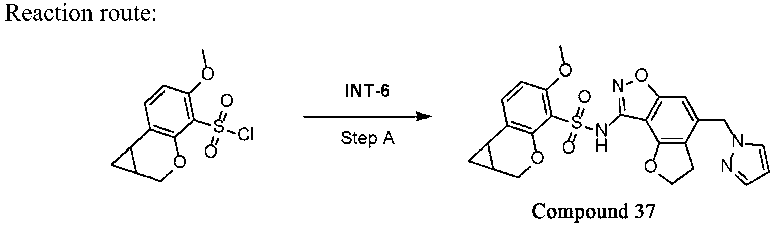

### Operating steps:

**Step A:** Under nitrogen protection, 4-((1H-pyrazol-1-yl)methyl)-2,3-dihydrobenzofuro[7,6-d]isoxazol-8-amine (12 mg, 0.047 mmol) and 5-methoxy-1,1a,2,7b-tetrahydrocyclopropa[c]chromene-4-sulfonyl chloride (26 mg, 0.095 mmol) were dissolved in pyridine (0.4 mL). The system was stirred at 100 °C under microwave conditions for 6 hours. After the complete reaction of the starting material as monitored by LCMS, the reaction mixture was added to water (5 mL), diluted with ethyl acetate (5 mL), washed with saturated brine (3 mL), dried over anhydrous sodium sulfate, filtered, and the filtrate was concentrated under reduced pressure. The resulting mixture was purified by silica gel column chromatography and reverse-phase preparative chromatography to afford 10 mg of N-(4-((1H-pyrazol-1-yl)methyl)-2,3-dihydrobenzofuro[7,6-d]isoxazol-8-yl)-5-methoxy-1,1a,2,7b -tetrahydrocyclopropa[c]chromene-4-sulfonamide.

MS (ESI) M/Z: 495.1 [M+H]⁺.

¹H NMR (400 MHz, DMSO-*d₆*) δ 7.82 (d, *J* = 2.3 Hz, 1H), 7.49 (d, *J=* 1.8 Hz, 1H), 7.36 (s, 1H), 6.72 - 6.50 (m, 2H), 6.30 (t, *J =* 2.1 Hz, 1H), 5.39 (s, 2H), 4.70 (t, *J* = 8.9 Hz, 2H), 4.16 (s, 1H), 3.67 (s, 3H), 3.64 - 3.49 (m, 1H), 3.09 (t, *J =* 8.9 Hz, 2H), 1.99 (q, *J =* 4.4 Hz, 1H), 1.72 (d, *J =* 7.0 Hz, 1H), 0.94 (td, *J =* 8.3, 4.3 Hz, 1H), 0.72 (q, *J=* 4.6 Hz, 1H).

### Example 38:

### N-(6-((1H-Pyrazol-1-yl)methyl)-5-methoxy-4-methylbenzo[d]isoxazol-3-yl)-7-methoxy-3,4-dihydro-2H-benzo[b][1,4]dioxepine-6-sulfonamide

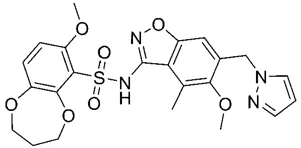

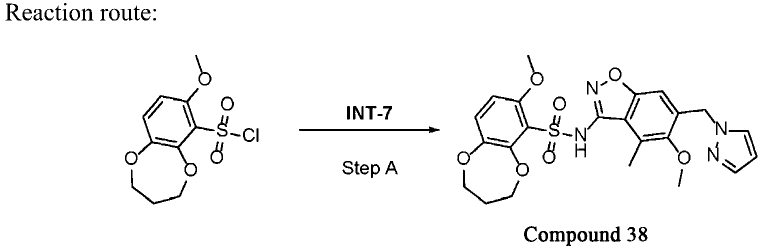

### Operating steps:

**Step A:** Under nitrogen protection, 6-((1H-pyrazol-1-yl)methyl)-5-methoxy-4-methylbenzo [d]isoxazol-3-amine (20 mg, 0.077 mmol) and 7-methoxy-3,4-dihydro-2H-benzo[b][1,4] dioxepine-6-sulfonyl chloride (0.042 g, 0.15 mmol) were dissolved in pyridine (0.5 mL). The system was stirred at 100 °C under microwave conditions for 6 hours. After the complete reaction of the starting material as monitored by LCMS, the reaction mixture was added to water (5 mL), diluted with ethyl acetate (5 mL), washed with saturated brine (3 mL), dried over anhydrous sodium sulfate, filtered, and the filtrate was concentrated under reduced pressure. The resulting mixture was purified by silica gel column chromatography and reverse-phase preparative chromatography to afford 20 mg of N-(6-((1H-pyrazol-1-yl)methyl)-5-methoxy-4-methylbenzo[d]isoxazol-3-yl)-7-methoxy-3,4-dihydro-2H-benzo[b][1,4]dioxepine-6-sulfonamide.

MS (ESI) M/Z: 497.1 [M+H]⁺.

¹H NMR (400 MHz, DMSO-*d₆*) δ 10.29 (s, 1H), 7.86 (d, *J =* 2.3 Hz, 1H), 7.50 (d, *J* = 1.9 Hz, 1H), 7.45 (d, *J* = 8.5 Hz, 1H), 6.88 (s, 1H), 6.72 (d, *J* = 8.6 Hz, 1H), 6.31 (t, *J =* 2.1 Hz, 1H), 5.48 (s, 2H), 4.26 (d, *J* = 10.8 Hz, 1H), 3.70 (d, *J* = 3.0 Hz, 6H), 2.56 (s, 3H), 2.10 - 1.96 (m, 2H), 1.77 (q, *J* = 7.7 Hz, 1H), 0.99 (q, *J* = 3.9 Hz, 1H), 0.75 (q, *J* = 4.7 Hz, 1H).

### Example 39:

### N-(6-((1H-Pyrazol-1-yl)methyl)-5-methoxy-4-methylbenzo[d]isoxazol-3-yl)-6-methoxy-2H-spiro[benzofuran-3,1'-cyclopropane]-7-sulfonamide

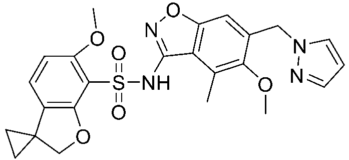

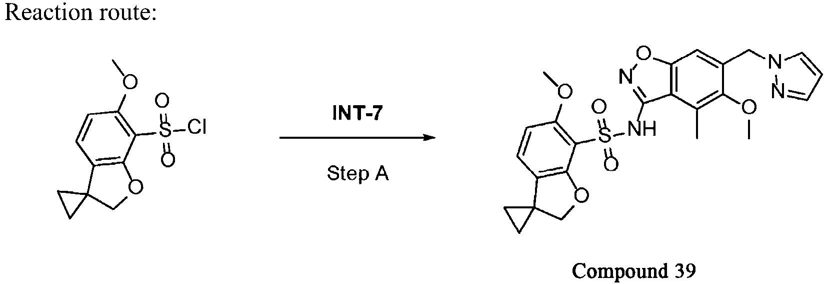

### Operating steps:

**Step A:** Under nitrogen protection, 6-((1H-pyrazol-1-yl)methyl)-5-methoxy-4-methylbenzo [d]isoxazol-3-amine (20 mg, 0.077 mmol) and 6-methoxy-2H-spiro[benzofuran-3,1'-cyclopropane]-7-sulfonyl chloride (0.042 g, 0.15 mmol) were dissolved in pyridine (0.5 mL). The system was stirred at 100 °C under microwave conditions for 6 hours. After the complete reaction of the starting material as monitored by LCMS, the reaction mixture was added to water (5 mL), diluted with ethyl acetate (5 mL), washed with saturated brine (3 mL), dried over anhydrous sodium sulfate, filtered, and the filtrate was concentrated under reduced pressure. The resulting mixture was purified by silica gel column chromatography and reverse-phase preparative chromatography to afford 20 mg of N-(6-((1H-pyrazol-1-yl)methyl)-5-methoxy-4-methylbenzo[d]isoxazol-3-yl)-6-methoxy-2H-spiro[benzofuran-3,1'-cyclopropane]-7-sulfonamide.

MS (ESI) M/Z: 497.1 [M+H]⁺.

¹H NMR (400 MHz, DMSO-*d₆*) δ 10.39 (s, 1H), 7.86 (d, *J =* 2.3 Hz, 1H), 7.50 (d, *J =* 1.8 Hz, 1H), 6.95 - 6.85 (m, 2H), 6.55 (d, *J =* 8.3 Hz, 1H), 6.31 (t, *J =* 2.0 Hz, 1H), 5.48 (s, 2H), 4.47 (s, 2H), 3.69 (d, *J =* 2.9 Hz, 6H), 2.54 (s, 3H), 1.01 (dd, *J =* 7.6, 2.2 Hz, 4H).

### Example 40:

### N-(6-((1H-Pyrazol-1-yl)methyl)-5-methoxy-4-methylbenzo[d]isoxazol-3-yl)-5-methoxy-1,1a,2,7b-tetrahydrocyclopropa[c]chromene-4-sulfonamide

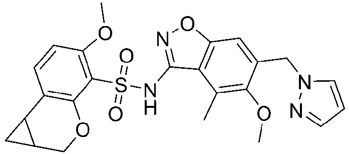

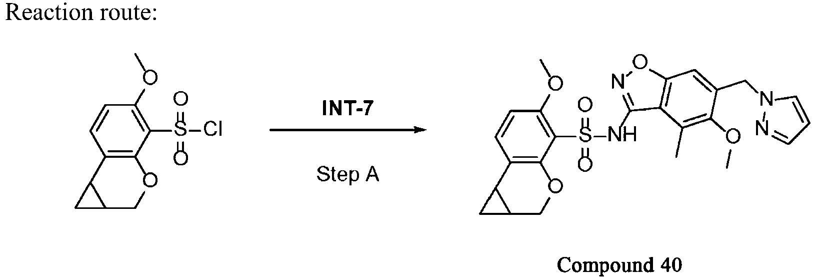

### Operating steps:

**Step A:** Under nitrogen protection, 6-((1H-pyrazol-1-yl)methyl)-5-methoxy-4-methylbenzo[d]isoxazol-3-amine (20 mg, 0.077 mmol) and 5-methoxy-1,1a,2,7b-tetrahydrocyclopropa[c]chromene-4-sulfonyl chloride (0.042 g, 0.15 mmol) were dissolved in pyridine (0.5 mL). The system was stirred at 100 °C under microwave conditions for 6 hours. After the complete reaction of the starting material as monitored by LCMS, the reaction mixture was added to water (5 mL), diluted with ethyl acetate (5 mL), washed with saturated brine (3 mL), dried over anhydrous sodium sulfate, filtered, and the filtrate was concentrated under reduced pressure. The resulting mixture was purified by silica gel column chromatography and reverse-phase preparative chromatography to afford 18 mg of N-(6-((1H-pyrazol-1-yl)methyl)-5-methoxy-4-methylbenzo[d]isoxazol-3-yl)-5-methoxy-1,1a,2,7 b-tetrahydrocyclopropa[c]chromene-4-sulfonamide.

MS (ESI) M/Z: 497.1 [M+H]⁺.

¹H NMR (400 MHz, DMSO-*d₆*) δ 10.29 (s, 1H), 7.86 (d, *J* = 2.3 Hz, 1H), 7.50 (d, *J =* 1.9 Hz, 1H), 7.45 (d, *J* = 8.5 Hz, 1H), 6.88 (s, 1H), 6.72 (d, *J* = 8.6 Hz, 1H), 6.31 (t, *J* = 2.1 Hz, 1H), 5.48 (s, 2H), 4.26 (d, *J =* 10.8 Hz, 1H), 3.70 (d, *J* = 3.0 Hz, 6H), 2.56 (s, 3H), 2.10 - 1.96 (m, 2H), 1.77 (q, *J =* 7.7 Hz, 1H), 0.99 (q, *J =* 3.9 Hz, 1H), 0.75 (q, *J =* 4.7 Hz, 1H).

### Example 41:

### N-(6-((1H-Pyrazol-1-yl)methyl)-4-methoxybenzo[d]isoxazol-3-yl)-8-methoxy-5-methyl-2,3,4,5-tetrahydrobenzo[b][1,4]oxazepine-9-sulfonamide

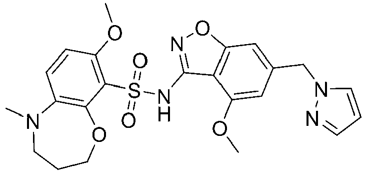

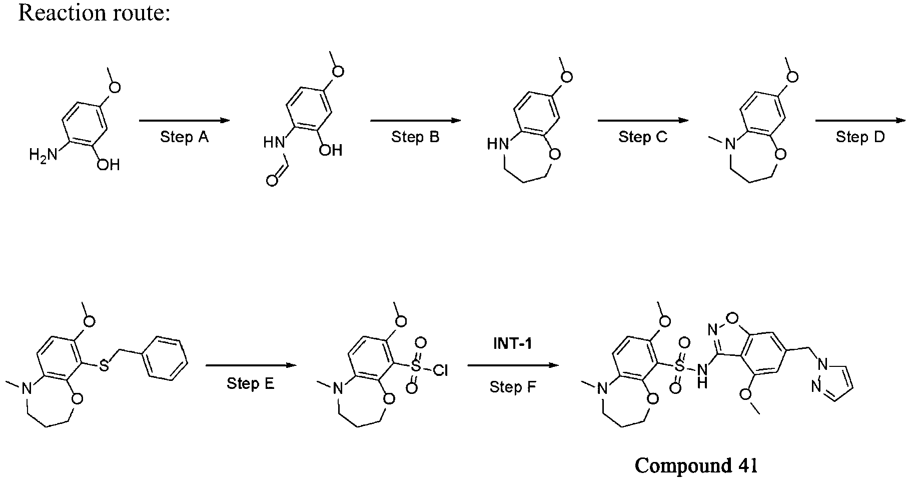

### Operating steps:

**Step A:** Under nitrogen protection, 2-amino-5-methoxyphenol (2 g, 14.39 mmol) was dissolved in formic acid (20 mL). Sodium formate (988 mg, 14.5 mmol) was added, and the mixture was heated to 90°C and reacted for 16 hours. The reaction was monitored by LCMS. After the reaction was complete, the mixture was extracted with ethyl acetate (60 mL) and water (60 mL). The organic phase was dried over anhydrous sodium sulfate and concentrated under reduced pressure. Column chromatography with petroleum ether/ethyl acetate = 3/1 afforded 1.8 g of N-(2-hydroxy-4-methoxyphenyl)formamide.

MS (ESI) M/Z: 168.1 [M+H]⁺.

**Step B:** N-(2-hydroxy-4-methoxyphenyl)formamide (1.8 g, 10.78 mmol) was dissolved in N,N-dimethylformamide (20 mL). Sodium hydride (2.59 g, 107.8 mmol, 60wt%) was added and reacted for 10 minutes, then 1,3-dibromopropane (1.64 mL, 16.17 mmol) was added. The reaction was carried out at 120 °C for 12 hours. The reaction was monitored by LCMS. After the starting material was consumed, the reaction mixture was quenched with water and extracted with ethyl acetate (60 mL) and water (60 mL). The organic phase was dried over anhydrous sodium sulfate. Column chromatography with petroleum ether/ethyl acetate = 3/1 afforded 400 mg of 8-methoxy-2,3,4,5-tetrahydrobenzo[b][1,4]oxazepine.

MS (ESI) M/Z: 180.1 [M+H]⁺.

**Step C:** Under ice-bath conditions, sodium hydride (117 mg, 4.86 mmol, 60wt%) was added to a solution of 8-methoxy-2,3,4,5-tetrahydrobenzo[b][1,4]oxazepine (350 mg, 1.944 mmol) in N,N-dimethylformamide (5 mL). After 10 minutes, iodomethane (121 µL, 2.92 mmol) was added, and the reaction proceeded at room temperature for 3 hours. TLC showed complete conversion of the starting material to the product. The reaction mixture was concentrated under reduced pressure, and the residue was extracted with ethyl acetate (20 mL) and water (20 mL), washed with saturated brine. The organic phase was dried over anhydrous sodium sulfate and concentrated under vacuum. Column chromatography with eluent petroleum ether/ethyl acetate = 3/1 afforded 218 mg of 8-methoxy-5-methyl-2,3,4,5-tetrahydrobenzo[b][1,4]oxazepine.

MS (ESI) M/Z: 194.2 [M+H]⁺.

**Step D:** At 0 °C, n-butyllithium (494 µL, 1.24 mmol) was added to a solution of 8-methoxy-5-methyl-2,3,4,5-tetrahydrobenzo[b][1,4]oxazepine (218 mg, 1.12 mmol) and N,N,N',N'-tetramethylethylenediamine (185 µL, 1.24 mmol) in tetrahydrofuran (5 mL). After 20 minutes, the temperature was lowered to -78°C, and a solution of dibenzyl disulfide (304 mg, 1.24 mmol) in tetrahydrofuran was added. The reaction proceeded at room temperature for 3 hours. TLC showed complete conversion of the starting material to the product. Water was added to quench the reaction, and the mixture was concentrated under reduced pressure. The residue was extracted with ethyl acetate (20 mL) and water (20 mL), washed with saturated brine. The organic phase was dried over anhydrous sodium sulfate and concentrated under vacuum. Column chromatography with eluent petroleum ether/ethyl acetate = 3/1 afforded 304 mg of 9-(benzylthio)-8-methoxy-5-methyl-2,3,4,5-tetrahydrobenzo[b][1,4]oxazepine.

MS (ESI) M/Z: 316.1 [M+H]⁺.

**Step E:** Under nitrogen protection, N-chlorosuccinimide (187.6 mg, 0.952 mmol) was added to a solution of 9-(benzylthio)-8-methoxy-5-methyl-2,3,4,5-tetrahydrobenzo[b][1,4] oxazepine (10 mg, 0.317 mmol) in acetic acid (1.5 mL) and water (0.5 mL) at 0 °C. The reaction proceeded at 0 °C for 3 hours. TLC showed complete conversion of the starting material to the product. The reaction mixture was concentrated under reduced pressure, and the residue was extracted with ethyl acetate (10 mL) and water (10 mL), washed with saturated brine. The organic phase was dried over anhydrous sodium sulfate and concentrated under vacuum. Column chromatography afforded 38 mg of 8-methoxy-5-methyl-2,3,4,5-tetrahydrobenzo[b][1,4] oxazepine-9-sulfonyl chloride.

MS (ESI) M/Z: 292.0 [M+H]⁺.

**Step F:** Under nitrogen protection, 6-((1H-pyrazol-1-yl)methyl)-4-methoxybenzo[d] isoxazol-3-amine (12 mg, 0.025 mmol) and 8-methoxy-5-methyl-2,3,4,5-tetrahydrobenzo[b][1,4] oxazepine-9-sulfonyl chloride (31 mg, 0.095 mmol) were dissolved in pyridine (0.4 mL). The system was stirred at 100 °C under microwave conditions for 6 hours. After the complete reaction of the starting material as monitored by LCMS, the reaction mixture was added to water (5 mL), diluted with ethyl acetate (5 mL), washed with saturated brine (3 mL), dried over anhydrous sodium sulfate, filtered, and the filtrate was concentrated under reduced pressure. The resulting mixture was purified by silica gel column chromatography and reverse-phase preparative chromatography to afford 6 mg of N-(6-((1H-pyrazol-1-yl)methyl)-4-methoxybenzo[d]isoxazol-3-yl)-8-methoxy-5-methyl-2,3,4,5-tetrahydrobenzo[b][1,4]oxazepine-9-sulfonamide.

MS (ESI) M/Z: 500.2 [M+H]⁺.

¹H NMR (400 MHz, DMSO-*d₆*) δ 7.87 (d, *J* = 2.2 Hz, 1H), 7.49 (d, *J* = 1.8 Hz, 1H), 7.04 (d, *J=* 9*.*0 Hz, 1H), 6.82 - 6.67 (m, 3H), 6.30 (t, *J* = 2.1 Hz, 1H), 5.43 (s, 2H), 3.97 (t, *J* = 5.2 Hz, 2H), 3.85 (s, 3H), 3.65 (s, 3H), 2.90 (t, *J =* 5.5 Hz, 2H), 2.74 (s, 3H), 1.85 (p, *J =* 5.4 Hz, 2H).

### Example 42:

### (1aR,7bS)-N-(6-((1H-Pyrazol-1-yl)methyl-d2)-4-methoxybenzo[d]isoxazol-3-yl)-5-meth oxy-1,1a,2,7b-tetrahydrocyclopropa[c]chromene-4-sulfonamide

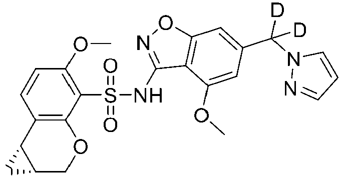

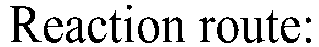

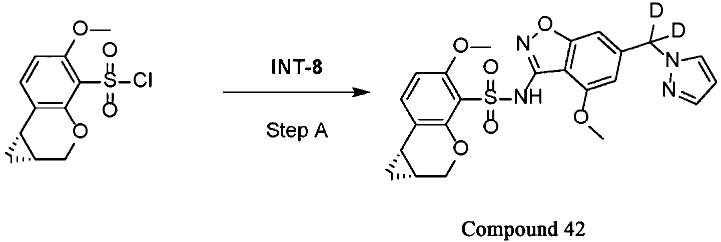

### Operating steps:

**Step A:** Under nitrogen protection, 6-((1H-pyrazol-1-yl)methyl-d2)-4-methoxybenzo[d] isoxazol-3-amine (20 mg, 0.081 mmol) and (1aR,7bS)-5-methoxy-1,la,2,7b-tetrahydrocyclopropa[c]chromene-4-sulfonyl chloride (33 mg, 0.12 mmol) were dissolved in pyridine (0.4 mL). The system was stirred at 100 °C under microwave conditions for 6 hours. After the complete reaction of the starting material as monitored by LCMS, the reaction mixture was added to water (5 mL), diluted with ethyl acetate (5 mL), washed with saturated brine (3 mL), dried over anhydrous sodium sulfate, filtered, and the filtrate was concentrated under reduced pressure. The resulting mixture was purified by silica gel column chromatography and reverse-phase preparative chromatography to afford 20 mg of (1aR,7bS)-N-(6-((1H-pyrazol-1-yl)methyl-d2)-4-methoxybenzo[d]isoxazol-3-yl)-5-methoxy-1,1 a,2,7b-tetrahydrocyclopropa[c]chromene-4-sulfonamide.

MS (ESI) M/Z: 485.1 [M+H]⁺.

¹H NMR (400 MHz, DMSO-*d₆*) δ 9.68 (s, 1H), 7.88 (d, *J =* 2.2 Hz, 1H), 7.50 (d, *J=* 1.8 Hz, 1H), 7.43 (d, *J =* 8.5 Hz, 1H), 6.83 (s, 1H), 6.75 (s, 1H), 6.70 (d, *J =* 8.5 Hz, 1H), 6.30 (t, *J =* 2.1 Hz, 1H), 4.25 (d, *J* = 10.8 Hz, 1H), 3.87 (s, 3H), 3.72 (s, 3H), 3.58 (d, *J =* 10.7 Hz, 1H), 2.01 (td, *J =* 8.6, 4.1 Hz, 1H), 1.75 (q, *J =* 7.5 Hz, 1H), 0.95 (td, *J =* 8.2, 4.5 Hz, 1H), 0.65 (q, *J =* 4.7 Hz, 1H).

### Example 43:

### N-(6-((1H-Pyrazol-1-yl)methyl-d2)-4-methoxybenzo[d]isoxazol-3-yl)-7-methoxy-3,4-di hydro-2H-benzo[b][1,4]dioxepine-6-sulfonamide

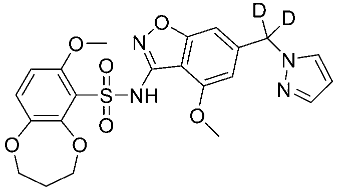

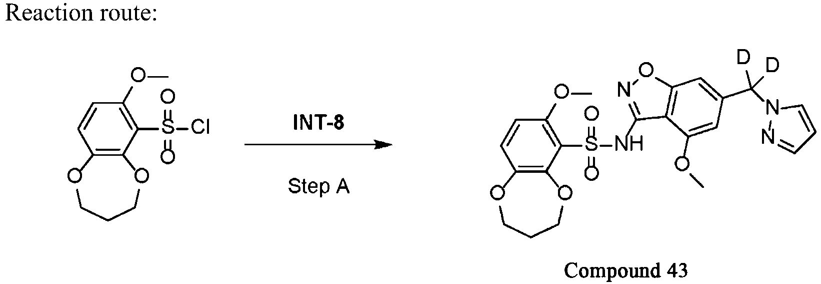

### Operating steps:

**Step A:** Under nitrogen protection, 6-((1H-pyrazol-1-yl)methyl-d2)-4-methoxybenzo[d] isoxazol-3-amine (20 mg, 0.081 mmol) and 7-methoxy-3,4-dihydro-2H-benzo[b][1,4]dioxepine-6-sulfonyl chloride (34 mg, 0.12 mmol) were dissolved in pyridine (0.4 mL). The system was stirred at 100 °C under microwave conditions for 6 hours. After the complete reaction of the starting material as monitored by LCMS, the reaction mixture was added to water (5 mL), diluted with ethyl acetate (5 mL), washed with saturated brine (3 mL), dried over anhydrous sodium sulfate, filtered, and the filtrate was concentrated under reduced pressure. The resulting mixture was purified by silica gel column chromatography and reverse-phase preparative chromatography to afford 16 mg of N-(6-((1H-pyrazol-1-yl)methyl-d2)-4-methoxybenzo[d] isoxazol-3-yl)-7-methoxy-3,4-dihydro-2H-benzo[b][1,4]dioxepine-6-sulfonamide.

MS (ESI) M/Z: 489.1 [M+H]⁺.

¹H NMR (400 MHz, DMSO-*d₆*) δ 9.94 (s, 1H), 7.88 (d, *J =* 2.3 Hz, 1H), 7.49 (d, *J* = 1.8 Hz, 1H), 7.20 (d, *J* = 9.0 Hz, 1H), 6.93 - 6.67 (m, 3H), 6.30 (t, *J =* 2.1 Hz, 1H), 4.02 (dt, *J* = 26.1, 5.2 Hz, 4H), 3.83 (s, 3H), 3.68 (s, 3H), 2.05 (q, *J =* 5.3 Hz, 2H).

### Example 44:

### 4-Methoxy-N-[4-methoxy-6-(pyrazol-1-ylmethyl)-1,2-benzoxazol-3-yl]-la,6b-dihydro-1 H-cyclopropa[b]benzofuran-3-sulfonamide

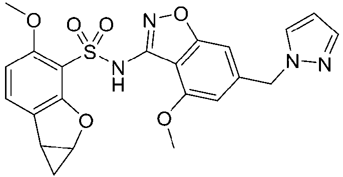

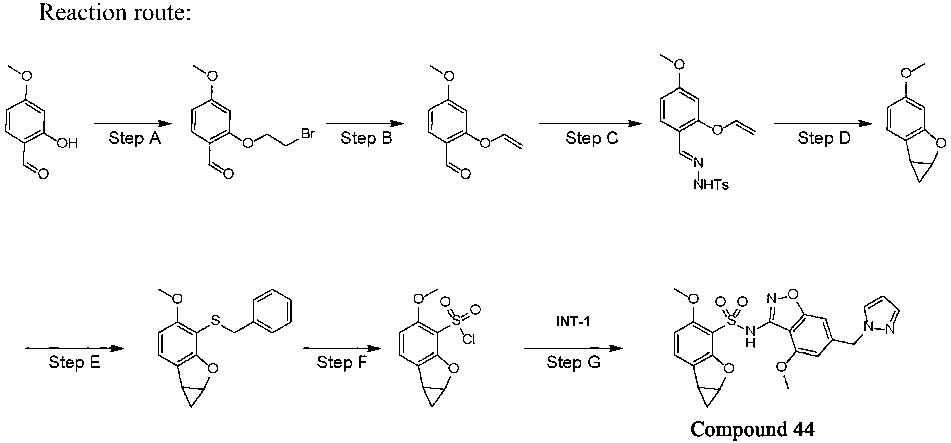

### Operating steps:

**Step A:** 2-Hydroxy-4-methoxybenzaldehyde (2 g, 13.15 mmol) was dissolved in acetonitrile (30 mL). Potassium carbonate (3.63 g, 26.29 mmol) and 1,2-dibromoethane (24.69 g, 131.45 mmol, 9.92 mL) were added. The system was stirred at 90°C for 12 hours. After the complete reaction of the starting material as monitored by LCMS, the reaction mixture was concentrated under reduced pressure. The mixture was extracted with ethyl acetate and water, dried over anhydrous sodium sulfate, filtered, and the filtrate was concentrated under reduced pressure. The resulting mixture was purified by silica gel column chromatography to afford 2.53 g of 2-(2-bromoethoxy)-4-methoxybenzaldehyde.

MS (ESI) M/Z: 260.5 [M+H]⁺.

¹H NMR (400 MHz, CHLOROFORM-d) δ 3.65 - 3.76 (m, 2 H), 3.83 - 3.94 (m, 3 H), 4.34 - 4.44 (m, 2 H), 6.38 - 6.46 (m, 1 H), 6.56 - 6.65 (m, 1 H), 7.81 - 7.89 (m, 1 H), 10.34 - 10.43 (m, 1 H).

**Step B:** 2-(2-Bromoethoxy)-4-methoxybenzaldehyde (2.5 g, 9.65 mmol) was dissolved in dimethyl sulfoxide (25 mL). Potassium tert-butoxide (1.30 g, 11.58 mmol) was added. The system was stirred at 25°C for 2 hours. After the complete reaction of the starting material as monitored by LCMS, the reaction mixture was extracted with ethyl acetate and water. The organic phase was washed with saturated brine, dried over anhydrous sodium sulfate, filtered, and the filtrate was concentrated under reduced pressure. The resulting mixture was purified by silica gel column chromatography to afford 495 mg of 4-methoxy-2-(vinyloxy)benzaldehyde.

MS (ESI) M/Z: 178.6 [M+H]⁺.

¹H NMR (400 MHz, CHLOROFORM-d) δ 3.85 - 3.93 (m, 3 H), 4.60 - 4.68 (m, 1 H), 4.86 - 4.96 (m, 1 H), 6.51 - 6.56 (m, 1 H), 6.64 - 6.75 (m, 2 H), 7.86 (d, *J =* 8.8 Hz, 1 H), 10.26 - 10.37 (m, 1 H).

**Step C:** 4-Methoxy-2-(vinyloxy)benzaldehyde (490 mg, 2.75 mmol) was dissolved in methanol (10 mL). 4-Methylbenzenesulfonohydrazide (563.34 mg, 3.02 mmol) was added. The system was stirred at 25°C for 12 hours. After the complete reaction of the starting material as monitored by LCMS, the reaction mixture was filtered. The filter cake was collected after concentration under reduced pressure, and the filtrate was concentrated under reduced pressure and purified by silica gel column chromatography to afford a total of 1.1 g of N-[(E)-(4-methoxy-2-(vinyloxy)phenyl)methylideneamino]-4-methylbenzenesulfonamide.

MS (ESI) M/Z: 346.7 [M+H]⁺.

¹H NMR (400 MHz, CHLOROFORM-d) δ 2.42 (s, 3 H), 3.82 (s, 3 H), 4.50 (dd, *J=* 6.0, 1.8 Hz, 1 H), 4.68 - 4.78 (m, 1 H), 6.46 (d, *J* = 2.3 Hz, 1 H), 6.55 (dd, *J =* 13.8, 6.0 Hz, 1 H), 6.65 (dd, *J =* 8.8, 2.3 Hz, 1 H), 7.29 - 7.35 (m, 2 H), 7.51 - 7.64 (m, 1 H), 7.79 - 7.91 (m, 3 H), 8.00 - 8.05 (m, 1 H).

**Step D:** Under nitrogen protection, N-[(E)-(4-methoxy-2-(vinyloxy)phenyl) methylideneamino]-4-methylbenzenesulfonamide (1090 mg, 3.15 mmol), lithium tert-butoxide (277.09 mg, 3.46 mmol), and bis[(α,α,α',α'-tetramethyl-1,3-benzenedipropionic acid)]rhodium (239.93 mg, 314.67 µmol) were dissolved in toluene (30 mL). The mixture was stirred at 110°C for 12 hours. After the complete reaction of the starting material as monitored by LCMS, the reaction mixture was concentrated under reduced pressure, extracted with ethyl acetate and water. The organic phase was dried over anhydrous sodium sulfate, filtered, and the filtrate was concentrated under reduced pressure. The resulting mixture was purified by silica gel column chromatography to afford 409 mg of 4-methoxy-1a,6b-dihydro-1H-cyclopropa[b]benzofuran.

MS (ESI) M/Z: 162.6 [M+H]⁺.

¹H NMR (400 MHz, CHLOROFORM-d) δ 0.24 - 0.35 (m, 1 H), 0.89 - 1.00 (m, 1 H), 2.56 (ddd, *J =* 8.9, 5.3, 4.1 Hz, 1 H), 3.73 (br d, *J* = 5.8 Hz, 3 H), 4.75 - 4.87 (m, 1 H), 6.39 - 6.47 (m, 2 H), 7.16 - 7.23 (m, 1 H).

**Step E:** Under nitrogen protection, 4-methoxy-1a,6b-dihydro-1H-cyclopropa[b]benzofuran (200 mg, 1.23 mmol) and N,N,N',N'-tetramethylethane-1,2-diamine (157.63 mg, 1.36 mmol, 204.71 µL) were dissolved in tetrahydrofuran (4 mL). The mixture was cooled to 0°C, and n-butyllithium (1.6 M, 847.80 µL) was added dropwise. After stirring at this temperature for 1 hour, the temperature was lowered to -78°C, and a solution of 1,2-dibenzyldisulfane (334.22 mg, 1.36 mmol) in tetrahydrofuran (2 mL) was added dropwise. After stirring at this temperature for 0.5 hours, the mixture was warmed to room temperature and stirred for 1 hour. After the complete reaction of most of the starting material as monitored by LCMS, the reaction mixture was slowly poured into ice water to quench, extracted with ethyl acetate, dried over anhydrous sodium sulfate, filtered, and the filtrate was concentrated under reduced pressure. The resulting mixture was purified by silica gel column chromatography to afford 295 mg of 3-(benzylthio)-4-methoxy-1a,6b-dihydro-1H-cyclopropa[b]benzofuran.

MS (ESI) M/Z: 284.7 [M+H]⁺.

¹H NMR (400 MHz, CHLOROFORM-d) δ 0.10 - 0.20 (m, 1 H), 0.86 - 0.96 (m, 1 H), 2.50 - 2.60 (m, 1 H), 3.75 - 3.82 (m, 3 H), 3.95 - 4.07 (m, 2 H), 4.77 - 4.86 (m, 1 H), 6.33 - 6.39 (m, 1 H), 7.12 - 7.23 (m, 6 H).

**Step F:** 3-(Benzylthio)-4-methoxy-1a,6b-dihydro-1H-cyclopropa[b]benzofuran (290 mg, 1.02 mmol) was dissolved in acetic acid (5 mL) and water (0.5 mL). The mixture was cooled to 10°C, and N-chlorosuccinimide (408.53 mg, 3.06 mmol) was added. The system was stirred at 10°C for 1 hour. After the complete reaction of the starting material as monitored by TLC, the reaction mixture was diluted with water, extracted with ethyl acetate, washed with saturated brine, dried over anhydrous sodium sulfate, filtered, and the filtrate was concentrated under reduced pressure. The resulting mixture was purified by silica gel column chromatography to afford 218 mg of 4-methoxy-1a,6b-dihydro-1H-cyclopropa[b]benzofuran-3-sulfonyl chloride.

¹H NMR (400 MHz, CHLOROFORM-d) δ 0.47 (ddd, *J* = 6.7, 4.4, 1.8 Hz, 1 H), 1.11 (ddd, *J=* 8.8, 6.8, 5.5 Hz, 1 H), 2.60 - 2.69 (m, 1 H), 3.98 (s, 3 H), 5.06 - 5.15 (m, 1 H), 6.46 - 6.57 (m, 1 H), 7.48 - 7.55 (m, 1 H).

**Step G:** 4-Methoxy-6-(pyrazol-1-ylmethyl)-1,2-benzoxazol-3-amine (170 mg, 696.01 µmol) and 4-methoxy-la,6b-dihydro-1H-cyclopropa[b]benzofuran-3-sulfonyl chloride (217.74 mg, 835.21 µmol, 1.2 eq) were dissolved in pyridine (1.3 mL). The system was stirred at 110°C for 12 hours. After the complete reaction of most of the starting material as monitored by LCMS, the reaction mixture was concentrated under reduced pressure. The resulting mixture was purified by High Performance Liquid Chromatography (column: Xtimate C18 150×40mm×10µm; mobile phase: [water(FA)-ACN]; gradient: 31%-61% B over 10 min) to afford 140 mg of 4-methoxy-N-[4-methoxy-6-(pyrazol-1-ylmethyl)-1,2-benzoxazol-3-yl]-1a,6b-dihydro-1H-cyclo propa[b]benzofuran-3-sulfonamide.

MS (ESI) M/Z: 468.9 [M+H]⁺.

¹H NMR (400 MHz, DMSO-*d₆*) δ 0.05 (ddd, J=6.1, 4.2, 1.5 Hz, 1 H), 0.96 - 1.09 (m, 1 H), 2.62 - 2.72 (m, 1 H), 3.62 - 3.72 (m, 3 H), 3.84 (s, 3 H), 4.98 - 5.08 (m, 1 H), 5.30 - 5.49 (m, 2 H), 6.23 - 6.33 (m, 1 H), 6.50 - 6.60 (m, 1 H), 6.68 - 6.77 (m, 1 H), 6.83 (s, 1 H), 7.33 - 7.55 (m, 2 H), 7.91 (s, 1 H), 9.73 - 10.04 (m, 1 H).

### Example 45:

### (1aR,7bS)-N-(6-((1H-Pyrazol-1-yl)methyl)-4-(methoxy-d3)benzo[d]isoxazol-3-yl)-5-met hoxy-1,1a,2,7b-tetrahydrocyclopropa[c]chromene-4-sulfonamide

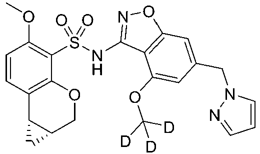

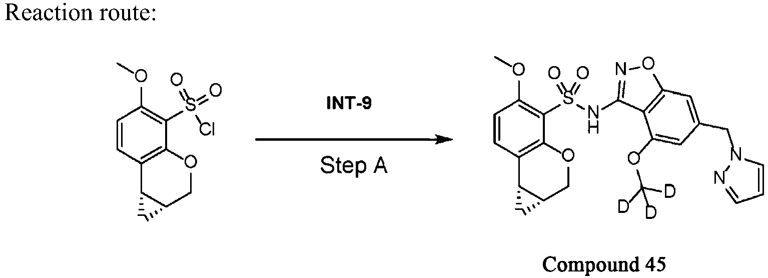

### Operating steps:

**Step A:** 6-(Pyrazol-1-ylmethyl)-4-(trideuteriomethoxy)-1,2-benzoxazol-3-amine (40 mg, 161.77 µmol) and (1aR,7bS)-5-methoxy-1,1a,2,7b-tetrahydrocyclopropa[c]chromene- 4-sulfonyl chloride (57.77 mg, 210.30 µmol) were dissolved in pyridine (0.3 mL). The system was stirred at 100 °C for 12 hours. After the complete reaction of the starting material as monitored by LCMS, the reaction mixture was concentrated under reduced pressure. The resulting mixture was purified by High Performance Liquid Chromatography (column: Xtimate C18 150×40mm×10µm; mobile phase: [water(FA)-ACN]; gradient: 31%-61% B over 10 min) to afford 23.13 mg of (1aR,7bS)-N-(6-((1H-pyrazol-1-yl)methyl)-4-(methoxy-d3)benzo[d]isoxazol-3-yl)-5-methoxy-1,1a,2,7b-tetrahydrocyclopropa[c]chromene-4-sulfonamide.

MS (ESI) M/Z: 485.9 [M+H]⁺.

¹H NMR (400 MHz, DMSO-*d₆*) δ ppm 0.58 - 0.71 (m, 1 H), 0.89 - 0.99 (m, 1 H), 1.68 - 1.82 (m, 1 H), 1.95 - 2.06 (m, 1 H), 3.51 - 3.63 (m, 1 H), 3.66 - 3.79 (m, 3 H), 4.16 - 4.31 (m, 1 H), 5.44 (s, 2 H), 6.27 - 6.34 (m, 1 H), 6.66 - 6.77 (m, 2 H), 6.78 - 6.85 (m, 1 H), 7.38 - 7.46 (m, 1 H), 7.47 - 7.52 (m, 1 H), 7.87 (d, *J =* 2.3 Hz, 1 H), 9.49 - 9.87 (m, 1 H).

### Example 46:

### 7-Methoxy-N-[6-(pyrazol-1-ylmethyl)-4-(trideuteriomethoxy)-1,2-benzoxazol-3-yl]-3,4-dihydro-2H-1,5-benzodioxepine-6-sulfonamide

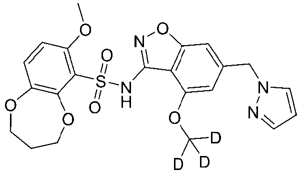

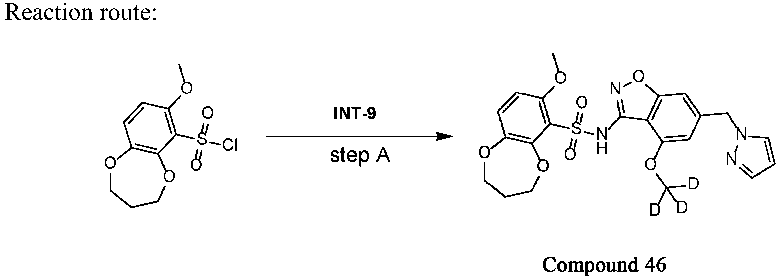

### Operating steps:

**Step A:** 6-(Pyrazol-1-ylmethyl)-4-(trideuteriomethoxy)-1,2-benzoxazol-3-amine (40 mg, 161.77 µmol) and 7-methoxy-3,4-dihydro-2H-1,5-benzodioxepine-6-sulfonyl chloride (54.10 mg, 194.12 µmol) were dissolved in pyridine (0.3 mL). The system was stirred at 100 °C for 12 hours.

After the complete reaction of most of the starting material as monitored by LCMS, the reaction mixture was concentrated under reduced pressure. The resulting mixture was purified by High Performance Liquid Chromatography to afford 17.82 mg of 7-methoxy-N-[6-(pyrazol-1-ylmethyl)-4-(trideuteriomethoxy)-1,2-benzoxazol-3-yl]-3,4-dihydro-2H-1,5-benzodioxepine-6-sulfonamide.

MS (ESI) M/Z: 489.9 [M+H]⁺.

¹H NMR (400 MHz, DMSO-*d₆*) δ ppm 1.98 - 2.12 (m, 2 H), 3.65 - 3.74 (m, 3 H), 3.94 - 4.13 (m, 4 H), 5.40 - 5.48 (m, 2 H), 6.26 - 6.33 (m, 1 H), 6.70 - 6.90 (m, 3 H), 7.17 - 7.27 (m, 1 H), 7.47 - 7.54 (m, 1 H), 7.84 - 7.92 (m, 1 H), 9.89 (br s, 1 H).

### Example 47:

### (1aR,7bS)-5-Methoxy-N-(4-methoxy-6-(thiazol-2-yloxy)benzo[d]isoxazol-3-yl)-1,1a,2,7 b-tetrahydrocyclopropa[c]chromene-4-sulfonamide

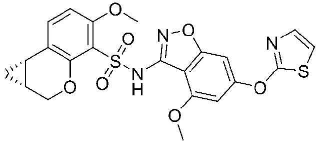

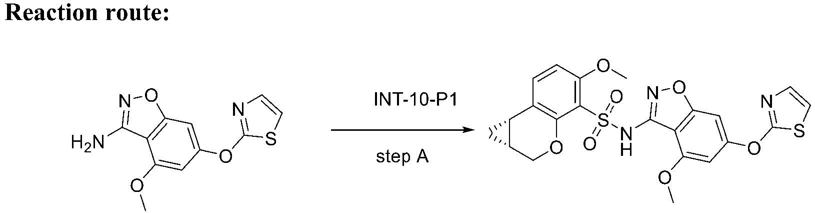

### Operating steps:

**Step A:** Under nitrogen protection, (1aR,7bS)-5-methoxy-1,1a,2,7b-tetrahydrocyclopropa[c] chromene-4-sulfonyl chloride (0.06 g, 0.228 mmol) and 4-methoxy-6-(thiazol-2-yloxy)benzo[d] isoxazol-3-amine (0.05 g, 0.19 mmol) were dissolved in pyridine (0.5 mL). The system was stirred at 100 °C under microwave conditions for 6 hours.

After the complete reaction of the starting material as monitored by LCMS, the reaction mixture was added to water (5 mL), diluted with ethyl acetate (5 mL), washed with saturated brine (3 mL), dried over anhydrous sodium sulfate, filtered, and the filtrate was concentrated under reduced pressure. The resulting mixture was purified by silica gel column chromatography and reverse-phase preparative chromatography (eluent: 0.01vol% formic acid in water / acetonitrile = 1/1) to afford 22 mg of (1aR,7bS)-5-methoxy-N-(4-methoxy-6-(thiazol-2-yloxy) benzo[d]isoxazol-3-yl)-1,1a,2,7b-tetrahydrocyclopropa[c]chromene-4-sulfonamide.

MS (ESI) M/Z: 502.0 [M+H]⁺.

¹H NMR (400 MHz, DMSO-*d₆*) δ 9.84 (s, 1H), 7.42 (s, 1H), 7.38 - 7.31 (m, 2H), 7.21 (s, 1H), 6.86 (s, 1H), 6.71 (d, *J* = 8.5 Hz, 1H), 4.24 (s, 1H), 3.90 (s, 3H), 3.72 (s, 3H), 3.59 (d, *J* = 10.6 Hz, 1H), 2.00 (d, *J* = 7.8 Hz, 1H), 1.76 (d, *J* = 7.2 Hz, 1H), 1.04 - 0.91 (m, 1H), 0.68 (q, *J* = 4.6 Hz, 1H).

### Example 48:

### (1aR,7bS)-5-Methoxy-N-(4-methoxy-6-(pyridin-2-yloxy)benzo[d]isoxazol-3-yl)-1,1a,2,7 b-tetrahydrocyclopropa [c] chromene-4-sulfonamide

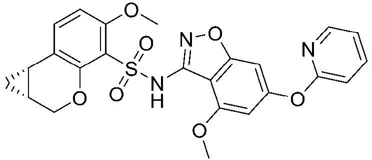

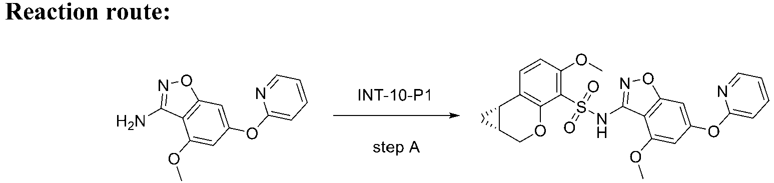

### Operating steps:

**Step A:** Under nitrogen protection, (1aR,7bS)-5-methoxy-1,1a,2,7b-tetrahydrocyclopropa[c] chromene-4-sulfonyl chloride (0.06 g, 0.228 mmol) and 4-methoxy-6-(pyridin-2-yloxy)benzo[d] isoxazol-3-amine (0.05 g, 0.19 mmol) were dissolved in pyridine (0.5 mL), and the system was stirred at 100 °C under microwave conditions for 6 hours.

After LCMS monitoring showed complete consumption of the starting materials, the reaction mixture was poured into water (5 mL), diluted with ethyl acetate (5 mL), washed with saturated brine (3 mL), dried over anhydrous sodium sulfate, filtered, and the filtrate was concentrated under reduced pressure. The resulting mixture was subjected to silica gel column chromatography and reverse-phase preparation (eluent: 0.01% formic acid in water / acetonitrile = 1/1) to afford 32 mg of (1aR,7bS)-5-methoxy-N-(4-methoxy-6-(pyridin-2-yloxy)benzo[d] isoxazol-3-yl)-1,1a,2,7b-tetrahydrocyclopropa[c]chromene-4-sulfonamide.

MS (ESI) M/Z: 496.1 [M+H]⁺.

¹H NMR (400 MHz, DMSO-*d₆*) δ 9.71 (s, 1H), 8.21 (dd, *J* = 5.0, 2.0 Hz, 1H), 7.90 (ddd, *J* = 9.1, 7.2, 2.0 Hz, 1H), 7.44 (d, *J =* 8.5 Hz, 1H), 7.20 (dd, *J =* 7.2, 5.0 Hz, 1H), 7.11 (d, *J =* 8.2 Hz, 1H), 6.92 (s, 1H), 6.73 (d, *J =* 8.5 Hz, 1H), 6.67 (s, 1H), 4.29 (d, *J =* 10.9 Hz, 1H), 3.87 (s, 3H), 3.75 (s, 3H), 3.63 (d, *J =* 10.8 Hz, 1H), 2.03 (td, *J =* 8.3, 4.0 Hz, 1H), 1.77 (q, *J =* 7.8 Hz, 1H), 0.99 (dd, *J =* 8.3, 4.5 Hz, 1H), 0.69 (q, *J =* 4.7 Hz, 1H).

### Example 49: N-(6-((1H-Pyrazol-1-yl)methyl)-4-methoxybenzo[d]isoxazol-3-yl)-6-methoxy-2H-spiro[benzofuran-3,3'-oxetane]-7-sulfonamide

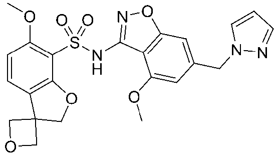

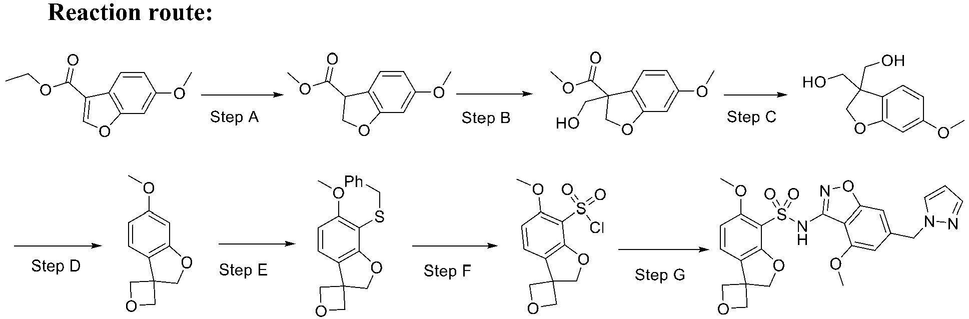

### Operating steps:

**Step A:** To a solution of ethyl 6-methoxy-1-benzofuran-3-carboxylate (5 g, 22.70 mmol) in methanol (80 mL) was added magnesium turnings (2.2 g, 90.8 mmol), and the reaction was stirred at room temperature for 12 hours.

TLC indicated consumption of the starting material and formation of a new spot. The reaction mixture was cooled to 0 °C, diluted hydrochloric acid was added until the solution became clear, followed by the addition of water (50 mL). The mixture was extracted with ethyl acetate (50 mL × 2). The combined organic phases were washed with saturated brine (50 mL × 2), dried over sodium sulfate, filtered, and concentrated to afford the product, 4.5 g of methyl 6-methoxy-2,3-dihydro-1-benzofuran-3-carboxylate.

¹H NMR (400 MHz, DMSO-*d₆*) δ 7.18 (dt, *J =* 7.8, 1.0 Hz, 1H), 6.47 - 6.41 (m, 2H), 4.76 (dd, *J =* 9.2, 5.9 Hz, 1H), 4.66 (t, *J* = 9.4 Hz, 1H), 4.37 (ddd, *J* = 9.6, 5.9, 1.1 Hz, 1H), 3.69 (d, *J* = 9.6 Hz, 6H).

**Step B:** To a solution of methyl 6-methoxy-2,3-dihydro-1-benzofuran-3-carboxylate (4.5 g, 21.61 mmol) in dimethyl sulfoxide (90 mL) were added paraformaldehyde (713.13 mg, 23.77 mmol) and sodium methoxide (128.41 mg, 2.38 mmol). The reaction was stirred at room temperature for 2 hours.

LCMS showed the formation of the target peak. The reaction mixture was cooled to 0 °C, and dilute hydrochloric acid (1M, 15 mL) was added, followed by water (60 mL). The mixture was extracted with ethyl acetate (80 mL × 2). The combined organic phases were washed with saturated brine (30 mL), dried over sodium sulfate, filtered, and concentrated. The residue was purified by column chromatography to afford the product, 4.2 g of methyl 3-(hydroxymethyl)-6-methoxy-2,3-dihydro-1-benzofuran-3-carboxylate.

¹H NMR (400 MHz, DMSO-*d₆*) δ 7.17 (d, *J* = 8.2 Hz, 1H), 6.46 - 6.40 (m, 2H), 5.32 (t, *J =* 5.4 Hz, 1H), 4.92 (d, *J =* 9.4 Hz, 1H), 4.57 (d, *J* = 9.3 Hz, 1H), 3.94 (dd, *J =* 10.4, 5.7 Hz, 1H), 3.69 (d, *J =* 6.7 Hz, 6H), 3.54 (dd, *J* = 10.4, 5.2 Hz, 1H).

**Step C:** To a solution of methyl 3-(hydroxymethyl)-6-methoxy-2,3-dihydro-1-benzofuran-3-carboxylate (2 g, 8.39 mmol) in tetrahydrofuran (30 mL) at 0 °C was added lithium aluminum hydride (0.32 g, 8.39 mmol) dropwise. The reaction was stirred at 0 °C for 1 hour.

After the disappearance of starting material and formation of the target peak as monitored by LCMS, water (32 mL), aqueous sodium hydroxide solution (32 mL), and water (96 mL) were sequentially added to the reaction mixture. The mixture was filtered and concentrated. The residue was purified by column chromatography to afford the product, 1.1 g of (3-(hydroxymethyl)-6-methoxy-2,3-dihydro-1-benzofuran-3-yl)methanol.

MS (ESI) M/Z: 211.1 [M+H]⁺.

**Step D:** To a solution of (3-(hydroxymethyl)-6-methoxy-2,3-dihydro-1-benzofuran-3-yl)methanol (1 g, 4.76 mmol) in N,N-dimethylformamide (20 mL) at 0 °C was added sodium hydride (0.23 g, 5.71 mmol, 60% purity). The mixture was stirred at 0 °C for 20 minutes. A solution of p-toluenesulfonyl chloride (952.87 mg, 5.00 mmol) in N,N-dimethylformamide (5 mL) was added to the reaction mixture, and stirring was continued at 0 °C for 1 hour. Sodium hydride (0.23 g, 5.71 mmol, 60% purity) was added to the reaction mixture at 0 °C, and stirring was continued at 0 °C for 40 minutes. The temperature was then raised to 80 °C, and stirring was continued for 1 hour.

After the disappearance of the starting material and formation of the target peak as monitored by LCMS, the reaction mixture was cooled to 0 °C, and saturated ammonium chloride solution (5 mL) and water (50 mL) were added. The mixture was extracted with ethyl acetate (60 mL × 2). The combined organic phases were washed with saturated brine (30 mL), dried over sodium sulfate, filtered, and concentrated. The residue was purified by column chromatography to afford the product, 410 mg of 6-methoxy-2H-spiro[1-benzofuran-3,3'-oxetane].

¹H NMR (400 MHz, DMSO-*d₆*) δ 7.50 (d, *J =* 8.2 Hz, 1H), 6.53 (dd, *J =* 8.3, 2.3 Hz, 1H), 6.39 (d, *J* = 2.3 Hz, 1H), 4.79 (s, 2H), 4.73 (s, 4H), 3.71 (s, 3H).

**Step E:** To a solution of 6-methoxy-2H-spiro[1-benzofuran-3,3'-oxetane] (400 mg, 2.08 mmol) and N,N,N',N'-tetramethylethylenediamine (265.87 mg, 2.29 mmol) in tetrahydrofuran (10 mL) at 0 °C was added n-butyllithium (0.92 mL, 2.29 mmol, 2.5M) dropwise. The reaction was stirred at 0 °C for 1 hour. The temperature was then lowered to -70 °C, and a solution of [(benzyldithio)methyl]benzene (563.74 mg, 2.29 mmol) in tetrahydrofuran (3 mL) was added dropwise to the reaction mixture. The reaction was maintained at this temperature for 0.5 hours, then warmed to 20 °C and stirred for 1.5 hours.

LCMS indicated remaining starting material and formation of the target peak. Saturated ammonium chloride solution (2 mL) and water (15 mL) were added to the reaction mixture. The mixture was extracted with ethyl acetate (20 mL × 2). The combined organic phases were washed with saturated brine, dried over sodium sulfate, filtered, and concentrated. The residue was purified by column chromatography to afford the product, 550 mg of 7-(benzylthio)-6-methoxy-2H-spiro[1-benzofuran-3,3'-oxetane].

MS (ESI) M/Z: 315.2 [M+H]⁺.

**Step F:** To a solution of 7-(benzylthio)-6-methoxy-2H-spiro[1-benzofuran-3,3'-oxetane] (100 mg, 0.32 mmol) in acetic acid (5 mL) and water (0.5 mL) at 0 °C was added N-chlorosuccinimide (128.19 mg, 0.96 mmol). The reaction was stirred between 0-10 °C for 1 hour.

TLC indicated consumption of the starting material and formation of a new spot. Water (10 mL) was added to the reaction mixture, and the mixture was extracted with ethyl acetate (15 mL × 2). The combined organic phases were washed with saturated brine (5 mL), dried over sodium sulfate, filtered, and concentrated. The residue was purified by column chromatography to afford the product, 80 mg of 6-methoxy-2H-spiro[1-benzofuran-3,3'-oxetane]-7-sulfonyl chloride.

¹H NMR (400 MHz, DMSO-*d₆*) δ 7.50 (d, *J =* 8.2 Hz, 1H), 6.56 (d, *J* = 8.3 Hz, 1H), 4.77 - 4.66 (m, 6H), 3.70 (s, 3H).

**Step G:** To a solution of 6-methoxy-2H-spiro[1-benzofuran-3,3'-oxetane]-7-sulfonyl chloride (20 mg, 0.069 mmol) and 4-dimethylaminopyridine (0.84 mg, 0.0069 mmol) in pyridine (1 mL) was added 4-methoxy-6-[(1H-pyrazol-1-yl)methyl]-1,2-benzoxazol-3-amine (13.48 mg, 0.055 mmol). The reaction was stirred at 75 °C for 12 hours.

LCMS indicated remaining starting material and detection of the target molecular peak. The solvent was removed under reduced pressure, and the product was purified by preparative separation to afford 3.5 mg of N-(6-((1H-pyrazol-1-yl)methyl)-4-methoxybenzo[d]isoxazol-3-yl)-6-methoxy-2H-spiro[benzofuran-3,3'-oxetane]-7-sulfonamide.

MS (ESI) M/Z: 499.0 [M+H]⁺.

¹H NMR (400 MHz, DMSO-*d₆*) δ 7.85 (d, *J=* 2.3 Hz, 1H), 7.62 (s, 1H), 7.48 (d, *J=* 1.8 Hz, 1H), 6.67 (s, 1H), 6.60 (d, *J =* 8.5 Hz, 2H), 6.29 (t, *J =* 2.1 Hz, 1H), 5.39 (s, 2H), 4.80 - 4.63 (m, 6H), 3.81 (s, 3H), 3.64 (s, 3H).

### Example 50: N-(6-((1H-Pyrazol-1-yl)methyl)-4-methoxybenzo[d]isoxazol-3-yl)-4-methoxy-1a,7a-dihydro-1H-benzo[b]cyclopropa[e][1,4]dioxine-3-sulfonamide

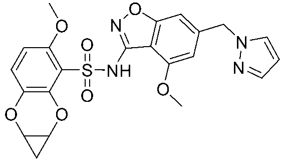

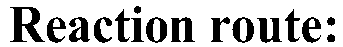

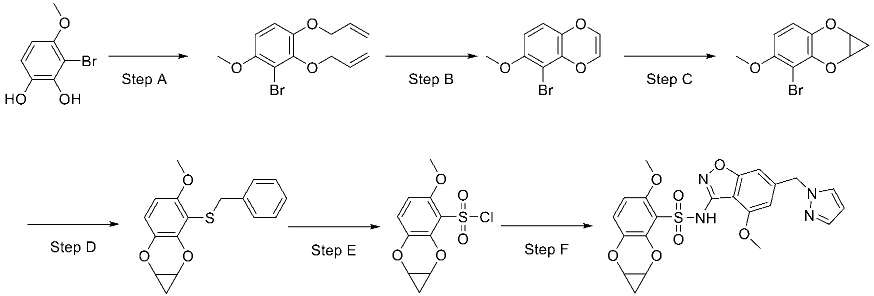

### Operating steps:

**Step A:** To a solution of 3-bromo-4-methoxybenzene-1,2-diol (6.4 g, 29.22 mmol) in N,N-dimethylformamide (120 mL) at 0 °C was added potassium carbonate (16.15 g, 116.88 mmol), followed by the addition of 3-bromoprop-1-ene (12.37 g, 102.27 mmol) to the reaction mixture. The temperature was raised to 20 °C, and the mixture was stirred for 12 hours.

TLC indicated consumption of the starting material and formation of a new spot. Water (20 mL) was added to the reaction mixture, and the mixture was extracted with ethyl acetate (20 mL × 2). The combined organic phases were washed with saturated brine (20 mL), dried over sodium sulfate, filtered, and concentrated. The residue was purified by column chromatography to afford 5.5 g of 1,2-bis(allyloxy)-3-bromo-4-methoxybenzene.

¹H NMR (400 MHz, DMSO-*d₆*) δ 7.04 (d, *J =* 9.1 Hz, 1H), 6.79 (d, *J* = 9.1 Hz, 1H), 6.14 - 5.97 (m, 2H), 5.38 (ddd, *J =* 19.2, 17.3, 1.8 Hz, 2H), 5.23 (ddd, *J =* 21.0, 10.5, 1.7 Hz, 2H), 4.53 (ddt, *J =* 18.7, 5.8, 1.5 Hz, 4H), 3.78 (s, 3H).

**Step B:** Under a nitrogen atmosphere, to a solution of 1,2-bis(allyloxy)-3-bromo-4-methoxybenzene (5.5 g, 18.38 mmol) in toluene (80 mL) was added tris(triphenylphosphine)carbonylhydridoruthenium(II) (1748.65 mg, 1.84 mmol). The reaction was stirred at 80 °C under nitrogen protection for 24 hours. Then, [1,3-bis(2,4,6-trimethylphenyl)imidazolin-2-ylidene]dichloro[(2-isopropoxyphenyl)methylene]ru thenium (1.15 g, 1.84 mmol) was added, and the reaction was continued at 80 °C for 1 hour.

LCMS indicated the consumption of the starting material and the formation of a new spot. The solvent was removed under reduced pressure, and the residue was purified by column chromatography to afford 3.5 g of 5-bromo-6-methoxybenzo[b][1,4]dioxine.

¹H NMR (400 MHz, DMSO-*d₆*) δ 6.74 (d, *J =* 9.0 Hz, 1H), 6.61 (d, *J =* 9.0 Hz, 1H), 6.32 (d, *J=* 3.6 Hz, 1H), 6.26 (d, *J=* 3.6 Hz, 1H), 3.77 (s, 3H).

**Step C:** To a solution of 5-bromo-6-methoxybenzo[b][1,4]dioxine (1 g, 4.11 mmol) in dichloromethane (20 mL) at 0 °C was added diethylzinc (20.55 mL, 20.55 mmol, 1M) dropwise, followed by the dropwise addition of chloroiodomethane (7.3 g, 41.1 mmol). The reaction was stirred at 0 °C for 2 hours and then at room temperature for 12 hours.

TLC indicated remaining starting material and formation of a new spot. Saturated ammonium chloride solution (5 mL) and water (50 mL) were added to the reaction mixture. The mixture was extracted with ethyl acetate (50 mL × 2). The combined organic phases were washed with saturated brine (20 mL), dried over sodium sulfate, filtered, and concentrated. The residue was purified by column chromatography to afford 280 mg of 3-bromo-4-methoxy-1a,7a-dihydro-1H-benzo[b]cyclopropa[e][1,4]dioxine.

¹H NMR (400 MHz, DMSO-*d₆*) δ 6.92 (d, *J* = 9.0 Hz, 1H), 6.64 (d, *J* = 9.0 Hz, 1H), 4.13 - 4.07 (m, 1H), 4.02 - 3.97 (m, 1H), 3.76 (s, 3H), 1.18 - 1.12 (m, 1H), 1.11 - 1.06 (m, 1H).

**Step D:** To a solution of 3-bromo-4-methoxy-1a,7a-dihydro-1H-benzo[b]cyclopropa[e] [1,4]dioxine (270 mg, 1.05 mmol) and N,N,N',N'-tetramethylethylenediamine (134.21 mg, 1.16 mmol) in tetrahydrofuran (10 mL) at -70 °C was added n-butyllithium (0.464 mL, 1.16 mmol, 2.5M) dropwise. The reaction was stirred at -70 °C for 0.5 hours. Then, a solution of [(benzyldithio)methyl]benzene (284.58 mg, 1.16 mmol) in tetrahydrofuran (3 mL) was added dropwise to the reaction mixture. The mixture was stirred for 0.5 hours, then warmed to room temperature and stirred for 1.5 hours.

After the disappearance of starting material and formation of the target peak as monitored by LCMS, ammonium chloride (2 mL) and water (20 mL) were added to the reaction mixture. The mixture was extracted with ethyl acetate (30 mL × 2). The combined organic phases were washed with saturated brine (20 mL), dried over sodium sulfate, filtered, and concentrated. The residue was purified by column chromatography to afford the product, 190 mg of 3-(benzylthio)-4-methoxy-1a,7a-dihydro-1H-benzo[b]cyclopropa[e][1,4]dioxine.

MS (ESI) M/Z: 301.2 [M+H]⁺.

**Step E:** To a solution of 3-(benzylthio)-4-methoxy-1a,7a-dihydro-1H-benzo[b]cyclopropa[e] [1,4]dioxine (70 mg, 0.23 mmol) in acetic acid (2 mL) and water (0.2 mL) at 0 °C was added N-chlorosuccinimide (92.14 mg, 0.69 mmol). The reaction was stirred between 0-10 °C for 1 hour.

TLC indicated the consumption of the starting material and the formation of a new spot. Water (10 mL) was added to the reaction mixture, and the mixture was extracted with ethyl acetate (15 mL × 2). The combined organic phases were washed with saturated brine, dried over sodium sulfate, filtered, and concentrated. The residue was purified by column chromatography to afford the product, 40 mg of 4-methoxy-la,7a-dihydro-1H-benzo[b]cyclopropa[e] [1,4]dioxine-3-sulfonyl chloride.

¹H NMR (400 MHz, DMSO-*d₆*) δ 6.84 (d, *J =* 8.8 Hz, 1H), 6.49 (d, *J =* 8.9 Hz, 1H), 3.93 (ddd, *J =* 6.2, 5.4, 3.4 Hz, 1H), 3.85 (ddd, *J =* 6.4, 5.4, 3.5 Hz, 1H), 3.64 (s, 3H), 1.04 (dt, *J =* 7.7, 6.2 Hz, 1H), 0.93 (dt, *J =* 7.4, 3.5 Hz, 1H).

**Step F:** To a solution of 4-methoxy-1a,7a-dihydro-1H-benzo[b]cyclopropa[e][1,4]dioxine-3-sulfonyl chloride (20 mg, 0.072 mmol) and 4-dimethylaminopyridine (0.88 mg, 0.0072 mmol) in pyridine (0.2 mL) was added 4-methoxy-6-[(1H-pyrazol-1-yl)methyl]-1,2-benzoxazol-3-amine (15.83 mg, 0.065 mmol). The reaction was stirred at 80 °C for 12 hours.

LCMS indicated remaining starting material and formation of the target peak. The solvent was removed under reduced pressure, and the product was purified by preparative separation to afford 9 mg of N-(6-((1H-pyrazol-1-yl)methyl)-4-methoxybenzo[d]isoxazol-3-yl)-4-methoxy-1a,7a-dihydro-1H-benzo[b]cyclopropa[e][1,4]dioxine-3-sulfonamide.

MS (ESI) M/Z: 485.0 [M+H]⁺.

¹H NMR (400 MHz, DMSO-*d₆*) δ 10.10 (s, 1H), 7.87 (d, *J =* 2.3 Hz, 1H), 7.49 (d, *J =* 1.8 Hz, 1H), 7.13 (s, 1H), 6.89 - 6.61 (m, 3H), 6.30 (t, *J =* 2.1 Hz, 1H), 5.44 (s, 2H), 3.84 (s, 5H), 3.68 (s, 3H), 1.06 (d, *J =* 7.5 Hz, 1H), 0.96 (s, 1H).

### Biological testing experiments

### I. KATs acetyltransferase enzyme activity assay

1. The inhibitory effect of compounds on the acetyltransferase activity of KATs was evaluated using the Time-Resolved Fluorescence Resonance Energy Transfer (TR-FRET) assay. The assay was conducted in 384-well plates using Tris-HCl buffer.
2. Compounds were diluted in DMSO and transferred to 384-well plates. Enzyme solutions (KAT6: active motif (kit), 81223; KAT6B: active motif, 81224; KAT5: SignalChem, K314-380G; KAT7: ICE, S2210F-H21HF; KAT8: Active motif, 81225) were added and the mixture was incubated at room temperature for 15 minutes.
3. Subsequently, 2.5 µL of a mixture containing KAT6A/KAT6B: Biotin-H3 (1-21, Genscript, C6829GL060) or KAT5/7/8: Bio-H4 (1-25, Genscript, C736BGL100) AcCOA (Sigma, A2056) was added, and the reaction proceeded at room temperature for 90 minutes.
4. After the reaction, a detection mixture containing Europium-anti-acetyl-Histone H4 Lysine (H4Kac pan) Antibody (Perkin Elmer, TRF0412) and LANCE Ultra ULight^{™}-Streptavidin in LANCE^{™} Detection buffer (10x) (Perkin Elmer, CR97-100) was added, followed by incubation at room temperature for 60 minutes.
5. HTRF signals were measured using a BMG PHERAstar FSX (Ratio: 665nm/620nm). IC₅₀ values were calculated by nonlinear regression using a four-parameter logistic equation in GraphPad Prism 8 software.

Test results are shown in Table 1 below:

**Table 1**

| **Compound NO.** | **KAT6A IC₅₀ (nM)** | **KAT6B IC₅₀ (nM)** |
|---|---|---|
| **Compound** 1 | 12.9 | 81.7 |
| **Compound** 2 | 3.2 | 25.2 |
| **Compound** 3 | 1.8 | 7.5 |
| **Compound** 4 | 2.0 | 15.0 |
| **Compound** 5 | 1.5 | 3.7 |
| **Compound** 5-P1 | 2.5 | 11.1 |
| **Compound** 5-P2 | 1.1 | 1.6 |
| **Compound** 6 | 1.3 | 11.8 |
| **Compound** 7 | 2.7 | 19.2 |
| **Compound** 8 | 2.7 | 40.8 |
| **Compound** 9 | 1.6 | 9.5 |
| **Compound** 10 | 2.2 | 19.9 |
| **Compound** 11 | 2.5 | 46.8 |
| **Compound** 12 | 6.5 | 40.0 |
| **Compound** 14 | 1.4 | 12.7 |
| **Compound** 15 | 1.7 | 9.3 |
| **Compound** 22 | 1.5 | 7.3 |
| **Compound** 24 | 3.1 | 34.4 |
| **Compound** 25 | 5.9 | 70.3 |
| **Compound** 26 | 4.7 | 80.3 |
| **Compound** 27 | 1.3 | 8.9 |
| **Compound** 28 | 2.2 | 12.3 |
| **Compound** 29 | 3.0 | 23.3 |
| **Compound** 30 | 0.8 | 1.8 |
| **Compound** 30-P1 | 1.4 | 1.4 |
| **Compound** 31 | 1.3 | 2.7 |
| **Compound** 33 | 1.1 | 1.2 |
| **Compound** 34 | 1.2 | 2.2 |
| **Compound** 35 | 4.3 | 45.9 |
| **Compound** 36 | 1.7 | 8.0 |
| **Compound** 37 | 1.6 | 4.1 |
| **Compound** 39 | 2.2 | 37.6 |
| **Compound** 40 | 1.7 | 12.9 |
| **Compound** 41 | 8.9 | 97.5 |
| **Compound** 42 | 1.1 | 1.6 |
| **Compound** 43 | 3.7 | 28.9 |
| **Compound** 44 | 1.7 | 8.6 |
| **Compound** 45 | 0.6 | 1.4 |
| **Compound** 46 | 5.4 | 33.8 |
| **Compound** 47 | 0.8 | 1.5 |
| **Compound** 48 | 1.7 | 15.6 |
| **Compound** 49 | 3.0 | 23.1 |
| **Compound** 50 | 6.8 | 51.6 |

The results showed that the compounds of the present application possess good enzyme inhibitory activity against KAT6A/6B.

### II. ZR-75-1 cell proliferation assay

1. ZR-75-1 cells were seeded in 96-well plates and cultured overnight in an incubator at 37°C & 5% CO₂.
2. Compounds were diluted using culture medium and added to the 96-well plates. The plates were placed back into the incubator for continued culture. Every 5 days, the cells were trypsinized, counted, and re-seeded. After the cells had fully adhered, compounds were added again for incubation, for a total culture period of 10 days.
3. After the culture period, 60 µL of CellTiter-Glo reagent (Promega, G7573) was added to each well. The plates were protected from light and shaken for 2 minutes to lyse the cells, followed by further incubation at room temperature, protected from light, for 30 minutes.
4. Luminescence signals were read using a BMG PHERAstar FSX. IC₅₀ values were calculated by nonlinear regression using a four-parameter logistic equation in GraphPad Prism 8 software.

Test results are shown in Table 2 below:

**Table 2**

| **Compound NO.** | **ZR-75-1 Proliferation Inhibition IC₅₀ (nM)** |
|---|---|
| **Compound** 1 | 6.7 |
| **Compound** 2 | 3.2 |
| **Compound** 5 | 0.2 |
| **Compound** 5-P1 | 0.3 |
| **Compound** 5-P2 | 0.1 |
| **Compound** 6 | 0.6 |
| **Compound** 7 | 2.4 |
| **Compound** 8 | 4.2 |
| **Compound** 10 | 1.4 |
| **Compound** 12 | 1.4 |
| **Compound** 13 | 14.2 |
| **Compound** 14 | 8.6 |
| **Compound** 15 | 0.6 |
| **Compound** 17 | 3.7 |
| **Compound** 18 | 22.1 |
| **Compound** 22 | 0.5 |
| **Compound** 27 | 11.5 |
| **Compound** 28 | 16.6 |
| **Compound** 29 | 0.6 |
| **Compound** 30 | 0.15 |
| **Compound** 30-P1 | 0.08 |
| **Compound** 31 | 0.3 |
| **Compound** 33 | 0.03 |
| **Compound** 34 | 0.04 |
| **Compound** 35 | 3.3 |
| **Compound** 36 | 0.4 |
| **Compound** 37 | 0.4 |
| **Compound** 39 | 2.6 |
| **Compound** 40 | 1.6 |
| **Compound** 41 | 4.8 |
| **Compound** 42 | 0.1 |
| **Compound** 43 | 1.0 |
| **Compound** 44 | 0.3 |
| **Compound** 45 | 0.04 |
| **Compound** 46 | 1.0 |

Conclusion: The compounds described in the present application possess good cell proliferation inhibitory activity.

### III. Histone H3 acetylation assay

1. ZR-75-1 cells were seeded at 5000 cells/40 µL per well in 384-well plates and cultured overnight in an incubator at 37°C & 5% CO₂.
2. 40 nL of serially diluted compounds were added to the 384-well plates, followed by further incubation for 72 hours.
3. The supernatant was discarded, and 8 w/v% paraformaldehyde (final concentration was 4 w/v%) was added directly to the wells to fix the cells at room temperature.
4. After washing twice with PBS, a permeabilization agent (40 µL of methanol) was added and incubated for 10 minutes to permeabilize the cell membranes.
5. After washing twice with PBS, a blocking buffer was added and incubated at room temperature for 1 hour for blocking.
6. The blocking buffer was discarded, and 20 µL of primary antibodies (recombinant Anti-Histone H3 (acetyl K23) antibody and Anti-Histone H4 antibody) diluted in blocking buffer (1:1000 by volume dilution) were added, followed by incubation at 4°C overnight.
7. After washing three times with PBST (0.05 vol% Tween-20 in PBS), 20 µL of fluorescent secondary antibodies (IRDye 680RD Goat anti-MOUSE, Licor, 926-68070; IRDye 800CW Goat anti-Rabbit, Licor, 926-32211) diluted in blocking buffer (1:2000 by volume dilution) were added, followed by incubation at room temperature, protected from light, for 1 hour. Then, the plates were washed three times with PBST.
8. Scanning was performed using an Odyssey CLx, and the fluorescence signal value for each well was quantified. IC₅₀ values were calculated by nonlinear regression using a four-parameter logistic equation in GraphPad Prism 8 software.

Test results are shown in Table 3 below:

**Table 3**

| **Compound NO.** | **ZR-75-1 H3K23 Acetylation Inhibition IC₅₀ (nM)** |
|---|---|
| **Compound** 1 | 9.0 |
| **Compound** 2 | 5.1 |
| **Compound** 5 | 0.5 |
| **Compound-P1** | 1.9 |
| **Compound** 5-P2 | 0.5 |
| **Compound** 6 | 5.3 |
| **Compound** 7 | 8.6 |
| **Compound** 8 | 10.3 |
| **Compound** 9 | 2.1 |
| **Compound** 10 | 2.3 |
| **Compound** 11 | 12.7 |
| **Compound** 12 | 4.5 |
| **Compound** 13 | 34.3 |
| **Compound** 14 | 11.5 |
| **Compound** 15 | 1.1 |
| **Compound** 27 | 27.1 |
| **Compound** 28 | 37.7 |
| **Compound** 29 | 4.6 |
| **Compound** 30 | 0.8 |
| **Compound** 30-P1 | 0.3 |
| **Compound** 31 | 1.1 |
| **Compound** 33 | 0.2 |
| **Compound** 34 | 0.4 |
| **Compound** 35 | 15.0 |
| **Compound** 36 | 3.7 |
| **Compound** 37 | 1.2 |
| **Compound** 39 | 23.7 |
| **Compound** 40 | 12.4 |
| **Compound** 41 | 20.2 |
| **Compound** 47 | 3.4 |
| **Compound** 48 | 17.9 |
| **Compound** 49 | 4.2 |
| **Compound** 50 | 6.5 |

Conclusion: The data indicate that the compounds of the present application exhibit significant target cell activity.

### IV. In vivo efficacy study of test drug Compound 2, Compound 5-P2, Compound 3, and Compound A in a BALB/c nude mouse model with subcutaneously implanted human breast cancer ZR-75-1 cell xenografts

### 1. Experimental objective

The objective of this experiment is to evaluate the in vivo efficacy of the test drug Compound 2, Compound 5-P2, Compound 3, and Compound A in a BALB/c nude mouse model with subcutaneously implanted human breast cancer ZR-75-1 cell xenografts.

### 2. Experimental animals

Species: Mouse;
Strain: BALB/c nude mice;
Age and weight: 6-8 weeks old, weighing 19-26 grams;
Gender: Female;
Supplier: GemPharmatech Co., Ltd.;
Compound source:
   Compound A: 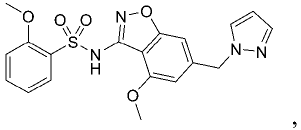 prepared with reference to patent CN114364672A.

### 3. Experimental methods and procedures

### 3.1 Cell culture

Human breast cancer ZR-75-1 (ATCC, CRL-1500) cells were cultured in vitro as adherent cultures. The culture conditions were: 1640 medium supplemented with 10 vol% FBS, 100 U/mL penicillin, and 100 µg/mL streptomycin, incubated in a 37°C, 5% CO₂ cell culture incubator. Routine passaging was performed twice a week. When cell confluence reached 80%-90% and the cell number met the requirements, cells were harvested, counted, and inoculated.

### 3.2 Estrogen pellet implantation and urination

Three days before cell implantation, a 17β-estrogen pellet (0.36 mg) was implanted into the left dorsal side of each mouse. Starting one week after pellet implantation, animals were urinated 2-3 times per week. If necessary, animals were urinated daily.

### 3.3 Tumor cell implantation

A mixture containing 10×10⁶ ZR-75-1 cells in 100 µL PBS and 100 µL Matrigel (final volume was 200 µL) was subcutaneously implanted into the right dorsal side (upper side) of each mouse. On day 18 after cell implantation, when the average tumor volume reached 138 mm³, grouping and administration were initiated. Mice with tumor volumes ranging from 100 to 212 mm³ were selected for the study. Mice were randomly assigned to groups based on body weight and tumor volume, with 7 mice per group. The day of grouping was designated as PG-D0, and administration for all groups started from PG-D0. Experimental groups and dosing regimens are shown in Table 4.

### 3.4 Preparation of Test Articles

**Table 4. Preparation Methods of Test Materials**

| **Compound** | **Preparation method** | **Concentration (mg/mL)** | **Storage conditions** |
|---|---|---|---|
| Vehicle (blank) | 5 vol% DMSO + 10 vol% Solutol (solubilizer) + 85 vol% Saline | - | 4°C |
| Compound 2 | Calculated based on a purity of 98.08%, salt factor = 1. 1.53 mg of Compound 2 was weighed, and 2.501 mL of DMSO was added. The resultant was dissolved, aliquoted into 7 vials with 0.120 mL each, and stored at -20°C. Before each use, one vial was thawed, mixed with 0.240 mL Solutol by vortexing, then 2.040 mL Saline was added and mixed by vortexing to obtain a clear solution. Solutol was thawed in advance. | 0.03 | Prepared before use |
| Compound 5-P2 | Calculated based on a purity of 98.33%, salt factor = 1. 1.53 mg of Compound 5-P2 was weighed, and 2.507 mL of DMSO was added. The resultant was dissolved, aliquoted into 7 vials with 0.120 mL each, and stored at -20°C. Before each use, one vial was thawed, mixed with 0.240 mL Solutol by vortexing, then 2.040 mL Saline was added and mixed by vortexing to obtain a clear solution. Solutol was thawed in advance. | 0.03 | Prepared before use |
| Compound 3 | Calculated based on a purity of 99.95%, salt factor = 1. 1.50 mg of Compound 3 was weighed, and 2.499 mL of | 0.03 | Prepared before use |
| | DMSO was added. The resultant was dissolved, aliquoted into 7 vials with 0.120 mL each, and stored at -20°C. Before each use, one vial was thawed, mixed with 0.240 mL Solutol by vortexing, then 2.040 mL Saline was added and mixed by vortexing to obtain a clear solution. Solutol was thawed in advance. | | |
| Compound A | Calculated based on a purity of 97.21%, salt factor = 1. 1.50 mg of QLS-CPO-018 was weighed, and 2.430 mL of DMSO was added. The resultant was dissolved, aliquoted into 7 vials with 0.120 mL each, and stored at -20°C. Before each use, one vial was thawed, mixed with 0.240 mL Solutol by vortexing, then 2.040 mL Saline was added and mixed by vortexing to obtain a clear solution. Solutol was thawed in advance. | 0.03 | Prepared before use |

| | | | |
|---|---|---|---|
| Note: The drug solution needs to be gently but thoroughly mixed before administration; "-" indicates 0. | | | |

### 3.5 Tumor measurement and experimental index

The experimental index is to investigate whether tumor growth is inhibited, delayed, or cured. Tumor diameters were measured three times per week using a vernier caliper. Tumor volume was calculated using the formula: V = 0.5a × b², where a and b represent the major and minor diameters of the tumor, respectively.

The anti-tumor efficacy of the compounds was evaluated using TGI (%) or the relative tumor proliferation rate T/C (%). TGI (%), reflects the tumor growth inhibition rate. TGI (%) was calculated as: TGI (%) = [1 - (Mean tumor volume of a treatment group at the end of administration - Mean tumor volume of the same treatment group at the start of administration) / (Mean tumor volume of the vehicle control group at the end of treatment - Mean tumor volume of the vehicle control group at the start of treatment)] × 100%.

Relative Tumor Proliferation Rate T/C (%): The calculation formula is as follows: T/C% = T_{RTV} / C_{RTV} ×100 % (T_{RTV}: RTV of the treatment group; C_{RTV}: RTV of the negative control group). The relative tumor volume (RTV) was calculated based on tumor measurement results using the formula RTV = Vt /V₀, where V₀ is the tumor volume of each mouse measured at the start of grouping and administration (i.e., d₀), and Vₜ is the tumor volume of each mouse at a specific measurement time. The mean value for each group was then calculated. V₀ and Vₜ correspond to tumor volume data from the same mouse.

At the end of the experiment, tumor weight was measured, and the T_{weight}/C_{weight} percentage was calculated, where T_{weight} and C_{weight} represent the tumor weight of the administration group and the vehicle control group, respectively.

### 4. Experimental results

### 4.1 Body weight changes

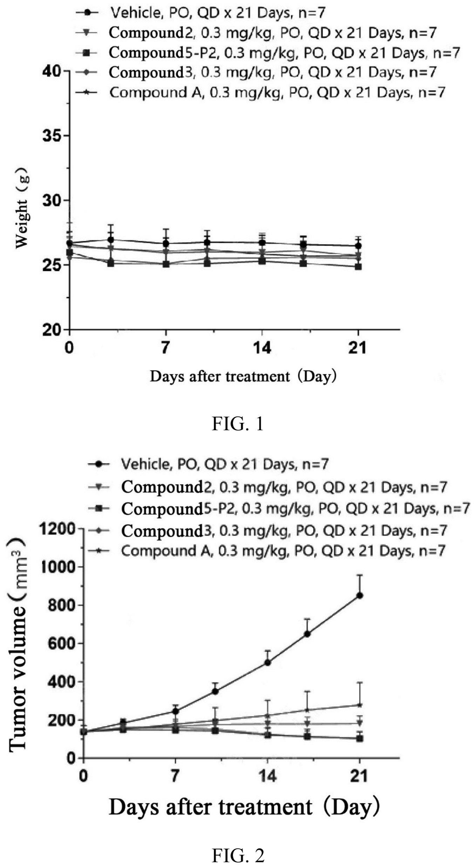

The effects of the test drugs Compound 2, Compound 5-P2, Compound 3, and Compound A on the body weight of the female BALB/c nude mouse model with subcutaneously implanted human breast cancer ZR-75-1 cell xenografts are shown in FIG. 1.

FIG. 1 shows the body weight changes of tumor-bearing mice in the human breast cancer ZR-75-1 cell xenograft model after administration of the test drugs Compound 2, Compound 5-P2, Compound 3, and Compound A. It can be seen from the figure that the differences in body weight changes among the groups were not significant.

### 4.2 Tumor growth curves

The tumor growth curves for each group in the female BALB/c nude mouse model with subcutaneously implanted ZR-75-1 cell xenografts after treatment with the test drugs Compound 2, Compound 5-P2, Compound 3, and Compound A are shown in FIG. 2.

FIG. 2 shows the tumor growth curves of tumor-bearing mice in the ZR-75-1 xenograft model after administration of the test drugs Compound 2, Compound 5-P2, Compound 3, and Compound A. It can be seen from the figure that Compound 2, Compound 5-P2, and Compound 3 all exhibited more significant anti-tumor effects compared to Compound A.

### 4.3 Anti-tumor Efficacy Evaluation Metrics

Table 5 shows the evaluation of the tumor inhibition efficacy of Compound 2, Compound 5-P2, Compound 3, and Compound A in the ZR-75-1 xenograft model (calculated based on tumor volume on day 21 post-administration).

**Table 5**

| **Group** | **Tumor Volume (mm³)^{a}** | **T/C^{a} (%)** | **TGI^{a} (%)** |
|---|---|---|---|
| Vehicle | 851 ± 106 | -- | -- |
| **Compound** 2 (0.3mg/kg) | 182 ± 39 | 20.83 | 93.97 |
| **Compound** 5-P2 (0.3 mg/kg) | 102 ± 14 | 12.10 | 105.10 |
| **Compound** 3 (0.3 mg/kg) | 106 ± 12 | 12.95 | 104.47 |
| Compound A (0.3 mg/kg) | 278 ± 44 | 33.17 | 80.40 |

| | | | |
|---|---|---|---|
| Note: a. Specific calculations for Tumor Growth Inhibition T/C and TGI are detailed in section 3.5; "--" indicates not applicable. | | | |

### 5. Conclusion

In summary, in this experiment, tumor-bearing mice in the human breast cancer ZR-75-1 model tolerated the test Compound 2, Compound 5-P2, and Compound 3, and all test substances demonstrated significant anti-tumor effects. Compared to Compound A, Compound 2, Compound 5-P2, and Compound 3 all exhibited more significant anti-tumor effects.

The foregoing descriptions are merely preferred embodiments of the present application and are not intended to limit the present application. Any modifications, equivalent substitutions, or improvements made within the spirit and principles of the present application shall be included within the scope of protection of the present application.

## Claims

1. A compound of formula (I), or an isomer, deuterated form, or pharmaceutically acceptable salt thereof, 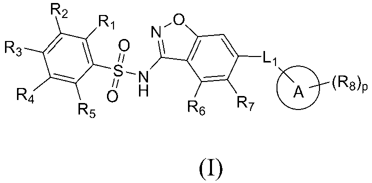 wherein,
R₁ and R₂ are each independently selected from the group consisting of hydrogen, halogen, C₁₋₄ alkyl, C₁₋₄ haloalkyl, C₁₋₄ alkoxy, substituted or unsubstituted C₃₋₆ cycloalkyl, -C₁₋₄ alkyl-O-C₁₋₄ alkyl, hydroxyl, -C₁₋₄ alkylhydroxy, -C₁₋₄ alkyl-S-C₁₋₄ alkyl, amino, and C₁₋₄ alkylamino;
R₃ is selected from the group consisting of hydrogen, halogen, C₁₋₄ alkyl, C₁₋₄ haloalkyl, C₁₋₄ alkoxy, hydroxyl, and amino; R₄ and R₅ together with carbon atoms to which they are bonded form a 7- or 8-membered heterocyclyl, the heterocyclyl is optionally substituted by m Rₐ; two Rₐ located on the same carbon atom can form a C₃₋₆ cycloalkyl or a 3- to 6-membered heterocyclyl, or two Rₐ located on adjacent carbon atoms can form a C₃₋₆ cycloalkyl or a 3- to 6-membered heterocyclyl;
or R₅ is selected from the group consisting of hydrogen, halogen, C₁₋₄ alkyl, C₁₋₄ haloalkyl, C₁₋₄ alkoxy, hydroxyl, and amino; R₃ and R₄ together with carbon atoms to which they are bonded form a 7- or 8-membered heterocyclyl, the heterocyclyl is optionally substituted by m Rₐ; two Rₐ located on the same carbon atom can form a C₃₋₆ cycloalkyl or a 3- to 6-membered heterocyclyl, or two Rₐ located on adjacent carbon atoms can form a C₃₋₆ cycloalkyl or a 3- to 6-membered heterocyclyl;
Rₐ is selected from the group consisting of hydrogen, halogen, C₁₋₄ alkyl, C₁₋₄ haloalkyl, C₁₋₄ alkoxy, hydroxyl, and amino;
m is 1, 2, 3, or 4;
R₆ and R₇ are each independently selected from the group consisting of hydrogen, halogen, C₁₋₄ alkyl, C₁₋₄ haloalkyl, C₁₋₄ alkoxy, C₁₋₄ haloalkoxy, C₃₋₆ cycloalkyl, C₃₋₆ cycloalkyloxy, and deuterated C₁₋₄ alkoxy; or R₆ and R₇ together with carbon atoms to which they are bonded form a 5- to 8-membered heterocyclyl, the heterocyclyl is optionally substituted by n R_{b};
R_{b} is selected from the group consisting of hydrogen, halogen, C₁₋₄ alkyl, C₁₋₄ haloalkyl, C₁₋₄ alkoxy, hydroxyl, and amino;
n is 1, 2, 3, or 4;
L₁ is selected from the group consisting of C₁₋₄ alkylene, -O-, and deuterated C₁₋₄ alkylene;
ring A is a 5- or 6-membered heteroaryl;
R₈ is selected from the group consisting of hydrogen, halogen, C₁₋₄ alkyl, C₁₋₄ haloalkyl, C₁₋₄ alkoxy, substituted or unsubstituted C₃₋₆ cycloalkyl, amino, -C₁₋₄ alkylamino, and hydroxyl; and
p is 1, 2, 3, or 4.

2. The compound according to claim 1, or an isomer, deuterated form, or pharmaceutically acceptable salt thereof, wherein R₁ is selected from the group consisting of CH₃O- and hydrogen.

3. The compound according to any one of claims 1 to 2, or an isomer, deuterated form, or pharmaceutically acceptable salt thereof, wherein the 7- or 8-membered heterocyclyl formed by R₃ and R₄ together with the carbon atoms to which they are bonded is selected from the group consisting of

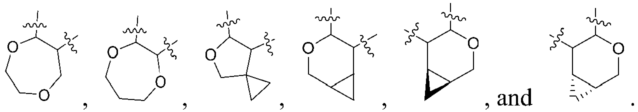

4. The compound according to any one of claims 1 to 2, or an isomer, deuterated form, or pharmaceutically acceptable salt thereof, wherein the 7- or 8-membered heterocyclyl formed by R₄ and R₅ together with the carbon atoms to which they are bonded is selected from the group consisting of

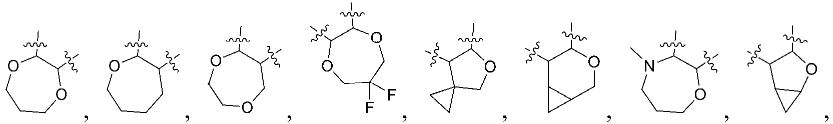

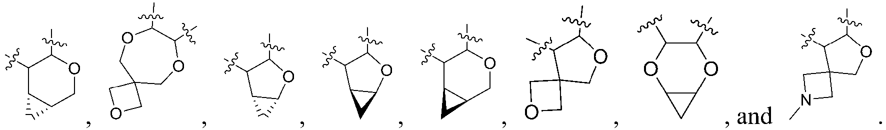

5. The compound according to any one of claims 1 to 4, or an isomer, deuterated form, or pharmaceutically acceptable salt thereof, wherein Rₐ is selected from the group consisting of -F and -CH₃.

6. The compound according to any one of claims 1 to 5, or an isomer, deuterated form, or pharmaceutically acceptable salt thereof, wherein 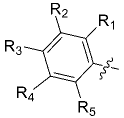 is selected from the group consisting of 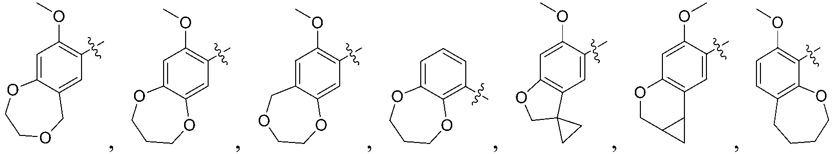 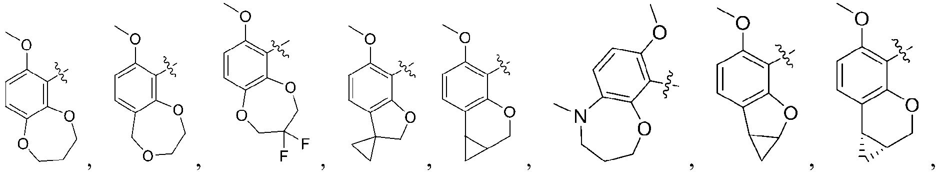 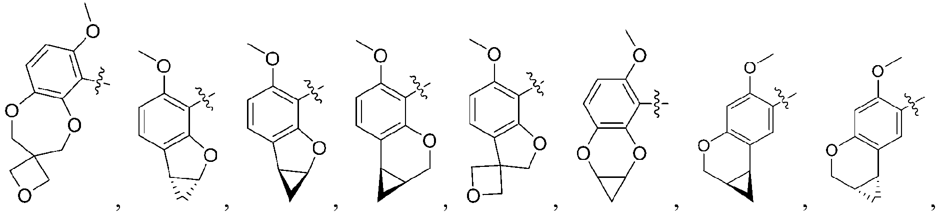 and

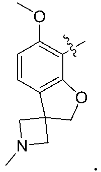

7. The compound according to any one of claims 1 to 6, or an isomer, deuterated form, or pharmaceutically acceptable salt thereof, wherein R₆ and R₇ are each independently selected from the group consisting of -F, -CH₃, CH₃O-, C₂H₅O-, CHF₂O-, 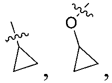 -C₂H₅, and -OCD₃.

8. The compound according to any one of claims 1 to 6, or an isomer, deuterated form, or pharmaceutically acceptable salt thereof, wherein the 5- to 8-membered heterocyclyl formed by R₆ and R₇ together with the carbon atoms to which they are bonded is selected from the group consisting of

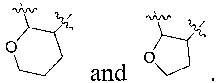

9. The compound according to any one of claims 1 to 8, or an isomer, deuterated form, or pharmaceutically acceptable salt thereof, wherein L₁ is selected from the group consisting of -CH₂-, -CD₂-, and -O-.

10. The compound according to any one of claims 1 to 9, or an isomer, deuterated form, or pharmaceutically acceptable salt thereof, wherein ring A is selected from the group consisting of

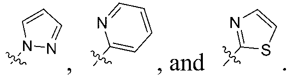

11. A compound, or an isomer, deuterated form, or pharmaceutically acceptable salt thereof, wherein the compound is selected from the group consisting of:

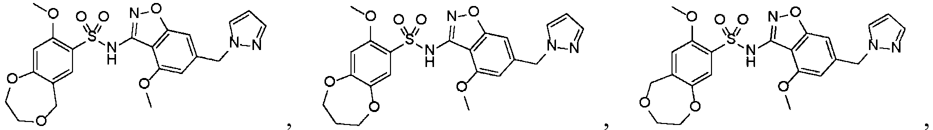

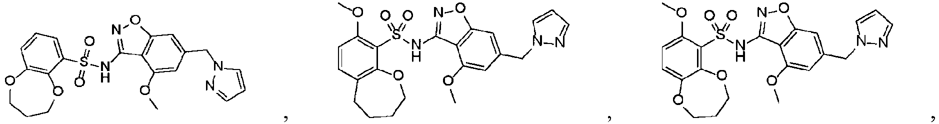

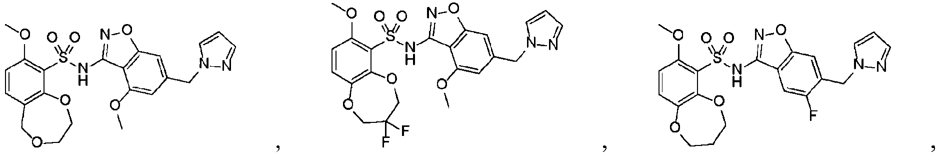

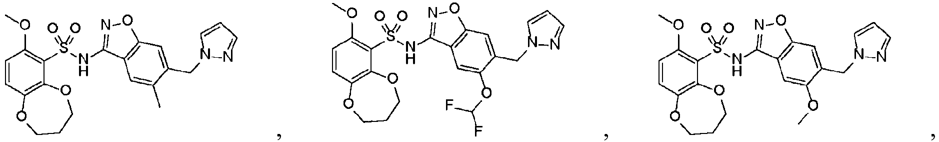

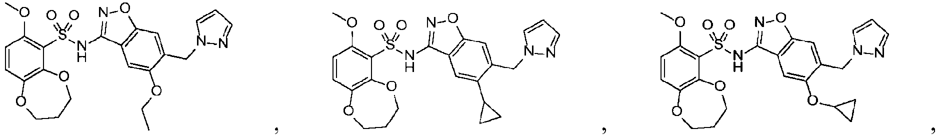

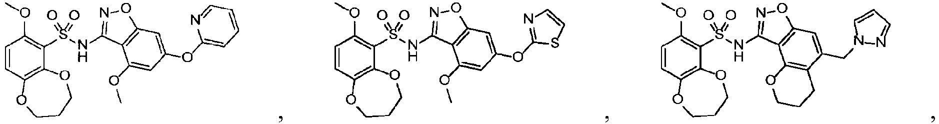

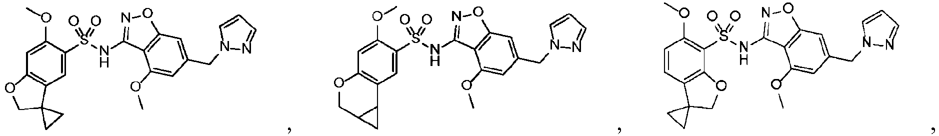

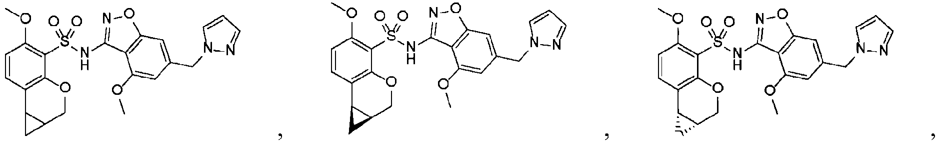

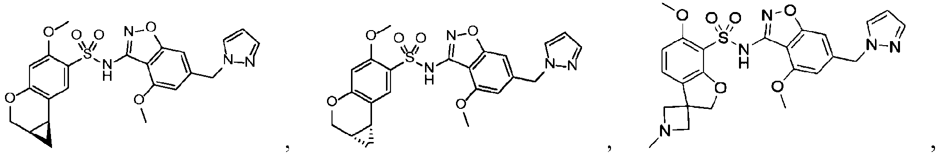

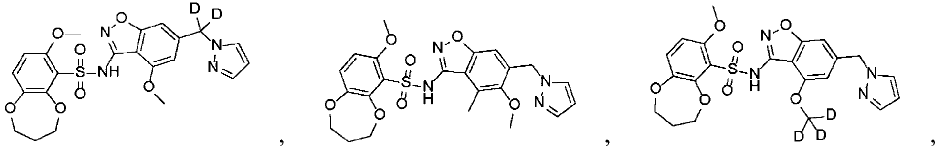

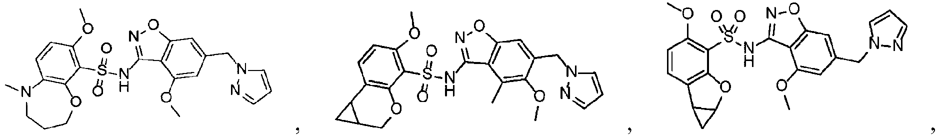

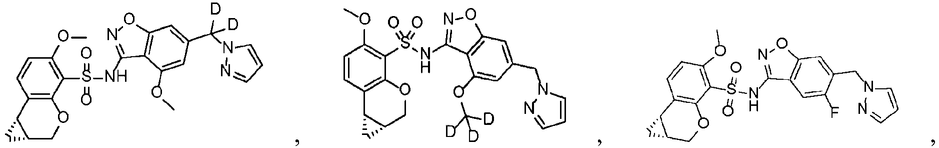

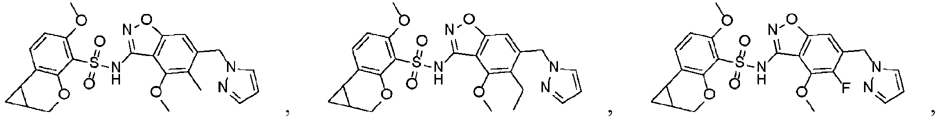

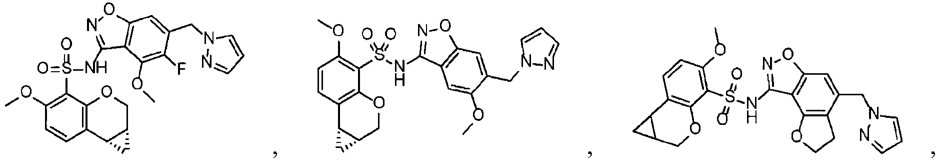

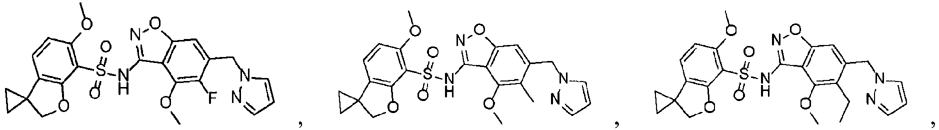

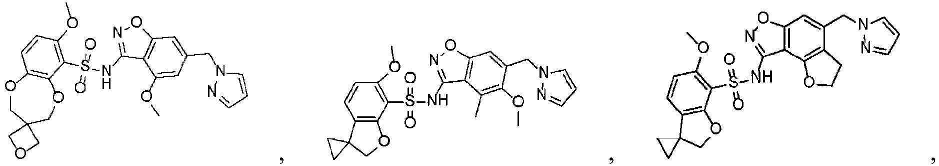

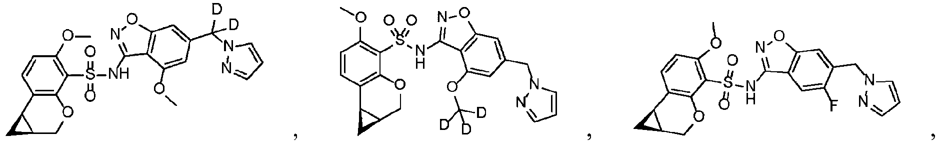

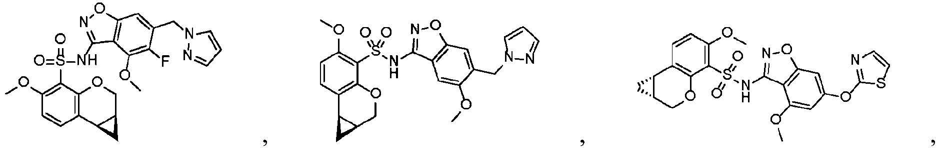

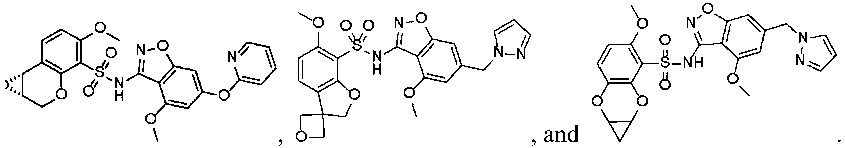

12. A pharmaceutical composition comprising the compound according to any one of claims 1 to 11, or an isomer, deuterated form, or pharmaceutically acceptable salt thereof, and a pharmaceutically acceptable carrier.

13. Use of the compound according to any one of claims 1 to 12, or an isomer, deuterated form, or pharmaceutically acceptable salt thereof, or the pharmaceutical composition according to claim 12, in the manufacture of a medicament for treating a disease mediated by a KAT6 target.
